(19) Europäisches Patentamt
European Patent Office
Office européen des brevets

(11) EP 1 666 600 B1

(12) **EUROPEAN PATENT SPECIFICATION**

(45) Date of publication and mention of the grant of the patent:
**30.12.2009 Bulletin 2009/53**

(21) Application number: **05023231.3**

(22) Date of filing: **10.07.2001**

(51) Int Cl.:
*C12N 15/85 (2006.01)* *C12N 15/10 (2006.01)*
*A61K 38/17 (2006.01)* *A61K 38/19 (2006.01)*
*A61K 39/395 (2006.01)* *C07K 16/28 (2006.01)*
*C07K 16/24 (2006.01)* *C07K 16/18 (2006.01)*
*G01N 33/50 (2006.01)* *C12Q 1/68 (2006.01)*

(54) **Methods for enhancing the efficacy of cancer therapy**

Verfahren zur Verbesserung von Krebstherapie

Procédés pour augmenter l'efficacité de la thérapie du cancer

(84) Designated Contracting States:
**AT BE CH CY DE DK ES FI FR GB GR IE IT LI LU MC NL PT SE TR**

(30) Priority: **29.08.2000 US 228914 P**
**11.01.2001 US 759056**
**10.07.2001 US 901812**

(43) Date of publication of application:
**07.06.2006 Bulletin 2006/23**

(62) Document number(s) of the earlier application(s) in accordance with Art. 76 EPC:
**01950996.7 / 1 341 923**

(73) Proprietor: **Genentech, Inc.**
**South San Francisco CA 94080-4990 (US)**

(72) Inventors:
- **Tice, David**
  **San Mateo, CA 94401 (US)**
- **Pennica, Diane**
  **Burlingame, CA 94010 (US)**
- **Polakis, Paul**
  **Mill Valley, CA 94941 (US)**
- **Szeto, Wayne**
  **San Francisco, CA 94107 (US)**

(74) Representative: **Tollervey, Rebecca Marie et al**
**Mewburn Ellis LLP**
**33 Gutter Lane**
**London**
**EC2V 8AS (GB)**

(56) References cited:
**WO-A-01/51635** **US-A- 4 997 852**

- **NAGPAL S ET AL: "RETINOIDS AS ANTI-CANCER AGENTS" CURRENT PHARMACEUTICAL DESIGN, BENTHAM SCIENCE PUBLISHERS, SCHIPHOL, NL, vol. 2, 1996, pages 295-316, XP000983109 ISSN: 1381-6128**
- **BOUILLET P ET AL: "DEVELOPMENTAL EXPRESSION PATTERN OF STRA6, A RETINOIC ACID-RESPONSIVE GENE ENCODING A NEW TYPE OF MEMBRANE PROTEIN" MECHANISMS OF DEVELOPMENT, ELSEVIER SCIENCE IRELAND LTD, IE, vol. 63, no. 2, May 1997 (1997-05), pages 173-186, XP001002219 ISSN: 0925-4773**
- **EASWARAN V ET AL: "Cross-regulation of beta-catenin-LEF/TCF and retinoid signaling pathways." CURRENT BIOLOGY: CB. ENGLAND 2 DEC 1999, vol. 9, no. 23, 2 December 1999 (1999-12-02), pages 1415-1418, XP002206444 ISSN: 0960-9822**
- **MCGREW L LYNN ET AL: "Direct regulation of the Xenopus engrailed-2 promoter by the Wnt signaling pathway, and a molecular screen for Wnt-responsive genes, confirm a role for Wnt signaling during neural patterning in Xenopus." MECHANISMS OF DEVELOPMENT, vol. 87, no. 1-2, 1999, pages 21-32, XP002206445 ISSN: 0925-4773**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- TANEJA R ET AL: "REEXPRESSION OF RETINOIC ACID RECEPTOR (RAR)GAMMA OR OVEREXPRESSIONOF RARALPHA OR RARBETA IN RARGAMMA-NULL F9 CELLS REVEALS A PARTIAL FUNCTIONAL REDUNDANCY BETWEEN THE THREE RAR TYPES" PROCEEDINGS OF THE NATIONAL ACADEMY OF SCIENCES OF USA, NATIONAL ACADEMY OF SCIENCE. WASHINGTON, US, vol. 92, no. 17, 1 August 1995 (1995-08-01), pages 7854-7858, XP000615317 ISSN: 0027-8424
- ST-ARNAUD R ET AL: "THE INT-1 PROTO-ONCOGENE IS TRANSCRIPTIONALLY ACTIVATED DURING NEUROECTODERMAL DIFFERENTIATION OF P19 MOUSE EMBRYONAL CARCINOMA CELLS" ONCOGENE, vol. 4, no. 9, 1989, pages 1077-1080, XP008005849 ISSN: 0950-9232
- POLAKIS P: "Wnt signaling and cancer" GENES AND DEVELOPMENT, COLD SPRING HARBOR LABORATORY PRESS, PLAINVIEW, NY, US, vol. 14, no. 15, 1 August 2000 (2000-08-01), pages 1837-1851, XP002240708 ISSN: 0890-9369
- SZETO WAYNE ET AL: "Overexpression of the retinoic acid-responsive gene Stra6 in human cancers and its synergistic induction by Wnt-1 and retinoic acid." CANCER RESEARCH, vol. 61, no. 10, 15 May 2001 (2001-05-15), pages 4197-4205, XP001074038 ISSN: 0008-5472
- TICE DAVID A ET AL: "Synergistic induction of tumor antigens by Wnt-1 signaling and retinoic acid revealed by gene expression profiling." THE JOURNAL OF BIOLOGICAL CHEMISTRY. UNITED STATES 19 APR 2002, vol. 277, no. 16, 19 April 2002 (2002-04-19), pages 14329-14335, XP002206447 ISSN: 0021-9258

SEQUENCE LISTING

<110> Genentech, Inc.

<120> METHODS FOR ENHANCING THE EFFICACY OF CANCER THERAPY

<130> RXT/FP6337281

<140> EP 05023231.3
<141> I2001-07-10

<150> EP 01950996.7
<151> 2001-07-10

<150> PCT/US01/21635
<151> 2001-07-10

<150> 60/228, 914
<151> 2000-08-29

<150> 09/759,056
<151> 2001-01-11

<150> 09/901,812
<151> 2001-07-10

<160> 28

<170> FastSEQ for Windows Version 4.0

<210> 1
<211> 2732
<212> DNA
<213> Homo sapiens

<400> 1

```
agtcccagac gggcttttcc cagagagcta aaagagaagg gccagagaat gtcgtcccag 60
ccagcaggga accagacctc ccccggggcc acagaggact actcctatgg cagctggtac 120
atcgatgagc cccagggggg cgaggagctc cagccagagg gggaagtgcc ctcctgccac 180
accagcatac cacccggcct gtaccacgcc tgcctggcct cgctgtcaat ccttgtgctg 240
ctgctcctgg ccatgctggt gaggcgccgc cagctctggc ctgactgtgt gcgtggcagg 300
cccggcctgc ccagccctgt ggatttcttg gctggggaca ggccccgggc agtgcctgct 360
gctgttttca tggtcctcct gagctccctg tgtttgctgc tccccgacga ggacgcattg 420
cccttcctga ctctcgcctc agcacccagc caagatggga aaactgaggc tccaagaggg 480
gcctggaaga tactgggact gttctattat gctgccctct actaccctct ggctgcctgt 540
gccacggctg gccacacagc tgcacacctg ctcggcagca cgctgtcctg ggcccacctt 600
ggggtccagg tctggcagag ggcagagtgt ccccaggtgc ccaagatcta caagtactac 660
tccctgctgg cctccctgcc tctcctgctg ggcctcggat tcctgagcct ttggtaccct 720
gtgcagctgg tgagaagctt cagccgtagg acaggagcag gctccaaggg gctgcagagc 780
agctactctg aggaatatct gaggaacctc ctttgcagga agaagctggg aagcagctac 840
cacacctcca agcatggctt cctgtcctgg gcccgcgtct gcttgagaca ctgcatctac 900
actccacagc caggattcca tctcccgctg aagctggtgc tttcagctac actgacaggg 960
acggccattt accaggtggc cctgctgctg ctggtgggcg tggtacccac tatccagaag 1020
gtgagggcag gggtcaccac ggatgtctcc tacctgctgg ccggctttgg aatcgtgctc 1080
tccgaggaca agcaggaggt ggtggagctg gtgaagcacc atctgtgggc tctggaagtg 1140
tgctacatct cagccttggt cttgtcctgc ttactcacct tcctggtcct gatgcgctca 1200
ctggtgacac acaggaccaa ccttcgagct ctgcaccgag gagctgccct ggacttgagt 1260
```

```
cccttgcatc ggagtcccca tccctcccgc caagccatat tctgttggat gagcttcagt 1320
gcctaccaga cagcctttat ctgccttggg ctcctggtgc agcagatcat cttcttcctg 1380
ggaaccacgg ccctggcctt cctggtgctc atgcctgtgc tccatggcag gaacctcctg 1440
ctcttccgtt ccctggagtc ctcgtggccc ttctggctga ctttggccct ggctgtgatc 1500
ctgcagaaca tggcagccca ttgggtcttc ctggagactc atgatggaca cccacagctg 1560
accaaccggc gagtgctcta tgcagccacc tttcttctct tccccctcaa tgtgctggtg 1620
ggtgccatgg tggccacctg gcgagtgctc ctctctgccc tctacaacgc catccacctt 1680
ggccagatgg acctcagcct gctgccaccg agagccgcca ctctcgaccc cggctactac 1740
acgtaccgaa acttcttgaa gattgaagtc agccagtcgc atccagccat gacagccttc 1800
tgctccctgc tcctgcaagc gcagagcctc ctacccagga ccatggcagc cccccaggac 1860
agcctcagac caggggagga agacgaaggg atgcagctgc tacagacaaa ggactccatg 1920
gccaagggag ctaggcccgg ggccagccgc ggcagggctc gctggggtct ggcctacacg 1980
ctgctgcaca acccaaccct gcaggtcttc cgcaagacgg ccctgttggg tgccaatggt 2040
gcccagccct gagggcaggg aaggtcaacc cacctgccca tctgtgctga ggcatgttcc 2100
tgcctaccat cctcctccct ccccggctct cctcccagca tcacaccagc catgcagcca 2160
gcaggtcctc cggatcactg tggttgggtg gaggtctgtc tgcactggga gcctcaggag 2220
ggctctgctc cacccacttg gctatgggag agccagcagg ggttctggag aaaaaaactg 2280
gtgggttagg gccttggtcc aggagccagt tgagccaggg cagccacatc caggcgtctc 2340
cctaccctgg ctctgccatc agccttgaag ggcctcgatg aagccttctc tggaaccact 2400
ccagcccagc tccacctcag ccttggcctt cacgctgtgg aagcagccaa ggcacttcct 2460
caccccctca gcgccacgga cctctctggg gagtggccgg aaagctcccg gtcctctggc 2520
ctgcagggca gcccaagtca tgactcagac caggtcccac actgagctgc ccacactcga 2580
gagccagata tttttgtagt tttatgcct ttggctatta tgaaagaggt tagtgtgttc 2640
cctgcaataa acttgttcct gagaaaaaaa aaaaaaaaaa aaaaaaaaaa aaaaaaaaaa 2700
aaaaaaaaaa aaaaaaaaaa aaaaaaaaaa aa                               2732
```

<210> 2
<211> 667
<212> PRT
<213> Homo sapiens

<400> 2

```
Met Ser Ser Gln Pro Ala Gly Asn Gln Thr Ser Pro Gly Ala Thr Glu
 1               5                  10                  15
Asp Tyr Ser Tyr Gly Ser Trp Tyr Ile Asp Glu Pro Gln Gly Gly Glu
            20                  25                  30
Glu Leu Gln Pro Glu Gly Glu Val Pro Ser Cys His Thr Ser Ile Pro
        35                  40                  45
Pro Gly Leu Tyr His Ala Cys Leu Ala Ser Leu Ser Ile Leu Val Leu
    50                  55                  60
Leu Leu Leu Ala Met Leu Val Arg Arg Arg Gln Leu Trp Pro Asp Cys
65                  70                  75                  80
Val Arg Gly Arg Pro Gly Leu Pro Ser Pro Val Asp Phe Leu Ala Gly
                85                  90                  95
Asp Arg Pro Arg Ala Val Pro Ala Ala Val Phe Met Val Leu Leu Ser
            100                 105                 110
Ser Leu Cys Leu Leu Leu Pro Asp Glu Asp Ala Leu Pro Phe Leu Thr
        115                 120                 125
Leu Ala Ser Ala Pro Ser Gln Asp Gly Lys Thr Glu Ala Pro Arg Gly
    130                 135                 140
Ala Trp Lys Ile Leu Gly Leu Phe Tyr Tyr Ala Ala Leu Tyr Tyr Pro
145                 150                 155                 160
Leu Ala Ala Cys Ala Thr Ala Gly His Thr Ala Ala His Leu Leu Gly
                165                 170                 175
Ser Thr Leu Ser Trp Ala His Leu Gly Val Gln Val Trp Gln Arg Ala
            180                 185                 190
Glu Cys Pro Gln Val Pro Lys Ile Tyr Lys Tyr Tyr Ser Leu Leu Ala
        195                 200                 205
Ser Leu Pro Leu Leu Leu Gly Leu Gly Phe Leu Ser Leu Trp Tyr Pro
```

```
    210                 215                 220
Val Gln Leu Val Arg Ser Phe Ser Arg Arg Thr Gly Ala Gly Ser Lys
225                 230                 235                 240
Gly Leu Gln Ser Ser Tyr Ser Glu Glu Tyr Leu Arg Asn Leu Leu Cys
                245                 250                 255
Arg Lys Lys Leu Gly Ser Ser Tyr His Thr Ser Lys His Gly Phe Leu
            260                 265                 270
Ser Trp Ala Arg Val Cys Leu Arg His Cys Ile Tyr Thr Pro Gln Pro
        275                 280                 285
Gly Phe His Leu Pro Leu Lys Leu Val Leu Ser Ala Thr Leu Thr Gly
    290                 295                 300
Thr Ala Ile Tyr Gln Val Ala Leu Leu Leu Leu Val Gly Val Val Pro
305                 310                 315                 320
Thr Ile Gln Lys Val Arg Ala Gly Val Thr Thr Asp Val Ser Tyr Leu
                325                 330                 335
Leu Ala Gly Phe Gly Ile Val Leu Ser Glu Asp Lys Gln Glu Val Val
            340                 345                 350
Glu Leu Val Lys His His Leu Trp Ala Leu Glu Val Cys Tyr Ile Ser
        355                 360                 365
Ala Leu Val Leu Ser Cys Leu Leu Thr Phe Leu Val Leu Met Arg Ser
    370                 375                 380
Leu Val Thr His Arg Thr Asn Leu Arg Ala Leu His Arg Gly Ala Ala
385                 390                 395                 400
Leu Asp Leu Ser Pro Leu His Arg Ser Pro His Pro Ser Arg Gln Ala
                405                 410                 415
Ile Phe Cys Trp Met Ser Phe Ser Ala Tyr Gln Thr Ala Phe Ile Cys
            420                 425                 430
Leu Gly Leu Leu Val Gln Gln Ile Ile Phe Phe Leu Gly Thr Thr Ala
        435                 440                 445
Leu Ala Phe Leu Val Leu Met Pro Val Leu His Gly Arg Asn Leu Leu
    450                 455                 460
Leu Phe Arg Ser Leu Glu Ser Ser Trp Pro Phe Trp Leu Thr Leu Ala
465                 470                 475                 480
Leu Ala Val Ile Leu Gln Asn Met Ala Ala His Trp Val Phe Leu Glu
                485                 490                 495
Thr His Asp Gly His Pro Gln Leu Thr Asn Arg Arg Val Leu Tyr Ala
            500                 505                 510
Ala Thr Phe Leu Leu Phe Pro Leu Asn Val Leu Val Gly Ala Met Val
        515                 520                 525
Ala Thr Trp Arg Val Leu Leu Ser Ala Leu Tyr Asn Ala Ile His Leu
    530                 535                 540
Gly Gln Met Asp Leu Ser Leu Leu Pro Pro Arg Ala Ala Thr Leu Asp
545                 550                 555                 560
Pro Gly Tyr Tyr Thr Tyr Arg Asn Phe Leu Lys Ile Glu Val Ser Gln
                565                 570                 575
Ser His Pro Ala Met Thr Ala Phe Cys Ser Leu Leu Leu Gln Ala Gln
            580                 585                 590
Ser Leu Leu Pro Arg Thr Met Ala Ala Pro Gln Asp Ser Leu Arg Pro
        595                 600                 605
Gly Glu Glu Asp Glu Gly Met Gln Leu Leu Gln Thr Lys Asp Ser Met
    610                 615                 620
Ala Lys Gly Ala Arg Pro Gly Ala Ser Arg Gly Arg Ala Arg Trp Gly
625                 630                 635                 640
Leu Ala Tyr Thr Leu Leu His Asn Pro Thr Leu Gln Val Phe Arg Lys
                645                 650                 655
Thr Ala Leu Leu Gly Ala Asn Gly Ala Gln Pro
            660                 665
```

<210> 3
<211> 676
<212> DNA
<213> Homo sapiens

<220>
<221> misc_feature
<222> (0)...(0)
<223> n = A, T, C or G

<400> 3

```
gtgctctccg aggacaagca ggaggnggtg gagctggtga agcaccatct gtgggctctg 60
gaagtgtgct acatctcagc cttggtcttg tcctgcttac tcaccttcct ggtcctgatg 120
cgctcactgg tgacacacag gaccaacctt cgagctctgc accgaggagc tgccctggac 180
ttgagtccct tgcatcggag tccccatccc tcccgccaag ccatattctg ttggatgagc 240
ttcagtgcct accagacagc ctttatctgc cttgggctcc tggtgcagca gatcatcttc 300
ttcctgggaa ccacggccct ggccttcctg gtgctcatgc ctgtgctcca tggcaggaac 360
ctcctgctct tccgttccct ggagtcctcg tggcccttct ggctgacttt ggccctggct 420
gtgatcctgc agaacatggc agcccattgg gtcttcctgg agactcatga tggacaccca 480
cagctgacca accggcgagt gctctatgca gccacctttc ttctcttccc cctcaatgtg 540
ctggtgggtg ccatggtggc cacctggcga gtgctcctct ctgccctcta caacgccatc 600
caccttggcc agatggacct cagcctgctg ccaccgagag ccgccactct cgaccccggc 660
tactacacgt accgaa                                                 676
```

<210> 4
<211> 2777
<212> DNA
<213> Homo sapiens

<400> 4

```
cacaaccagc caccccctcta ggatcccagc ccagctggtg ctgggctcag aggagaaggc 60
cccgtgttgg gagcaccctg cttgcctgga gggacaagtt tccgggagag atcaataaag 120
gaaaggaaag agacaaggaa gggagaggtc aggagagcgc ttgattggag gagaagggcc 180
agagaatgtc gtcccagcca gcagggaacc agacctcccc cggggccaca gaggactact 240
cctatggcag ctggtacatc gatgagcccc aggggggcga ggagctccag ccagaggggg 300
aagtgccctc ctgccacacc agcataccac ccggcctgta ccacgcctgc ctggcctcgc 360
tgtcaatcct tgtgctgctg ctcctggcca tgctggtgag gcgccgccag ctctggcctg 420
actgtgtgcg tggcaggccc ggcctgccca ggccccgggc agtgcctgct gctgttttca 480
tggtcctcct gagctccctg tgtttgctgc tccccgacga ggacgcattg cccttcctga 540
ctctcgcctc agcacccagc caagatggga aaactgaggc tccaagaggg gcctggaaga 600
tactgggact gttctattat gctgccctct actaccctct ggctgcctgt gccacggctg 660
gccacacagc tgcacacctg ctcggcagca cgctgtcctg ggcccacctt ggggtccagg 720
tctggcagag ggcagagtgt ccccaggtgc ccaagatcta caagtactac tccctgctgg 780
cctccctgcc tctcctgctg ggcctcggat tcctgagcct ttggtaccct gtgcagctgg 840
tgagaagctt cagccgtagg acaggagcag gctccaaggg gctgcagagc agctactctg 900
aggaatatct gaggaacctc ctttgcagga agaagctggg aagcagctac cacacctcca 960
agcatggctt cctgtcctgg gcccgcgtct gcttgagaca ctgcatctac actccacagc 1020
caggattcca tctcccgctg aagctggtgc tttcagctac actgacaggg acggccattt 1080
accaggtggc cctgctgctg ctggtgggcg tggtacccac tatccagaag gtgagggcag 1140
gggtcaccac ggatgtctcc tacctgctgg ccggctttgg aatcgtgctc tccgaggaca 1200
agcaggaggt ggtggagctg gtgaagcacc atctgtgggc tctggaagtg tgctacatct 1260
cagccttggt cttgtcctgc ttactcacct tcctggtcct gatgcgctca ctggtgacac 1320
acaggaccaa ccttcgagct ctgcaccgag gagctgccct ggacttgagt cccttgcatc 1380
ggagtcccca tccctcccgc caagccatat tctgttggat gagcttcagt gcctaccaga 1440
cagcctttat ctgccttggg ctcctggtgc agcagatcat cttcttcctg ggaaccacgg 1500
ccctggcctt cctggtgctc atgcctgtgc tccatggcag gaacctcctg ctcttccgtt 1560
ccctggagtc ctcgtggccc ttctggctga ctttggccct ggctgtgatc ctgcagaaca 1620
tggcagccca ttgggtcttc ctggagactc atgatggaca cccacagctg accaaccggc 1680
gagtgctcta tgcagccacc tttcttctct tccccctcaa tgtgctggtg ggtgccatag 1740
```

```
tggccacctg gcgagtgctc ctctctgccc tctacaacgc catccacctt ggccagatgg 1800
acctcagcct gctgccaccg agagccgcca ctctcgaccc cggctactac acgtaccgaa 1860
acttcttgaa gattgaagtc agccagtcgc atccagccat gacagccttc tgctccctgc 1920
tcctgcaagc gcagagcctc ctacccagga ccatggcagc cccccaggac agcctcagac 1980
caggggagga agacgaaggg atgcagctgc tacagacaaa ggactccatg gccaagggag 2040
ctaggcccgg ggccagccgc ggcagggctc gctggggtct ggcctacacg ctgctgcaca 2100
acccaaccct gcaggtcttc cgcaagacgg ccctgttggg tgccaatggt gcccagccct 2160
gagggcaggg aaggtcaacc cacctgccca tctgtgctga ggcatgttcc tgcctaccac 2220
ctcctccctc cccggctctc ctcccagcat cacaccagcc atgcagccag caggtcctcc 2280
ggatcactgt ggttgggtgg aggtctgtct gcactgggag cctcaggagg gctctgctcc 2340
acccacttgg ctatgggaga gccagcaggg gttctggaga aagaaactgg tgggttaggg 2400
ccttggtcca ggagccagtt gagccagggc agccacatcc aggcgtctcc ctaccctggc 2460
tctgccatca gccttgaagg gcctcgatga agccttctct ggaaccactc cagcccagct 2520
ccacctcagc cttggccttc acgctgtgga agcagccaag gcacttcctc acccccctcag 2580
cgccacggac ctctctgggg agtggccgga aagctcccgg gcctctggcc tgcagggcag 2640
cccaagtcat gactcagacc aggtcccaca ctgagctgcc cacactcgag agccagatat 2700
ttttgtagtt tttatgcctt tggctattat gaaagaggtt agtgtgttcc ctgcaataaa 2760
cttgttcctg agaaaaa                                                2777
```

<210> 5
<211> 658
<212> PRT
<213> Homo sapiens

<400> 5

```
Met Ser Ser Gln Pro Ala Gly Asn Gln Thr Ser Pro Gly Ala Thr Glu
 1               5                   10                  15
Asp Tyr Ser Tyr Gly Ser Trp Tyr Ile Asp Glu Pro Gln Gly Gly Glu
            20                  25                  30
Glu Leu Gln Pro Glu Gly Glu Val Pro Ser Cys His Thr Ser Ile Pro
        35                  40                  45
Pro Gly Leu Tyr His Ala Cys Leu Ala Ser Leu Ser Ile Leu Val Leu
    50                  55                  60
Leu Leu Leu Ala Met Leu Val Arg Arg Arg Gln Leu Trp Pro Asp Cys
65                  70                  75                  80
Val Arg Gly Arg Pro Gly Leu Pro Arg Pro Arg Ala Val Pro Ala Ala
                85                  90                  95
Val Phe Met Val Leu Leu Ser Ser Leu Cys Leu Leu Leu Pro Asp Glu
            100                 105                 110
Asp Ala Leu Pro Phe Leu Thr Leu Ala Ser Ala Pro Ser Gln Asp Gly
        115                 120                 125
Lys Thr Glu Ala Pro Arg Gly Ala Trp Lys Ile Leu Gly Leu Phe Tyr
    130                 135                 140
Tyr Ala Ala Leu Tyr Tyr Pro Leu Ala Ala Cys Ala Thr Ala Gly His
145                 150                 155                 160
Thr Ala Ala His Leu Leu Gly Ser Thr Leu Ser Trp Ala His Leu Gly
                165                 170                 175
Val Gln Val Trp Gln Arg Ala Glu Cys Pro Gln Val Pro Lys Ile Tyr
            180                 185                 190
Lys Tyr Tyr Ser Leu Leu Ala Ser Leu Pro Leu Leu Leu Gly Leu Gly
        195                 200                 205
Phe Leu Ser Leu Trp Tyr Pro Val Gln Leu Val Arg Ser Phe Ser Arg
    210                 215                 220
Arg Thr Gly Ala Gly Ser Lys Gly Leu Gln Ser Ser Tyr Ser Glu Glu
225                 230                 235                 240
Tyr Leu Arg Asn Leu Leu Cys Arg Lys Lys Leu Gly Ser Ser Tyr His
                245                 250                 255
Thr Ser Lys His Gly Phe Leu Ser Trp Ala Arg Val Cys Leu Arg His
            260                 265                 270
```

```
Cys Ile Tyr Thr Pro Gln Pro Gly Phe His Leu Pro Leu Lys Leu Val
        275                 280                 285
Leu Ser Ala Thr Leu Thr Gly Thr Ala Ile Tyr Gln Val Ala Leu Leu
    290                 295                 300
Leu Leu Val Gly Val Val Pro Thr Ile Gln Lys Val Arg Ala Gly Val
305                 310                 315                 320
Thr Thr Asp Val Ser Tyr Leu Leu Ala Gly Phe Gly Ile Val Leu Ser
                325                 330                 335
Glu Asp Lys Gln Glu Val Val Glu Leu Val Lys His His Leu Trp Ala
            340                 345                 350
Leu Glu Val Cys Tyr Ile Ser Ala Leu Val Leu Ser Cys Leu Leu Thr
        355                 360                 365
Phe Leu Val Leu Met Arg Ser Leu Val Thr His Arg Thr Asn Leu Arg
    370                 375                 380
Ala Leu His Arg Gly Ala Ala Leu Asp Leu Ser Pro Leu His Arg Ser
385                 390                 395                 400
Pro His Pro Ser Arg Gln Ala Ile Phe Cys Trp Met Ser Phe Ser Ala
                405                 410                 415
Tyr Gln Thr Ala Phe Ile Cys Leu Gly Leu Leu Val Gln Gln Ile Ile
            420                 425                 430
Phe Phe Leu Gly Thr Thr Ala Leu Ala Phe Leu Val Leu Met Pro Val
        435                 440                 445
Leu His Gly Arg Asn Leu Leu Leu Phe Arg Ser Leu Glu Ser Ser Trp
    450                 455                 460
Pro Phe Trp Leu Thr Leu Ala Leu Ala Val Ile Leu Gln Asn Met Ala
465                 470                 475                 480
Ala His Trp Val Phe Leu Glu Thr His Asp Gly His Pro Gln Leu Thr
                485                 490                 495
Asn Arg Arg Val Leu Tyr Ala Ala Thr Phe Leu Leu Phe Pro Leu Asn
            500                 505                 510
Val Leu Val Gly Ala Ile Val Ala Thr Trp Arg Val Leu Leu Ser Ala
        515                 520                 525
Leu Tyr Asn Ala Ile His Leu Gly Gln Met Asp Leu Ser Leu Leu Pro
    530                 535                 540
Pro Arg Ala Ala Thr Leu Asp Pro Gly Tyr Tyr Thr Tyr Arg Asn Phe
545                 550                 555                 560
Leu Lys Ile Glu Val Ser Gln Ser His Pro Ala Met Thr Ala Phe Cys
                565                 570                 575
Ser Leu Leu Leu Gln Ala Gln Ser Leu Leu Pro Arg Thr Met Ala Ala
            580                 585                 590
Pro Gln Asp Ser Leu Arg Pro Gly Glu Glu Asp Glu Gly Met Gln Leu
        595                 600                 605
Leu Gln Thr Lys Asp Ser Met Ala Lys Gly Ala Arg Pro Gly Ala Ser
    610                 615                 620
Arg Gly Arg Ala Arg Trp Gly Leu Ala Tyr Thr Leu Leu His Asn Pro
625                 630                 635                 640
Thr Leu Gln Val Phe Arg Lys Thr Ala Leu Leu Gly Ala Asn Gly Ala
                645                 650                 655
Gln Pro
```

<210> 6
<211> 23
<212> DNA
<213> Artificial Sequence

<220>
<223> Artificial Sequence = synthetic oligonucleotide

<400> 6
cacactcgag agccagatat ttt 23

<210> 7
<211> 23
<212> DNA
<213> Artificial Sequence

<220>
<223> Artificial Sequence = synthetic oligonucleotide

<400> 7
aacaagttta ttgcagggaa cac 23

<210> 8
<211> 36
<212> DNA
<213> Artificial Sequence

<220>
<223> Artificial Sequence = synthetic oligonucleotide

<400> 8
tgtagttttt atgcctttgg ctattatgaa agaggt 36

<210> 9
<211> 19
<212> DNA
<213> Artificial Sequence

<220>
<223> Artificial Sequence = synthetic oligonucleotide

<400> 9
agaccaggtc ccacactga 19

<210> 10
<211> 24
<212> DNA
<213> Artificial Sequence

<220>
<223> Artificial Sequence = synthetic oligonucleotide

<400> 10
ttcataatag ccaaaggcat aaaa 24

<210> 11
<211> 23
<212> DNA
<213> Artificial Sequence

<220>
<223> Artificial Sequence = synthetic oligonucleotide

<400> 11
ctgcccacac tcgagagcca gat 23

<210> 12
<211> 17
<212> PRT
<213> Artificial Sequence

<220>
<223> Artificial Sequence = synthetic peptide

<400> 12

```
Met Lys His Gln His Gln His Gln His Gln His Gln His Gln Met His
 1               5                  10                  15
Gln
```

<210> 13
<211> 9
<212> PRT
<213> Homo sapiens

<400> 13

```
Ser Pro Val Asp Phe Leu Ala Gly Asp
 1               5
```

<210> 14
<211> 20
<212> DNA
<213> Artificial Sequence

<220>
<223> Artificial Sequence = synthetic oligonucleotide

<400> 14
gaagatggtg atgggatttc 20

<210> 15
<211> 19
<212> DNA
<213> Artificial Sequence

<220>
<223> Artificial Sequence = synthetic oligonucleotide

<400> 15
gaaggtgaag gtcggagtc 19

<210> 16
<211> 20
<212> DNA
<213> Artificial Sequence

<220>
<223> Artificial Sequence = synthetic oligonucleotide

<400> 16
caagcttccc gttctcagcc 20

<210> 17
<211> 19
<212> DNA
<213> Artificial Sequence

<220>
<223> Artificial Sequence = synthetic oligonucleotide

<400> 17
tgcaccacca actgcttag 19

<210> 18
<211> 19
<212> DNA
<213> Artificial Sequence

<220>
<223> Artificial Sequence = synthetic oligonucleotide

<400> 18
ggatgcaggg atgatgttc 19

<210> 19
<211> 23
<212> DNA
<213> Artificial Sequence

<220>
<223> Artificial Sequence = synthetic oligonucleotide

<400> 19
cagaagactg tggatggccc ctc 23

<210> 20
<211> 20
<212> DNA
<213> Artificial Sequence

<220>
<223> Artificial Sequence = synthetic oligonucleotide

<400> 20
gtaggccagg gctatttctg 20

<210> 21
<211> 20
<212> DNA
<213> Artificial Sequence

<220>
<223> Artificial Sequence = synthetic oligonucleotide

<400> 21
tgtctcatga gggtccaaaa 20

<210> 22
<211> 21
<212> DNA

<213> Artificial Sequence

<220>
<223> Artificial Sequence = synthetic oligonucleotide

<400> 22
tggcgctctt ctctcctcgg t 21

<210> 23
<211> 19
<212> DNA
<213> Artificial Sequence

<220>
<223> Artificial Sequence = synthetic oligonucleotide

<400> 23
tcgccaagct actggctaa 19

<210> 24
<211> 18
<212> DNA
<213> Artificial Sequence

<220>
<223> Artificial Sequence = synthetic oligonucleotide

<400> 24
cttggctgtg ccagttca 18

<210> 25
<211> 28
<212> DNA
<213> Artificial Sequence

<220>
<223> Artificial Sequence = synthetic oligonucleotide

<400> 25
aaccaagcat cgttgtgcaa tacaactc 28

<210> 26
<211> 21
<212> DNA
<213> Artificial Sequence

<220>
<223> Artificial Sequence = synthetic oligonucleotide

<400> 26
gccagtacag gatctggaaa g 21

<210> 27
<211> 26
<212> DNA
<213> Artificial Sequence

<220>

<223> Artificial Sequence = synthetic oligonucleotide

<400> 27
catatctcat cagagagcat ctaaaa 26

<210> 28
<211> 22
<212> DNA
<213> Artificial Sequence

<220>
<223> Artificial Sequence = synthetic oligonucleotide

<400> 28
aagcttttag cctgcccagc ca 22

## Description

### Background of the Invention

### Field of the Invention

The present invention concerns the identification of drug targets for cancer therapy. In particular, the invention concerns the identification of tumor antigens the expression of which is selectively enhanced by retinoid treatment, and treatment methods targeting such antigens.

### Description of the Related Art

One approach to understanding the molecular basis of cancer and to devise effective treatment strategies is to identify differences in gene expression between cancer cells and normal cells. Strategies based on assumptions that steady-state mRNA levels will differ between normal and malignant cells have been used to clone differentially expressed genes (Zhang et al., Science 276:1268-1272 (1997)), and have lead to the identification of novel drug targets.

The aberrant growth and survival of neoplastically transformed cells is attributed to underlying genetic defects that alter normal cellular homeostasis. Wnt signaling represents a mechanism that contributes to the progression in a high percentage of human cancers for which appropriate animal and cell culture models are available. β-catenin, a key component of the Wnt signaling pathway, interacts with the TCF/LEF family of transcription factors and activates transcription of Wnt target genes. Recent studies have revealed that a number of proteins such as, the adenomatous polyposis (APC) tumor suppressor, and axin are involved in the regulation of the Wnt signaling pathway. Furthermore, mutations in APC or β-catenin have been found to be responsible for the genesis of many human cancers.

For example, in the case of colorectal cancer, inactivation of the APC tumor suppressor occurs early in tumor progression and provides a growth advantage resulting from the inappropriate activation of genes such as cyclin D and c-myc (He et al., Science 281:1509-1512 (1998)); Tetsu and McCormick, Nature 38:422-426 (1999)). These genes are targets of LEF/TCF transcription factors that are activated by their interaction with β-catenin, a protein that is normally down-regulated by APC (Barker et al, Adv Cancer Res 77:1-24 (2000); Polakis, Genes Dev 14:1837-1851 (2000)). The upregulation of this signaling pathway in cancer can also result from missense mutations in the β-catenin gene (Rubinfeld et al., Science 275:1790-1792 (1997)); Korinek et al., Science 275:1784-1787 (1997)). These mutations render the β-catenin protein refractory to down-regulation by APC. Mutations in β-catenin have recently been identified in a wide variety of human tumors and are particularly prevalent in human hepatocellular cancers (Polakis, 2000, *supra*). Activation of a β-catenin signaling also occurs when the cell surface frizzled receptors are stimulated by the secreted Wnt ligands (Wodarz and Nusse, Annu Rev Cell Dev Biol 14:59-88 (1998)). Although it is not known whether Wnt ligands *per se* contribute to human cancers, early experiments demonstrated that their over-expression in murine mammary tissue was tumorigenic (Nusse and Varmus, Cell 31:99-109 (1982)). The Wnt signaling pathway has been implicated in the pathogenesis of a variety of cancers, including colon cancer, breast cancer, gastric cancer, lung cancer, and melanoma. Thus, the vast majority of colorectal tumors contain mutations in the genes coding for either the APC tumor suppressor or β-catenin (Polakis, Curr. Opin. Genet. Dev. 9:15-21 (1999)). Activating mutations in β-catenin have also been identified in cancers of the ovary and endometrium, Wilm's kidney tumors and melanomas, demonstrating that defects in Wnt-1 signaling contribute to the progression of these cancers (Kobayashi et al., Jpn J Cancer Res 90:55-9 (1999); Koesters et al, Cancer Res 59:3880-2 (1999); Palacios and Hamallo, Cancer Res 58:1344-7 (1998); Rimm et al., Am J Pathol 154:325-9 (1999); Rubinfeld et al., Science 262:1731-1734 (1993); Wright et al, Int J Cancer 82:625-9 (1999)).

Signals emanating from the Wnt receptors are thought to proceed via the activation of disheveled, which in turn, negatively regulates glycogen synthase kinase 3b (GSK3b) (Peifer and Polakis, Science 287:1606-1609 (2000)). This kinase normally phosphorylates the regulatory sequence of β-catenin that targets the protein for ubiquitin-dependent degradation (Miller and Moon, Genes & Dev. 10:2527-2539 (1996)). Negative regulation of GSK3b thus increases the stability of β-catenin and prolongs its activation of the TCF/LEF transcription factors (Molenaar, et ai, Cell 86:391-399 (1996); Behrens, et al., Nature 382:638-642 (1996)). Although the activation of the TCF/LEF transcription factors by β-catenin is well established, there remain additional mechanisms independent of these transcription factors by which β-catenin might engage gene activation. One of these alternative mechanisms was recently proposed by Byers and colleagues in a study investigating the potential for cross talk between signaling by retinoic acid receptors (RAR) and β-catenin (Easwaran, et al., Curr Biol 9:1415-1418 (1999)). A synthetic reporter gene containing a retinoic acid response element was activated more robustly by retinoids following the over-expression of β-catenin in a cultured cell line.

The use of monoclonal antibodies as therapeutics has gained increased acceptance with several monoclonal antibodies (mAbs) either approved for human use or in late stage clinical trials. The first mAb approved by the US Food and Drug Administration (FDA) for the treatment of allograft rejection was anti-CD3 (OKT3) in 1986. Since then the pace of progress in the field of mAbs has been considerably accelerated, particularly from 1994 onwards which led to approval of additional seven mAbs for human treatment. These include ReoPro® for the management of complications of coronary angioplasty in 1994, Zenapax® (anti-CD25) for the prevention of allograft rejection in 1997, Rituxan® (anti-CD20) for the treatment of B cell non-Hodgkin's lymphoma in 1997, Infliximab® (anti-TNF-$\alpha$) initially for the treatment of Crohn's disease in 1998 and subsequently for the treatment of rheumatoid arthritis in 1999, Simulect® (anti-CD25) for the prevention of allograft rejection in 1998, Synagis® (anti-F protein of respiratory syncitial virus) for the treatment of respiratory infections in 1998, and Herceptin® (anti-HER2/neu) for the treatment of HER2 overexpressing metastatic breast tumors in 1998 (Glennie and Johnson, Immunol Today 21: 403-410 (2000)).

The proven utility of therapeutic antibodies in the treatment of human cancer in the clinic has recently spurred intense activity aimed at the development and refinement of immunotherapeutics. Ideally, these therapies require the presence of cell surface antigens expressed on the cancer cells at significantly higher levels than that present on normal tissues throughout the body. Such criteria for differential expression on tumors relative to normal tissue will obviously limit the number of antigens considered desirable as targets for cancer treatment, such as immunotherapy. Therefore, it would be desirable to find ways of selectively enhancing cell surface antigen expression on tumor cells. In particular, reagents that would selectively enhance the level of antigen expression of cancel cells relative to normal cells would have great potential in improving the therapeutic index for treatment, such as immunotherapeutics directed against these antigens.

<u>Summary of the invention</u>

Novel drug targets can be identified by differential analysis of RNA transcripts isolated from cancer cell lines and tissues. In the work underlying the present invention, this approach has been extended by analyzing differences in gene expression resulting from the drug treatment of transformed and non-transformed cancer cells. A breast epithelial cell line, which conditionally expresses the Wnt-1 proto-oncogen, was left untreated or treated with 9-cis retinoic acid in the presence of absence of Wnt-1 expression. A number of genes, including several cell surface antigens were selectively up-regulated by the combination of Wnt-1 and retinoic acid. This observation indicates that the efficacy of cancer treatment, and in particular, the immunotherapy of cancers characterized by the involvement of the Wnt signaling pathway can be enhanced by co-administration of retinoic acid or other retinoids.

Accordingly, in one aspect the present invention concerns use of an anti-tumour agent for the manufacture of a medicament for the treatment in combination with a retinoid of a tumor of a human cancer selected from ovarian cancer, endometrial cancer, Wilm's kidney tumour, colon cancer, breast cancer, prostate cancer, gastric cancer, lung cancer, hepatocellular cancer, melanoma or pheochromocytoma, wherein said anti-tumour agent is an antibody, an antisense molecule, a ribozyme or a triple-helix DNA that binds to a target protein selected from the group consisting of autotaxin, 4.1BB ligand, ephrin b1, ISLR, M-ras, and ZO-1 or to a nucleic acid encoding a target protein.

In another aspect, the invention concerns use of a retinoid and an anti-tumour agent for the manufacture of a medicament for the treatment of a tumor of a human cancer selected from ovarian cancer, endometrial cancer, Wilm's kidney tumour, colon cancer, breast cancer, prostate cancer, gastric cancer, lung cancer, hepatocellular cancer, melanoma or pheochromocytoma , wherein said anti-tumour agent is an antibody, an antisense molecule, a ribozyme or a triple-helix DNA that binds to a target protein selected from the group consisting of autotaxin, 4-1BB ligand, ephrin b1, ISLR, M-ras, and ZO-1 or to a nucleic acid encoding a target protein.

The treatment can be performed with any retinoid, including retinoic acid.

The retinoid may be administered prior to, concurrently with, or following the administration of the anti-tumor agent.

In a preferred embodiment, the anti-tumor agent is an antibody. Throughout the disclosure, the term "antibody" is used in the broadest sense, and includes antibody fragments, Such as Fab, Fab', $F(ab')_2$ and Fv fragments, diabodies, single-chain antibody molecules, and multispecific antibodies formed from antibody fragments. The antibody may be a chimeric, humanized, or human antibody, including antibody fragments.

The treatment may optionally include additional treatment, for example with a chemotherapeutic agent and/or radiation treatment.

In another aspect, the invention concerns a method for diagnosing a tumor of a human cancer selected from ovarian cancer, endometrial cancer, Wilm's kidney tumour, colon cancer, breast cancer, prostate cancer, gastric cancer, lung cancer, hepatocellular cancer, melanoma or pheochromocytoma comprising:

a) contacting a biological sample obtained from said patient with retinoic acid; and

b) detecting enhanced expression of a target protein in the biological sample treated with the retinoid relative to a normal tissue, wherein the target protein is selected from the group consisting of autotaxin, 4-1BB ligand, ephrin b1, ISLR, M-ras, and ZO-1.

In another aspect, the invention concerns pharmaceutical composition comprising a retinoid - and an anti-tumour agent, wherein the anti-tumour agent is an antibody, an antisense molecule, a ribozyme or a triple-helix DNA that binds to a target protein selected from the group consisting of autotaxin, 4-1BB ligand, ephrin b1, ISLR, M-ras, and ZO-1 or to a nucleic acid encoding the target protein.

In a further aspect, the invention concerns anti-tumour agent for use in the treatment in combination with a retinoid of a tumor of a human cancer selected from ovarian cancer, endometrial cancer, Wilm's kidney tumour, colon cancer, breast cancer, prostate cancer, gastric cancer, lung cancer, hepatocellular cancer, melanoma or pheochromocytoma, wherein said anti-tumour agent is an antibody, an antisense molecule, a ribozyme or a triple-helix DNA that binds to a target protein selected from the group consisting of autotaxin, 4-IBB ligand, ephrin b1, ISLR, M-ras, and ZO-1 or to a nucleic acid encoding a target protein.

Brief Description of the Drawings

Figure 1 shows the nucleotide sequence (SEQ ID NO:1) of a cDNA containing a nucleotide sequence (nucleotides 1-2732) encoding native sequence PRO10282 (stra6), wherein the nucleotide sequence (SEQ ID NO:1) is a clone designated herein as "DNA148380-2827," Also presented in bold font and underlined are the positions of the respective start and stop codons.

Figure 2 shows the amino acid sequence (SEQ ID NO:2) of a native sequence PRO10282 (stra6) polypeptide as derived from the coding sequence of SEQ ID ND;1. Also shown are the approximate locations of various other important polypeptide domains.

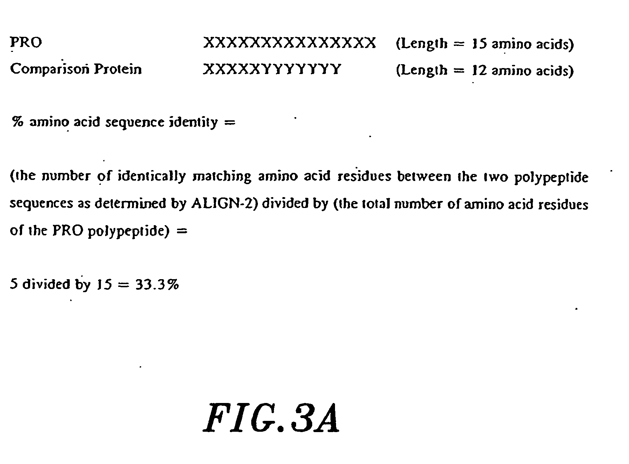

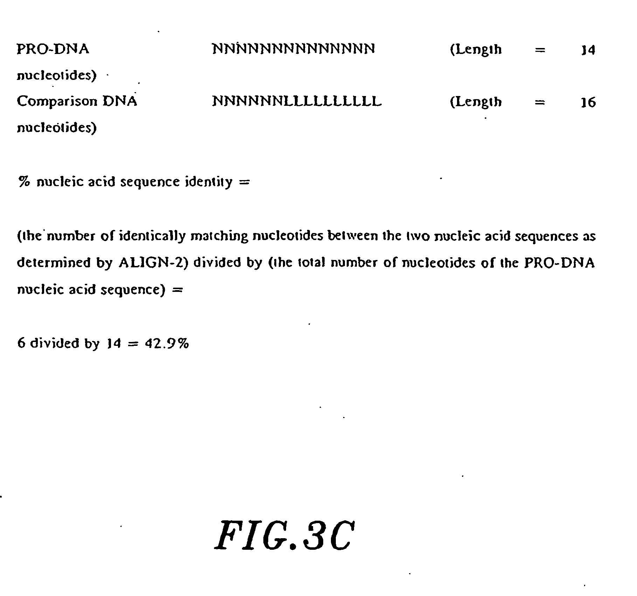

Figures 3A-D show hypothetical exemplifications for using the below described method to determined % amino acid sequence identity [Figures 3A-B) and % nucleic acid sequence identity (Figures 3C-D) using the ALIGN-2 sequence comparison computer program, wherein "PRO" represents the amino acid sequence of a hypothetical PRO10282 polypeptide of interest, "Comparison Protein" represents the amino acid sequence of a polypeptide against which the "PRO" polypeptide of interest is being compared, "PRO-DNA" represents a hypothetical PRO10282-encoding nucleic acid sequence of interest, "Comparison DNA" represents the nucleotide sequence of a nucleic acid molecule against which the PRO-DNA" nucleic acid molecule of interest is being compared, "X, "Y" and "Z" each represent different hypothetical amino acid residues and "N", "L" and "V" each represent different hypothetical nucleotides.

Figures 4A-Q provide the complete source code for the ALISN-2 sequence comparison computer program. This source code may be routinely compiled for use on a UNIX operating system to provide the ALIGN-2 sequence comparison computer program.

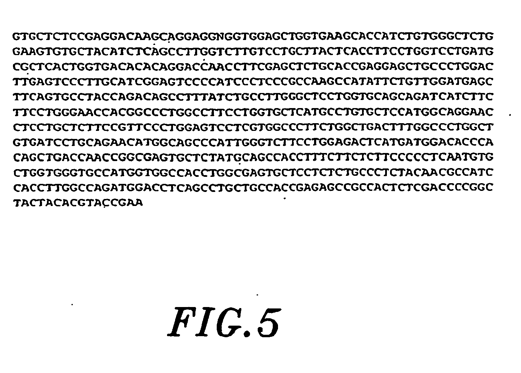

Figure 5 shows a nucleotide sequence designated herein as DNA100038 SEQ ID NO:3).

Figure 6 shows the nucleotide sequence (SEQ ID NO: 4) of a cDNA containing a nucleotide sequence (nucleotides 1-2778) encoding a native sequence human Stra6 polypeptide variant, wherein the nucleotide sequence (SEQ ID NO:4) is a clone designated herein as "DNA148389-2827-1." Also presented in bold font and underlined are the positions of the respective start and stop codons.

Figure 7 shows the amino acid sequence (SEQ ID NO:5) of a native sequence human Stra6 polypeptide variant as derived from the coding sequence of SEQ ID NO:4. Also shown are the approximate locations of various other important polypeptide domains.

Figure 8 is a schematic representation of mouse Stra6, and the human Stra6 protein encoded by DNA148380.2827 (native human PRO10282).

Figure 9 shows the hydrophobicity plot of the native sequence human Stra6 protein encoded by DNA 148380-2827 (native human PRO10282).

Figure 10 shows the relative RNA expression profile for the native sequence human Stra6 protein encoded by DNA148380-2827 in various normal human tissues.

Figure 11 shows the RNA fold expression for the native sequence human Stra6 protein encoded by DNA 148380-2827 in human colon tumor tissue relative to RNA expression in normal mucose from the same patient assayed by quantitative PCR. The data are from one experiment done in triplicate. The experiment was repeated at least twice with a different set of PCR primers.

Figure 12A shows the RNA expression for the native sequence human Stra6 protein encoded by DNA148380-2827 in human colon tissue relative to RNA expression in normal mucose from the same patient, using the housekeeping gene, GAPDH as a control.

Figure 12B shows the localization of Stra6 to the epithelial tumor cells in a colon adenocarcinoma by in situ hybridization.

Figure 13 shows the RNA expression for the native human Stra6 protein encoded by DNA148380-2827 in human breast, kidney, colon and lung tumor cell lines, relative to corresponding normal cell lines.

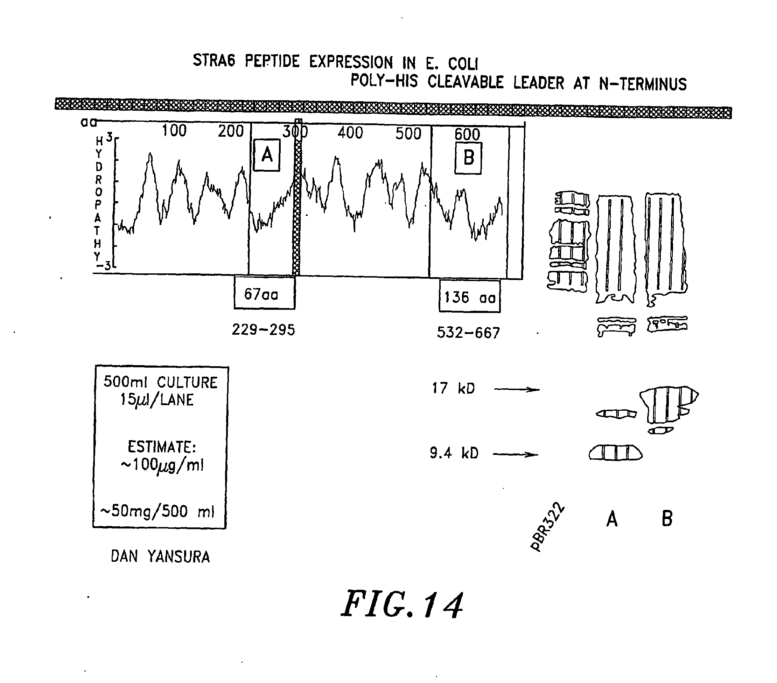

Figure 14 illustrates the expression of peptide fragments derived from the native sequence human Stra6 protein encoded by DNA148380-2827 in *E.coli.*

Figure 15 illustrates Stra6 RNA expression in human colon carcinoma cells in the presence and absence of all-trans-retinoic acid (ATRA) and 9-cis-retinoic acid (9cRA), respectively.

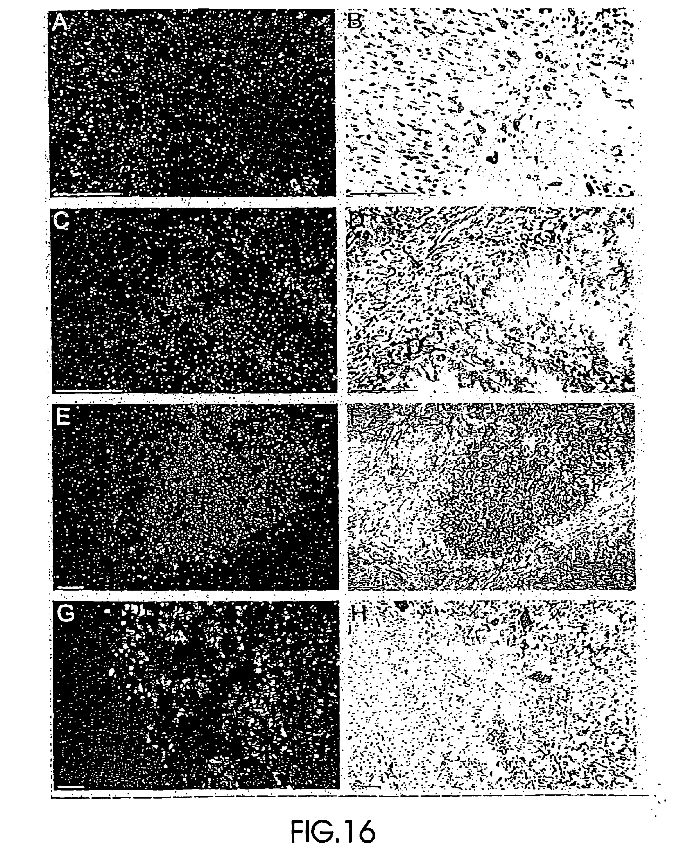

Figure 16 In situ hybridization for Stra6 in tumor sections. Darkfield images demonstrating silver grains (A, C, E, G) are shown with corresponding hemataxylinleosin-stained brightfield images (B, D, F, H). Moderate densities of silver grains overlie tumor cells but not a blood vessel in a malignant melanoma (A, B). Neoplastic epithelium in an endometrial adenocarcinoma is moderately labeled whereas tumor stroma is negative (C, D). Blastemal regions in a Wilm's tumor display high expression levels whereas tumor stroma is negative (E, F). A pheochromocytoma shows very high Stra6 mRNA expression while adjacent normal adrenal cortex is negative (G, H). Scale bars=100 microns.

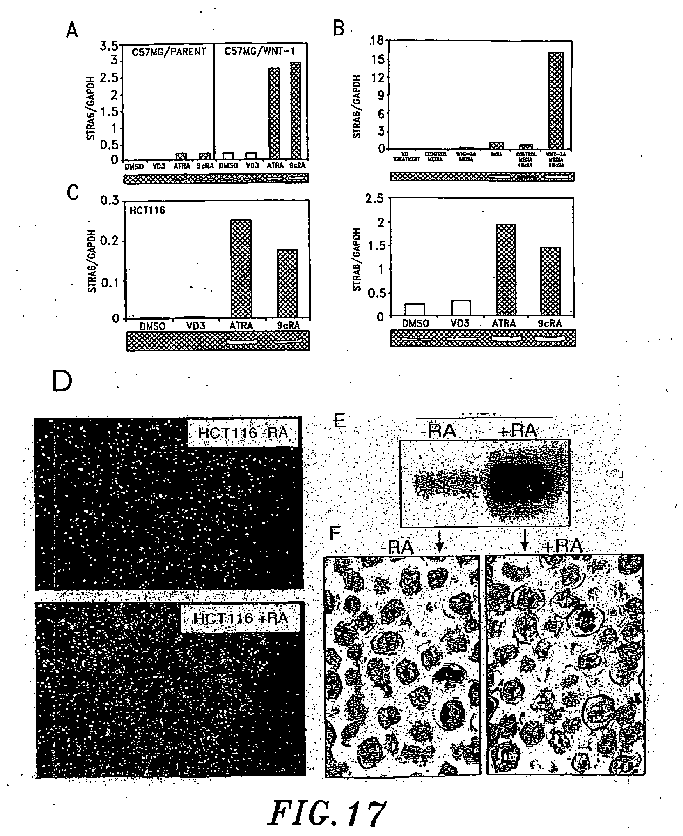

Figure 17 (A) Induction of Stra6 mRNA expression in response to *9-cis*-RA or all-*trans*-RA in C57MG/Parent and C57MG/Wnt-1 cells. (B) Induction of Stra6 mRNA expression in C57MG/Parent cells in response to Wnt-3A conditioned media and 9-*cis*-RA. (C) Induction of Stra6 mRNA expression after retinoic acid treatment in HCT116 and WiDr colon adenocarcinoma cells. (D) Darkfield images demonstrating Stra6 expression by in situ hybridization in HCT116 cells before (top panel) and after (lower panel) treatment with retinoic acid. (E) Stra6 protein expression in WiDr cells before (-RA) and after (+RA) treatment with retinoic acid as visualized by Western blot with a monoclonal antibody directed against human Stra6 peptide B. (F) Stra6 membrane localization in WiDr cells untreated (left panel) or treated (right panel) with retinoic acid. Immunohistochemistry was performed with an anti-human Stra6 peptide B monoclonal hybridoma culture supernatant (clone 12F4.2H9.1D5). For the experiments shown in A-F, cells were treated with retinoic acid for 48 hours. Stra6 products obtained after completion of the quantitative PCR reactions (40 cycles each) are shown below each graph A-C.

Figure 18 (A) Wnt-1 induces RARγ-1 expression. Protein-equivalent amounts of whole cell lysate from tetracycline repressible C57MG/Wnt-1 cells in the absence of tetracycline for 0, 24, 48, or 72 hours, were subjected to SDS-PAGE and immunoblotted for RARγ-1 and ERK2. (B) RARγ-1 mRNA expression in hyperplastic mammary glands and mammary gland tumors from Wnt-1 transgenic mice. mRNA expression was determined by quantitative RT-PCR and the data are expressed as fold expression relative to mRNA expression in wild-type mammary glands.

Figure 19 illustrates the synergistic induction of stra6 by retinoic acid (RA) and Wnt-1 (Wnt) treatment.

Figure 20 illustrates the synergistic induction of autotaxin by retinoic acid (RA and Wnt-1 (Wnt).

Figure 21 illustrates the synergistic induction of the 41 BB ligand (41 bb) by retinoic acid (RA) and Wnt-1 (Wnt).

Figure 22 illustrates the synergistic induction of ephrinb1 by retinoic aid (RA) and Wnt-1 (Wnt).

Figure 23 shows that the ISRL gene is responsive to retinoic acid (RA), but not to Wnt-1 (Wnt), while the combination of both agents results in activation that exceeds that of retinoic acid alone.

Figure 24 shows that the tight junction protein ZO-1 and the M-ras gene do not respond significantly either to retinoic acid (RA) or Wnt-1 (Wnt) alone, but are activated by combined treatment.

Figure 25A illustrates the activation of an RAR response element (RARE) by β-catenin, as determined by the increased production of luciferase.

Figure 25B shows that the LEF responsive element TopFlash is activated not only by β-catenin, was further activated by coexpression of LEF with β-catenin.

Figure 26 shows the upregulation of Stra6 mRNA in tumors and normal mammary gland by various doses of all-*trans*-retinoic acid (ATRA).

Figure 27 shows the upregulation of Stra6 mRNA in WiDr xenografts from mice orally dosed with 400 mg/kg of ATRA.

## <u>Detailed Description of the Preferred Embodiment</u>

### A. <u>Definitions</u>

Unless defined otherwise, technical and scientific terms used herein have the same meaning as commonly understood by one of ordinary skill in the art to which this invention belongs. See, e.g. Singleton et al., Dictionary of Microbiology and Molecular Biology 2nd ed, J. Wiley & Sons (New York, NY 1994); Sambrook et al., Molecular Cloning, A laboratory Manual, Cold Springs Harbor Press (Cold Springs Harbor, NY 1989). For purposes of the present invention, the following terms are defined below.

**[0020]** - The terms "PRO10282 polypeptide", "PRO10282 protein", "PRO10282", "Stra6 polypeptide", "Stra6 protein" and "Stra6" are used interchangeably, and encompass native sequence PRO10282 (Stra6) and PRO10282 (Stra6) polypeptide variants (which are further defined herein). The PRO10282 (Stra6) polypeptide may be isolated from a variety of sources, such as from human tissue types or from another source, or prepared by recombinant and/or synthetic methods.

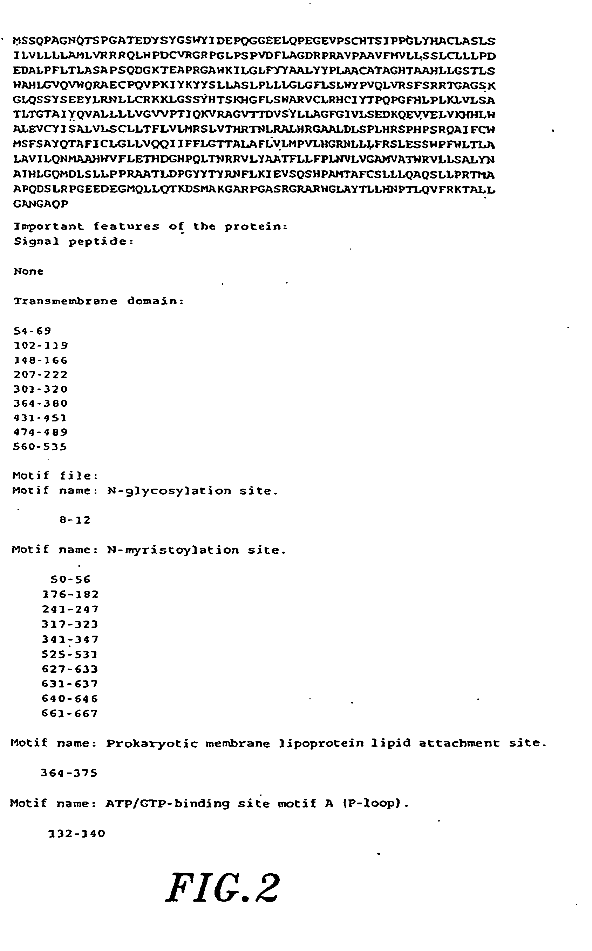

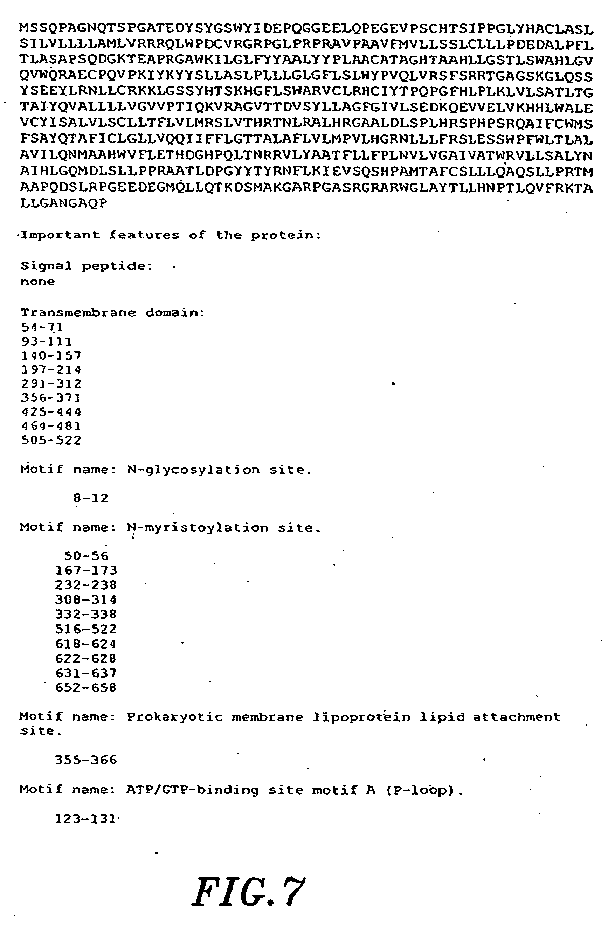

A "native sequence PRO10282" or "native sequence Stra6" comprises a polypeptide having the same amino acid sequence as a PRO10282 derived from nature. Such native sequence PRO10282 (Stra6) can be isolated from nature or can be produced by recombinant and/or synthetic means. The term "native sequence PRO 10282" or "native sequence Stra6" specifically encompasses naturally-occuring truncated or secreted forms (*e.g.*, an extracellular domain sequence), naturally-occurring variant forms (*e.g.*, alternatively spliced forms) and naturally-occuring allelic variants of the PRO10282. The native sequence PRO10282 may be a mature or full-length native sequence PRO10282 comprising amino acids 1 to 667 of Figure 2 (SEQ ID NO:2). The native sequence PRO10282 polypeptide may be a mature or full-length PRO19578 polypeptide comprising amino acids 1 to 658 of SED ID NO: 5, which is believed to be an alternatively spliced form of the native sequence PRO10282 polypeptide of SEQ ID NO: 2. Also, while the PRO10282 polypeptides disclosed in Figure 2 (SEQ ID NO:2) and in Figure 7, SEQ ID NO: 5 are shown to begin with the methionine residue designated herein as amino acid position 1, it is conceivable and possible that another methionine residue located either upstream or downstream from amino acid position in Figure 2 (SEO ID NO:2) or in Figure 7 (SEQ ID NO: 5) may be employed as the starting amino acid residue for the PRO10282 polypeptide. All Stra6 molecules which might be alternatively spliced at their 5' end are specifically inlcuded within the definition herein.

The PRO10282 (Stra6) polypeptide "extracellular domain" or "ECO" refers to a form of the PRO10282 (Stra68) polypeptide which is essentially free of the transmembrane and cytoplasmic domains. Ordinarily, a PRO10282 polypeptide ECD will have lass than about 1% of such transmembrane and/or cytoplasmic domains and preferably, will have less than about 0.5% of such domains. It will be understood that any transmembrane domains) identified for the PRO10282 polypeptides of the present invention are identified pursuant to criteria routinely employed in the art for identifying that type of hydrophobic domain. The exact boundaries of a transmembrane domain may vary but most likely by no more than about 5 amino acids at either end of the domain as initially identified. As such, in one embodiment of the present invention, the extracellular domain of a PRO10282 polypeptide comprises amino acids 1 to X, wherein X is any amino acid from amino acid 49 to 59 of Figure 2 (SEQ ID NO:2) or of Figure 7 (SEQ ID NO: 5).

"PRO10282 variant polypeptide" or "Stra6 variant polypeptide", which terms are used interchangeably, means an active PRO10282 (Stra6) polypeptide as defined below having at least about 80% amino acid sequence identity with the amino acid sequence of (a) residues 1 to 667 of the PRO10282 polypeptide shown in Figure 2 (SEQ ID NO:2), or residues 1 to 658 of Figure 7 (SEQ ID NO: 5), (b) 1 to X of Figure 2 (SEQ ID NO:2) or Figure 7 (SEQ ID NO: 5), wherein X is any amino acid from amino acid 49 to amino acid 59 of Figure 2 (SEQ ID NO:2) or of Figure 7 (SEG ID NO: 5), or (c) another specifically derived fragment of the amino acid sequence shown in Figure 2 (SEQ ID NO:2), or Figure 7 (SEQ ID NO: 5). Such PRO10282 (Stra6) variant polypeptides include for instance, PRO10282 (Stra6) polypeptides wherein one or more amino acid residues are added, or deleted, at the N- and/or C-terminus, as well as within one or more internal domains, of the sequence of Figure 2 (SEQ ID NO:2) or Figure 7 (SEQ ID NO: 5). Ordinarily, a PRO10282 variant polypeptide will have at least about 80% amino acid sequence identity, more preferably at least about 81% amino acid sequence identity, more preferably at least about 82% amino acid sequence identity, more preferably at least about 83% amino acid sequence identity, more preferably at least about 84% amino acid sequence identity, more preferably at least about 85% amino acid sequence identity, more preferably at least about 86% amino acid sequence identity, more preferably at least about 87% amino acid sequence identity, more preferably at least about 88% amino acid sequence identity, more preferably at least about 89% amino acid sequence identity, more preferably at least about 90% amino acid sequence identity, more preferably at least about 91% amino acid sequence identity, more preferably at least about 92% amino acid sequence identity, more preferably at least about 93% amino acid sequence identity, more preferably at least about 94% amino acid sequence identity, more preferably at least about 95% amino acid sequence identity, more preferably at least about 96% amino acid sequence identity, more preferably at least about 97% amino acid sequence identity, more preferably at least about 98% amino acid sequence identity and yet more preferably at least about 99% amino acid sequence identity with (a) residues 1 to 667 of the PRO10282 polypeptide shown in Figure 2 (SEQ ID NO:2) or residues 1 to 658 of the PRO19578 polypeptide of Figure 7 (SEQ ID NO: 5), (b) 1 to X of Figure 2 (SEQ ID NO:2) or Figure 7 (SEQ ID NO: 5), wherein X is any amino acid from amino acid 49 to amino acid 59 of Figure 2 (SEQ ID NO:2) or Figure 7 (SEQ ID NO: 5), or (c) another specifically derived fragment of the amino acid sequence shown in Figure 2 (SEQ ID NO:2) or Figure 7 (SEQ 10 NO: 5). PRO10282 variant polypeptides do not encompass the native PRO10282 polypeptide sequence. Ordinarily, PRO10282 variant polypeptides are at least about 10 amino acids in length, often at least about 20 amino acids in length, more often at least about 30 amino acids in length, more often at least about 40 amino acids in length, more often at least about 50 amino acids in length, more often at least about 60 amino acids in length, more often at least about 70 amino acids in length, more often at least about 80 amino acids in length, more often at least about 90 amino acids in length, more often at least about 100 amino acids in length, more often at least about 150 amino acids in length, more often at least about 200 amino acids in length, more often at least about 250 amino acids in length, more often at least about 300 amino acids in length, or more, and are different from fragments of the murine Stra6 sequence, as disclosed in Bouillet et al., Mechanisms of Development 63, 173-186 (1997). Variants of other polypeptides disclosed herein are defined in an analogous manner.

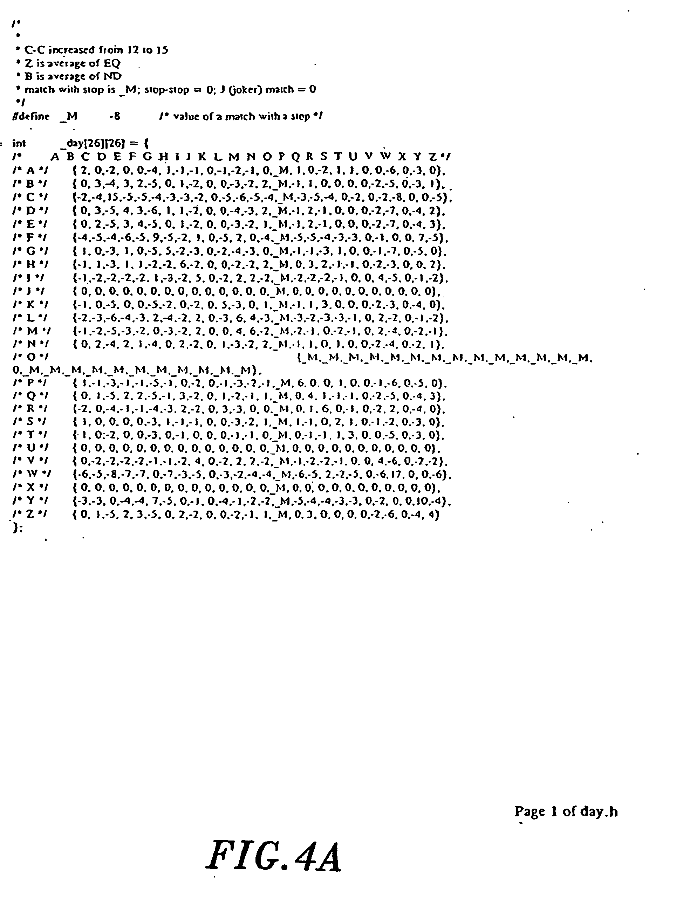

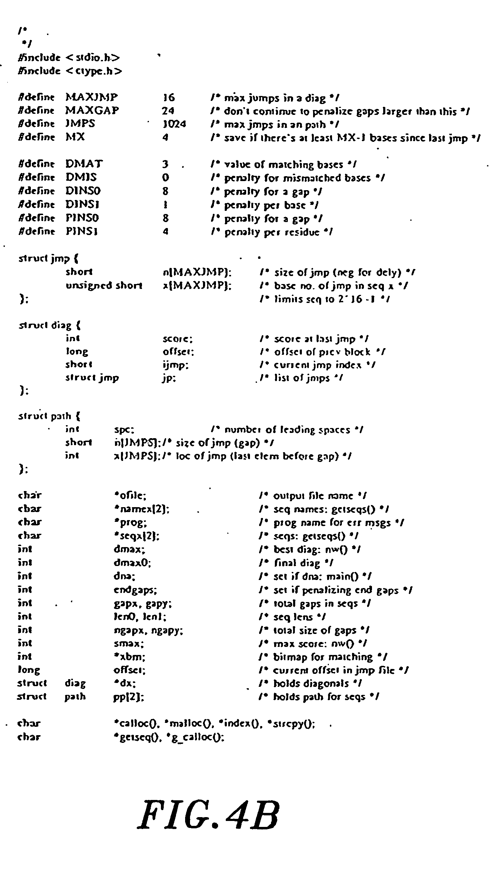

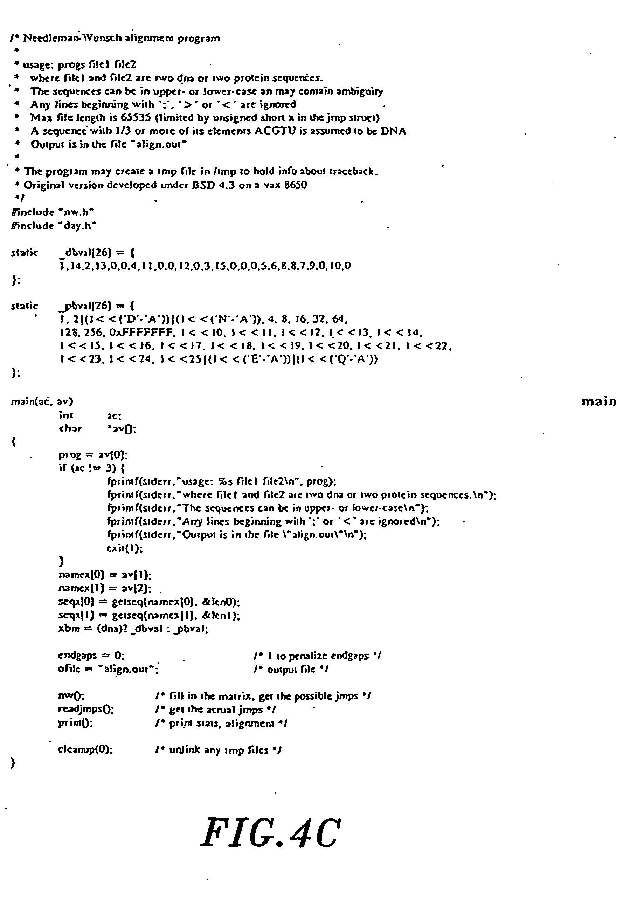

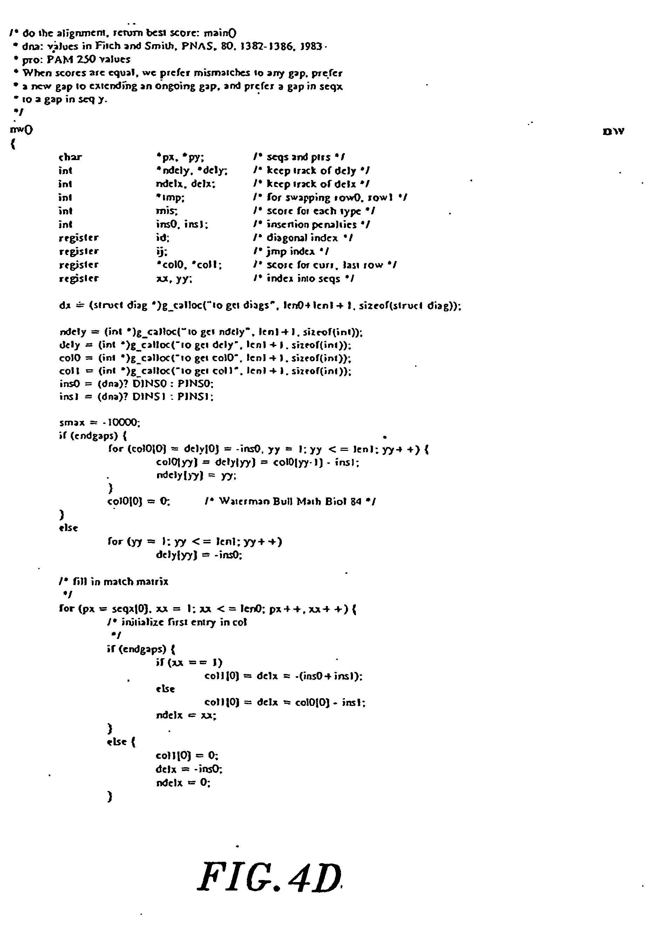

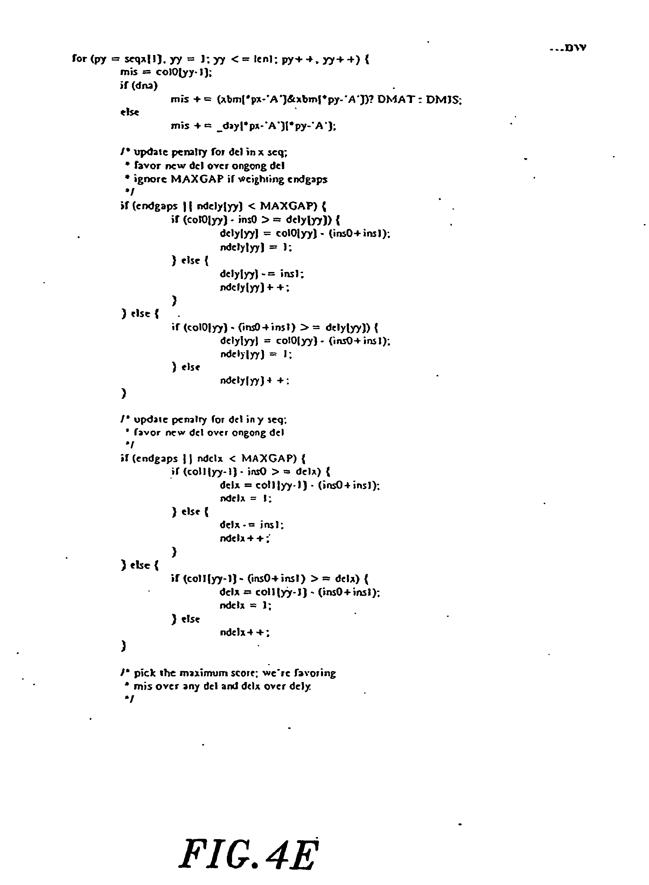

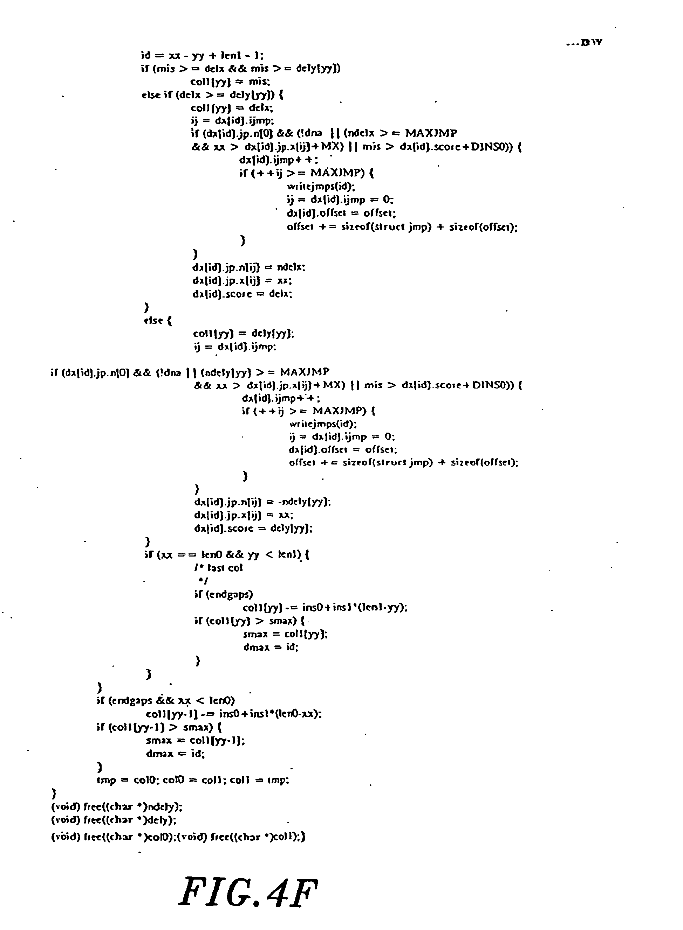

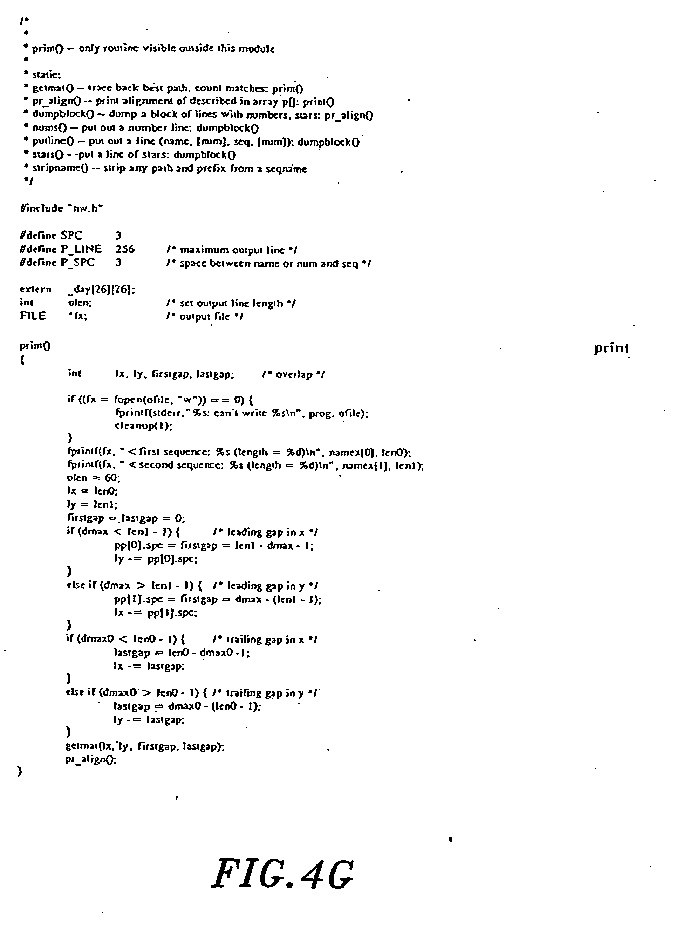

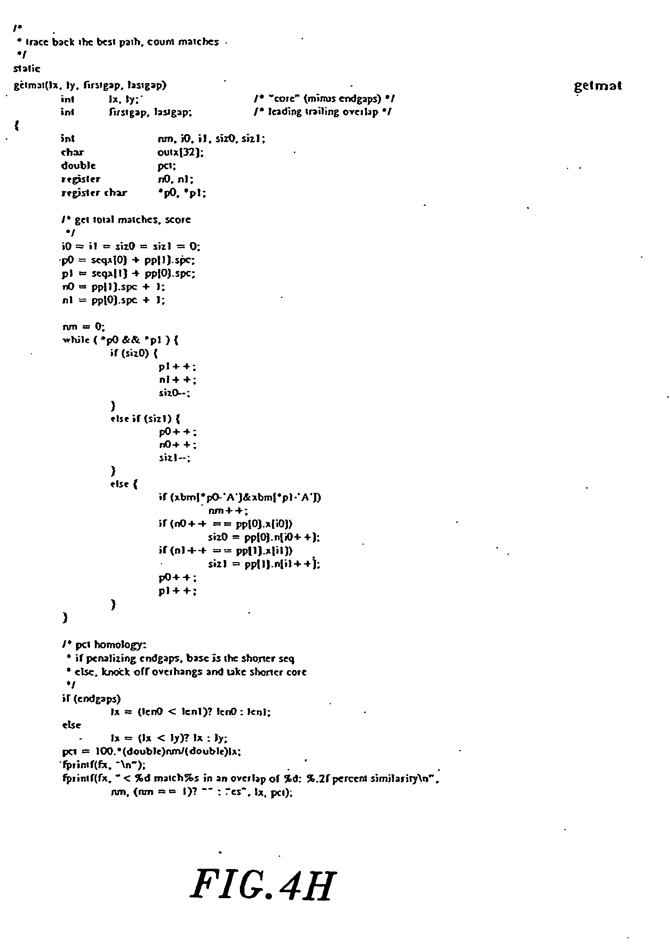

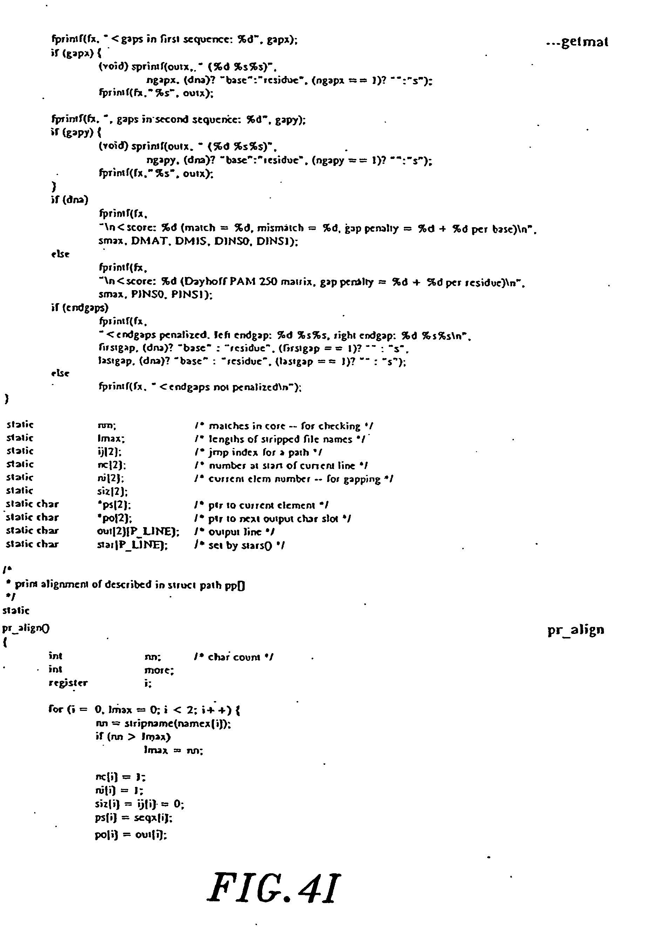

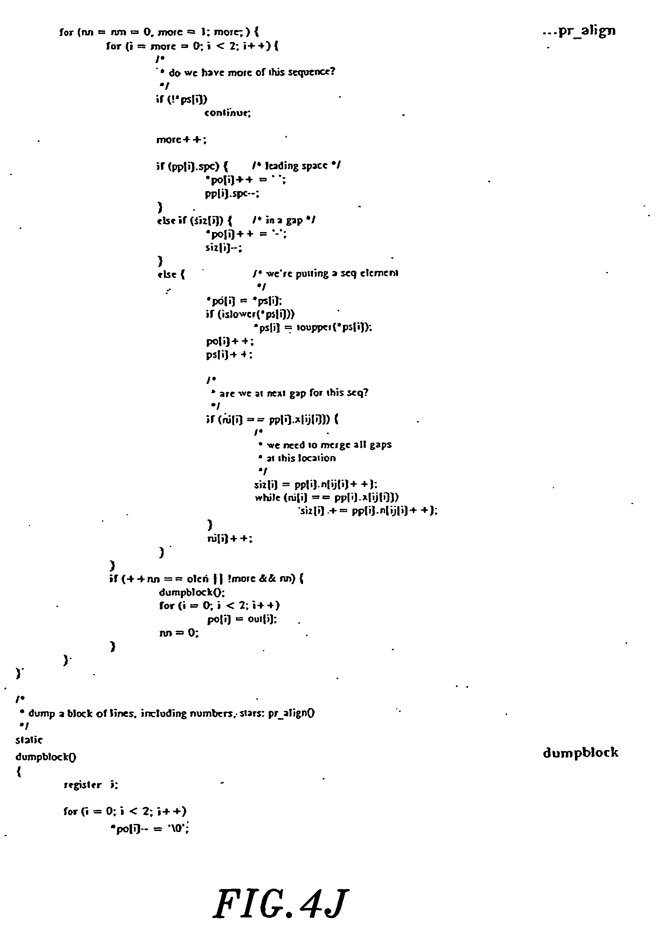

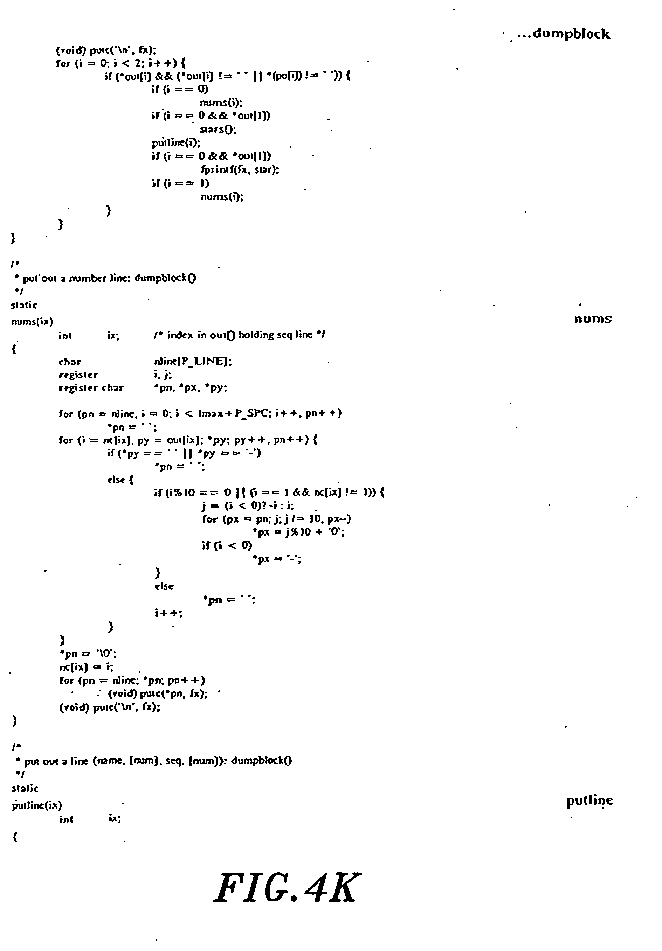

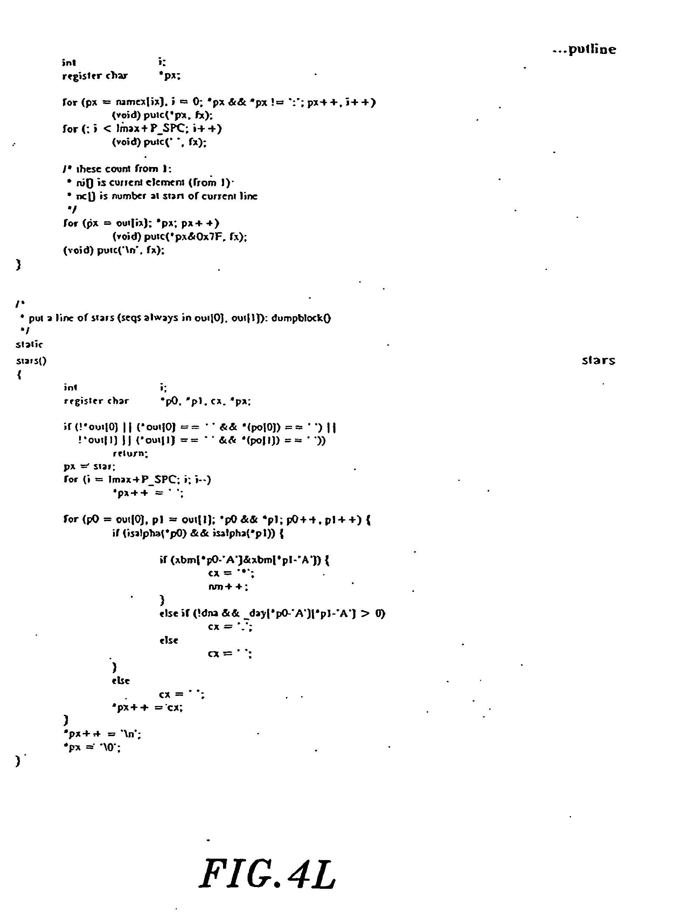

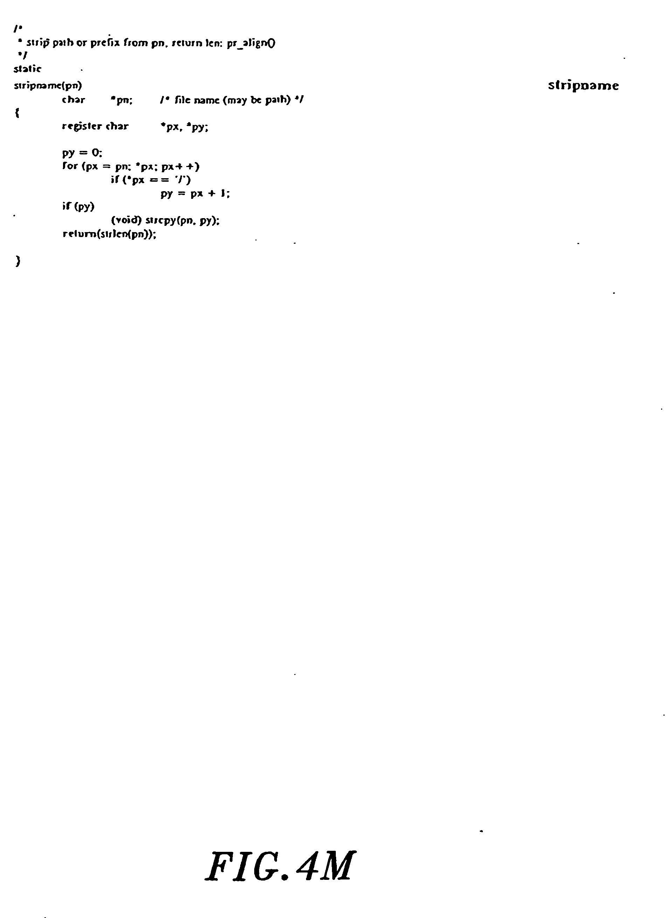

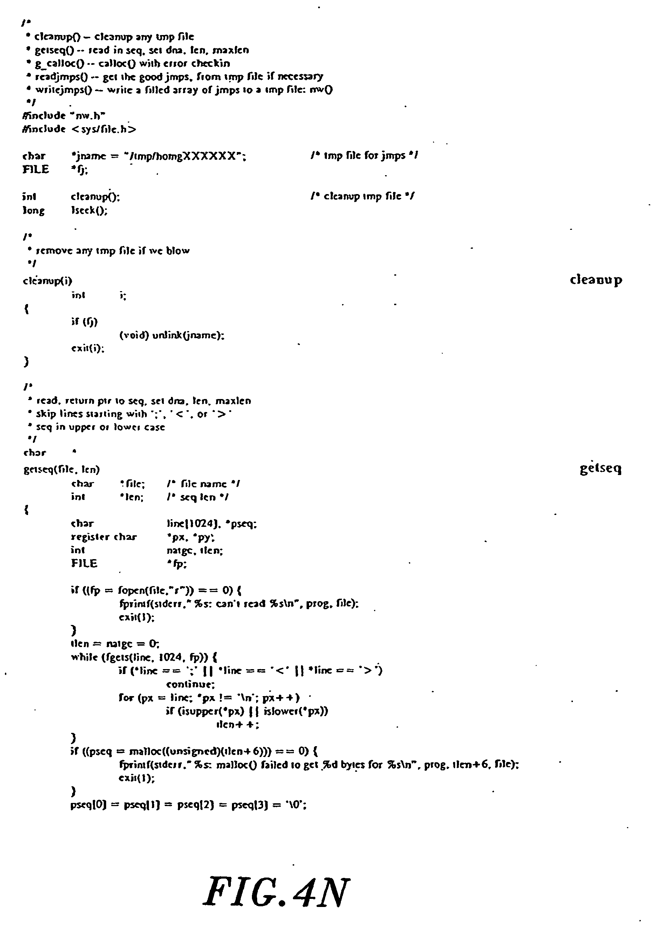

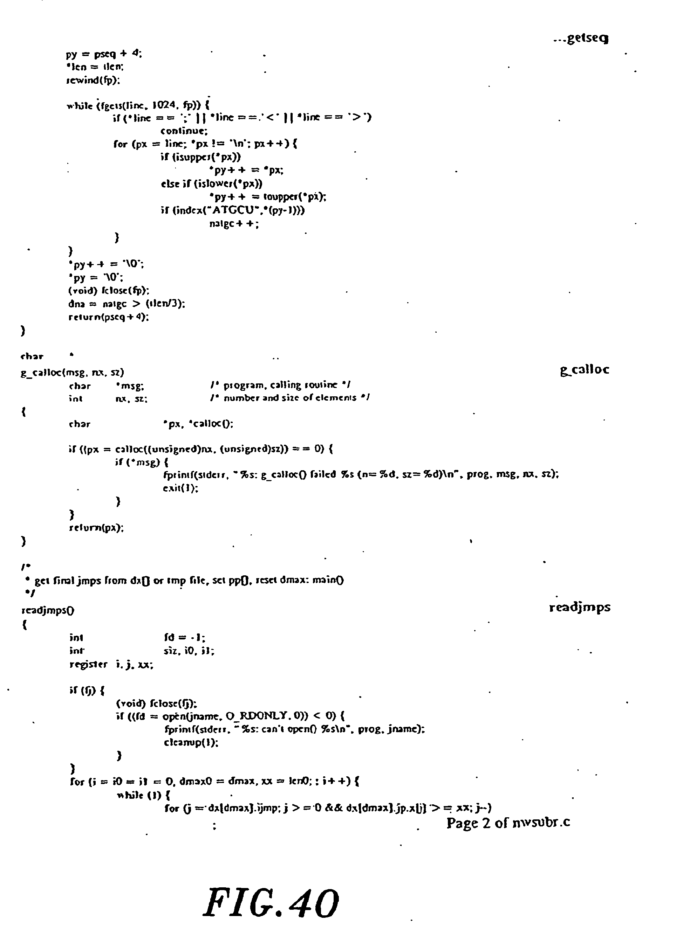

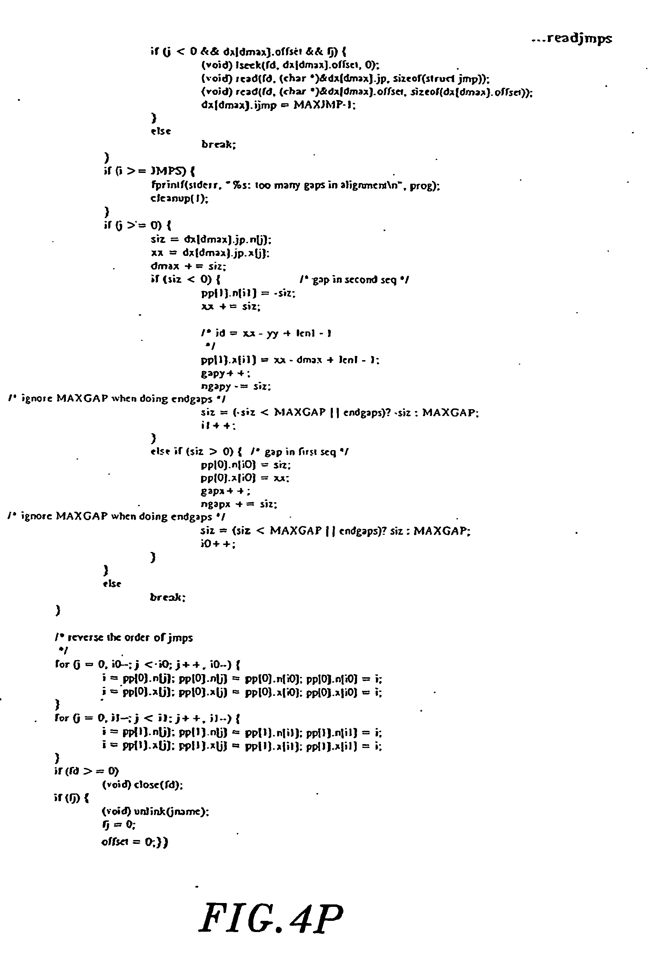

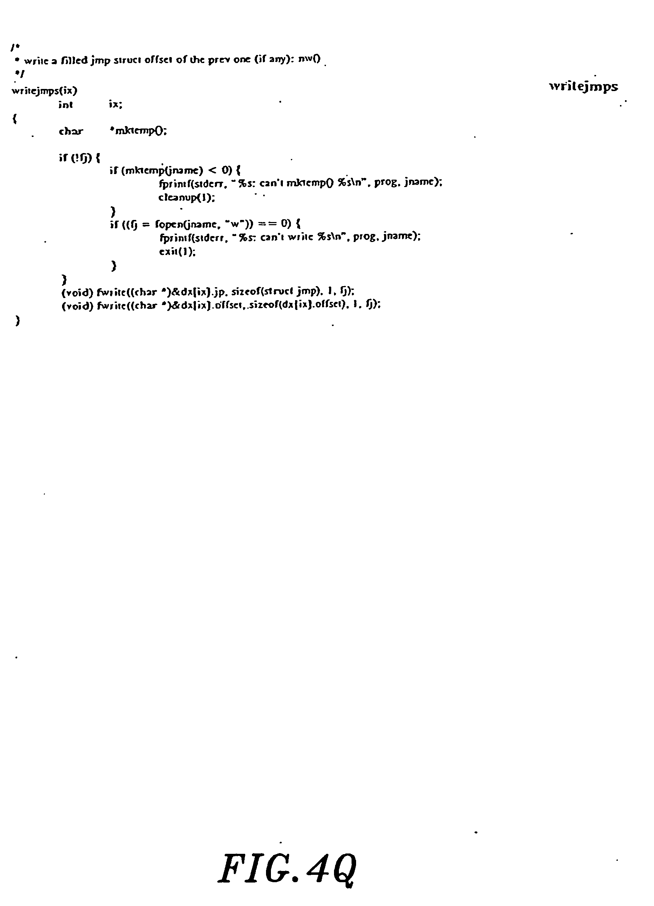

"Percent (%) amino acid sequence identity" is defined as the percentage of amino acid residues in a candidate sequence that are identical with the amino acid residues in a reference (e.g. native polypeptide) sequence, after aligning the sequences and introducing gaps, if necessary, to achieve the maximum percent sequence identity, and not considering any conservative substitutions as part of the sequence identity. Alignment for purposes of determining percent amino acid sequence identity can be achieved in various ways that are within the skill in the art, for instance, using publicly available computer software such as BLAST, BLAST-2, ALIGN, ALIGN-2 or Megalign (DNASTAR) software. Those skilled in the art can determine appropriate parameters for measuring alignment, including any algorithms needed to achieve maximal alignment over the full-length of the sequences being compared. For purposes herein, however, % amino acid sequence identity values are obtained as described below by using the sequence comparison computer program ALIGN-2, wherein the complete source code for the ALIGN-2 program is provided in Figures 4A-Q. The ALIGN-2 sequence comparison computer program was authored by Genentech, Inc. and the source code shown in Figures 4A-Q has been filed with user documentation in the U.S. Copyright Office, Washington D.C., 20559, where it is registered under U.S. Copyright Registration No. TXU510087. The ALIGN-2 program is publicly available through Genentech, Inc., South San Francisco, California or may be compiled from the source code provided in Figures 4A-Q. The ALIGN-2 program should be compiled for use on a UNIX operating system, preferably digital UNIX V4.0D. All sequence comparison parameters are set by the ALIGN-2 program and do not vary.

For purposes herein, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

**100 times the fraction X/Y**

where X is the number of amino acid residues scored as identical matches by the sequence alignment program ALIGN. 2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A. As examples of % amino acid sequence identity calculations, Figures 3A-B demonstrate how to calculate the % amino acid sequence identity of the amino acid sequence designated "Comparison Protein" to the amino acid sequence designated "PRO".

Unless specifically stated otherwise, all % amino acid sequence identity values used herein are obtained as described above using the ALIGN-2 sequence comparison computer program. However, % amino acid sequence identity may also be determined using the sequence comparison program NCBI-BLAST2 (Altschul et al., Nucleic Acids Res. 25:3389-3402 (1997)). The NCBI-BLAST2 sequence comparison program may be downloaded from http://www.ncbi.nlm.nih.gov. NCBI-BLAST2 uses several search parameters, wherein all of those search parameters are set to default values including, for example, unmask = yes, strand = all, expected occurrences = 10, minimum low complexity length - 15/5, multi-pass e-value = 0.01, constant for multi-pass = 25, dropoff for final gapped alignment = 25 and scoring matrix = BLOSUM62.

In situations where NCBI-BLAST2 is employed for amino acid sequence comparisons, the % amino acid sequence identity of a given amino acid sequence A to, with, or against a given amino acid sequence B (which can alternatively be phrased as a given amino acid sequence A that has or comprises a certain % amino acid sequence identity to, with, or against a given amino acid sequence B) is calculated as follows:

**100 times the fraction X/Y**

where X is the number of amino acid residues scored as identical matches by the sequence alignment program NCBI-BLAST2 in that program's alignment of A and B, and where Y is the total number of amino acid residues in B. It will be appreciated that where the length of amino acid sequence A is not equal to the length of amino acid sequence B, the % amino acid sequence identity of A to B will not equal the % amino acid sequence identity of B to A.

The terms "4-1BB ligand" and "4-1BBL" are used interchangeably and refer to a native sequence membrane protein belonging to the tumor necrosis factor superfamily, typically expressed on antigen presenting cells, and its variants. The nucleic acid and deduced amino acid sequences of a native human 4-1BB ligand are disclosed in Zhou et al, Immunol Lett 45:67-73 (1995). The amino acid sequence variants of native sequence 4-1BB ligands are defined on the analogy of the Stra6 protein discussed above, using the same algorithm to determine sequence identity. It has been shown that antibodies to the receptor of 4-1BBL (4-1BB) can eradicate established tumors in mice, and the 4-1 BB T cell stimulation pathway has been implicated in the amplification of an antitumor immune response.

The term "ephrin b1" is used herein to describe native sequence ephrin b1 molecules (also known as "lerk2") and their variants, which are defined as described above for Stra6. Human ephrin b1 is a 45 kDa, 346 amino acid glycosylated polypeptide that contains a 24 amino acid signal sequence, a 211 amino acid extracellular region, a 26 amino acid transmembrane (TM) region, and an 83 amino acid cytoplasmic segment (Davis, et al., Science 266:816 (1994); Beckmann, et al., EMBO J 13:3657 (1994)). There is an about 95% amino acid sequence identity between the extracellular regions of the mouse and human ephrin b1 polypeptides. A potential proteolytic cleavage site on ephrin b1 has been identified (Beckmann *et al., supra*). The role of ephrins in angiogenesis and in tumor progression is discussed, for example, in L'Allemain et al., Bull Cancer 87:529-530 (2000).

The terms "ISLR," "immunoglobulin superfamily containing leucine rich repeat," and "immunoglobulin superfamily containing LRR" are used interchangeably, and refer to native sequence ISRL proteins and their variants, as hereinabove defined. The cloning and sequencing of a native human ISLR was reported by Nagasawa et al., Genomics 44:173-179 (1997). Further human and mouse ISLR genes are described in Nagasawa et al., Genomics 61:37-43 (1999)..

The terms "autotaxin" and "ATX" are used interchangeably, and refer to native sequence autotaxin molecules of any animal species, and autotaxin variants, as hereinabove defined. Autotaxin is a cancer-related autocrine motility factor, which shows signifiant homology to the plasma cell membrane differentiation antigen PC-1. Human autotaxin is a 125-kDa, 915 aa glycoprotein originally isolated from a human melanoma cell line (Murata et al., J Biol Chem 269: 30479-84 (1994)). The cloning of autotaxin from human teratoarcinoma cells has been reported by Lee et al, Biochem Biophys Res Commun 218:714-719 (1996). Autotoxin has been described to catalyze the hydrolysis of the phosphodiester bond on either side of the beta-phosphate of ATP, and also to catalyze the hydrolysis of GTP to GDP and GMP, of either AMP or PPi to Pi, and the hydrolysis of NAD to AMP. Each of these substrates can serve as a phosphate donor in the phosphorylation of autotoxin. Autotoxin is believed to be a potent tumor motogen, which augments invasive and metastatic potential of ras-transformed cells (Nam et al., Oncogene 19:241-247 (2000)).

The term "retinoid" is used in the broadest sense and specifically includes, without limitation, retinoic acid (also known as tretinoin, vitamin A acid or vitamin $A_1$), and retinoic acid derivatives consisting of four isoprenoid units joined in a head-to-tail manner, such as retinol, retinal, substituted retinoids, seco-, nor-, and retro-retinoids. All retinoids may be formally derived from a monocyclic parent compound containing five carbon-carbon double bonds and a functional group at the terminus of the acyclic portion. For the nomenclature of retinoids see Moss, G.P., Arch. Biochem. Biophys., 224:728-731 (1983); Eur. J. Biochem., 129:1-5 (1982); J. Biol. Chem., 258:5329-5333 (1983); Pure Appl. Chem., 55: 721-726 (1983); Biochemical Nomenclature and Related Documents, 2nd edition, Portland Press, 1992, pages 247-251. The term "retinoid" specifically includes molecules occurring in nature and their derivatives, as long as they retain retinoid biological activity.

The term "retinoid biological activity," as defined herein, refers to involvement in embryogenesis, (epithelial) cell proliferation, cell differentiation and/or carcinogenesis, especially oncogenic effect associated with cancers characterized by aberrant Wnt signaling.

The term "Wnt" refers to a family of highly conserved, cysteine-rich, secreted glycoproteins that are involved in critical aspects of early embryonic development. Wnt genes are also implicated in cancer. "Wnt" as used herein specifically includes Wnt genes of all human and non-human animal species, including, but not limited to, mammals, such as human, mouse, rat and other rodents, etc. The term "Wnt" specifically includes native human Wnt genes and encoded polypeptides, including human Wnt-1 (previously called int-1) (van Ooyen et al., EMBO J 4:2905-9 (1985)); Wnt-2 (previously called Dint-1) (Wainwright et al., EMBO J 7:1743-1748 (1988)); Wnt-13 (Katoh et al., Oncogene 13: 873-876 (1996)); Wnt-3 (Roelink et al., Genomics 17:790-792 (1993)); Huguet et al., Cancer Res 54:2615-2521 (1994)); Wnt-4 (Huguet *et al.,* 1994, *supra*); Wnt-5A (Clark et al., Genomics 18:249-260 (1993)); Wnt-6 (Rankin et al., Cytogenet Cell Genet 84:50-52 (1999)); Wnt-7A (Ikegawa et al., Cytogenet Cell Genet 74:149-152 (1996)); Wnt-7B (Huguet *et al.,* 1994, *supra*); Wnt-8B (Lako et al., Genomics 35:386-388 (1996)); Wnt-10B (Bui et al., Oncogene 14:1249-1253 (1997)); Wnt-11 (Lako et al., Gene 219:101-110 (1998)); Wnt-14 (Bergstein et al., Genomics 46:450-458 (1997)); and Wnt-16 (McWhirter et al., Proc Natl Acad Sci USA 96:11464-11469 (1999); Fear et al., Biochem Biophys Res Commun 278: 814-820 (2000)), and their variants, in particular amino acid sequence variants, which have at least about 80%, more preferably at least about 85%, more preferably at least about 90%, even more preferably at least about 95%, yet more preferably at least about 98%, more preferably at least about 99% percent amino acid sequence identity with a native Wnt polypeptide, wherein sequence identity is determined as hereinabove defined. In a preferred embodiment, the term "Wnt" refers to any transforming (oncogenic) Wnt polypeptide, as discussed in Wong et al., Mol Cell Biol 14:6278-86 (1994).

A cancer is "characterized by aberrant Wnt signaling" if it harbors genetic defects and/or shows altered expression patterns (including mutations, amplification, over-expression and/or suppression) of any member of a Wnt signaling pathway. Wnts exert their effects through interaction with cell surface receptors, named "Frizzled." Structurally, the Frizzled receptors have an extracellular Wnt-binding domain, seven transmembrane-spanning regions, and an intracellular C-terminal tail. Additional components of the Frizzled receptor-Wnt interaction pathway are the soluble Wnt inhibitors which consist of secreted proteins containing a cysteine-rich domain (CRD) similar to that in the ligand binding domain of the Frizzled receptors. These Wnt inhibitory proteins are collectively referred to as Frizzled Receptor-like Proteins (FRPs). Since the Frizzled receptors have no enzymatic motifs on their intracellular domains, the Wnt signals are believed to be transmitted through receptor coupling to Dishevelled (dsh) proteins. The next step in the Wnt signaling cascade is the inhibition of the serine/threonine kinase, glycogen synthetase kinase-3b (GSK-3b) by Dishevelled. The consequence of the Wnt-induced inhibition of GSK-3b activity is that the next protein in the cascade, β-catenin, is stabilized. The central region of the β-catenin protein contains a positively charged groove which is believed to interact with acidic regions in the adenometous polyposis coli (APC) locus encoded protein, the transcription factor TCF/LEF1, and the cadherin family of calcium-dependent cell-adhesion molecules. Members of the Wnt signaling pathway, including APC and β-catenin, have been implicated in the pathogenesis of a series of human cancers, including colon cancer, breast cancer, hepatocellular cancers, and melanoma (Morin et al., Science 275:1787-1790 (1997)). Mutations in specific regions of either gene can cause the stabilization and accumulation of cytoplasmic β-catenin, which is believed to contribute to human carcinogenesis through the activation of target genes such as the WISP genes (Pennica et al., Proc. Natl. Acad. Sci. USA 95:14717-14722 (1998)). Mutations in the Wnt-1 gene are also involved in the development of Wilm's tumors, a kidney cancer found in children. As discussed above, further members of the Wnt signaling pathway include, without limitation, other members of the Wnt gene family, Frizzled receptors, the cytoplasmic protein Dishevelled (Dsh), glycogen synthase kinase-3β (GSK-3β), the transcription factor TCFILEF1, and transcriptionally activated downstream components of the pathway, such as the nodal-related 3 gene, Xnr3, the homeobox genes, engrailed, goosecoid, twin (Xtwn), and siamois, c-myc, and the WISP genes, e.g. WISP-1 and WISP-2. The term "characterized by aberrant Wnt signaling" includes genetic defects and/or altered expression patterns (including mutations, amplification, overexpression and/or suppression) of any of these members of the Wnt signaling pathway, or any other members, known today or hereinafter identified.

The enhancement of the expression of an antigen in a tumor, e.g. cancer, is "selective," if the enhancement of the expression level of such antigen in tumor cells is significantly higher than in normal cells. Preferably, the enhancement of the expression level of a target antigen (e.g. a cell surface antigen) in tumor cells results in at least about two-fold, more preferably at least about three-fold, even more preferably at least about five-fold, most preferably at least about ten-fold over-expression in tumor cells or tissues relative to corresponding normal (non-tumorigenic) cells or tissues.

"Tumor", as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Examples of cancer include but are not limited to, carcinoma, lymphoma, blastoma, sarcoma, and leukemia. More particular examples of such cancers-include breast cancer, prostate cancer, colon cancer, squamous cell cancer, small-cell lung cancer, non-small cell lung cancer, gastrointestinal cancer, pancreatic cancer, glioblastoma, cervical cancer, ovarian cancer, liver cancer, bladder cancer, hepatoma, colorectal cancer, endometrial carcinoma, salivary gland carcinoma, kidney cancer, liver cancer, vulval cancer, thyroid cancer, hepatic carcinoma and various types of head and neck cancer. Cancers particularly amenable to treatment in accordance with the present invention include ovarian cancer, endometrial cancer, Wilm's kidney tumor, colon cancer, breast cancer, prostate cancer, gastric cancer, lung cancer, hepatocellular cancer, and melanoma.

"Treatment" refers to both therapeutic treatment and prophylactic or preventative measures, wherein the object is to prevent or slow down (lessen) the targeted pathologic condition or disorder. Those in need of treatment include those already with the disorder as well as those prone to have the disorder or those in whom the disorder is to be prevented. In tumor (*e.g.,* cancer) treatment, a therapeutic agent may directly decrease the pathology of tumor cells, or render the tumor cells more susceptible to treatment by other therapeutic agents, *e.g.*, radiation and/or chemotherapy.

The "pathology" of cancer includes all phenomena that compromise the well-being of the patient. This includes, without limitation, abnormal or uncontrollable cell growth, metastasis, interference with the normal functioning of neighboring cells, release of cytokines or other secretory products at abnormal levels, suppression or aggravation of inflammatory or immunological response, etc.

"Chronic" administration refers to administration of the agent(s) in a continuous mode as opposed to an acute mode, so as to maintain the initial therapeutic effect (activity) for an extended period of time. "Intermittent" administration is treatment that is not consecutively done without interruption, but rather is cyclic in nature.

"Mammal" for purposes of treatment refers to any animal classified as a mammal, including humans and other higher mammals, domestic and farm animals, and zoo, sports, or pet animals, such as dogs, cats, cattle, horses, sheep, pigs, goats, rabbits, etc. Preferably, the mammal is human.

Administration "in combination with" one or more further therapeutic agents, or "co-administration," which terms are used interchangeably, includes simultaneous (concurrent) and consecutive administration in any order.

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

"Native antibodies and immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain (VH) followed by a number of constant domains. Each light chain has a variable domain at one end (VL) and a constant domain at its other end; the constant domain of the light chain is aligned with the first constant domain of the heavy chain, and the light chain variable domain is aligned with the variable domain of the heavy chain. Particular amino acid residues are believed to form an interface between the light-and heavy-chain variable domains (Chothia et al., J. Mol. Biol. 186:651 [1985]; Novotny and Haber, Proc. Natl. Acad. Sci. U.S.A. 82:4592 [1985]; Chothia et al., Nature 342: 877-883 [1989]).

The term "variable" refers to the fact that certain portions of the variable domains differ extensively in sequence among antibodies and are used in the binding and specificity of each particular antibody for its particular antigen. However, the variability is not evenly distributed throughout the variable domains of antibodies. It is concentrated in three segments called complementarity-determining regions (CDRs) or hypervariable regions both in the light-chain and the heavy-chain variable domains. The more highly conserved portions of variable domains are called the framework (FR). The variable domains of native heavy and light chains each comprise four FR regions, largely adopting a β-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen-binding site of antibodies (see Kabat *et al.* (1991) *supra*). The constant domains are not involved directly in binding an antibody to an antigen, but exhibit various effector functions, such as participation of the antibody in antibody-dependent cellular toxicity.

The "light chains" of antibodies (immunoglobulins) from any vertebrate species can be assigned to one of two clearly distinct types, called κ and λ, based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG, and IgM, and several of these can be further divided into subclasses (isotypes), e.g, $IgG_1$, $IgG_2$, $IgG_3$, $IgG_4$, $IgA_1$, and $IgA_2$. The heavy-chain constant domains that correspond to the different classes of immunoglobulins are called α, δ, ε γ, and μ respectively. The subunit structures and three-dimensional configurations of different classes of immunoglobulins are well known.

The term "antibody" includes all classes and subclasses of intact immunoglobulins. The term "antibody" also covers antibody fragments. The term "antibody" specifically covers monoclonal antibodies, including antibody fragment clones.

"Antibody fragments" comprise a portion of an intact antibody that contains the antigen binding or variable region of the intact antibody. Examples of antibody fragments include Fab, Fab', $F(ab')_2$, and Fv fragments; diabodies; single-chain antibody molecules, including single-chain Fv (scFv) molecules; and multispecific antibodies formed from antibody fragments.

The term "monoclonal antibody" as used herein refers to an antibody (or antibody fragment) obtained from a population of substantially homogeneous antibodies, *i.e.,* the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, and are not contaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., Nature, 256:495 (1975), or may be made by recombinant DNA methods (see, *e.g.,* U.S. Patent No. 4,816,567). The "monoclonal antibodies" also include clones of antigen-recognition and binding-site containing antibody fragments (Fv clones) isolated from phage antibody libraries using the techniques described in Clackson et al., Nature, 352:624-628 (1991) and Marks et al., J. Mol. Biol., 222:581-597 (1991), for example.

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (U.S. Patent No. 4,816,567 to Cabilly et al.*;* Morrison et al., Proc. Natl. Acad Sci. USA, 81:6851-6855 [1984]).

"Humanized" forms of non-human (*e.g.*, murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. For the most part, humanized antibodies are human immunoglobulins (recipient antibody) in which residues from part or all of a complementarity-determining region (CDR) of the recipient are replaced by residues from a CDR of a non-human species (donor antibody) such as mouse, rat or rabbit having the desired specificity, affinity, and capacity. In some instances, Fv framework region (FR) residues of the human immunoglobulin are replaced by corresponding non-human residues. Furthermore, humanized antibodies may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin sequence. The humanized antibody optimally also will comprise at least a portion of an immunoglobulin constant region (Fc), typically that of a human immunoglobulin. For further details, see Jones et al., Nature, 321:522-525 (1986); Reichmann et al., Nature, 332:323-329 (1988); Presta, Curr. Op. Struct. Biol., 2:593-596 (1992); and Clark, Immunol. Today 21: 397-402 (2000). The humanized antibody includes a Primatized™ antibody wherein the antigen-binding region of the antibody is derived from an antibody produced by immunizing macaque monkeys with the antigen of interest.

"Single-chain Fv" or "scFv" antibody fragments comprise the VH and VL domains of antibody, wherein these domains are present in a single polypeptide chain. Generally, the scFv polypeptide further comprises a polypeptide linker between the VH and VL domains, which enables the scFv to form the desired structure for antigen binding. For a review of scFv see Pluckthun, in The Pharmacology of Monoclonal Antibodies, vol. 113, Rosenburg and Moore eds., Springer-Verlag, New York, pp. 269-315 (1994), Dall'Acqua and Carter, Curr. Opin. Struct. Biol. 8: 443-450 (1998), and Hudson, Curr. Opin. Immunol. 11: 548-557 (1999).

The term "diabodies" refers to small antibody fragments with two antigen-binding sites, which fragments comprise a heavy-chain variable domain (VH) connected to a light-chain variable domain (VL) in the same polypeptide chain (VH - VL). By using a linker that is too short to allow pairing between the two domains on the same chain, the domains are forced to pair with the complementary domains of another chain and create two antigen-binding sites. Diabodies are described more fully in, for example, EP 404,097; W0 93/11161; and Hollinger et al., Proc. Natl. Acad. Sci. USA, 90:6444-6448 (1993).

An "isolated" antibody is one which has been identified and separated and/or recovered from a component of its natural environment. Contaminant components of its natural environment are materials which would interfere with diagnostic or therapeutic uses for the antibody, and may include enzymes, hormones, and other proteinaceous or nonproteinaceous solutes. In preferred embodiments, the antibody will be purified (1) to greater than 95% by weight of antibody as determined by the Lowry method, and most preferably more than 99% by weight, (2) to a degree sufficient to obtain at least 15 residues of N-terminal or internal amino acid sequence by use of a spinning cup sequenator, or (3) to homogeneity by SDS-PAGE under reducing or nonreducing conditions using Coomassie blue or, preferably, silver stain. Isolated antibody includes the antibody *in situ* within recombinant cells since at least one component of the antibody's natural environment will not be present. Ordinarily, however, isolated antibody will be prepared by at least one purification step.

By "neutralizing antibody" is meant an antibody molecule which is able to eliminate or significantly reduce an effector function of a target antigen to which it binds.

"Carriers" as used herein include pharmaceutically acceptable carriers, excipients, or stabilizers which are nontoxic to the cell or mammal being exposed thereto at the dosages and concentrations employed. Often the physiologically acceptable carrier is an aqueous pH buffered solution. Examples of physiologically acceptable carriers include buffers such as phosphate, citrate, and other organic acids; antioxidants including ascorbic acid; low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, arginine or lysine; monosaccharides, disaccharides, and other carbohydrates including glucose, mannose, or dextrins; chelating agents such as EDTA; sugar alcohols such as mannitol or sorbitol; salt-forming counterions such as sodium; and/or nonionic surfactants such as TWEEN™, polyethylene glycol (PEG), and PLURONICS™.

The word "label" when used herein refers to a detectable compound or composition which is conjugated directly or indirectly to the antibody so as to generate a "labeled" antibody. The label may be detectable by itself (e.g. radioisotope labels or fluorescent labels) or, in the case of an enzymatic label, may catalyze chemical alteration of a substrate compound or composition which is detectable.

By "solid phase" is meant a non-aqueous matrix to which the antibody of the present invention can adhere. Examples of solid phases encompassed herein include those formed partially or entirely of glass (e.g., controlled pore glass), polysaccharides (e.g., agarose), polyacrylamides, polystyrene, polyvinyl alcohol and silicones. In certain embodiments, depending on the context, the solid phase can comprise the well of an assay plate; in others it is a purification column (e.g., an affinity chromatography column). This term also includes a discontinuous solid phase of discrete particles, such as those described in U.S. Patent No. 4,275,149.

A "liposome" is a small vesicle composed of various types of lipids, phospholipids and/or surfactant which is useful for delivery of a drug (such as a PRO10282 polypeptide or antibody thereto) to a mammal. The components of the liposome are commonly arranged in a bilayer formation, similar to the lipid arrangement of biological membranes.

A "small molecule" is defined herein to have a molecular weight below about 500 Daltons.

The term "upregulation" is used in the broadest sense and refers to the induction and/or enhancement of gene expression as measured, for example, by quantification of mRNA levels.

The phrases "gene amplification" and "gene duplication" are used interchangeably and refer to a process by which multiple copies of a gene or gene fragment are formed in a particular cell or cell line. The duplicated region (a stretch of amplified DNA) is often referred to as "amplicon." Usually, the amount of the messenger RNA (mRNA) produced, *i.e.,* the level of gene expression, also increases in the proportion of the number of copies made of the particular gene expressed.

The term "anti-tumor agent" or "anti-cancer agent" is used in the broadest sense and including any molecule useful in the treatment of cancer. Such molecules include, without limitation, polypeptides (including proteins), such as antibodies, peptides, organic and inorganic small molecules, DNA and RNA molecules, etc.

The term "cytotoxic agent" as used herein refers to a substance that inhibits or prevents the function of cells and/or causes destruction of cells. Without limitation, the term is intended to include radioactive isotopes (e,g., $I^{131}$, $I^{125}$, $Y^{90}$ and $Re^{166}$), chemotherapeutic agents, and toxins such as enzymatically active toxins of bacterial, fungal, plant or animal origin, or fragments thereof. The term specifically includes maytansines and maytansinoids, BCNU, streptozoicin, vincristine, and the family of agents described in U.S. patent Nos. 5,053,395 and 5,770,710, as well as esperamicins (U.S. patent No. 5,877,296).

A "chemotherapeutic agent" is a chemical compound useful in the treatment of cancer. Examples of chemotherapeutic agents include adriamycin, doxorubicin, epirubicin, 5-fluorouracil, cytosine arabinoside ("Ara-C"), cyclophosphamide, thiotepa, busulfan, cytoxin, taxoids, *e.g.,* paclitaxel (Taxol, Bristol-Myers Squibb Oncology, Princeton, NJ), and doxetaxel (Taxotere, Rhône-Poulenc Rorer, Antony, Rnace), toxotere, methotrexate, cisplatin, melphalan, vinblastine, bleomycin, etoposide, ifosfamide, mitomycin C, mitoxantrone, vincristine, vinorelbine, carboplatin, teniposide, daunomycin, carminomycin, aminopterin, dactinomycin, mitomycins, esperamicins (see U.S. Pat. No. 4,675,187), 5-FU, 6-thioguanine, 6-mercaptopurine, actinomycin D, VP-16, chlorambucil, melphalan, and other related nitrogen mustards. Also included in this definition are hormonal agents that act to regulate or inhibit hormone action on tumors such as tamoxifen and onapristone.

A "growth inhibitory agent" when used herein refers to a compound or composition which inhibits growth of a cell, especially cancer cell overexpressing any of the genes identified herein, either *in vitro* or *in vivo.* Thus, the growth inhibitory agent is one which significantly reduces the percentage of cells overexpressing such genes in S phase. Examples of growth inhibitory agents include agents that block cell cycle progression (at a place other than S phase), such as agents that induce G1 arrest and M-phase arrest. Classical M-phase blockers include the vincas (vincristine and vinblastine), taxol, and topo II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogens, and antineoplastic drugs" by Murakami et al., (WB Saunders: Philadelphia, 1995), especially p. 13.

"Doxorubicin" is an anthracycline antibiotic. The full chemical name of doxorubicin is (8S-cis)-10-[(3-amino-2,3,6-trideoxy-L-lyxo-hexapyranosyl) oxy]-7,8,9,10-tetrahydro-6,8,11-trihydroxy-8-(hydroxyacetyl)-1-methoxy-5,12-naphthacenedione.

The term "cytokine" is a generic term for proteins released by one cell population which act on another cell as intercellular mediators. Examples of such cytokines are lymphokines, monokines, and traditional polypeptide hormones. Included among the cytokines are growth hormone such as human growth hormone, N-methionyl human growth hormone, and bovine growth hormone; parathyroid hormone; thyroxine; insulin; proinsulin; relaxin; prorelaxin; glycoprotein hormones such as follicle stimulating hormone (FSH), thyroid stimulating hormone (TSH), and luteinizing hormone (LH); hepatic growth factor; fibroblast growth factor; prolactin; placental lactogen; tumor necrosis factor-$\alpha$ and -$\beta$; mullerian-inhibiting substance; mouse gonadotropin-associated peptide; inhibin; activin; vascular endothelial growth factor; integrin; thrombopoietin (TPO); nerve growth factors such as NGF; platelet-growth factor; transforming growth factors (TGFs) such as TGF-$\alpha$ and TGF-$\beta$; insulin-like growth factor-I and -II; erythropoietin (EPO); osteoinductive factors; interferons such as interferon-$\alpha$, -$\beta$, and -$\gamma$; colony stimulating factors (CSFs) such as macrophage-CSF (M-CSF); granulocyte-macrophage-CSF (GM-CSF); and granulocyte-CSF (G-CSF); interleukins (ILs) such as IL-1, IL- 1a, IL-2, IL-3, IL-4, IL-5, IL-6, IL-7, IL-8, IL-9, IL-11, IL-12; a tumor necrosis factor such as TNF-$\alpha$ or TNF-$\beta$; and other polypeptide factors including LIF and kit ligand (KL). As used herein, the term cytokine includes proteins from natural sources or from recombinant cell culture and biologically active equivalents of the native sequence cytokines.

The term "prodrug" as used in this application refers to a precursor or derivative form of a pharmaceutically active substance that is less cytotoxic to tumor cells compared to the parent drug and is capable of being enzymatically activated or converted into the more active parent form. *See, e.g.,* Wilman, "Prodrugs in Cancer Chemotherapy", Biochemical Society Transactions, 14:375-382, 615th Meeting, Belfast (1986), and Stella et al., "Prodrugs: A Chemical Approach to Targeted Drug Delivery", Directed Drug Delivery, Borchardt et al., (ed.), pp. 147-267, Humana Press (1985). The prodrugs of this invention include, but are not limited to, phosphate-containing prodrugs, thiophosphate-containing prodrugs, sulfate-containing prodrugs, peptide-containing prodrugs, D-amino acid-modified prodrugs, glysocylated prodrugs, β-lactam-containing prodrugs, optionally substituted phenoxyacetamide-containing prodrugs or optionally substituted phenylacetamide-containing prodrugs, 5-fluorocytosine and other 5-fluorouridine prodrugs which can be converted into the more active cytotoxic free drug. Examples of cytotoxic drugs that can be derivatized into a prodrug form for use in this invention include, but are not limited to, those chemotherapeutic agents described above.

An "effective amount" of a polypeptide disclosed herein or an antagonist thereof, in reference to inhibition of neoplastic cell growth, tumor growth or cancer cell growth, is an amount capable of inhibiting, to some extent, the growth of target cells. The term includes an amount capable of invoking a growth inhibitory, cytostatic and/or cytotoxic effect and/or apoptosis of the target cells. An "effective amount" of a polypeptide for the purposes of inhibiting neoplastic cell growth, tumor cell or cancer cell growth, may be determined empirically and in a routine manner.

A "therapeutically effective amount", in reference to the treatment of tumor, refers to an amount capable of invoking one or more of the following effects: (1) inhibition, to some extent, of tumor growth, including, slowing down and complete growth arrest; (2) reduction in the number of tumor cells; (3) reduction in tumor size; (4) inhibition (*i.e.*, reduction, slowing down or complete stopping) of tumor cell infiltration into peripheral organs; (5) inhibition (*i.e.*, reduction, slowing down or complete stopping) of metastasis; (6) enhancement of anti-tumor immune response, which may, but does not have to, result in the regression or rejection of the tumor; and/or (7) relief, to some extent, of one or more symptoms associated with the disorder. A "therapeutically effective amount" of an anti-tumor agent may be determined empirically and in a routine manner.

A "growth inhibitory amount" of an anti-tumor agent is an amount capable of inhibiting the growth of a cell, especially tumor, e.g. cancer cell, either *in vitro* or *in vivo.*

A "cytotoxic amount" of an anti-tumor agent is an amount capable of causing the destruction of a cell, especially tumor, e.g. cancer cell, either *in vitro* or *in vivo.* The "cytotoxic amount" for purposes of inhibiting neoplastic cell growth may be determined empirically and in a routine manner.

"Antisense oligodeoxynucleotides" or "antisense oligonucleotides" (which terms are used interchangeably) are defined as nucleic acid molecules that can inhibit the transcription and/or translation of target genes in a sequence-specific manner. The term "antisense" refers to the fact that the nucleic acid is complementary t the coding ("sense") genetic sequence of the target gene. Antisense oligonucleotides hybridize in an antiparallel orientation to nascent mRNA through Watson-Crick base-pairing. By binding the target mRNA template, antisense oligonucleotides block the successful translation of the encoded protein. The term specifically includes antisense agents called "ribozomes" that have been designated to induce catalytic cleavage of a target RNA by addition of a sequence that has natural self-splicing activity (Warzocha and Wotowiec, "Antisense strategy" biological utility and prospects int he treatment of hematological malignancies." Leuk. Lymphoma 24:267-281 [1997]).

The term "therapeutic index" refers to the ratio between the toxic concentration and the effective concentration of a drug, such a therapeutic antibody.

B. Detailed Description

As discussed above, several components of the Wnt signaling pathway have been implicated in human tumors or experimental cancer models. Wnt-1 was found as an oncogene activated by the Mouse Mammary Tumor Virus (MMTV) in murine breast cancer (Nusse and Varnus, Cell 31;99-109 (1982)), Another member of the Wnt pathway, the adenomatous polyposis coll (APC) tumor suppressor was first isolated in human colon cancer (reviewed in Polakis, Biochem. Biophys. Acta 1332(3):F127-47 (1997)). After establishing that APC and β-catenin bind each other, activating mutations in the human β-catenin gene were found in human colon cancer end melanomes (Morin et al., Science 275: 1752-53 (1997)). For review of the role of the Wnt signaling in cancer see, e.g. Polakis, Genes Dev. 14;1837-51 (2000) and Bienz and Clevers, Cell 103:311-20 (2000)).

The present invention concerns the enhancement of the efficacy of treatment, such as immunotherapy, of tumors driven by Wnt signaling.

1. Identification of Gene Targets for Tumor Treatment

Drug targets for tumor treatment may be identified by analyzing differences in gene expression resulting from retinoid treatment of tumor cells. In particular, the invention concerns the identification of antigens that are preferentially upregulated by the treatment of Wnt-transformed cells with retinoids, such as retinoic acid, as targets for cancer therapy. The preferred gene targets are those which express cell surface proteins, and which are synergistically upregulated by retinoid treatment and Wnt signaling.

*a. Gene Expression Profiling*

The most commonly used methods known in the art for the quantification of mRNA expression in a sample include northern blotting and *in situ* hybridization (Parker & Barnes, Methods in Molecular Biology 106:247-283 (1999)); RNAse protection assays (Hod, Biotechniques 13:852-854 (1992)); and reverse transcription polymerase chain reaction (RT-PCR) (Weis et al., Trends in Genetics 8:283-264 (1992)). Alternatively, antibodies may be employed that can recognize specific duplexes, including DNA duplexes, RNA duplexes, and DNA-RNA hybrid duplexes or DNA-protein duplexes.

*Reverse Transcriptase PCR (RT-PCR)*

Of the techniques listed above, the most sensitive and most flexible quantitative method is RT-PCR, which can be used to compare mRNA levels in different sample populations, in normal and tumor tissues, with or without drug treatment, to characterize patterns of gene expression, to discriminate between closely related mRNAs, and to analyze RNA structure.

The first step is the isolation of mRNA from a target sample. The starting material is typically total RNA isolated from human tumors or tumor cell lines, and corresponding normal tissues or cell lines, respectively. Thus RNA can be isolated from a variety of primary tumors, including breast, lung, colon, prostate, brain, liver, kidney, pancreas, spleen, thymus, testis, ovary, uterus, etc., tumor, or tumor cell lines, with pooled DNA from healthy donors. If the source of mRNA is a primary tumor, mRNA can be extracted, for example, from frozen or archived paraffin-embedded and fixed (e.g. formalin-fixed) tissue samples.

Tumor cell lines, include, for example, human lung carcinoma cell lines, such as A549 (SRCC768), Calu-1 (SRCC769), Calu-6 (SRCC770), H157 (SRCC771), H441 (SRCC772), H460 (SRCC773), SKMES-1 (SRCC774), SW900 (SRCC775), H522 (SRCC832),and H810 (SRCC833), all available from ATCC. Primary human lung tumor cells usually derive from adenocarcinomas, squamous cell carcinomas, large cell carcinomas, non-small cell carcinomas, small cell carcinomas, and broncho alveolar carcinomas, and include, for example, SRCC724 (adenocarcinoma, abbreviated as "AdenoCa") (LT1), SRCC725 (squamous cell carcinoma, abbreviated as "SqCCa) (LT1a), SRCC726 (adenocarcinoma) (LT2), SRCC727 (adenocarcinoma) (LT3), SRCC728 (adenocarcinoma) (LT4), SRCC729 (squamous cell carcinoma) (LT6), SRCC730 (adeno/squamous cell carcinoma) (LT7), SRCC731 (adenocarcinoma) (LT9), SRCC732 (squamous cell carcinoma) (LT10), SRCC733 (squamous cell carcinoma) (LT11), SRCC734 (adenocarcinoma) (LT12), SRCC735 (adeno/squamous cell carcinoma) (LT13), SRCC736 (squamous cell carcinoma) (LT15), SRCC737 (squamous cell carcinoma) (LT16), SRCC738 (squamous cell carcinoma) (LT17), SRCC739 (squamous cell carcinoma) (LT18), SRCC740 (squamous cell carcinoma) (LT19), SRCC741 (lung cell carcinoma, abbreviated as "LCCa") (LT21), SRCC811 (adenocarcinoma)(LT22), SRCC825(adenocarcinoma) (LT8), SRCC886 (adenocarcinoma) (LT25), SRCC887 (squamous cell carcinoma) (LT26), SRCC888 (adeno-BAC carcinoma) (LT27), SRCC889 (squamous cell carcinoma) (LT28), SRCC890 (squamous cell carcinoma) (LT29), SRCC891 (adenocarcinoma) (LT30), SRCC892 (squamous cell carcinoma) (LT31), SRCC894 (adenocarcinoma) (LT33). Also included are human lung tumors designated SRCC1125 [HF-000631], SRCC1127 [HF-000641], SRCC1129 [HF-000643], SRCC1133 [HF-000840], SRCC1135 [HF-000842], SRCC1227 [HF-001291], SRCC1229 [HF-001293], SRCC1230 [HF-001294], SRCC1231 [HF-001295], SRCC1232 [HF-001296], SRCC1233 [HF-001297], SRCC1235 [HF-001299], and SRCC1236 [HF-001300].

Colon cancer cell lines include, for example, ATCC cell lines SW480 (adenocarcinoma, SRCC776), SW620 (lymph node metastasis of colon adenocarcinoma, SRCC777), Colo320 (carcinoma, SRCC778), HT29 (adenocarcinoma, SRCC779), HM7 (a high mucin producing variant of ATCC colon adenocarcinoma cell line, SRCC780, obtained from Dr. Robert Warren, UCSF), CaWiDr (adenocarcinoma, SRCC781), HCT116 (carcinoma, SRCC782), SKC01 (adenocarcinoma, SRCC783), SW403 (adenocarcinoma, SRCC784), LS174T (carcinoma, SRCC785), Colo205 (carcinoma, SRCC828), HCT15 (carcinoma, SRCC829), HCC2998 (carcinoma, SRCC830), and KM12 (carcinoma, SRCC831). Primary colon tumors include colon adenocarcinomas designated CT2 (SRCC742), CT3 (SRCC743), CT8 (SRCC744), CT10 (SRCC745), CT12 (SRCC746), CT14 (SRCC747), CT15 (SRCC748), CT16 (SRCC749), CT17 (SRCC750), CT1 (SRCC751), CT4 (SRCC752), CT5 (SRCC753), CT6 (SRCC754), CT7 (SRCC755), CT9 (SRCC756), CT11 (SRCC757), CT18 (SRCC758), CT19 (adenocarcinoma, SRCC906), CT20 (adenocarcinoma, SRCC907), CT21 (adenocarcinoma, SRCC908), CT22 (adenocarcinoma, SRCC909), CT23 (adenocarcinoma, SRCC910), CT24 (adenocarcinoma, SRCC911), CT25 (adenocarcinoma, SRCC912), CT26 (adenocarcinoma, SRCC913), CT27 (adenocarcinoma, SRCC914),CT28 (adenocarcinoma, SRCC915), CT29 (adenocarcinoma, SRCC918), CT30 (adenocarcinoma, SRCC917), CT31 (adenocarcinoma, SRCC918), CT32(adenocarcinoma, SRCC919), CT33 (adenocarcinoma, SRCC920), CT35 (adenocarcinoma, SRCC921), and CT36 (adenocarcinoma, SRCC922). Also included are human colon tumors designated SRCC1051 [HF-000499], SRCC1052 [HF-000539], SRCC1053 [HF-000575], SHCC1054 [HF-000698], SRCC1142 [HF-000782], SRCC1144 [HF-000769], SRCC1148 [HF-000795] and SRCC1148[HF-000811].

Human breast carcinoma cell lines include, for example, HBL100 (SRCC759), MB435s (SRCC780), T47D (SRCC761), MB468 (SRCC762), MB175 (SRCC763), MB361 (SRCC764), BT20 (SRCC785), MCP7 (SRCC766), and SKBR3 (SRCC767), and human breast tumor center designated SRCC1057 [HF-000545]. Also included are human breast tumors designated SRCC1094, SRCC1095, SRCC1098, SRCC1097, SRCC1098, SRCC1099, SRCC1100, SRCC1101, and human breast-met-lung-NS tumor designated SRCC893 [LT 32].

Human kidney tumor centers include SRCC989 [HF-000611] and SRCC1014 [HF-000613].

Human testis tumor center includes SRCC1001 [HF-000733] and testis tumor margin SRCC989 [HF-000716].

Human parathyroid tumor includes SRCC1002 [HF-000831] and SRCC1003 [HF-000832].

Methods for mRNA extraction are well known in the art end are disclosed in standard textbooks of molecular biology, including Ausubel et al., Current Protocols of Molecular Biology, John Wiley and Sons (1997). Methods for RNA extraction from paraffin embedded tissues are disclosed, for example, in Rupp and Locker, Lab Invest. 56:A67 (1987), and De Andrés et al., BioTechniques 18:42044 (1995). In particular, RNA isolation can be performed using purification kit, buffer set and protease from commercial manufacturers, such as Qiagen, according to the manufacturer's instructions. For example, total RNA from cells in culture can be isolated using Qiagen RNeasy mini-columns. Total RNA from tissue samples can be isolated using RNA Stat-60 (Tel-Test). RNA prepared from tumor can be isolated, for example, by cesium chloride density gradient centrifugation.

The present application describes methods for identifying genes, preferably cell surface antigens, the expression of which is selectively enhanced by retinoid treatment in tumor calls driven by Wnt signalling. Accordingly, a preferred source of the mRNA is a tissue sample or cell line derived from a tumor the development and/or progression of which is associated with defects in the Wnt signaling pathway. As discussed above, for example, activating mutations in β-catenin have been identified in cancers of the overy and endometrium, Wilm's kidney tumors and melanomas, demonstrating that defects in Wnt-1 signaling contribute to the progression of these cancers (Kobayashi et al., Jpn J Cancer Res 90:55-9 (1999); Koesters et al., Cancer Res 59:3880-2 (1999); Palacios and Hamallo, Cancer Res 58:1344-7 (1998); Rimm et al. Am J Pathol 154:325-9 (1999); Rubinfeld et al., Science 262:1731-1734 (1993); Wright et al., Int J Cancer 82:825-9 (1989)). Similarly, certain colorectal cancers are characterized by defective Wnt-1 signaling. Accordingly, frozen or paraffin-embedded samples or tumor cell lines from such tumors are a preferred source of mRNA for the expression profiling assays of the present invention. Alternatively, tumor and normal cells engineered to conditionally express a Wnt (e.g. Wnt-1) proto-oncogene can be a source of mRNA, before and after retinoid treatment, and in the presence of absence of Wnt (e.g. Wnt-1) expression.

As RNA cannot serve as a template for PCR, the first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo.dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

TaqMan RT-PCR can be performed using commercially available equipments, such as, for example, ABI PRIZM 7700™ Sequence Detection System™ (Perkin-Elmer-Applied Biosystems, Foster City, CA, USA), or Lightcycler (Roche Molecular Biochemicals, Mannheim, Germany). In a preferred embodiment, the 5' nuclease procedure is run on a real-time quantitative PCR device such as the ABI PRIZM 7700™ Sequence Detection System™. The system consists of a thermocycler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle ($C_t$). The ΔCt values are used as quantitative measurement of the relative number of starting copies of a particular target sequence in a nucleic acid sample when comparing the expression of RNA in a cancer cell with that from a normal cell.

To minimize errors and the effect of sample-to-sample variation, RT-PCR is usually performed using an internal standard. The ideal internal standard is expressed at a constant level among different tissues, and is unaffected by the experimental treatment. RNAs most frequently used to normalize patterns of gene expression are mRNAs for the housekeeping genes glyceraldehyde-3-phosphate-dehydrogenase (GAPDH) and β-actin.

*Microarrays*

Differential gene expression can also be identified, or confirmed using the microarray technique. In this method, nucleotide sequences of interest are plated, or arrayed, on a microchip substrate. The arrayed sequences are then hybridized with specific DNA probes from cells or tissues of interest. The genes targeted by the present invention can be identified by constructing normalized and subtracted cDNA libraries from mRNA extracted from tumor cells or tissues, and the cells or tissue of healthy subjects; purifying the DNA from the cDNA; treating both tumor and healthy samples under otherwise identical conditions with a retinoid; microarraying the purified DNA for expression analysis; and probing microarrays to identify the genes from the clones that are selectively upregulated by retinoid treatment in the tumor cells or tissues, relative to corresponding normal cells or tissues. Just as in the RT-PCR method, the source of mRNA typically is total RNA isolated from human tumors or tumor cell lines, and corresponding normal tissues or cell lines. Thus RNA can be isolated from a variety of primary tumors or tumor cell lines, including those listed above. If the source of mRNA is a primary tumor, mRNA can be extracted, for example, from frozen or archived paraffin-embedded and fixed (e.g. formalin-fixed) tissue samples, which are routinely prepared and preserved in everyday clinical practice.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes may be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy. Quantitation of hybridization of each arrayed element allows for assessment of corresponding mRNA abundance. With dual color fluorescence, separately labeled cDNA probes generated from two sources of RNA are hybridized pairwise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad Sci. USA 93(20): 106-49 (1996)).

The methodology of hybridization of nucleic acids and microarray technology is well known in the art. In one example, the specific preparation of nucleic acids for hybridization and probes, slides, and hydridization conditions are all detailed in PCT Application Serial No. PCT/US01/10482, filed on March 30, 2001.

b. *Retinoid Treatment*

Regardless of the method chosen for gene expression profiling, and to quantify mRNA transcription, the crux underlying the present invention is the identification of genes the expression of which is selectively enhanced by retinoid treatment in cancer cells driven by Wnt signaling, relative to normal cells. In particular, the gene expression profile of tumor cells characterized by the involvement of the Wnt signaling pathway is determined in the presence and absence of Wnt, e.g. Wnt-1 expression, and in the presence and absence of retinoid treatment. After quantitation of the gene expression data, genes are identified, the expression of which is selectively upregulated by retinoid treatment of Wnt-expressing tumor cells, relative to normal cells treated with the same retinoid, and, preferably, also relative to tumor cells not expressing Wnt, with or without retinoid treatment. As discussed before, the retinoid may be a retinoic acid (also known as tretinoin, vitamin A acid or vitamin $A_1$), including both all-*trans*-retinoic acid (all-*trans*-RA) and 9-*cis*-retinoic acid (9-cis-RA), and retinoic acid derivatives consisting of four isoprenoid units joined in a head-to-tail manner, such as retinol, retinal, substituted retinoids, seco-, nor-, and retro-retinoids. All retinoids may be formally derived from a monocyclic parent compound containing five carbon-carbon double bonds and a functional group at the terminus of the acyclic portion.

C. *Cell-Based Tumor Assays*

The gene targets identified and the treatment methods herein can be further validated in cell-based tumor assays. The role of genes and gene products identified herein in the development and pathology of tumor or cancer can be tested by using primary tumor cells or cell lines that have been identified to amplify the genes herein. Such cells include, for example, the breast, colon and lung cancer cells and cell lines listed above.

In a different approach, cells of a cell type known to be involved in a particular tumor are transfected with the cDNAs encoding the cell surface proteins identified herein, and the ability of these cDNAs to induce excessive growth is analyzed. Suitable cells include, for example, stable tumor cells lines such as, the B104-1-1 cell line (stable NIH-3T3 cell line transfected with the *neu* protooncogene) and *res*-transfected NIH-3T3 cells, which can be transfected with the desired gene, and monitored for tumorigenic growth. Such transfected cell lines can than be used to test the ability of anti-tumor agents, such as poly- or monoclonal antibodies or antibody compositions in combination with retinoids to inhibit tumorigenic cell growth by exerting cytostatic or cytotoxic activity on the growth of the transformed calls, or by mediating antibody-dependent cellular cytotoxicity (ADCC), in the presence of absence of retinoid treatment. Cells transfected with the coding sequences of the genes identified herein can further be used to identify drug candidates for the treatment of cancer.

In addition, primary cultures derived from tumors in transgenic animals (as described below) can be used in the cell-based assays herein, although stable cell lines are preferred. Techniques to derive continuous cell lines from transgenic animals are well known in the art (*see, e.g.,* Small et al., Mol. Cell. Biol., 5:642-648 (1985)).

d. *Animal Models*

A variety of well known animal models can be used to further understand the role of the genes identified herein in the development and pathogenesis of tumors, and to test the efficacy of retinoid treatment in combination with anti-cancer, e.g. antibody therapy. The *in vivo* nature of such models makes them particularly predictive of responses in human patients. Animal models of tumors and cancers (*e.g.*, breast cancer, colon cancer, prostate cancer, lung cancer, etc.) include both non-recombinant and recombinant (transgenic) animals. Non-recombinant animal models include, for example, rodent, *e.g.*, murine models. Such models can be generated by introducing tumor cells into syngeneic mice using standard techniques, *e.g.*, subcutaneous injection, tail vein injection, spleen implantation, intraperitoneal implantation, implantation under the renal capsule, or orthopin implantation, *e.g.*, colon cancer cells implanted in colonic tissue. *(See, e.g.,* PCT publication No. W0 97133551, published September 18, 1997).

Probably the most often used animal species in oncological studies are immunodeficient mice and, in particular, nude mice. The observation that the nude mouse with hypoaplasia could successfully act as a host for human tumor xenografts has lead to its widespread use for this purpose. The autosomal recessive *nu* gene has been introduced into a very large number of distinct congenic strains of nude mouse, including, for example, ASW, A/He, AKR, BALB/c, B10.LP, C17, C3H, C57BL, C57, CBA, DBA, ODD, I/st, NC, NFR, NFS, NFS/N, NZB, NZC, NZW, P, RIII and SJL. In addition, a wide variety of other animals with inherited immunological defects other than the nude mouse have been bred and used as recipients of tumor xenografts. For further details *see, e.g.,* The Nude Mouse in Oncology Research, E. Boven and B. Winograd, eds., CRC Press, Inc., 1991.

The cells introduced into such animals can be derived from known tumor/cancer cell lines, such as, for example, the Wnt-1 transfected murine C57MG breast epithelial cell line used in the examples herein, stably expressing Wnt-1 (see also Diatchenko et al., Proc. Natl. Acad. Sci. USA 93:6025-6030 (1996)), or from the following ATCC human cell lines, after transfection with Wnt-1: SW40, COL0320DM, HT-29, WiDr, and SW403 (colon adenocarcinomas), SW620 (lympho node metastasis, colon adenocarcinoma), HT 116 (colon carcinoma), SK-Co-1 (colon adenocarcinoma, ascites), and HM7 (a variant of ATCC colon adenocarcinoma cell line LS 174T). Samples of tumor or cancer cells can also be obtained from patients undergoing surgery, using standard conditions, involving freezing and storing in liquid nitrogen (Karmali et al., Br. J. Cancer, 48:689-696 (1983)), or from paraffin-embedded, formalin-fixed samples.

Tumor cells can be introduced into animals, such as nude mice, by a variety of procedures. The subcutaneous (s.c.) space in mice is very suitable for tumor implantation. Tumors can be transplanted s.c. as solid blocks, as needle biopsies by use of a trochar, or as cell suspensions. For solid block or trochar implantation, tumor tissue fragments of suitable size are introduced into the s.c. space. Cell suspensions are freshly prepared from primary tumors or stable tumor cell lines, and injected subcutaneously. Tumor cells can also be injected as subdermal implants. In this location, the inoculum is deposited between the lower part of the dermal connective tissue and the s.c. tissue. Boven and Winograd (1991), *supra.*

Animal models of breast cancer can be generated, for example, by implanting rat neuroblastoma cells (from which the *neu* oncogene was initially isolated), or *neu*-transformed NIH-3T3 cells into nude mice, essentially as described by Drebin et al., PNAS USA, 83:9129-9133 (1986).

Similarly, animal models of colon cancer can be generated by passaging colon cancer cells in animals, eg.,

nude mice, leading to the appearance of tumors in these animals. An orthotopic transplant model of human colon cancer in nude mice has been described, for example, by Wang et al., Cancer Research, 54:4726-4728 (1994) and Too et al., Cancer Research, 55:681-684 (1995). This model is based on the so-called "METAMOUSE" sold by AntiCancer, Inc., (San Diego, California).

Tumors that arise in animals can be removed and cultured *in vitro.* Cells from the *in vitro* cultures can then be passaged to animals. Such tumors can serve as targets for further testing or drug screening. Alternatively, the tumors resulting from the passage can be isolated and RNA from pre-passage cells and cells isolated after one or more rounds of passage analyzed for differential expression of genes of interest. Such passaging techniques can be performed with any known tumor or cancer cell lines.

For example, Meth A, CMS4, CMS5, CMS21, and WEHI-164 are chemically induced fibrosarcomas of BALB/c female mice (DeLeo et al., J. Exp. Med., 146:720 (1977)), which provide a highly controllable model system for studying the anti-tumor activities of various agents (Palladino et al., J. Immunol., 138:4023-4032 (1987)). Briefly, tumor cells are propagated *in vitro* in cell culture. Prior to injection into the animals, the cell lines are washed and suspended in buffer, at a cell density of about $10x10^8$ to $10x10^7$ cells/ml. The animals are then infected subcutaneously with 10 to 100 μl of the cell suspension, allowing one to three weeks for a tumor to appear.

In addition, the Lewis lung (3LL) carcinoma of mice, which is one of the most thoroughly studied experimental tumors, can be used as an investigational tumor model. Efficacy in this tumor model has been correlated with beneficial effects in the treatment of human patients diagnosed with small cell carcinoma of the lung (SCCL). This tumor can be introduced in normal mice upon injection of tumor fragments from an affected mouse or of cells maintained in culture (Zupi et al., Br. J. Cancer, 41:suppl. 4:309 (1980)), and evidence indicates that tumors can be started from injection of even a single cell and that a very high proportion of infected tumor cells survive. For further information about this tumor model *see,* Zacharski, Haemostasis, 16:300-320 (1986)).

One way of evaluating the efficacy of a test compound in an animal model on an implanted tumor is to measure the size of the tumor before and after treatment. Traditionally, the size of implanted tumors has been measured with a slide caliper in two or three dimensions. The measure limited to two dimensions does not accurately reflect the size of the tumor, therefore, it is usually converted into the corresponding volume by using a mathematical formula. However, the measurement of tumor size is very inaccurate. The therapeutic effects of a drug candidate can be better described as treatment-induced growth delay and specific growth delay. Another important variable in the description of tumor growth is the tumor volume doubling time. Computer programs for the calculation and description of tumor growth are also available, such as the program reported by Rygaard and Spang-Thomsen, Proc. 6th Int. Workshop on Immune-Deficient Animals, Wu and Sheng eds., Basel, 1989, 301. It is noted, however, that necrosis and inflammatory responses following treatment may actually result in an increase in tumor size, at least initially. Therefore, these changes need to be carefully monitored, by a combination of a morphometric method and flow cytometric analysis.

Recombinant (transgenic) animal models can be engineered by introducing the coding portion of the genes identified herein into the genome of animals of interest, using standard techniques for producing transgenic animals. Animals that can serve as a target for transgenic manipulation include, without limitation, mice, rats, rabbits, guinea pigs, sheep, goats, pigs, and non-human primates, *e.g.*, baboons, chimpanzees and monkeys. Techniques known in the art to introduce a transgene into such animals include pronucleic microinjection (Hoppe and Wanger, U.S. Patent No. 4,873,191); retrovirus-mediated gene transfer into germ lines (*e.g.*, Van der Putten et al., Proc. Natl. Acad. Sci. USA, 82:6148-615 (1985)); gene targeting in embryonic stem cells (Thompson et al., Cell, 56:313-321 (1989)); electroporation of embryos (Lo, Mol. Cell Biol., 3:1803-1814 (1983)); sperm-mediated gene transfer (Lavitrano et al., Cell, 57:717-73 (1989)). For review, *see,* for example, U.S. Patent No. 4,736,866.

For the purpose of the present invention, transgenic animals include those that carry the transgene only in part of their cells ("mosaic animals"). The transgene can be integrated either as a single transgene, or in concatamers, *e.g.*, head-to-head or head-to-tail tandems. Selective introduction of a transgene into a particular cell type is also possible by following, for example, the technique of Lasko et al., Proc. Natl. Acad. Sci. USA, 89:6232-636 (1992).

The expression of the transgene in transgenic animals can be monitored by standard techniques. For example, Southern blot analysis or PCR amplification can be used to verify the integration of the transgene. The level of mRNA expression can then be analyzed using techniques such as *in situ* hybridization, Northern blot analysis, PCR, or immunocytochemistry. The animals are further examined for signs of tumor or cancer development.

Alternatively, "knock out" animals can be constructed which have a defective or altered gene encoding a protein (e.g. a cell surface protein) identified herein, as a result of homologous recombination between the endogenous gene encoding the protein and altered genomic DNA encoding the same protein introduced into an embryonic cell of the animal. For example, cDNA encoding such protein can be used to clone genomic DNA encoding the protein in accordance with established techniques. A portion of the genomic DNA encoding a particular protein can be deleted or replaced with another gene, such as a gene encoding a selectable marker which can be used to monitor integration. Typically, several kilobases of unaltered flanking DNA (both at the 5' and 3' ends) are included in the vector [*see, e.g.,* Thomas and Capecchi, Cell, 51:503 (1987) for a description of homologous recombination vectors]. The vector is introduced into an embryonic stem cell line (*e.g.*, by electroporation) and cells in which the introduced DNA has homologously recombined with the endogenous DNA are selected (*see, e.g.,* Li et al., Cell, 69:915 (1992)). The selected cells are then injected into a blastocyst of an animal *(e.g.,* a mouse or rat) to form aggregation chimeras [see, *e.g.*, Bradley, in Teratocarcinomas and Embryonic Stem Cells: A Practical Approach, E. J. Robertson, ed. (IRL, Oxford, 1987), pp. 113-152]. A chimeric embryo can then be implanted into a suitable pseudopregnant female foster animal and the embryo brought to term to create a "knock out" animal. Progeny harboring the homologously recombined DNA in their germ cells can be identified by standard techniques and used to breed animals in which all cells of the animal contain the homologously recombined DNA. Knockout animals can be characterized for instance, by their ability to defend against certain pathological conditions and by their development of pathological conditions due to absence of the absent protein.

The efficacy of antibodies specifically binding the proteins identified in accordance with the present invention and other drug candidates, can be tested also in the treatment of spontaneous animal tumors. A suitable target for such studies is the feline oral squamous cell carcinoma (SCC). Feline oral SCC is a highly invasive, malignant tumor that is the most common oral malignancy of cats, accounting for over 60% of the oral tumors reported in this species. It rarely metastasizes to distant sites, although this low incidence of metastasis may merely be a reflection of the short survival times for cats with this tumor. These tumors are usually not amenable to surgery, primarily because of the anatomy of the feline oral cavity. At present, there is no effective treatment for this tumor. Prior to entry into the study, each cat undergoes complete clinical examination, biopsy, and is scanned by computed tomography (CT). Cats diagnosed with sublingual oral squamous cell tumors are excluded from the study. The tongue can become paralyzed as a result of such tumor, and even if the treatment kills the tumor, the animals may not be able to feed themselves. Each cat is treated repeatedly, over a longer period of time. Photographs of the tumors will be taken daily during the treatment period, and at each subsequent recheck. After treatment, each cat undergoes another CT scan. CT scans and thoracic radiograms are evaluated every 8 weeks thereafter. The data are evaluated for differences in survival, response and toxicity as compared to control groups. Positive response may require evidence of tumor regression, preferably with improvement of quality of life and/or increased life span.

In addition, other spontaneous animal tumors, such as fibrosarcoma, adenocarcinoma, lymphoma, chrondroma, leiomyosarcoma of dogs, cats, and baboons can also be tested. Of these mammary adenocarcinoma in dogs and cats is a preferred model as its appearance and behavior are very similar to those in humans. However, the use of this model is limited by the rare occurrence of this type of tumor in animals.

2. Agents Targeting the Tumor Antigens Identified

The tumor antigens identified herein are desirable targets for cancer therapy, since selective enhancement of their expression by retinoid treatment relative to normal cells is expected to improve the efficacy and therapeutic index of cancer therapeutics directed against these antigens. This is generally true for any antagonist of a tumor antigen identified in accordance with the present invention.

*a. Antagonists*

Antagonists include oligonucleotides that bind to a tumor antigen, and, in particular, antibodies including, without limitation, poly- and monoclonal antibodies and antibody fragments, single-chain antibodies, anti-idiotypic antibodies, and chimeric or humanized versions of such antibodies or fragments, as well as human antibodies and antibody fragments. Alternatively, a potential antagonist may be a closely related protein, for example, a mutated form of the tumor antigen that recognizes its receptor but imparts no effect (e.g. a stra6, ephrin b1, 4.1BB ligand, autotaxin or ISRL variant), thereby competitively inhibiting the action of the tumor antigen.

Other antagonist may be an antisense RNA or DNA construct prepared using antisense technology, where, *e.g.,* an antisense RNA or DNA molecule acts to block directly the translation of mRNA by hybridizing to targeted mRNA and preventing protein translation. Antisense technology can be used to control gene expression through triple-helix formation or antisense DNA or RNA, both of which methods are based on binding of a polynucleotide to DNA or RNA. For example, the 5' coding portion of the polynucleotide sequence, which encodes a mature tumor antigen herein, is used to design an antisense RNA oligonucleotide of from about 10 to 40 base pairs in length. A DNA oligonucleotide is designed to be complementary to a region of the gene involved in transcription (triple helix - *see,* Lee et al., Nucl. Acids Res., 6:3073 (1979); Cooney et al., Science, 241: 456 (1988); Dervan et al., Science, 251:1360 (1991)), thereby preventing transcription and the production of the target polypeptide. The antisense RNA oligonucleotide hybridizes to the mRNA *in vivo* and blocks translation of the mRNA molecule into the target polypeptide (antisense - Okano, Neurochem., 56:560 (1991); Oligodeoxynucleotides as Antisense Inhibitors of Gene Expression (CRC Press: Boca Raton, FL, 1988). The oligonucleotides described above can also be delivered to cells such that the antisense RNA or DNA may be expressed *in vivo* to inhibit production of the target polypeptide. When antisense DNA is used, oligodeoxyribonucleotides derived from the translation-initiation site, *e.g.*, between about -10 and +10 positions of the target gene nucleotide sequence, are preferred.

Antisense RNA or DNA molecules are generally at least about 5 bases in length, about 10 bases in length, about 15 bases in length, about 20 bases in length, about 25 bases in length, about 30 bases in length, about 35 bases in length, about 40 bases in length, about 45 bases in length, about 50 bases in length, about 55 bases in length, about 60 bases in length, about 65 bases in length, about 70 bases in length, about 75 bases in length, about 80 bases in length, about 85 bases in length, about 90 bases in length, about 95 bases in length, about 100 bases in length, or more.

Potential antagonists include small molecules that bind to the active site, the receptor binding site, or growth factor or other relevant binding site of a tumor antigen identified herein, thereby blocking its normal biological activity. Examples of small molecules include, but are not limited to, small peptides or peptide-like molecules, preferably soluble peptides, and synthetic non-peptidyl organic or inorganic compounds. These small molecules can be identified by screening techniques well known for those skilled in the art.

Ribozymes are enzymatic RNA molecules capable of catalyzing the specific cleavage of RNA. Ribozymes act by sequence-specific hybridization to the complementary target RNA, followed by endonucleolytic cleavage. Specific ribozyme cleavage sites within a potential RNA target can be identified by known techniques. For further details *see, e.g.,* Rossi, Current Biology, 4:469-471 (1994), and PCT publication No. WO 97/33551 (published September 18, 1997).

Nucleic add molecules in triple-helix formation used to inhibit transcription should be single-stranded and composed of deoxynucleotides. The base composition of these oligonucleotides is designed such that it promotes triple-helix formation via Hoogsteen base-pairing rules, which generally require sizeable stretches of purines or pyrimidines on one strand of a duplex. For further details *see, e.g.,* PCT publication No. WO 97/33551, *supra,*

In a preferred embodiment, the antagonists for use in the treatments methods of the present invention are antibodies that are capable of specific binding a tumor antigen identified in accordance with the present invention. Retinoid (e.g. retinoic acid) treatment will selectively upregulate the tumor antigen identified, and improve the efficacy and therapeutic index of antibodies directed against such tumor antigens,

*b. Antibodies*

Methods of preparing polyclonal antibodies are known to the skilled artisan. Polyclonal antibodies can be raised in a mammal, for example, by one or more injections of an immunizing agent and, if desired, an adjuvant. Typically, the immunizing agent and/or adjuvant will be injected in the mammal by multiple subcutaneous or intraperitoneal injections. The immunizing agent may include the polypeptide identified in accordance with the present invention or a fusion protein thereof. It may be useful to conjugate the immunizing agent to a protein known to be immunogenic in the mammal being immunized. Examples of such immunogenic proteins include but are not limited to keyhole limpet hemocyanin, serum albumin, bovine thyroglobulin, and soybean trypsin inhibitor. Examples of adjuvants which may be employed include Freund's complete adjuvant and MPL-TDM adjuvant (monophosphoryl Lipid A, synthetic trehalose dicorynomycolate). The immunization protocol may be selected by one skilled in the art without undue experimentation.

*Monoclonal Antibodies*

The antibodies may, alternatively, be monoclonal antibodies. Monoclonal antibodies may be prepared using hybridoma methods, such as those described by Kohler and Milstein, Nature, 256:495(1975). In a hybridoma method, a mouse, hamster, or other appropriate host animal, is typically immunized with an immunizing agent to elicit lymphocytes that produce or are capable of producing antibodies that will specifically bind to the immunizing agent. Alternatively, the lymphocytes may be immunized *in vitro.*

The immunizing agent will typically include the polypeptide to which the antibody binds or a fusion protein thereof. Generally, either peripheral blood lymphocytes ("PBLs") are used if cells of human origin are desired, or spleen cells or lymph node cells are used if non-human mammalian sources are desired. The lymphocytes are then fused with an immortalized cell line using a suitable fusing agent, such as polyethylene glycol, to form a hybridoma cell (Goding, Monoclonal Antibodies: Principles and Practice, Academic Press, (1986) pp. 59-103). Immortalized cell lines are usually transformed mammalian cells, particularly myeloma cells of rodent, bovine and human origin. Usually, rat or mouse myeloma cell lines are employed. The hybridoma cells may be cultured in a suitable culture medium that preferably contains one or more substances that inhibit the growth or survival of the unfused, immortalized cells. For example, if the parental cells lack the enzyme hypoxanthine guanine phosphoribosyl transferase (HGPRT or HPRT), the culture medium for the hybridomas typically will include hypoxanthine, aminopterin, and thymidine ("HAT medium"), which substances prevent the growth of HGPRT-deficient cells.

Preferred immortalized cell lines are those that fuse efficiently, support stable high level expression of antibody by the selected antibody-producing cells, and are sensitive to a medium such as HAT medium. More preferred immortalized cell lines are murine myeloma lines, which can be obtained, for instance, from the Salk Institute Cell Distribution Center, San Diego, California and the American Type Culture Collection, Manassas, Virginia. Human myeloma and mouse-human heteromyeloma cell lines also have been described for the production of human monoclonal antibodies (Kozbor, J. Immunol,, 133:3001 (1984); Brodeur et al., Monoclonal Antibody Production Techniques and Applications Marcel Dekker, Inc., New York, (1987) pp. 51-63).

The culture medium in which the hybridoma cells are cultured can then be assayed for the presence of monoclonal antibodies directed against PRO10282. Preferably, the binding specificity of monoclonal antibodies produced by the hybridoma cells is determined by immunoprecipitation or by an *in vitro* binding assay, such as radioimmunoassay (RIA) or enzyme-linked immunoabsorbent assay (ELISA). Such techniques and assays are known in the art. The binding affinity of the monoclonal antibody can, for example, be determined by the Scatchard analysis of Munson and Pollard, Anal. Biochem., 107:220 (1980).

After the desired hybridoma cells are identified, the clones may be subcloned by limiting dilution procedures and grown by standard methods [Goding, supra]. Suitable culture media for this purpose include, for example, Dulbecco's Modified Eagle's Medium and RPMI-1640 medium. Alternatively, the hybridoma cells may be grown *in vivo* as ascites in a mammal.

The monoclonal antibodies secreted by the subclones may be isolated or purified from the culture medium or ascites fluid by conventional immunoglobulin purification procedures such as, for example, protein A-Sepharose, hydroxylapatite chromatography, gel electrophoresis, dialysis, or affinity chromatography.

The monoclonal antibodies may also be made by recombinant DNA methods, such as those described in U.S. Patent No. 4,816,567. DNA encoding the monoclonal antibodies of the invention can be readily isolated and sequenced using conventional procedures (e.g., by using oligonucleotide probes that are capable of binding specifically to genes encoding the heavy and light chains of murine antibodies). The hybridoma cells of the invention serve as a preferred source of such DNA. Once isolated, the DNA may be placed into expression vectors, which are then transfected into host cells such as simian COS cells, Chinese hamster ovary (CHO) cells, or myeloma cells that do not otherwise produce immunoglobulin protein, to obtain the synthesis of monoclonal antibodies in the recombinant host cells. Prokaryotic hosts, such as *E. coli* are also suitable for the recombinant production of the antibodies herein. The DNA also may be modified, for example, by substituting the coding sequence for human heavy and light chain constant domains in place of the homologous murine sequences (U.S. Patent No. 4,816,567; Morrison et al., *supra)* or by covalently joining to the immunoglobulin coding sequence all or part of the coding sequence for a non-immunoglobulin polypeptide. Such a non-immunoglobulin polypeptide can be substituted for the constant domains of an antibody of the invention, or can be substituted for the variable domains of one antigen-combining site of an antibody of the invention to create a chimeric bivalent antibody.

The antibodies may be monovalent antibodies. Methods for preparing monovalent antibodies are well known in the art. For example, one method involves recombinant expression of immunoglobulin light chain and modified heavy chain. The heavy chain is truncated generally at any point in the Fc region so as to prevent heavy chain crosslinking. Alternatively, the relevant cysteine residues are substituted with another amino acid residue or are deleted so as to prevent crosslinking.

*In vitro* methods are also suitable for preparing monovalent antibodies. Digestion of antibodies to produce fragments thereof, particularly, Fab fragments, can be accomplished using routine techniques known in the art.

*Human and Humanized Antibodies*

The antibodies suitable for use in the treatment methods of the invention may further comprise humanized antibodies or human antibodies. Humanized forms of non-human (e.g., murine) antibodies are chimeric immunoglobulins, immunoglobulin chains or fragments thereof (such as Fv, Fab, Fab', $F(ab')_2$ or other antigen-binding subsequences of antibodies) which contain minimal sequence derived from non-human immunoglobulin. Humanized antibodies include human immunoglobulins (recipient antibody) in which residues from a complementary determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Humanized antibodies may also comprise residues which are found neither in the recipient antibody nor in the imported CDR or framework sequences. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin (Jones et al., Nature, 321:522-525 (1986); Riechmann et al., Nature, 332:323-329 (1988); and Presta, Curr. Op. Struct. Biol., 2:593-596 (1992)).

Methods for humanizing non-human antibodies are well known in the art. Generally, a humanized antibody has one or more amino acid residues introduced into it from a source which is non-human. These non-human amino acid residues are often referred to as "import" residues, which are typically taken from an "import" variable domain.

Humanization can be essentially performed following the method of Winter and co-workers (Jones et al., Nature, 321: 522-525 (1986); Riechmann et al., Nature, 332:323-327 (1988); Verhoeyen et al., Science, 239:1534-1536 (1988)), by substituting rodent CDRs or CDR sequences for the corresponding sequences of a human antibody. Accordingly, such "humanized" antibodies are chimeric antibodies (U.S. Patent No. 4,816,567), wherein substantially less than an intact human variable domain has been substituted by the corresponding sequence from a non-human species. In practice, humanized antibodies are typically human antibodies in which some CDR residues and possibly some FR residues are substituted by residues from analogous sites in rodent antibodies.

Human antibodies can also be produced using various techniques known in the art, including phage display libraries (Hoogenboom and Winter, J. Mol. Biol., 227:381 (1991); Marks et al., J. Mol. Biol., 222:581 (1991)). The techniques of Cole et al. and Boerner et al. are also available for the preparation of human monoclonal antibodies (Cole et al., Monoclonal Antibodies and Cancer Therapy, Alan R. Liss, p. 77 (1985) and Boerner et al., J. Immunol., 147(1): 86-95 (1991)). Similarly, human antibodies can be made by introducing of human immunoglobulin loci into transgenic animals, e.g., mice in which the endogenous immunoglobulin genes have been partially or completely inactivated. Upon challenge, human antibody production is observed, which closely resembles that seen in humans in all respects, including gene rearrangement, assembly, and antibody repertoire. This approach is described, for example, in U.S. Patent Nos. 5,545,807; 5,545,806; 5,569,825; 5,625,126; 5,633,425; 5,661,016, and in the following scientific publications: Marks et al, Bio/Technology 10:779-783 (1992); Lonberg et al., Nature 368:856-859 (1994); Morrison, Nature 368:812-13 (1994); Fishwild et al., Nature Biotechnology 14:845-51 (1996); Neuberger, Nature Biotechnology 14:826 (1996); Lonberg and Huszar, Intern. Rev. Immunol. 13:65-93 (1995).

*Bispecific Antibodies*

Bispecific antibodies are monoclonal, preferably human or humanized, antibodies that have binding specificities for at least two different antigens. In the present case, one of the binding specificities is for the target protein, the other one is for any other antigen, and preferably for a cell-surface protein or receptor or receptor subunit.

Methods for making bispecific antibodies are known in the art. Traditionally, the recombinant production of bispecific antibodies is based on the co-expression of two immunoglobulin heavy-chain/light-chain pairs, where the two heavy chains have different specificities (Milstein and Cuello, Nature, 305:537-539 (1983)). Because of the random assortment of immunoglobulin heavy and light chains, these hybridomas (quadromas) produce a potential mixture of ten different antibody molecules, of which only one has the correct bispecific structure. The purification of the correct molecule is usually accomplished by affinity chromatography steps. Similar procedures are disclosed in WO 93/08829, published 13 May 1993, and in Traunecker et al., EMBO J., 10:3655-3659 (1991).

Antibody variable domains with the desired binding specificities (antibody-antigen combining sites) can be fused to immunoglobulin constant domain sequences. The fusion preferably is with an immunoglobulin heavy-chain constant domain, comprising at least part of the hinge, CH2, and CH3 regions. It is preferred to have the first heavy-chain constant region (CH1) containing the site necessary for light-chain binding present in at least one of the fusions. DNAs encoding the immunoglobulin heavy-chain fusions and, if desired, the immunoglobulin light chain, are inserted into separate expression vectors, and are co-transfected into a suitable host organism. For further details of generating bispecific antibodies see, for example, Suresh et al., Methods in Enzymology, 121:210 (1986).

According to another approach described in WO 96/27011, the interface between a pair of antibody molecules can be engineered to maximize the percentage of heterodimers which are recovered from recombinant cell culture. The preferred interface comprises at least a part of the CH3 region of an antibody constant domain. In this method, one or more small amino acid side chains from the interface of the first antibody molecule are replaced with larger side chains (e.g. tyrosine or tryptophan). Compensatory "cavities" of identical or similar size to the large side chain(s) are created on the interface of the second antibody molecule by replacing large amino acid side chains with smaller ones (e.g. alanine or threonine). This provides a mechanism for increasing the yield of the heterodimer over other unwanted end-products such as homodimers.

Bispecific antibodies can be prepared as full length antibodies or antibody fragments (e.g. $F(ab')_2$ bispecific antibodies). Techniques for generating bispecific antibodies from antibody fragments have been described in the literature. For example, bispecific antibodies can be prepared can be prepared using chemical linkage. Brennan et al., Science 229:81 (1985) describe a procedure wherein intact antibodies are proteolytically cleaved to generate $F(ab')_2$ fragments. These fragments are reduced in the presence of the dithiol complexing agent sodium arsenite to stabilize vicinal dithiols and prevent intermolecular disulfide formation. The Fab' fragments generated are then converted to thionitrobenzoate (TNB) derivatives. One of the Fab'-TNB derivatives is then reconverted to the Fab'-thiol by reduction with mercaptoethylamine and is mixed with an equimolar amount of the other Fab'-TNB derivative to form the bispecific antibody. The bispecific antibodies produced can be used as agents for the selective immobilization of enzymes.

Fab' fragments may be directly recovered from *E. coli* and chemically coupled to form bispecific antibodies. Shalaby et al., J. Exp. Med. 175:217-225 (1992) describe the production of a fully humanized bispecific antibody $F(ab')_2$

molecule. Each Fab' fragment was separately secreted from *E. coli* and subjected to directed chemical coupling *in vitro* to form the bispecific antibody. The bispecific antibody thus formed was able to bind to cells overexpressing the ErbB2 receptor and normal human T cells, as well as trigger the lytic activity of human cytotoxic lymphocytes against human breast tumor targets.

Various technique for making and isolating bispecific antibody fragments directly from recombinant cell culture have also been described. For example, bispecific antibodies have been produced using leucine zippers. Kostelny et al., J. Immunol. 148(5):1547-1553 (1992). The leucine zipper peptides from the Fos and Jun proteins were linked to the Fab' portions of two different antibodies by gene fusion. The antibody homodimers were reduced at the hinge region to form monomers and then re-oxidized to form the antibody heterodimers. This method can also be utilized for the production of antibody homodimers. The "diabody" technology described by Hollinger et al., Proc. Natl. Acad. Sci. USA 90:6444-6448 (1993) has provided an alternative mechanism for making bispecific antibody fragments. The fragments comprise a heavy-chain variable domain ($V_H$) connected to a light-chain variable domain ($V_t$) by a linker which is too short to allow pairing between the two domains on the same chain. Accordingly, the $V_H$ and $V_L$ domains of one fragment are forced to pair with the complementary $V_L$ and $V_H$ domains of another fragment, thereby forming two antigen-binding sites. Another strategy for making bispecific antibody fragments by the use of single-chain Fv (sFv) dimers has also been reported. See, Gruber et al., J. Immunol.152:5368 (1994). Antibodies with more than two valencies are contemplated. For example, trispecific antibodies can be prepared. Tutt et al., J. Immunol 147:60 (1991).

Exemplary bispecific antibodies may bind to two different epitopes on a given polypeptide (e.g. cell surface antigen) identified herein. Alternatively, one arm of the antibody, which binds to target polypeptide, may be combined with an arm which binds to a triggering molecule on a leukocyte such as a T-cell receptor molecule (e.g. CD2, CD3, CD28, or B7), or Fc receptors for IgG (Fc R), such as Fc RI (CD64), Fc RII (CD32) and Fc RIII (CD16) so as to focus cellular defense mechanisms to the cell expressing the particular polypeptide. Bispecific antibodies may also be used to localize cytotoxic agents to cells which express a particular antigen identified in accordance with the present invention. These antibodies possess an antigen-binding arm and an arm which binds a cytotoxic agent or a radionuclide chelator, such as EOTUBE, DPTA, DOTA, or TETA. Another bispecific antibody of interest binds the target polypeptide and further binds tissue factor (TF).

*Heteroconjugate Antibodies*

The use of heteroconjugate antibodies is also within the scope of the present invention. Heteroconjugate antibodies are composed of two covalently joined antibodies. Such antibodies have, for example, been proposed to target immune system cells to unwanted cells (U.S. Patent No. 4,676,980), and for treatment of HIV infection (WO 91/00360; WO 92/200373; EP 03089). It is contemplated that the antibodies may be prepared *in vitro* using known methods in synthetic protein chemistry, including those involving crosslinking agents. For example, immunotoxins may be constructed using a disulfide exchange reaction or by forming a thioether bond. Examples of suitable reagents for this purpose include iminothiolate and methyl-4-mercaptobutyrimidate and those disclosed, for example, in U.S. Patent No. 4,676,980.

*Effector Function Engineering*

It may be desirable to modify the antibody of the invention with respect to effector function, so as to enhance, *e.g.,* the effectiveness of the antibody in treating cancer. For example, cysteine residue(s) may be introduced into the Fc region, thereby allowing interchain disulfide bond formation in this region. The homodimeric antibody thus generated may have improved internalization capability and/or increased complement-mediated cell killing and antibody-dependent cellular cytotoxicity (ADCC). See Caron et al., J. Exp Med., 176: 1191-1195 (1992) and Shopes, J. Immunol., 148: 2918-2922 (1992). Homodimeric antibodies with enhanced anti-tumor activity may also be prepared using heterobifunctional cross-linkers as described in Wolff et al. Cancer Research, 53: 2560-2565 (1993). Alternatively, an antibody can be engineered that has dual Fc regions and may thereby have enhanced complement lysis and ADCC capabilities. See Stevenson et al., Anti-Cancer Drug Design, 3: 219-230 (1989).

*Immunoconjugates*

The invention also includes the use of immunoconjugates comprising an antibody conjugated to a cytotoxic agent such as a chemotherapeutic agent, toxin (*e.g.,* an enzymatically active toxin of bacterial, fungal, plant, or animal origin, or fragments thereof), or a radioactive isotope (*i.e.,* a radioconjugate).

Chemotherapeutic agents useful in the generation of such immunoconjugates have been described above. Enzymatically active toxins and fragments thereof that can be used include diphtheria A chain, nonbinding active fragments of diphtheria toxin, exotoxin A chain (from *Pseudomonas aeruginosa*), ricin A chain, abrin A chain, modeccin A chain, alpha-sarcin, *Aleurites fordii* proteins, dianthin proteins, *Phytolaca americana* proteins (PAPI, PAPII, and PAP-S), momordica charantia inhibitor, curcin, crotin, sapaonaria officinalis inhibitor, gelonin, mitogellin, restrictocin, phenomycin, enomycin, and the tricothecenes. A variety of radionuclides are available for the production of radioconjugated antibodies. Examples include $^{212}Bi$, $^{131}I$, $^{131}In$, $^{90}Y$, and $^{168}Re$.

Conjugates of the antibody and cytotoxic agent are made using a variety of bifunctional protein-coupling agents such as N-succinimidyl-3-(2-pyridyldithiol) propionate (SPDP), iminothiolane (IT), bifunctional derivatives of imidoesters (such as dimethyl adipimidate HCL), active esters (such as disuccinimidyl suberate), aldehydes (such as glutareldehyde), bis-azido compounds (such as bis (p-azidobenzoyl) hexanediamine), bis-diazonium derivatives (such as bis-(p-diazoniumbenzoyl)-ethylenediamine), diisocyanates (such as tolyene 2,6-diisocyanate), and bis-active fluorine compounds (such as 1,5-difluoro-2,4-dinitrobenzene). For example, a ricin immunotoxin can be prepared as described in Vitetta et al., Science, 238: 1098 (1987). Carbon-14-labeled 1-isothiacyanatobenzyl-3-methyldiethylene triaminepentaacetic acid (MX-DTPA) is an exemplary chelating agent for conjugation of radionucleotide to the antibody. See WO94/11026.

In another embodiment, the antibody may be conjugated to a "receptor" (such streptavidin) for utilization in tumor pretargeting wherein the antibody-receptor conjugate is administered to the patient, followed by removal of unbound conjugate from the circulation using a clearing agent and then administration of a "ligand" (*e.g.*, avidin) that is conjugated to a cytotoxic agent *(e.g.,* a radionucleotide).

Preferred immunoconjugates are antibody-maytansinoid conjugates. Immunoconjugates containing maytansinoids and their therapeutic use are disclosed, for example, in U.S. patent Nos. 5,208,020; 5,416,064; and European Patent EP 0 425 235 B1. Liu et al., Proc Natl Acad Sci USA 93:8618-8623 (1996) described immunoconjugates comprising a maytansinoid designated DM1 linked to the monoclonal antibody C242 directed against human colorectal cancer. The conjugate was found to be highly cytotoxic towards cultured colon cancer cells, and showed antitumor activity in an *in vivo* tumor growth assay. Chari et al., Cancer Research 52:127-131 (1992) describe immunoconjugates in which a maytansinoid was conjugated via a disulfide linked to the murine antibody A7 binding to an antigen on human colon cancer cell lines, or to another murine monoclonal antibody TA.1 that binds the HER-2/neu oncogene.

The antibody-maytansinoid conjugates are prepared by chemically linking an antibody to a maytansinoid molecule without significantly diminishing the biological activity of either the antibody or the maytansinoid molecule. An average of 3-4 maytansinoid molecules conjugates per antibody molecule has shown efficacy in enhancing cytotoxicity of target cells without negatively affecting the function or solubility of the antibody, although even one molecule of toxin/antibody would be expected to enhance cytotoxicity over the use of naked antibody. Maytansinoids are well known in the art and can be synthesized by known techniques or isolated from natural sources. Suitable maytansinoids are disclosed, for example, in U.S. Patent Nos 5,208,020; 4,137,230; 4,248,870; 4,256,746; 4,260,608; 4,265,814; 4,294,747; 4,307,016; 4,308,268; 4,308,269; 4,309,428; 4,313,946; 4,315,929; 4,317,821; 4,322,348; 4,331,598; 4,361,650; 4,364,866; 4,424,219; 4,450,254; 4,362,663; and 4,371,533. Preferred maytansinoids are maytansinol and maytansinol analogues modified in the aromatic ring or at other positions of the maytansinol molecule, such as various maytansinol esters.

Another immunoconjugate of particular interest comprises an antibody conjugated to one or more celicheamicin molecules. The alicheamicin family of antibiotics are capable of producing double-stranded DNA breaks at sub-picomolar concentrations. For the preparation of conjugates of the calicheamicin family, see U.S. patent Nos. 5,712,374; 5,714,586; 5,739,116; 5,767,285; 5,770,701; 5,773,001; and 5,877,296. Structural analogues of calicheamicin which may be used include, but are not limited to, $\gamma_1^1$,$\alpha_2^1$,$\alpha^1_3$,N-acetyl-$\gamma_1^1$, PSAG and $\theta^1_1$ (Hinman et al, CancerRes 53:3336-3342 (1993); Lode et al., Cancerres 58:2925-2928 (1998) and the aforementioned U.S. patents.

Another preferred anti-tumor drug that the antibody can be conjugated to is QFA, an antifolate. Both calicheamicin and QFA have intracellular sites of action and do not readily cross the plasma membrane. Therefore, cellular uptake of these agents through antibody mediated internalization greatly enhances their cytotoxic effects.

The upregulation of the target polypeptides, e.g. cell surface antigens, in accordance with the present invention lowers the effective dose of the immunoconjugates and thus eliminates or reduces the toxicity problems often associated with the administration of cytotoxic agents or immunconjugates containing cytotoxic agents.

*Immunoliposomes*

The antibodies used in the treatment methods of the present invention may also be formulated as immunoliposomes. Liposomes containing the antibody are prepared by methods known in the art, such as described in Epstein et al., Proc. Natl. Acad Sci. USA, 82: 3688 (1985); Hwang et al, Proc. Natl Acad. Sci. USA, 77: 4030 (1980); and U.S. Pat. Nos. 4,485,045 and 4,544,545. Liposomes with enhanced circulation time are disclosed in U.S. Patent No. 5,013,556.

Particularly useful liposomes can be generated by the reverse-phase evaporation method with a lipid composition comprising phosphatidylcholine, cholesterol, and PEG-derivatized phosphatidylethanolamine (PEG-PE). Liposomes are extruded through filters of defined pore size to yield liposomes with the desired diameter. Fab' fragments of the antibody of the present invention can be conjugated to the liposomes as described in Martin et al., J. Biol. Chem.,

257: 286-288 (1982) via a disulfide-interchange reaction. A chemotherapeutic agent (such as Doxorubicin) is optionally contained within the liposome. See Gabizon et al., J. National Cancer Inst., 81(19): 1484 (1989).

*Antibody-Dependent Enzyme Mediated Prodrug Therapy (ADEPT)*

The antibodies herein may also be used in ADEPT by conjugating the antibody to a prodrug-activating enzyme which converts a prodrug (*e.g.,* a peptidyl chemotherapeutic agent, *see* WO 81/01145) to an active anti-cancer drug. *See,* for example, WO 88/07378 and U. S. Patent No. 4,975,278.

The enzyme component of the immunoconjugate useful for ADEPT includes any enzyme capable of acting on a prodrug in such as way so as to convert it into its more active, cytotoxic form.

Enzymes that are useful in the method of this invention include, but are not limited to, glycosidase, glucose oxidase, human lysosyme, human glucuronidase, alkaline phosphatase useful for converting phosphate-containing prodrugs into free drugs; arylsulfatase useful for converting sulfate-containing prodrugs into free drugs; cytosine deaminase useful for converting non-toxic 5-fluorocytosine into the anti-cancer drug 5-fluorouracil; proteases, such as serratia protease, thermolysin, subtilisin, carboxypeptidases (*e.g.,* carboxypeptidase G2 and carboxypeptidase A) and cathepsins (such as cathepsins B and L), that are useful for converting peptide-containing prodrugs into free drugs; D-alanylcarboxypeptidases, useful for converting prodrugs that contain D-amino acid substituents; carbohydrate-cleaving enzymes such as -galactosidase and neuraminidase useful for converting glycosylated prodrugs into free drugs; - lactamase useful for converting drugs derivatized with -lactams into free drugs; and penicillin amidases, such as penicillin Vamidase or penicillin G amidase, useful for converting drugs derivatized at their amine nitrogens with phenoxyacetyl or phenylacetyl groups, respectively, into free drugs. Alternatively, antibodies with enzymatic activity, also known in the art as "abzymes" can be used to convert the prodrugs of the invention into free active drugs (see, *e.g.,* Massey, Nature, 328:457-458 (1987)). Antibody-abzyme conjugates can be prepared as described herein for delivery of the abzyme to a tumor cell population.

The enzymes can be covalently bound to the antibodies by techniques well known in the art such as the use of the heterobifunctional cross-linking agents discussed above. Alternatively, fusion proteins comprising at least the antigen binding region of the antibody of the invention linked to at least a functionally active portion of an enzyme of the invention can be constructed using recombinant DNA techniques well known in the art (*see, e.g.,* Neuberger et al., Nature, 312:604-608 (1984)).

3. Pharmaceutical Compositions

The antibodies specifically binding the tumor antigens identified in accordance with the present invention, as well as other molecules targeting such tumor antigens, can be administered for the treatment of various disorders in the form of pharmaceutical compositions.

If the target polypeptide (tumor antigen) is intracellular and whole antibodies are used as inhibitors, internalizing antibodies are preferred. However, lipofections or liposomes can also be used to deliver the antibody, or an antibody fragment, into cells. Where antibody fragments are used, the smallest inhibitory fragment that specifically binds to the binding domain of the target protein is preferred. For example, based upon the variable-region sequences of an antibody, peptide molecules can be designed that retain the ability to bind the target protein sequence. Such peptides can be synthesized chemically and/or produced by recombinant DNA technology. See, *e.g.,* Marasco et al., Proc. Natl. Acad Sci. USA, 90: 7889-7893 (1993). The formulation herein may also contain more than one active compound as necessary for the particular indication being treated, preferably those with complementary activities that do not adversely affect each other. Alternatively, or in addition, the composition may comprise an agent that enhances its function, such as, for example, a cytotoxic agent, cytokine, chemotherapeutic agent, or growth-inhibitory agent. Such molecules are suitably present in combination in amounts that are effective for the purpose intended.

The active ingredients may also be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nano-particles, and nanocapsules) or in macroemulsions. Such techniques are disclosed in Remington's Pharmaceutical Sciences, *supra.*

The formulations to be used for *in vivo* administration must be sterile. This is readily accomplished by filtration through sterile filtration membranes.

Sustained-release preparations may be prepared. Suitable examples of sustained-release preparations include semipermeable matrices of solid hydrophobic polymers containing the antibody, which matrices are in the form of shaped articles, *e.g.*, films, or microcapsules. Examples of sustained-release matrices include polyesters, hydrogels (for example, poly(2-hydroxyethyl-methacrylate), or poly(vinylalcohol)), polylactides (U.S. Pat. No. 3,773,919), copolymers of L-gtutamic acid and ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S-S bond formation through thio-disulfide interchange, stabilization may be achieved by modifying sulfhydryl residues, lyophilizing from acidic solutions, controlling moisture content, using appropriate additives, and developing specific polymer matrix compositions.

4. Treatment Methods

It is contemplated that the antibodies and other anti-tumor compounds for use in the present invention may be used to treat various conditions, including those characterized by overexpression and/or activation of the tumor antigens identified herein. Particularly preferred targets for treatment with antibodies and other antagonists of the present invention are tumors that herbor genetic defects in the Wnt-1 pathway (e.g. leading to abnormal activation of β-catenin signalling) and/or overexpress a tumor antigen identified herein. Thus, for example, it has been found that human cancers harboring genetic defects in the Wnt-1 pathway typically also exhibit overexpression of the tumor antigen Stra6, but not all Stra6 overexpressing tumors have been known to be associated with mutations in the Wnt-1 pathway. The antagonists of the present invention have great potential in the treatment of tumors characterized by aberrant Wnt signaling, especially when the target antigen is characterized by synergistic enhancement of its expression by a combination of Wnt-1 and retinoid treatment. Tumours for treatment herein are tumours of a human cancer selected from colorectal, ovary, endometrium, and Wilm's kidney tumor, breast cancer, prostate cancer, gastric cancer, lung cancer, hepatocellular cancer, melanoma, and pheochromocytome (a tumor derived from the adrenal medullal.

The anti-tumor agents of the present invention, *e.g.,* antibodies, are administered to a mammal, preferably a human, in combination with a retinoid, *e.g.*, retinoic acid, in accord with known methods, such as intravenous administration as a bolus or by continuous infusion over a period of time, by intramuscular, intraperitoneal, intracerobrospinal, subcutaneous, intra-articular, intrasynovial, intrathecal, oral, topical, or inhalation routes. Intravenous administration of the antibody is preferred. Retinoids can be administered by the same of different routes of administration. In Example 15 all-trans-retinol aid (ATRA) has been shown to be effective when administered orally. Based on this experimental finding, and also in view of its convenience, oral administration of the retinoids is preferred. Alternatively or in addition, the retinoids may also be administered by peri-tumoral insection.

Retinoid treatment may precede or follow the treatment with the anti-tumor agent, *e.g.,* antibody, or may occur simultaneously. Since the primary goal of the retinoid treatment is to upregulate the target antigen in order to enhance the efficacy of tumor therapy, the retinoid is preferably administered prior to or simultaneously with treatment with an anti-tumor agent. In the case of concurrent treatment, the anti-tumor agent and the retinoid may be formulated separately, or may be included in the same pharmaceutical composition. The effective amount of a retinoid can be determined by routine testing, and typically is between about 15 mg/kg and about 400 mg/kg, preferably between about 20 kg/kg and about 200 mg/kg, more preferably between about 25 mg/kg and about 150 mg/kg of body weight. In addition, the treatment may involve the administration of a Wnt, e.g. Wnt-1, since the coadministration of a retinoid and Wnt, e.g. Wnt-1 may synergistically induce the genes identified herein. Coadministration includes simultaneous administration, or consecutive administration of the two agents in either order.

Currently, depending on the stage of the cancer, cancer treatment involves one or a combination of the following therapies: surgery to remove the cancerous tissue, radiation therapy, and chemotherapy. The treatment methods of the present invention improve the therapeutic index of anti-tumor agents, such as antibodies, and thereby allow the reduction of the effective dose to be administered. Accordingly, the therapeutic methods herein are especially useful in the treatment of elderly patients and others who do not tolerate well the toxicity and side effects of chemotherapy and in metastatic disease where radiation therapy has limited usefulness.

Other therapeutic regimens may be combined with the administration of the anti-cancer agents, *e.g.,* antibodies and retinoids. For example, the patient to be treated with such anti-cancer agents may also receive radiation therapy and/or may undergo surgery. Alternatively, or in addition, a chemotherapeutic agent may be administered to the patient. Preparation and dosing schedules for such chemotherapeutic agents may be used according to manufacturers' instructions or as determined empirically by the skilled practitioner. Preparation and dosing schedules for such chemotherapy are also described in Chemotherapy Service Ed., M.C. Perry, Williams & Wilkins, Baltimore, MD (1992). The chemotherapeutic agent may precede, or follow administration of the anti-tumor agent, *e.g.,* antibody, or may be given simultarieously therewith. The antibody may be combined with an anti-estrogen compound such as tamoxifen or an anti-progesterone such as onapristone (*see,* EP 616812) in dosages known for such molecules.

It may be desirable to also administer antibodies against other tumor associated antigens, such as antibodies which bind to the ErbB2, EGFR, ErbB3, ErbB4, or vascular endothelial factor (VEGF). Alternatively, or in addition, two or more antibodies binding the same or two or more different antigens disclosed herein may be co-administered to the patient. Sometimes, it may be beneficial to also administer one or more cytokines to the patient. In a preferred embodiment, the antibodies herein are co-administered with a growth inhibitory agent. For example, the growth inhibitory agent may be administered first, followed by an antibody of the present invention. However, simultaneous administration or administration of the antibody of the present invention first is also contemplated. Suitable dosages for the growth inhibitory agent are those presently used and may be lowered due to the combined action (synergy) of the growth inhibitory agent and the antibody herein.

For the prevention or treatment of disease, the appropriate dosage of an anti-tumor agent, *e.g.*, an antibody herein will depend on the type of disease to be treated, as defined above, the severity and course of the disease, whether the agent is administered for preventive or therapeutic purposes, previous therapy, the patient's clinical history and response to the agent, and the discretion of the attending physician. The agent is suitably administered to the patient at one time or over a series of treatments.

For example, depending on the type and severity of the disease, about 1 g/kg to 15 mg/kg (*e.g.,* 0.1-20 mg/kg) of antibody is an initial candidate dosage for administration to the patient, whether, for example, by one or more separate administrations, or by continuous infusion. A typical daily dosage might range from about 1 g/kg to 100 mg/kg or more, depending on the factors mentioned above. For repeated administrations over several days or longer, depending on the condition, the treatment is sustained until a desired suppression of disease symptoms occurs. However, other dosage regimens may be useful. The progress of this therapy is easily monitored by conventional techniques and assays.

6. Articles of Manufacture

In another embodiment of the invention, a pharmaceutical composition comprising the antibodies or other antagonist herein, in combination with a retinoid, is provided. This may be contained in an article of manufacturing comprising a container and a label. Suitable containers include, for example, bottles, vials, syringes, and test tubes. The containers may be formed from a variety of materials such as glass or plastic. The container holds a composition which is effective for treating a condition targeted and may have a sterile access port (for example the container may be an intravenous solution bag or a vial having a stopper pierceable by a hypodermic injection needle). The active agent in the composition is usually an anti-tumor agent capable of interfering with the activity of a gene product identified herein, *e.g.,* an antibody. The label on, or associated with, the container indicates that the composition used for diagnosing or treating the condition of choice. The article of manufacture will further comprise, within the same or a separate container, a retinoid and optionally a pharmaceutically-acceptable buffer, such as phosphate-buffered saline, Ringer's solution and dextrose solution. It may further include other materiels desirable from a commercial and user standpoint, including other buffers, diluents, filters, needles, syringes, and package inserts with instructions for use.

6. Diagnostic Methods

While cell surface proteins, such as growth receptors overexpressed in certain tumors are excellent targets for drug candidates or tumor (*e.g.,* cancer) treatment, the same proteins along with secreted proteins encoded by the genes amplified in tumor cells find additional use in the diagnosis and prognosis of tumors, For example, antibodies directed against the protein products of genes amplified in tumor cells can be used as tumor diagnostics or prognostics.

For example, antibodies, including antibody fragments, can be used to qualitatively or quantitatively detect the expression of proteins encoded by the amplified genes ("marker gene products"). The antibody preferably is equipped with a detectable, *e.g.,* fluorescent label, and binding can be monitored by light microscopy, flow cytometry, fluorimetry, or other techniques known in the art. These techniques ere particularly suitable, if the amplified gene encodes a cell surface protein, *e.g.*, a growth factor. Such binding assays are performed essentially as described hereinabove.

The diagnostic assays of the present invention take advantage of the finding that retinoids, e.g. retinoic acid, selectively upregulate the tumor antigens identified herein. Accordingly, the diagnostic assays are performed in conjunction with retinoid treatment, where the retinoid treatment may precede, occur concurrently with, or follow the administration of the diagnostic agent targeting a tumor antigen, such as a corresponding antibody. This selective upregulation of the tumor antigen by retinoid treatment greatly improves the sensitivity of the diagnostic assay, and may allow the detection of tumor antigens which would otherwise not be detectable This, in turn, allows early detection of the tumor, and early intervention using the most appropriate treatment, including targeting the tumor antigen identified.

*In situ* detection of antibody binding to the marker gene products can be performed, for example, by immunofluorescence or immunoelectron microscopy. For this purpose, a histological specimen is removed from the patient, and a labeled antibody is applied to it, preferably by overlaying the antibody on a biological sample. This procedure also allows for determining the distribution of the marker gene product in the tissue examined. It will be apparent for those skilled in the art that a wide variety of histological methods are readily available for *in situ* defection.

EXAMPLES

Commercially available reagents referred to in the examples were used according to manufacturer's instructions unless otherwise indicated. The source of those cells identified in the following examples, and throughout the specification, by ATCC accession numbers is the American Type Culture Collection, Manassas, VA. In the following Examples, unless otherwise specified, "Stra6" will refer to native sequencePRO10282 polypeptide.

EXAMPLE 1

Isolation of cDNA Clones Encoding a Human PRO10282 and PRO19578 polypeptides

cDNA clones (DNA148380-2827 and DNA148399-2827-1) encoding native human PRO10282 and PRO19578 polypeptides were identified using a yeast screen, in a human fetal brain cDNA library that preferentially represents the 5' ends of the primary cDNA clones.

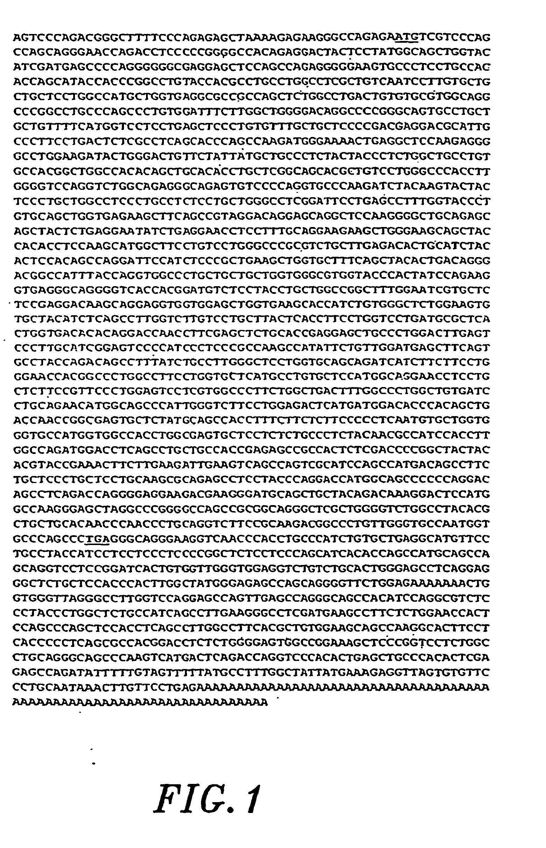

Clone DNA148380-2827 contains a single open reading frame with an apparent translational initiation site at nucleotide positions 49-51 and ending at the stop codon at nucleotide positions 2050-2052 (Figure 1). The predicted polypeptide precursor is 667 amino acids long Figure 2). The full-length PRO10282 protein shown in Figure 2 has an estimated molecular weight of about 73502 daltons and a pl of about 8.26. Analysis of the full-length PRO10282 sequence shown in Figure 2 (SEQ ID NO:2) evidences the presence of a variety of important polypeptide domains as shown in Figure 2, wherein the locations given for those important polypeptide domains are approximate as described above. Clone DNA148380-2827 has been deposited with ATCC on January 11, 2000 and is assigned ATCC Deposit No. PTA-1181.

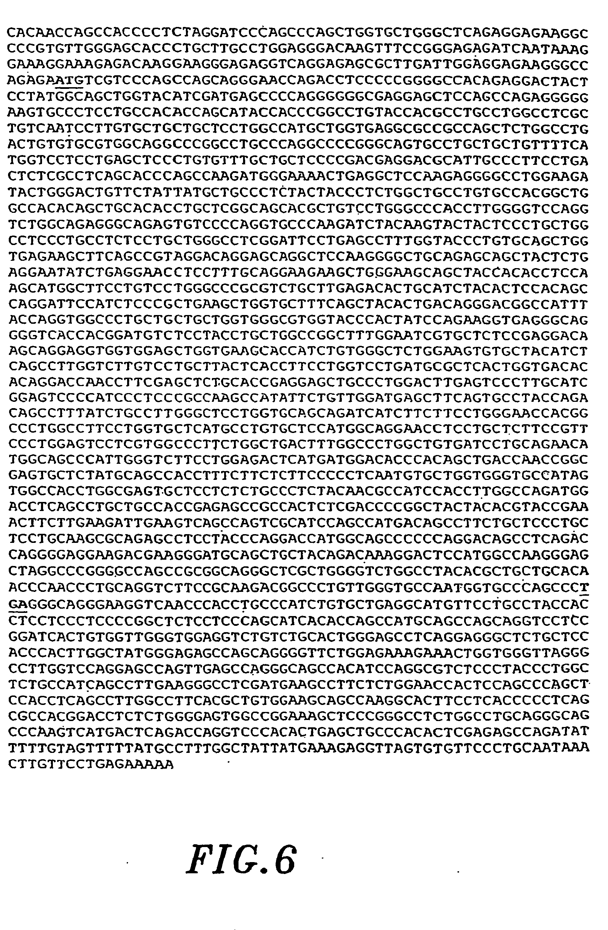

Clone DNA148389-2827-1 contains a single open reading frame with an apparent translational initiation site at nucleotide positions 186-188 and ending at the stop codon at nucleotide positions 2160-2162 (Figure 6, SEQ ID NO: 4). The predicted polypeptide precursor is 658 amino acids long (Figure 7, SEQ ID NO: 5). The full-length PRO19578 protein shown in Figure 7 has an estimated molecular weight of about 72583 daltons and a pl of about 9.36. Analysis of the full-length PRO19578 sequence shown in Figure 7 (SEQ ID NO: 5) evidences the presence of a variety of important polypeptide domains as shown in Figure 7, wherein the locations given for those important polypeptide domains are approximate as described above. Noteworthy is the presence of nine potential transmembrane domains and fourteen cysteine residues conserved between the human and the corresponding mouse sequence. While mouse Stra6 has three potential N-linked glycosylation sites, the human PRO19578 (native human Stra6) polypeptide has one. Clone 148389-2827-1 has been deposited with ATCC on February 23, 2000, and is assigned ATCC Deposit No. PTA-1405.

An analysis of the Dayhoff database (version 35.45 SwissProt 35), using the ALIGN-2 sequence alignment analysis of the full-length sequence shown in Figure 2 (SEQ ID NO:2), and of the full-length sequence shown in Figure 7 (SEQ ID NO: 5) evidenced sequence identity between the PRO10282 amino acid sequence and the following Dayhoff sequences: AF062476, P_W88559 and HGS_RE259.

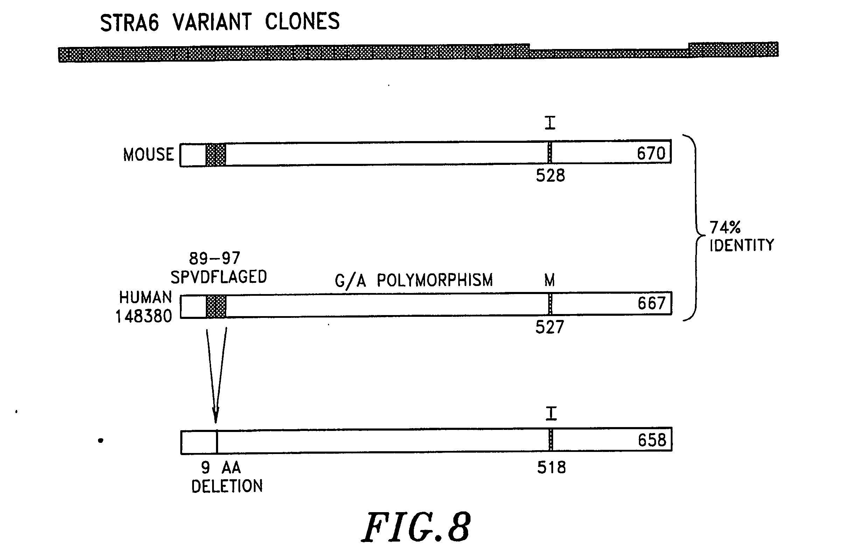

As shown in Figure 8, comparison of the full-length human PRO10282 and PRO19578 polypeptides shows that PRO19578 contains a deletion of nine amino acids (SPVDFLAGD; SEQ ID NO: 13) at positions 89-97 of the PRO10282 amino acid sequence. In addition, PRO19578 contains an isoleucine (I) at amino acid position 518 in place of methionine (M) at the corresponding position (position 527) of PRO10282, which results from a G/A polymorphism at this position. Both the PRO10282 and the native sequence PRO19578 polypeptides are believed to be the human homologues of the mouse Stra6 polypeptide, and are, therefore, also referred to as "Stra6." Mouse Stra6 and the native sequence human full-length PRO10282 polypeptide encoded by DNA148340-2827 show about 74% amino acid sequence identity.

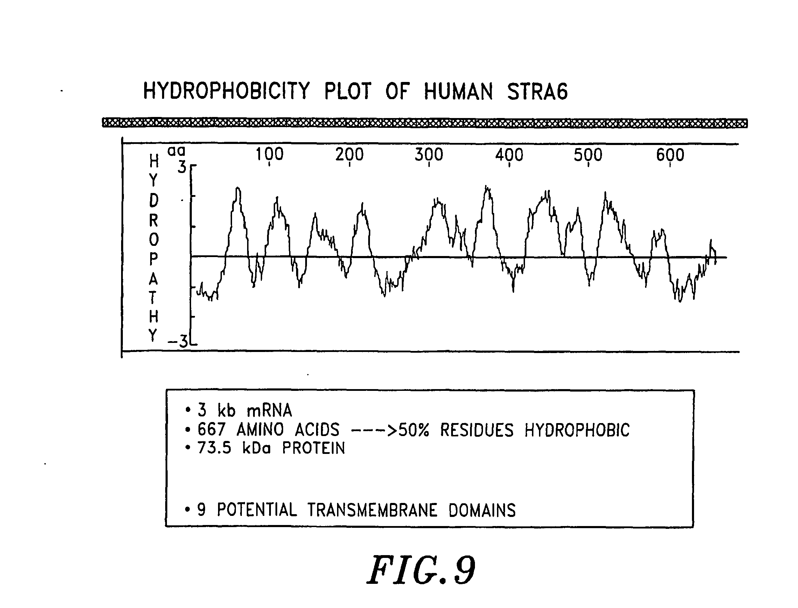

Figure 9 shows the hydrophobicity plot of the native sequence human full-length PRO10282 polypeptide encoded by DNA148380-2827, briefly referred to as "human Stra6." As shown in Figure 9, about 50% of the amino acid residues in this 667 amino acids long polypeptide are hydrophobic.

The human Stra6 gene was localized to chromosome 15q23 as determined by UNIGENE. Preliminary fine mapping indicates that Stra6 is located in the STS interval D15S124-D15S160 and the GeneMap'98 position corresponds to 244.52 on the G3 map.

EXAMPLE 2

Use of PRO10282 and PRO19578 as a hybridization probe

The following method describes use of a nucleotide sequence encoding PRO10282 and PRO19578 as a hybridization probe.

DNA comprising the coding sequence of full-length or mature PRO10282 or PRO19578 is employed as a probe to screen for homologous DNAs (such as those encoding naturally-occurring variants of PRO10282 or PRO19578) in human tissue cDNA libraries or human tissue genomic libraries.

Hybridization and washing of filters containing either library DNAs is performed under the following high stringency conditions. Hybridization of radiolabeled PRO10282-derived probe to the filters is performed in a solution of 50% formamide, 5x SSC, 0.1% SDS, 0.1% sodium pyrophosphate, 50 mM sodium phosphate, pH 6.8, 2x Denhardt's solution, and 10% dextran sulfate at 42°C for 20 hours. Washing of the filters is performed in an aqueous solution of 0.1x SSC and 0.1% SDS at 42°C.

DNAs having a desired sequence identity with the DNA encoding full-length native sequence PRO10282 or PRO19578 can then be identified using standard techniques known in the art.

EXAMPLE 3

Expression of PRO10282 and PRO19578 in *E. coli*

This example illustrates preparation of an unglycosylated form of PRO10282 or PRO19578 by recombinant expression in *E. coli.*

The DNA sequence encoding PRO10282 or PRO19578 is initially amplified using selected PCR primers. The primers should contain restriction enzyme sites which correspond to the restriction enzyme sites on the selected expression vector. A variety of expression vectors may be employed. An example of a suitable vector is pBR322 (derived from *E. coli;* see Bolivar et al., Gene, 2:95 (1977)) which contains genes for ampicillin and tetracycline resistance. The vector is digested with restriction enzyme and dephosphorylated. The PCR amplified sequences are then ligated into the vector. The vector will preferably include sequences which encode for an antibiotic resistance gene, a trp promoter, a polyhis leader (including the first six STII codons, polyhis sequence, and enterokinase cleavage site), the PRO10282 or PRO19578 coding region, lambda transcriptional terminator, and an argU gene.

The ligation mixture is then used to transform a selected *E. coli* strain using the methods described in Sambrook et al., supra. Transformants are identified by their ability to grow on LB plates and antibiotic resistant colonies are then selected. Plasmid DNA can be isolated and confirmed by restriction analysis and DNA sequencing.

Selected clones can be grown overnight in liquid culture medium such as LB broth supplemented with antibiotics. The overnight culture may subsequently be used to inoculate a larger scale culture. The cells are then grown to a desired optical density, during which the expression promoter is turned on.

After culturing the cells for several more hours, the cells can be harvested by centrifugation. The cell pellet obtained by the centrifugation can be solubilized using various agents known in the art, and the solubilized PRO10282 protein can then be purified using a metal chelating column under conditions that allow tight binding of the protein.

PRO10282 or PRO19578 may be expressed in *E. coli* in a poly-His tagged form, using the following procedure. The DNA encoding PRO10282 or PRO19578 is initially amplified using selected PCR primers. The primers will contain restriction enzyme sites which correspond to the restriction enzyme sites on the selected expression vector, and other useful sequences providing for efficient and reliable translation initiation, rapid purification on a metal chelation column, and proteolytic removal with enterokinase. The PCR-amplified, poly-His tagged sequences are then ligated into an expression vector, which is used to transform an *E. coli* host based on strain 52 (W3110 fuhA(tonA) lon galE rpoHts (htpRts) clpP(laclq). Transformants are first grown in LB containing 50 mg/ml carbenicillin at 30°C with shaking until an O.D.600 of 3-5 is reached. Cultures are then diluted 50-100 fold into CRAP media (prepared by mixing 3.57 g $(NH_4)_2SO_4$, 0.71 g sodium citrate 2H20, 1.07 g KCl, 5.36 g Difco yeast extract, 5.36 g Sheffield hycase SF in 500 mL water, as well as 110 mM MPOS, pH 7.3, 0.55% (w/v) glucose and 7 mM $MgSO_4$) and grown for approximately 20-30 hours at 30°C with shaking. Samples are removed to verify expression by SDS-PAGE analysis, and the bulk culture is centrifuged to pellet the cells. Cell pellets are frozen until purification and refolding.

*E. coli* paste from 0.5 to 1 L fermentations (6-10 g pellets) is resuspended in 10 volumes (w/v) in 7 M guanidine, 20 mM Tris, pH 8 buffer. Solid sodium sulfite and sodium tetrathionate is added to make final concentrations of 0.1 M and 0.02 M, respectively, and the solution is stirred overnight at 4°C. This step results in a denatured protein with all cysteine residues blocked by sulfitolization. The solution is centrifuged at 40,000 rpm in a Beckman Ultracentrifuge for 30 min. The supernatant is diluted with 3-5 volumes of metal chelate column buffer (6 M guanidine, 20 mM Tris, pH 7.4) and filtered through 0.22 micron filters to clarify. The clarified extract is loaded onto a 5 ml Qiagen Ni-NTA metal chelate column equilibrated in the metal chelate column buffer. The column is washed with additional buffer containing 50 mM imidazole (Calbiochem, Utrol grade), pH 7.4. The protein is eluted with buffer containing 250 mM imidazole. Fractions containing the desired protein are pooled and stored at 4°C. Protein concentration is estimated by its absorbance at 280 nm using the calculated extinction coefficient based on its amino acid sequence.

The proteins are refolded by diluting the sample slowly into freshly prepared refolding buffer consisting of: 20 mM Tris, pH 8.6, 0.3 M NaCl, 2.5 M urea, 5 mM cysteine, 20 mM glycine and 1 mM EDTA. Refolding volumes are chosen so that the final protein concentration is between 50 to 100 micrograms/ml. The refolding solution is stirred gently at 4°C for 12-36 hours. The refolding reaction is quenched by the addition of TFA to a final concentration of 0.4% (pH of approximately 3). Before further purification of the protein, the solution is filtered through a 0.22 micron filter and acetonitrile is added to 2-10% final concentration. The refolded protein is chromatographed on a Poros R11H reversed phase column using a mobile buffer of 0.1% TFA with elution with a gradient of acetonitrile from 10 to 80%. Aliquots of fractions with A280 absorbance are analyzed on SDS polyacrylamide gels and fractions containing homogeneous refolded protein are pooled. Generally, the properly refolded species of most proteins are eluted at the lowest concentrations of acetonitrile since those species are the most compact with their hydrophobic interiors shielded from interaction with the reversed phase resin. Aggregated species are usually eluted at higher acetonitrile concentrations. In addition to resolving misfolded forms of proteins from the desired form, the reversed phase step also removes endotoxin from the samples.

Fractions containing the desired folded PRO10282 or PRO19578 polypeptide are pooled and the acetonitrile removed using a gentle stream of nitrogen directed at the solution. Proteins are formulated into 20 mM Hepes, pH 6.8 with 0.14 M sodium chloride and 4% mannitol by dialysis or by gel filtration using G25 Superfine (Pharmacia) resins equilibrated in the formulation buffer and sterile filtered.

Specifically, two extracellular domains (ECD) of the native human Stra6 protein PRO10282, Pepetide A (amino acids 229-295) and Pepetide B (amino acids 532-667) were expressed separately as peptides in the *E. coli* cytoplasm with an N-terminal polyhistidine leader having the amino acid sequence MKHQHQHQHQHQHQMHQ (SEQ ID NO: 12). This leader provides for optimal translation initiation, purification on a nickel chelation column, and efficient removal if desired with the TAGZyme system (Unizyme Laboratories). Transcription was controlled by the *E. coli* alkaline phosphatase promoter (Kikuchi et al., Nucleic Acids Res. 9:5671-5678 [1981]) and the *trp* operon ribosome binding site (Yanofsky et al., Nucleic Acids Res. 9:6647-6668 [1981]) provided for translation. Downstream of the translation termination codon, is the λto transcriptional terminator (Scholtissek and Grosse, Nucleic Acids Res. 15:3185 [1987]) followed by the rare codon tRNA genes *pro2, argU,* and *glyT* (Komine et al., J. Mol. Biol. 212:579-598 [1990]; Fournier and Ozeki, Microbiol. Rev. 49:379.397 [1985]).

The two Stra6 ECD coding sequence DNA fragments were prepared by PCR from a full length cDNA clone, and inserted into the expression vector described above, which was designated as pST239. After DNA sequence verification, the new Stra6 expression plasmids, designated PE148380A and PE148380B, were transformed into the *E coli* strain 58F3 ((fhuAΔ(tonAΔ) lonΔ galE rpoHts (htpRts) ΔclpP laclq ΔompTΔ (nmpc-fepE) ΔslyD). Luria Broth cultures of these transformants were first grown overnight at 30 °C, and then diluted 100 fold into a phosphate limiting media to induce the alkaline phosphatase promoter. After 24 hours at 30 °C with shaking, the cultures were centrifuged, and the cell pastes frozen until the start of peptide purification.

For purification, *E. coli* pastes (6-10 gm pellets) were resuspended in 10 volumes (w/v) of 7 M guanidine HCl, 20 mM Tris, pH 8, buffer. Solid sodium sulfite and sodium tetrathionate were added to make final concentrations of 0.1 M and 0.02 M, respectively, and the solution was stirred overnight at 4 °C. The solution was clarified by centrifugation and loaded onto a Qiagen Ni-NTA metal chelate column equilibrated in 6 M guanidine, HCl, 20 mM Tris, pH 7.4. The column was washed with additional buffer containing 50 mM imidazole (Calbiochem, Utrol grade). The protein was eluted with buffer containing 250 mM imidazole. Fractions containing the desired protein were pooled, dialyzed against 1 mM HCl and stored at 4 °C.

EXAMPLE 4

Expression of PRO10282 and PRO19578 in mammalian cells

This example illustrates preparation of a potentially glycosylated form of PRO10282 and PRO19578 by recombinant expression in mammalian cells.

The vector, pRK5 (see EP 307,247, published March 15, 1989), is employed as the expression vector. Optionally, the PRO10282 or PRO19578 DNA is ligated into pRK5 with selected restriction enzymes to allow insertion of the PRO10282 DNA using ligation methods such as described in Sambrook et al., supra. The resulting vector is called pRK5-PRO10282 and pRK5-PRO19578, respectively.

In one embodiment, the selected host cells may be 293 cells. Human 293 cells (ATCC CCL 1573) are grown to confluence in tissue culture plates in medium such as DMEM supplemented with fetal calf serum and optionally, nutrient components and/or antibiotics. About 10 g pRKS-PRO10282 or pRK5-PRO19578 DNA is mixed with about 1 g DNA encoding the VA RNA gene [Thimmappaya et al., Cell, 31:543 (1982)] and dissolved in 500 1 of 1 mM Tris-HCl, 0.1 mM EDTA, 0.227 M $CaCl_2$. To this mixture is added, dropwise, 500 1 of 50 mM HEPES (pH 7.35), 280 mM NaCl, 1.5 mM $NaPO_4$, and a precipitate is allowed to form for 10 minutes at 25°C. The precipitate is suspended and added to the 293 cells and allowed to settle for about four hours at 37°C. The culture medium is aspirated off and 2 ml of 20% glycerol in PBS is added for 30 seconds. The 293 cells are then washed with serum free medium, fresh medium is added and the cells are incubated for about 5 days.

Approximately 24 hours after the transfections, the culture medium is removed and replaced with culture medium (alone) or culture medium containing 200 Ci/ml $^{35}$S-cysteine and 200 Ci/ml $^{35}$S-methionine. After a 12 hour incubation, the conditioned medium is collected, concentrated on a spin filter, and loaded onto a 15% SDS gel. The processed gel may be dried and exposed to film for a selected period of time to reveal the presence of PRO10282 polypeptide. The cultures containing transfected cells may undergo further incubation (in serum free medium) and the medium is tested in selected bioassays.

In an alternative technique, PRO10282 or PRO19578 may be introduced into 293 cells transiently using the dextran sulfate method described by Somparyrac et al., Proc. Natl. Acad. Sci., 12:7575 (1981). 293 cells are grown to maximal density in a spinner flask and 700 g pRK5-PRO10282 or pRKS-PRO19578 DNA is added. The cells are first concentrated from the spinner flask by centrifugation and washed with PBS. The DNA-dextran precipitate is incubated on the cell pellet for four hours. The cells are treated with 20% glycerol for 90 seconds, washed with tissue culture medium, and re-introduced into the spinner flask containing tissue culture medium, 5 g/ml bovine insulin and 0.1 g/ml bovine transferrin. After about four days, the conditioned media is centrifuged and filtered to remove cells and debris. The sample containing expressed PRO10282 or PRO19578 can then be concentrated and purified by any selected method, such as dialysis and/or column chromatography.

In another embodiment, PRO10282 or PRO19578 can be expressed in CHO cells. The pRK5-PRO10282 or pRK5-PRO19578 can be transfected into CHO cells using known reagents such as $CaPO_4$ or DEAE-dextran. As described above, the cell cultures can be incubated, and the medium replaced with culture medium (alone) or medium containing a radiolabel such as $^{35}$S-methionine. After determining the presence of PRO10282 or PRO19578 polypeptide, the culture medium may be replaced with serum free medium. Preferably, the cultures are incubated for about 6 days, and then the conditioned medium is harvested. The medium containing the expressed PRO10282 or PRO19578 can then be concentrated and purified by any selected method.

Epitope-tagged PRO10282 or pRO19578 may also be expressed in host CHO cells. The PRO10282 or PRO19578 may be subcloned out of the pRK5 vector. The subclone insert can undergo PCR to fuse in frame with a selected epitope tag such as a poly-his tag into a Baculovirus expression vector. The poly-his tagged PRO10282 or PRO19578 insert can then be subcloned into a SV40 driven vector containing a selection marker such as DHFR for selection of stable clones. Finally, the CHO cells can be transfected (as described above) with the SV40 driven vector. Labeling may be performed, as described above, to verify expression. The culture medium containing the expressed poly-His tagged PRO10282 or PRO19578 can then be concentrated and purified by any selected method, such as by $Ni^{2+}$-chelate affinity chromatography.

PRO10282 or PRO19578 may also be expressed in CHO and/or COS cells by a transient expression procedure or in CHO cells by another stable expression procedure.

Stable expression in CHO cells is performed using the following procedure. The proteins are expressed as an IgG construct (immunoadhesin), in which the coding sequences for the soluble forms (e.g. extracellular domains) of the respective proteins are fused to an IgG1 constant region sequence containing the hinge, CH2 and CH2 domains and/or is a poly-His tagged form.

Following PCR amplification, the respective DNAs are subcloned in a CHO expression vector using standard techniques as described in Ausubel et al., Current Protocols of Molecular Biology, Unit 3.16, John Wiley and Sons (1997). CHO expression vectors are constructed to have compatible restriction sites 5' and 3' of the DNA of interest to allow the convenient shuttling of cDNA's. The vector used expression in CHO cells is as described in Lucas et al., Nucl. Acids Res. 24:9 (1774-1779 (1996), and uses the SV40 early promoter/enhancer to drive expression of the cDNA of interest and dihydrofolate reductase (DHFR). DHFR expression permits selection for stable maintenance of the plasmid following transfection.

Twelve micrograms of the desired plasmid DNA is introduced into approximately 10 million CHO cells using commercially available transfection reagents Superfect® (Quiagen), Dosper® or Fugene® (Boehringer Mannheim). The cells are grown as described in Lucas et al., supra. Approximately 3 x $10^{-7}$ cells are frozen in an ampoule for further growth and production as described below.

The ampoules containing the plasmid DNA are thawed by placement into water bath and mixed by vortexing. The contents are pipetted into a centrifuge tube containing 10 mLs of media and centrifuged at 1000 rpm for 5 minutes. The supernatant is aspirated and the cells are resuspended in 10 mL of selective media (0.2 $\mu$m filtered PS20 with 5% 0.2 $\mu$m diafiltered fetal bovine serum). The cells are then aliquoted into a 100 mL spinner containing 90 mL of selective media. After 1-2 days, the cells are transferred into a 250 mL spinner filled with 150 mL selective growth medium and incubated at 37°C. After another 2-3 days, 250 mL, 500 mL and 2000 mL spinners are seeded with 3 x $10^5$ celis/ml. The cell media is exchanged with fresh media by centrifugation and resuspension in production medium. Although any suitable CHO media may be employed, a production medium described in U.S. Patent No. 5,122,469, issued June 16, 1992 may actually be used. A 3L production spinner is seeded at 1.2 x $10^8$ cells/mL. On day 0, the cell number pH is determined. On day 1, the spinner is sampled and sparging with filtered air is commenced. On day 2, the spinner is sampled, the temperature shifted to 33°C, and 30 mL of 500 g/L glucose and 0.6 mL of 10% antifoam (e.g., 35% polydimethylsiloxane emulsion, Dow Corning 365 Medical Grade Emulsion) taken. Throughout the production, the pH is adjusted as necessary to keep it at around 7.2. After 10 days, or until the viability dropped below 70%, the cell culture is harvested by centrifugation and filtering through a 0.22 μm filter. The filtrate was either stored at 4°C or immediately loaded onto columns for purification.

For the poly-His tagged constructs, the proteins are purified using a Ni-NTA column (Qiagen). Before purification, imidazole is added to the conditioned media to a concentration of 5 mM. The conditioned media is pumped onto a 6 ml Ni-NTA column equilibrated in 20 mM Hepes, pH 7.4, buffer containing 0.3 M NaCl and 5 mM imidazole at a flow rate of 4-5 ml/min. at 4°C. After loading, the column is washed with additional equilibration buffer and the protein eluted with equilibration buffer containing 0.25 M imidazole. The highly purified protein is subsequently desalted into a storage buffer containing 10 mM Hepes, 0.14 M NaCl and 4% mannitol, pH 6.8, with a 25 ml G25 Superfine (Pharmacia) column and stored at -80°C.

Immunoadhesin (Fc-containing) constructs are purified from the conditioned media as follows. The conditioned medium is pumped onto a 5 ml Protein A column (Pharmacia) which had been equilibrated in 20 mM Na phosphate buffer, pH 6.8. After loading, the column is washed extensively with equilibration buffer before elution with 100 mM citric acid, pH 3.5. The eluted protein is immediately neutralized by collecting 1 ml fractions into tubes containing 275 μL of 1 M Tris buffer, pH 9. The highly purified protein is subsequently desalted into storage buffer as described above for the poly-His tagged proteins. The homogeneity is assessed by SDS polyacrylamide gels and by N-terminal amino acid sequencing by Edman degradation.

EXAMPLE 5

Expression of PRO10282 and PRO19578 in Yeast

The following method describes recombinant expression of PRO10282 and PRO19578 in yeast.

First, yeast expression vectors are constructed for intracellular production or secretion of PRO10282 polypeptides from the ADH2/GAPDH promoter. DNA encoding a PRO10282 polypeptide (including PRO19578) and the promoter is inserted into suitable restriction enzyme sites in the selected plasmid to direct intracellular expression of PRO10282, For secretion, DNA encoding PRO10282 can be cloned into the selected plasmid, together with DNA encoding the ADH2/GAPDH promoter, a native PRO10282 signal peptide or other mammalian signal peptide, or, for example, a yeast alpha-factor or invertase secretory signal/leader sequence, and linker sequences (if needed) for expression of PRO10282.

Yeast calls, such as yeast strain AB110, can then be transformed with the expression plasmids described above and cultured in selected fermentation media. The transformed yeast supernatants can be analyzed by precipitation with 10% trichloroacetic acid and separation by SDS-PAGE, followed by staining of the gels with Coomassie Blue stain.

Recombinant PRO10282 (including PRO19578) can subsequently be isolated and purified by removing the yeast cells from the fermentation medium by centrifugation and then concentrating the medium using selected cartridge filters. The concentrate containing PRO10282 (e.g. PRO19578) may further be purified using selected column chromatography resins.

EXAMPLE 6

Expression of PRO10282 and PRO19578 in Baculovirus-Infected Insect Cells

The following method describes recombinant expression of PRO10282 and PRO19578 in Baculovirus-infected insect cells.

The sequence coding for PRO10282 or PRO19578 is fused upstream of an epitope tag contained within a baculovirus expression vector. Such epitope tags include poly-his tags and immunoglobulin tags (like Fc regions of IgG). A variety of plasmids may be employed, including plasmids derived from commercially available plasmids such as pVL1393 (Novagen). Briefly, the sequence encoding PRO10282 or PRO19578 or the desired portion of the coding sequence of PRO10282 or PRO19578, such as the sequence encoding the extracellular domain of a transmembrane protein or the sequence encoding the mature protein if the protein is extracellular, is amplified by PCR with primers complementary to the 5' and 3' regions. The 5' primer may incorporate flanking (selected) restriction enzyme sites. The product is then digested with those selected restriction enzymes and subcloned into the expression vector.

Recombinant baculovirus is generated by co-transfecting the above plasmid and BaculoGold™ virus DNA (Pharmingen) into *Spodoptera frugiperda* ("Sf9") cells (ATCC CRL 1711) using lipofectin (commercially available from GIBCO-BRL). After 4 - 5 days of incubation at 28°C, the released viruses are harvested and used for further amplifications. Viral infection and protein expression are performed as described by O'Reilley et al., Baculovirus expression vectors: A Laboratory Manual, Oxford: Oxford University Press (1994).

Expressed poly-his tagged PRO10282 or PRO19578 can then be purified, for example, by $Ni^{2+}$-chelate affinity chromatography as follows. Extracts are prepared from recombinant virus-infected Sf9 cells as described by Rupert et al., Nature, 362:175-179 (1993). Briefly, Sf9 cells are washed, resuspended in sonication buffer (25 mL Hepes, pH 7.9; 12.5 mM $MgCl_2$; 0.1 mM EDTA; 10% glycerol; 0.1% NP-40; 0.4 M KCl), and sonicated twice for 20 seconds on ice. The sonicates are cleared by centrifugation, and the supernatant is diluted 50-fold in loading buffer (50 mM phosphate, 300 mM NaCl, 10% glycerol, pH 7.8) and filtered through a 0.45 μm filter. A $Ni^{2+}$-NTA agarose column (commercially available from Qiagen) is prepared with a bed volume of 5 mL, washed with 25 mL of water and equilibrated with 25 mL of loading buffer. The filtered cell extract is loaded onto the column at 0.5 mL per minute. The column is washed to baseline $A_{280}$ with loading buffer, at which point fraction collection is started. Next, the column is washed with a secondary wash buffer (50 mM phosphate; 300 mM NaCl, 10% glycerol, pH 6.0), which elutes nonspecifically bound protein. After reaching $A_{280}$ baseline again, the column is developed with a 0 to 500 mM Imidazole gradient in the secondary wash buffer. One mL fractions are collected and analyzed by SDS-PAGE and silver staining or Western blot with $Ni^{2+}$-NTA-conjugated to alkaline phosphatase (Qiagen). Fractions containing the eluted $His_{10}$-tagged PRO10282 or PRO19578 are pooled and dialyzed against loading buffer.

Alternatively, purification of the IgG tagged (or Fc tagged) PRO10282 or PRO19578 can be performed using known chromatography techniques, including for instance, Protein A or protein G column chromatography.

EXAMPLE 7

Preparation of Antibodies that Bind PRO10282 or PRO19578

This example illustrates preparation of monoclonal antibodies which can specifically bind PRO10282 or PRO19578.

Techniques for producing the monoclonal antibodies are known in the art and are described, for instance, in Goding, supra. Immunogens that may be employed include purified PRO10282 and PRO19578, fusion proteins containing PRO10282 or PRO19578, and cells expressing recombinant PRO10282 or PRO19578 on the cell surface. Selection of the immunogen can be made by the skilled artisan without undue experimentation.

Mice, such as Balb/c, are immunized with the PRO10282 or PRO19578 immunogen emulsified in complete Freund's adjuvant and injected subcutaneously or intraperitoneally in an amount from 1-100 micrograms. Alternatively, the immunogen is emulsified in MPL-TDM adjuvant (Ribi Immunochemical Research, Hamilton, MT) and injected into the animal's hind foot pads. The immunized mice are then boosted 10 to 12 days later with additional immunogen emulsified in the selected adjuvant. Thereafter, for several weeks, the mice may also be boosted with additional immunization injections. Serum samples may be periodically obtained from the mice by retro-orbital bleeding for testing in ELISA assays to detect anti-PRO10282 antibodies or anti-PRO19578 antibodies.

After a suitable antibody titer has been detected, the animals "positive" for antibodies can be injected with a final intravenous injection of PRO10282 or PRO19578. Three to four days later, the mice are sacrificed and the spleen cells are harvested. The spleen cells are then fused (using 35% polyethylene glycol) to a selected murine myeloma cell line such as P3X63AgU.1, available from ATCC, No. CRL 1597. The fusions generate hybridoma cells which can then be plated in 96 well tissue culture plates containing HAT (hypoxanthine, aminopterin, and thymidine) medium to inhibit proliferation of non-fused cells, myeloma hybrids, and spleen cell hybrids.

The hybridoma cells will be screened in an ELISA for reactivity against PRO10282 or PRO19578. Determination of "positive" hybridoma cells secreting the desired monoclonal antibodies against PRO10282 or PRO19578 is within the skill in the art.

The positive hybridoma cells can be injected intraperitoneally into syngeneic Balb/c mice to produce ascites containing the anti-PRO10282 or anti-PRO19578 monoclonal antibodies. Alternatively, the hybridoma cells can be grown in tissue culture flasks or roller bottles. Purification of the monoclonal antibodies produced in the ascites can be accomplished using ammonium sulfate precipitation, followed by gel exclusion chromatography. Alternatively, affinity chromatography based upon binding of antibody to protein A or protein G can be employed.

Specifically, five Balb/c mice (Charles River Laboratories, Wilmington, DE) were hyper-immunized with purified Unizyme-conjugated amino acid peptide, corresponding to amino acids 532-667 of human Stra6, in Ribi adjuvant (Ribi Immunochem Research, Inc., Hamilton, MO). B-celis from popliteal lymph nodes were fused with mouse myeloma cells (X63.Ag8.653; American Type Culture Collection, Rockville, MD) as previously described (Hongo et al., Hybridoma 14: 253-260 [1995]). After 10-14 days, supernatants were harvested and screened for antibody production by direct enzyme-linked immunosorbant assay (ELISA). Eight positive clones, showing the highest immunobinding by direct ELISA and immunohistochemistry after two rounds of subcloning by limiting dilution, were injected into Pristine-primed mice for *in vivo* production of mAb (Freud and Blair, J. Immunol. 129:2826-2830 [1982]). The ascites fluids were pooled and purified by Protein A affinity chromatography (Pharmacia fast protein liquid chromatography (FPLC); Pharmacia, Uppsala, Sweden) as previously described (Hongo *et al., supra).* The purified antibody preparations were sterile filtered (0.2-μm pore size; Nalgene, Rochester, NY) and stored at 4 °C in phosphate buffered saline (PBS).

EXAMPLE 8

Purification of PRO10282 and PRO19578 Polypeptides Using Specific Antibodies

Native or recombinant PRO10282 and PRO19578 polypeptides may be purified by a variety of standard techniques in the art of protein purification. For example, pro-PRO10282 or pro-PRO19578 polypeptide, mature PRO10282 or PRO19578 polypeptide, or pre-PRO10282 or pre-PRO19578 polypeptide is purified by immunoaffinity chromatography using antibodies specific for the PRO10282 polypeptide of interest. In general, an immunoaffinity column is constructed by covalently coupling the anti-PRO10282 or anti-PRO19578 polypeptide antibody to an activated chromatographic resin.
Polyclonal immunoglobulins are prepared from immune sera either by precipitation with ammonium sulfate or by purification on immobilized Protein A (Pharmacia LKB Biotechnology, Piscataway, N.J.). Likewise, monoclonal antibodies are prepared from mouse ascites fluid by ammonium sulfate precipitation or chromatography on immobilized Protein A. Partially purified immunoglobulin is covalently attached to a chromatographic resin such as CnBr-activated SEPHAROSE™ (Pharmacia LKB Biotechnology). The antibody is coupled to the resin, the resin is blocked, and the derivative resin is washed according to the manufacturer's instructions.
Such an immunoaffinity column is utilized in the purification of PRO10282 or PRO19578 polypeptide by preparing a fraction from cells containing PRO10282 or PRO19578 polypeptide in a soluble form. This preparation is derived by solubilization of the whole cell or of a subcellular fraction obtained via differential centrifugation by the addition of detergent or by other methods well known in the art. Alternatively, soluble PRO10282 or PRO19578 polypeptide containing a signal sequence may be secreted in useful quantity into the medium in which the cells are grown.
A soluble PRO10282 or PRO19578 polypeptide-containing preparation is passed over the immunoaffinity column, and the column is washed under conditions that allow the preferential absorbance of PRO10282 or PRO19578 polypeptide (e.g., high ionic strength buffers in the presence of detergent). Then, the column is eluted under conditions that disrupt antibody/PRO10282 or PRO19578 polypeptide binding (e.g., a low pH buffer such as approximately pH 2-3, or a high concentration of a chaotrope such as urea or thiocyanate ion), and PRO10282 or pR019578 polypeptide is collected.

EXAMPLE 9

Drug Screening

This invention is particularly useful for screening compounds by using PRO10282 (Stra6) polypeptides (including PRO19578) or binding fragment thereof in any of a variety of drug screening techniques. The PRO10282 polypeptide or fragment employed in such a test may either be free in solution, affixed to a solid support, borne on a cell surface, or located intracellularly. One method of drug screening utilizes eukaryotic or prokaryotic host cells which are stably transformed with recombinant nucleic acids expressing the PRO10282 polypeptide or fragment. Drugs are screened against such transformed cells in competitive binding assays. Such cells, either in viable or fixed form, can be used for standard binding assays. One may measure, for example, the formation of complexes between PRO10282 polypeptide or a fragment and the agent being tested. Alternatively, one can examine the diminution in complex formation between the PRO10282 polypeptide and its target cell or target receptors caused by the agent being tested.
Thus, the present invention provides methods of screening for drugs or any other agents which can affect a PRO10282 (Stra6) polypeptide-associated disease or disorder. These methods comprise contacting such an agent with a Stra6 polypeptide or fragment thereof and assaying (i) for the presence of a complex between the agent and the Stra6 polypeptide or fragment, or (ii) for the presence of a complex between the Stra6 polypeptide or fragment and the cell, by methods well known in the art. In such competitive binding assays, the Stra6 polypeptide or fragment is typically labeled. After suitable incubation, free Stra6 polypeptide or fragment is separated from that present in bound form, and the amount of free or uncomplexed label is a measure of the ability of the particular agent to bind to Stra6 polypeptide or to interfere with the Stra6 polypeptide/cell complex.
Another technique for drug screening provides high throughput screening for compounds having suitable binding affinity to a polypeptide and is described in detail in WO 84/03564, published on September 13, 1984. Briefly stated, large numbers of different small peptide test compounds are synthesized on a solid substrate, such as plastic pins or some other surface. As applied to a PRO10282 (Stra6) polypeptide, the peptide test compounds are reacted with Stra6 polypeptide and washed. Bound Stra6 polypeptide is detected by methods well known in the art. Purified Stra6 polypeptide can also be coated directly onto plates for use in the aforementioned drug screening techniques. In addition, non-neutralizing antibodies can be used to capture the peptide and immobilize it on the solid support.
This invention also contemplates the use of competitive drug screening assays in which neutralizing antibodies capable of binding a Stra6 polypeptide specifically compete with a test compound for binding to a Stra6 polypeptide or fragments thereof. In this manner, the antibodies can be used to detect the presence of any peptide which shares one or more antigenic determinants with the Stra6 polypeptide.

**EXAMPLE 10**

Rational Drung Design

The goal of rational drug design is to produce structural analogs of biologically active polypeptide of interest *(i.e.,* a PR010282 (Stra6) polypeptide) or of small molecules with which they interact, *e.g.,* agonists, antagonists, or inhibitors. Any of these examples can be used to fashion drugs which are more active or stable forms of the native sequence PR010282 polypeptide or PR019578 polypeptide, or which enhance or interfere with the function of the native sequence PR010282 or PR019578 polypeptide *in vivo* (*c.f.*, Hodgson, Bio/Technology, 9:19-21 (1991)).

In one approach, the three-dimensional structure of the native sequence PR010282 or PR019578 polypeptide, or of a PR010282 or PR019578 polypeptide-inhibitor complex, is determined by x-ray crystallography, by computer modeling or, most typically, by a combination of the two approaches. Both the shape and charges of the native PR010282 or PR019578 polypeptide must be ascertained to elucidate the structure and to determine active site(s) of the molecule. Less often, useful information regarding the structure of the PR010282 or PR019578 polypeptide may be gained by modeling based on the structure of homologous proteins. In both cases, relevant structural information is used to design analogous PR010282 polypeptide-like molecules or to identify efficient inhibitors. Useful examples of rational drug design may include molecules which have improved activity or stability as shown by Braxton and Wells, Biochemistry, 31: 7796-7801 (1992) or which act as inhibitors, agonists, or antagonists of native peptides as shown by Athauda et al., J. Biochem., 113:742-746(1993).

It is also possible to isolate a target-specific antibody, selected by functional assay, as described above, and then to solve its crystal structure. This approach, in principle, yields a pharmacore upon which subsequent drug design can be based. It is possible to bypass protein crystallography altogether by generating anti-idiotypic antibodies (anti-ids) to a functional, pharmacologically active antibody. As a mirror image of a mirror image, the binding site of the anti-ids would be expected to be an analog of the original receptor. The anti-id could then be used to identify and isolate peptides from banks of chemically or biologically produced peptides. The isolated peptides would then act as the pharmacore.

By virtue of the present invention, sufficient amounts of the PR010282 polypeptides (including PR019578) may be made available to perform such analytical studies as X-ray crystallography. In addition, knowledge of the PR010282 polypeptide amino acid sequences provided herein will provide guidance to those employing computer modeling techniques in place of or in addition to x-ray crystallography.

**EXAMPLE 11**

Tissue Expression Distribution

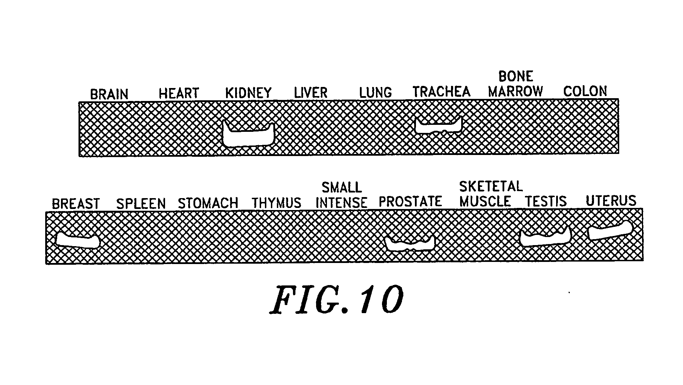

Oligonucleotide probes were constructed from the PR010282 polypeptide-encoding nucleotide sequence shown in the accompanying figures for use in quantitative PCR amplification reactions. The oligonucleotide probes were chosen so as to give an approximately 200-600 base pair amplified fragment from the 3' end of its associated template in a standard PCR reaction. The oligonucleotide probes were employed in standard quantitative PCR amplification reactions with Clontech RNA isolated from different adult human tissue sources and analyzed by agarose gel electrophoresis so as to obtain a quantitative determination of the level of expression of the PR010282 polypeptide-encoding nucleic acid in the various tissues tested. Knowledge of the expression pattern or the differential expression of the PR010282 polypeptide-encoding nucleic acid, in various different human tissue types provides a diagnostic marker useful for tissue typing, with or without other tissue-specific markers, for determining the primary tissue source of a metastatic tumor, and the like. The results of these assays (shown in Figure 10) demonstrated that the DNA148380-2827 molecule is highly expressed in the adult kidney, testis and uterus; significantly expressed in breast, prostate and trachea; weakly expressed in brain, heart, lung and thymus; and not expressed in liver, bone marrow, colon, skeletal muscle, small intestine, spleen and stomach.

Total RNA was purchased from Clontech (Palo Alto, California) and analyzed using the following primer/probe set for PCR amplification:

| | |
|---|---|
| h.Stra6.tmf3: | 5' CACACTCGAGAGCCAGATATTTT (SEO ID NO: 6) |
| h.Stra5.tmr4: | 5' AACAAGTTTATTGCAGGGAACAC (SEQ ID NO: 7) |

(continued)

| | |
|---|---|
| h.Stra6.tmp4: | 5' TGTAGTTTTTATGCCTTTGGCTATTATGAAAGAGGT (SEQ ID NO: 8) |
| tmf = forward primer<br>tmr = reverse primer<br>tmp = probe | |

In situ hybridization, performed as described in Example 12 below, confirmed Stra6 expression in kidney tubular epithelial cells, myometrium, and stromal cells surrounding breast ducts and lobules, whereas little or no expression was detected in sections of brain, liver, spleen, pancreas, heart, lung, stomach, small intestine, colon, prostate, spleen, and adrenal cortex (data not shown). Of the normal tissues examined by in situ hybridization, highest expression levels were seen in placenta and adrenal medulla, which were not included in the PCR analysis.

**EXAMPLE 12**

Over-expression of native human PR010282 (Stra6) transcript in Human Tumors

This example shows that the gene encoding native human full-length PRO10282 (Stra6) is significantly over-expressed in certain human colon tumors and also in cell lines derived from various human tumors such as colon, lung, kidney and breast.

The starting material for the screen was total RNA isolated from human colon tumors, or various human colon, kidney, breast, or lung tumor cell lines. In colon tumor tissue, Stra6 RNA expression was determined relative to RNA from normal colon tissue (mucosa) from the same patient. Stra6 RNA expression in various tumor cell lines was determined in comparison with various normal cell lines (i.e., normal colon, kidney and lung cell lines).

Real-time quantitative PCR (RT-PCR, for example, TAQMAN ABI PRIZM 7700™ Sequence Detection System™ [Perkin Elmer, Applied Biosystems Division, Foster City, California]), was used to monitor quantitative differences in the level of expression of the PRO10292 (Stra6) encoding gene (corresponding to DNA148380-2827) in normal cells and cells derived from certain cancers or cancer cell lines, using Taqman assay reagents. 50 μl RT-PCR reactions consisted of 5 μl 10x Taqman Buffer A, 300 μM of each dNTF, 5 mM $MgCl_2$, 10 units of RNAse inhibitor, 12.5 units ofMuLV Reverse Transcriptase, 1.25 units of AmpliTaq Gold DNA Polymerase, 200 nM probe, 500 nM primers and 100 ng RNA. Reaction conditions consisted of reverse transcription at 48 °C for 30 minutes, denaturation at 95 °C for 25 seconds and 65 °C for one minute. Reaction products were analyzed on 4-20% polyacrylamide gels (Novex). Standard curves were used to determine relative levels of expression for each gene of interest as well as the glyceraldehyde-3-phosphate dehydrogenase (GAPDH) housekeeping gene for each sample analyzed. Relative normalized units were obtained by dividing the gene of interest mRNA level by the GAPDH mRNA level. Relative normalized units were compared between experimental sample and control to determine fold induction.

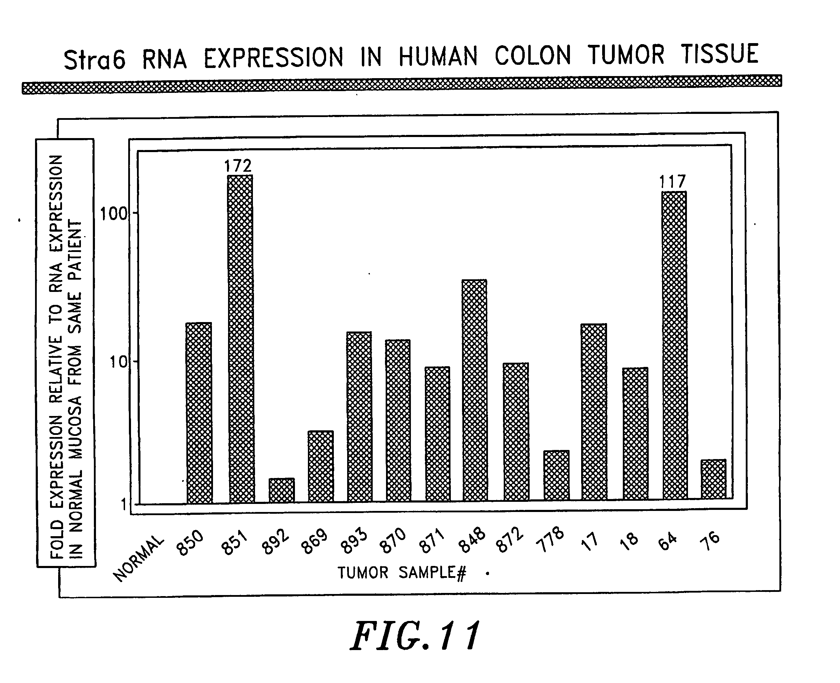

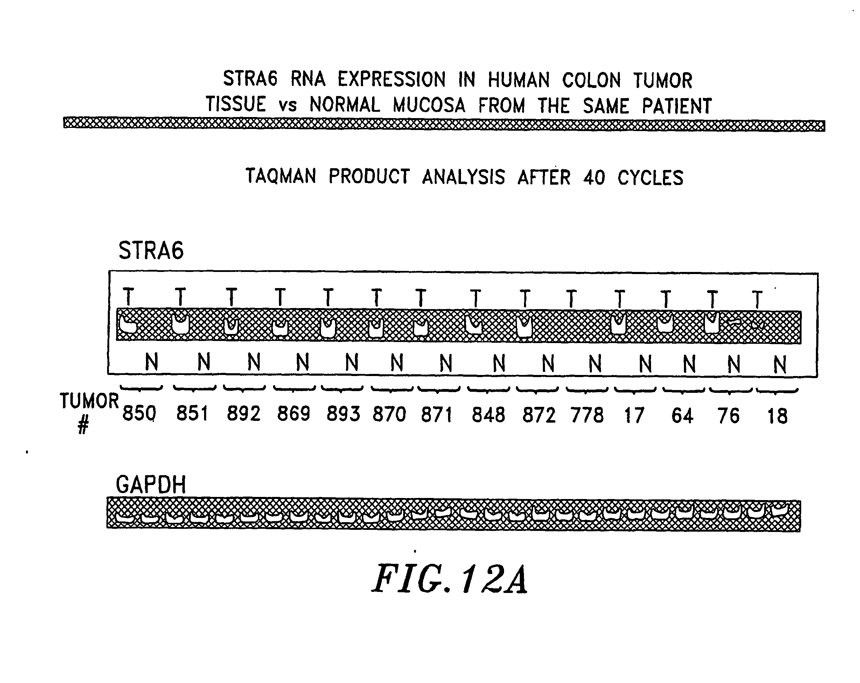

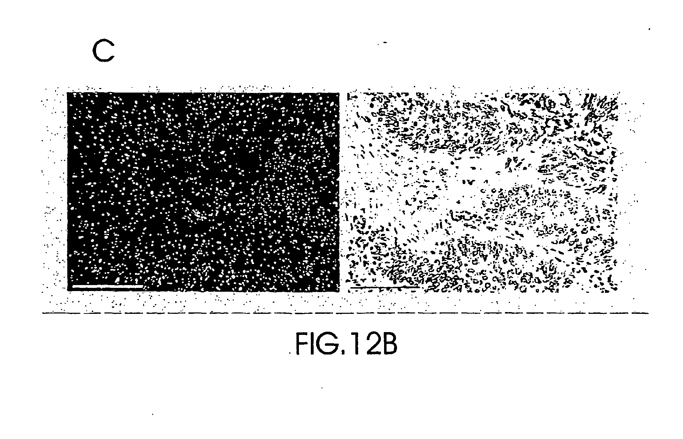

The results were used to determine whether the mRNA encoding PR010282 is over-expressed in any of the primary colon cancers or colon, kidney, breast, or lung cancer cell lines that were screened. The histology of some matched human normal and colon tumor samples used for the analysis (see Figures 11 and 12) is shown below:

| Number | Histology |
|---|---|
| 850 | Invasive adenocarcinoma; no necrosis; good preservation. |
| 851 | Invasive adenocarcinoma; minimal necrosis; good condition. |
| 892 | Invasive adenocarcinoma; minimal necrosis; good condition. |
| 869 | Invasive adenocarcinoma; minimal necrosis; good condition. |
| 893 | Normal mucosa - dysplasia - invasive adenocarcinoma; minor necrosis; good condition. |
| 870 | Adenocarcinoma - severe dysplasia; minimal necrosis; good condition. |
| 871 | Adenocarcinoma - dysplasia - normal mucosa; no necrosis; good condition. |
| 848 | Adenocarcinoma - appears to be arising in villous adenoma; normal mucosa/submucosa; no necrosis, good condition. |
| 872 | Invasive adenocarcinoma; about 70% of tumor is necrotic;<br>overall good preservation. |
| 778 | Adenocarcinoma - unusually papillary morphology; normal/hyperplastic mucosa; minimal necrosis; acceptable preservation. |
| 17 | Moderately well-differentiated adenocarcinoma. |
| 18 | Well-differentiated adenocarcinoma. |

(continued)

| Number | Histology |
|---|---|
| 64 | Moderately well-differentiated adenocarcinoma. |
| 76 | Moderately well-differentiated adenocarcinoma. |

Human lung cell lines include the normal human lung fibroblast cell lines MRC5 (CCL-171) and IMR90 (CCL-186), the human lung carcinoma epithelial cell line A549 (SRCC768, CCL-185), the human epidermoid lung carcinoma cell line Calu-1 (SRCC769; HTB-54), the human anaplastic carcinoma cell line Calu-6 (SRCC770, HTB-56 - probably lung), the human epithelial cell line NCI-H441 (SRCC772; HTB-174) which was derived from pericardial fluid of a patient with papillary adenocarcinoma of the lung, and the human lung squamous cell carcinoma cell line SW900 (SRCC775; HTB-59), all available from ATCC.

Colon cell lines include, for example, the normal colon fibroblast cell line CCD112Co (CRL-1541), the human colorectal adenocarcinoma cell line CaCo-2 (HTB-37), the human colorectal adenocarcinoma cell line WiDr (CCL-218), the human colorectal carcinoma cell line HCT116 (CCL-247), the human colorectal adenocarcinoma cell line SK-Co1 (HTB-39), the human colorectal adenocarcinoma cell line COLO320 (SRCC778; CCL-220), the human colorectal adenocarcinoma cell line HT29 (SRCC779; HTB-38), the human colorectal adenocarcinoma cell line SW403 (CCL-230), the human colon cancer cell line NCI/HCC2998, and the human colorectal adenocarcinoma cell line Colo320DM (CCL-220), all available from ATCC or other public sources.

Human breast carcinoma cell lines include the human breast adenocarcinoma cell line MCF7 (SRCC766; HTB-22), and the human breast cancer cell line NCI/ADR-RES, both of which are publicly available.

Kidney lines include the 293 cell line (CRL-1573) which is transformed with adenovirus 5 DNA. Two Wilm's tumor cell lines were also included in the analysis, G401 (CRL-1441) and SK-NEP-1 (CRL-1573).

<u>RNA preparation:</u>

RNA was prepared from the foregoing cultured cell lines. The isolation was performed using purification kit, buffer set and protease from Qiagen, according to the manufacturer's instructions and the description below. More specifically, total RNA from cells in culture was isolated using Qiagen RNeasy midi-columns. Total RNA from tissue samples was isolated using RNA Stat-60 (Tel-Test). RNA prepared from tumor was isolated by cesium chloride density gradient centrifugation.

<u>Solid human tumor sample preparation and lysis:</u>

Tumor samples were weighed and placed into 50 ml conical tubes and held on ice. Processing was limited to no more than 250 mg tissue per preparation (1 tip/preparation). The protease solution was freshly prepared by diluting into 6.25 ml cold $ddH_2O$ to a final concentration of 20 $\mu$g/ml and stored at 4°C. G2 buffer (20 ml) was prepared by diluting DNAse A to a final concentration of 200 $\mu$g/ml (from 100 mg/ml stock). The tumor tissue was homogenized in 10 ml G2 buffer for 60 seconds using the large tip of the polytron in a laminar-flow TC hood in order to avoid inhalation of aerosols, and held at room temperature. Between samples, the polytron was cleaned by spinning at 2 x 30 seconds each in 2L, $ddH_2O$, followed by G2 buffer (50 ml). If tissue was still present on the generator tip, the apparatus was disassembled and cleaned.

Qiagen protease (prepared as indicated above, 1.0 ml) was added, followed by vortexing and incubation at 50 °C for 3 hours. The incubation and centrifugation were repeated until the lysates were clear (e.g., incubating additional 30-60 minutes, pelleting at 3000 x g for 10 minutes, 4 °C).

<u>Quantitation</u>

The results obtained from the real-time PCR analysis of RNA were initially expressed as delta CT units. One unit corresponds to one PCR cycle or approximately a 2-fold amplification relative to normal, two units correspond to 4-fold, 3 units to 8-fold amplification and so on. The data is converted to fold difference and presented as such. Initially, reverse transcriptase was used to synthesize cDNA from 100 ng total RNA or polyA+ RNA using oligo(dT) as a primer. The resultant cDNA was then used as a template for PCR. Qantitation was obtained using primers derived from the 3'-untranslated regions of the PR010282 encoding sequence and a TAQMAN™ fluorescent probe corresponding to the respective intervening sequences. Using the 3' region tends to avoid crossing intron-exon boundaries in the genomic DNA, an essential requirement for accurate assessment of RNA expression using this method. The sequences for the primers and probes (forward, reverse, and probe) using for the PR010282 encoding gene amplification were as follows:

One set included the forward and reverse primers and probe described in Example 11 above as SEQ ID Nos: 6, 7 and 8, respectively.

Another set included:

hStra6.tmfl1: 5' AGACCAGGTCCCACACTGA (SEQ ID NO:9)
hStra6.tmr1: 5' TTCATAATAGCCAAAGGCATAAAA (SEQ ID NO:10), and
h.Stra6.tmp1:5' CTGCCCACACTCGAGAGCCAGAT 3' (SEQ ID NO: 11)

Human GAPDH:

forward primer: 5'-GAAGATGGTGATGGGATTTC-3' (SEQ ID NO: 14)
reverse primer; 5'-GAAGGTGAAGGTCGGAGTC-3' (SEQ ID NO: 15)
probe: 5'-CAAGCTTCCCGTTCTCAGCC-3' (SEQ ID NO: 16)

The 5' nuclease assay reaction is a fluorescent PCR-based technique which makes use of the 5' exonuclease activity of Taq DNA polymerase enzyme to monitor amplification in real time. Two oligonucleotide primers are used to generate an amplicon typical of a PCR reaction. A third oligonucleotide, or probe, is designed to detect nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

As noted above, the 5' nuclease procedure is run on a real-time quantitative PCR device such as the ABI PRIZM 7700™ Sequence Detection System™. The system consists of a thermocyler, laser, charge-coupled device (CCD), camera and computer. The system amplifies samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data are initially expressed as Ct, or the threshold cycle. This is defined as the cycle at which the reporter signal accumulates above the background level of fluorescence. The Ct values are used as quantitative measurement of the relative number of starting copies of a particular target sequence in a nucleic acid sample when comparing the expression of RNA in a cancer cell with that from a normal cell.

<u>Results</u>

The human Stra6 RNA (corresponding to DNA148380-2827) shows strong over-expression in human colon tumor tissues, when compared with corresponding normal human colon tissues. As shown in Figure 11, human Stra6 RNA (corresponding to DNA148380-2827) was found to be over-expressed in all 14 human tumor colon tissues examined, relative to RNA from matched normal colorectal mucosa from the same patient. The over-expression varied between two- and 170-fold, and in 7 out of 14 tumor tissue samples was at least about 10-fold. The cycle threshold values obtained by quantitative PCR indicated that Stra6 mRNA levels were extremely low or possibly absent in many of the normal mucosa samples.

As a second method of detection, the products obtained after completion of the quantitative PCR reactions (40 cycles each) were subjected to electrophoresis in polyacrylamide gels and visualized by ethidium bromide staining. As shown in Figure 12A, using expression of a housekeeping gene, glyceraldehyde 3-phosphate dehydrogenase (GAPDH) as the standard, substantially greater amounts of PCR products were generated off Stra6 mRNA in tumor samples compared to their normal counterparts. By contrast, a comparable level of product from the internal control GAPDH mRNA was generated from all samples.

Stra6 expression in colon adenocarcinomas was localized to the epithelial tumor cells by in situ hybridization (ISH) performed as follows. $^{32}$P-labeled sense and antisense riboprobes were transcribed from an 874 bp PCR product corresponding to nucleotides 432-1247 of the coding sequence of human Stra6 polypeptide encoded by DNA148380-2827. Formalin-fixed, paraffin-embedded tissue sections were processes as described previously (Pennica et al., Proc. Natl. Acad. Sci USA 95:14717-22 [1998]). The results are shown in Figure 12B.

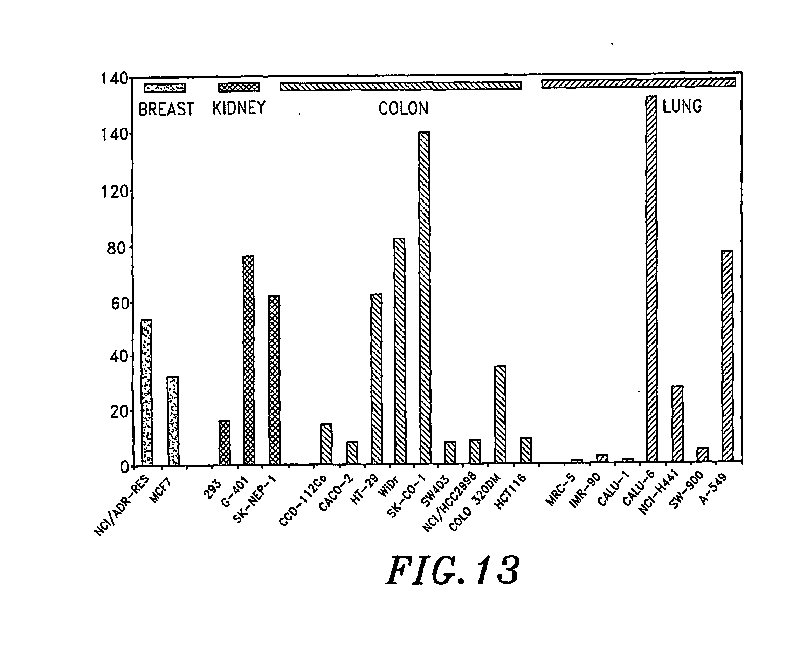

As shown in Figure 13, the human Stra6 RNA (corresponding to DNA148380-2827) is also significantly over-expressed in various breast, kidney, colon and lung tumor cell lines. "Relative RNA Expression" means that the RNA expression in normal and tumor tissues is shown relative to expression in an arbitrarily chosen standard cell line SW480.

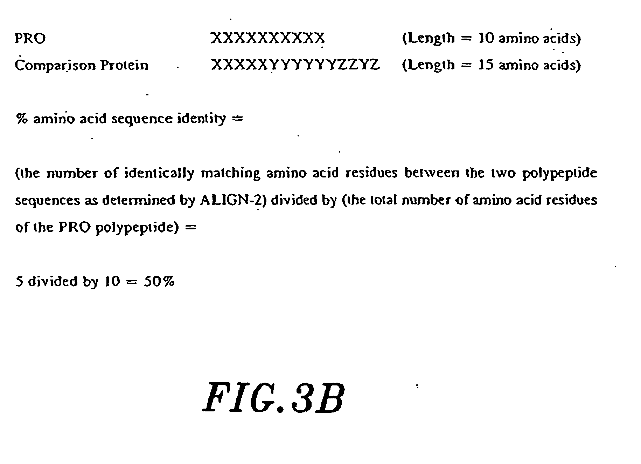

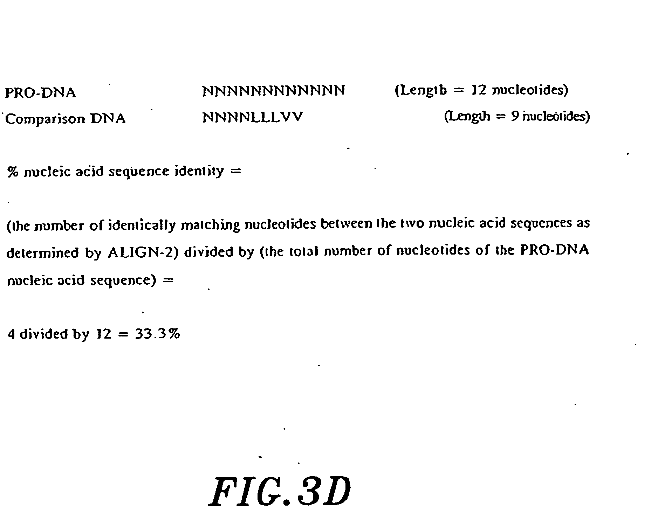

In situ hybridization results obtained in various tumor sections are also shown in Figure 16. Several tumor types other than colon adenocarcinomas also showed high levels of Stra6 expression. These included 3 of 3 melanomas (Figures 16A and B), 3 of 4 endometrial adenocarcinomas (Figures 16C and D), 2 of 3 ovarian adenocarcinomas, and a Wilm's tumor of the kidney (Figures 16E and F). The Stra6 in situ hybridization signal in these various tumors was considerably greater than in colon adenocarcinomas consistent with data showing relatively high expression levels in normal kidney and uterus and low levels in normal colon. Since Stra6 was detected in normal adrenal medulla, we also examined two pheochromocytomas, which are tumors derived from this tissue. In these tumors, Stra6 expression exceeded that of any other tumor or tissue examined in this study (Figures 16G and H). Although Stra6 was detected in normal kidney and was strongly expressed in Wilm's tumor, it was not detected in renal cell carcinomas. In kidney transitional cell carcinomas, tumor-associated stromal cells rather than tumor epithelial cells expressed Stra6 (data not shown).

Because mRNA DNA148380-2827 encoding PRO10282 is overexpressed in various tumors as well as in a number of tumor derived cell lines, it is likely associated with tumor formation and/or growth. As a result, antagonists (e.g., antibodies, organic and inorganic small molecules, peptides and polypeptides, such as Stra6 variants, antisense oligonucleotides) directed against the protein encoded by DNA148380-2827 (PR010282) or other naturally occurring variants of this protein, such as PRO19578 encoded by DNA148389-2827-1, are expected to be useful in diagnosis, prevention and/or treatment of cancer particularly, without limitation, colon, lung, breast and/or kidney cancer.

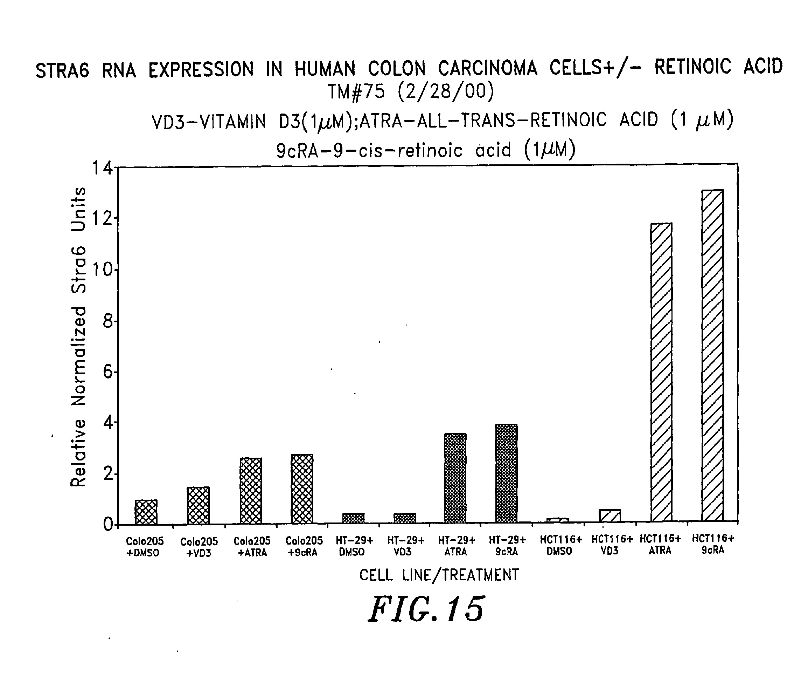

The efficacy of antagonists such as therapeutic antibodies directed against the protein encoded by DNA148380-2827 (PR010282) could be enhanced by agents that stimulate the expression of the gene encoding PR010282. For example, treatment of human colorectal cancer cell lines with 9-cis-retinoic acid or all-trans retinoic acid resulted in a dramatic enhancement of the expression of the Stra6 mRNA (Figure 15). Thus, the treatment of cancer patients with therapeutic antibodies directed against PRO10282 in combination with the appropriate retinoids would be expected to enhance tumor killing by the antibodies. The same will be true to antibodies directed against other native Stra6 polypeptides over-expressed in various tumors, such as the human splice variant encoded by DNA148389-2827-1.

## EXAMPLE 13

Synergistic Induction of Stra6 by Wnt-1 and Retinoids

Materials and Methods

*Cell Culture*

C57MG and C57MG/Wnt-1 cells were grown in Dulbecco's Modified Eagle medium supplemented with 10% fetal bovine serum, 2 mM L-glutamine, and 2.5 μg/ml puromycin (Edge Biosystems). C57MG cells with tetracycline-repressible Wnt-1 expression were grown in complete medium without puromycin, supplemented with 400 μg/ml G418 (Gibco BRL), 100 μg/ml hygromycin B (Gibco BRL), and 50 ng/ml tetracycline (Korinek et al., Mol. Cell Biol. 18:1248-56 [1998]). For Wnt-1 induction studies, cells were washed with phosphate buffered saline, cultured in tetracycline-free media for 10, 24, 48, 72, and 96 hours and then harvested. A 0-hour control dish was maintained entirely in media containing tetracycline. All dishes were simultaneously harvested and total RNA was extracted for each time point. RT-PCR was carried out with Wnt-1, Stra6, and GAPDH specific primers and probes on 100 ng total RNA from each sample.

Human colon adenocarcinoma cell lines HCT116 and WiDr cells were obtained from the American Type Culture Collection. HCT116 cells were maintained in McCoy's 5A media supplemented with 10% fetal bovine serum (FBS). WiDr cells were maintained in Dulbecco's minimal essential media (DMEM) supplemented with 10% FBS. For retinoic acid induction studies, cells were plated at $10^5$ cells/60 mm dish containing 2.5 ml of medium and allowed to grow for 24 hours. Cells were treated with Vitamin 03, all-*trans*-RA (Spectrum Laboratory Products), or 9-*cis*-RA (Toronto Research Chemicals Inc.) (1 μM final concentration in DMSO) for the indicated times. Control cells were treated with an equal volume of DMSO.

For the treatment of C57MG/Parent cells with Wnt-3a conditioned media, cells were incubated in regular media or conditioned media from L-cells or L-W3A cells in the presence or absence of 1 μM 9-*cis*-RA. Conditioned media was prepared as previously described (Willert et al.,Genes Dev. 13:1768-73 [1999]). At 48 hours, cells were harvested by scraping into PBS containing sodium fluoride and vanadate and quick frozen in liquid nitrogen. RNA was prepared using the RNAeasy kit (Qiagen), including the additional DNaseI treatment on the columns according to manufacturer's instructions.

*Western Blotting*

For the C57MG/Wnt-1 TET cell line, Wnt-1 expression was induced by culturing cells in the absence of tetracycline for 0, 24, 48, or 72 hours. Cells were lysed in Triton X-100 lysis buffer [20 mM tris-HCl (pH 8.0), 137 mM NaCl, 1% Triton X-100, 1 mM EGTA, 10% glycerol, 1.5 mM $MgCl_2$, 1 mM dithiothreitol, 1 mM sodium vanadate, 50 mM sodium fluoride, and complete protease inhibitor cocktail (Boehringer Mannheim) and protein-equivalents were subjected to SDS-PAGE and immunoblotting. Blots were incubated with either 0.2 μg/ml affinity purified rabbit polyclonal antibody against β-catenin (Rubinfeld et al., Science 262:1731-1734 [1993]), 0.1 μg/ml anti-ERK2 monoclonal antibody (Transduction Laboratories), or 1:2000 rabbit polyclonal antisera against RARγ-1 (Affinity Bioreagents). For the WiDr cell line, cells were treated with 1 μM all-*trans*- RA for 48 hours and then lysed in Triton X-100 lysis buffer and processed as indicated above. Blots were incubated with 1:50 anti-Stra6 peptide B monoclonal hybridoma culture supernatant (clone 12F4.2H9.1D5).

*Immunohistochemistry*

WiDr cells were treated with 9-*cis*-retinoic acid or DMSO, then detached and pelleted by low-speed centrifugation. Cell pellets were fixed overnight in 10% neutral buffered formalin, dehydrated, and embedded in paraffin. Immunohistochemistry was performed using anti-Stra6 peptide B monoclonal hybridoma culture supernatant (clone 12F4.2H9.1D5) or nonspecific mouse isotype IgG2A as primary antibodies, followed by detection using avidin-biotin complex method with diaminobenzidine as chromogen (Vectastain Elite Kit, Vector Laboratories) as described previously (Eberhard et al., Am. J. Pathol. 145:640-9 [1994]). Sections were counterstained with hematoxylin.

*Wnt-1 Transgenic Mice*

Transgenic mice that express the Wnt-1 proto-oncogene in the mammary gland typically exhibit hyperplastic lesions and develop neoplasms in this tissue (Tsukamoto et al., Cel/55:619-625 [1988]). Such mice were used in the following experiments.

Results

Easwaran *et al.* previously reported enhanced activation of a synthetic retinoic acid responsive reporter gene when MCF-7 cells were co-transfected with mutant β-catenin and treated with retinoids (Easwaran et al., Curr. Biol. 9: 1415-1418 [1999]). Considering this, together with the original identification of Stra6 as a retinoic acid inducible gene (Bouillet et al., Mech Dev. 63:173-186 [1997]), we asked whether retinoic acid could synergize with Wnt-1 to increase the level of Stra6 in the C57MG cell line. Treatment of parental C57MG cells with either 9-*cis*-RA or all-*trans*-RA (ATRA) for 48 hours significantly increased the level of Stra6 mRNA while DMSO and vitamin D3 had no effect (Figure 17A). As expected, the C57MG/Wnt-1 cells treated with either DMSO or vitamin D3 exhibited enhanced levels of Stra6 mRNA relative to the parent cell line. The level of Stra6 induction by Wnt-1 was comparable to that observed on stimulation of the parental C57MG cells with 9-*cis*-RA. However, 9-*cis*-RA treatment of the C57MG/Wnt-1 cell line induced a further 10-fold increase in Stra6 mRNA relative to either untreated C57MG/Wnt-1 or 9-*cis*-RA treated C57MG parent cells. Similar results were obtained with all-*trans*-RA.

It was possible that potential clonal variations in the C57MG/Wnt-1 cell line, that were unrelated to Wnt-1 expression, accounted for their differential response to retinoic acid relative to parental control cells. To address this, we tested the response of the parental C57MG cells to stimulation by soluble Wnt-3a in the presence or absence of 9-*cis*-RA. Wnt-3a is a Wnt-1 homolog that exhibits transforming properties similar to those of -Wnt-1 and can be produced as a soluble ligand in mouse L-cells. Conditioned media from cultured L-cells expressing Wnt-3a, but not from control L-cells, induced the expression of Stra6 in the C57MG cells (Figure 17B). A slightly higher level of induction was observed on treatment of C57MG cells with 9-*cis*-RA. However, the combination of 9-*cis*-RA and Wnt-3a resulted in levels of Stra6 transcript vastly exceeding that seen with either agent alone.

If the induction of Stra6 in the retinoic acid treated C57MG /Wnt-1cells was potentiated by increased β-catenin levels, then one might expect a similar induction of Stra6 in response to retinoic acid in human colon carcinoma cells containing mutations in either β-catenin or APC. To determine whether this occurs, Stra6 mRNA levels were measured before and after retinoic acid treatment in HCT116 cells, which carry an activating mutation in β-catenin, and in WiDr cells, which have lost both copies of wild-type APC. In both cell lines, a significant increase in Stra6 mRNA levels was seen following treatment with either ATRA or 9-cis-RA compared to DMSO or vitamin D3 (Figure 17C). The activation of the Stra6 gene by ATRA in the HCT116 cell line was confirmed by in situ hybridization (Figure 17D). Induction of the predicted 73kDa Stra6 protein band in WiDr cells treated with ATRA was detected by Western blot analysis with a Stra6 specific monoclonal antibody (Figure 17E). Immunohistochemical analysis of the WiDr cells revealed that the increase in Stra6 protein in response to retinoic acid was localized to the plasma membrane (Fig. 17F).

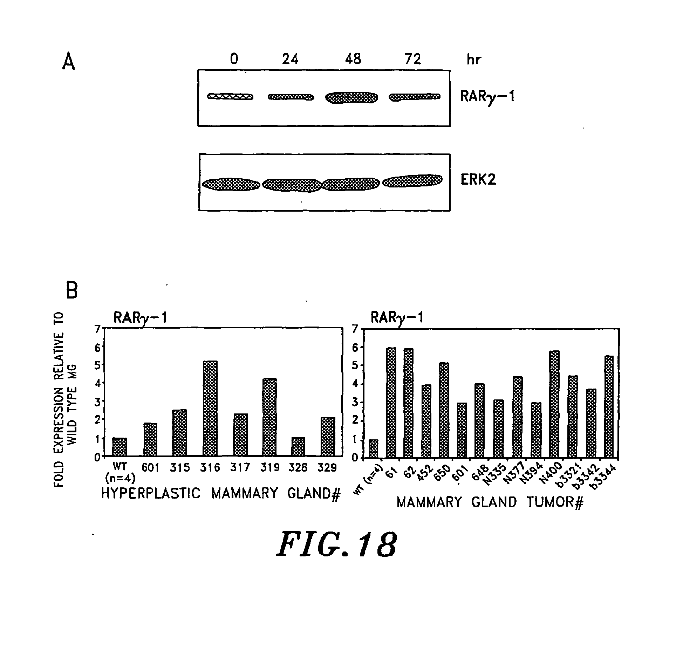

The RARγ gene has been proposed as a target for Wnt signaling in Xenopus embryos, and the induction of Stra6 by retinoids was shown to be dependent upon the presence of this retinoic acid receptor subtype (McGrew et al., Mech. Dev. 87:21-32 [1999]; Taneja et al., Proc. Natl. Acad. Sci. USA 92:7854-8 [1995]). Together, these observations suggest that the synergistic activation of Stra6 by Wnt and retinoids might be due to the activation of RARγ expression by Wnt signaling. To determine whether Wnt-1 signaling had any influence over the levels of RARγ in mammalian cells, we performed Western blots for the receptor on lysates prepared from C57MG cells that conditionally express Wnt-1. Upon Wnt-1 expression, a protein reactive with antibody specific to RARγ-1, and migrating with an apparent molecular mass of 64 kDa, was induced at 24 hours and its expression was increased at 48 hours (Figure 18A). We also analyzed hyperplastic mammary glands and mammary gland tumors obtained from Wnt-1 transgenic mice and detected elevated levels of RARγ mRNA in these tissues relative to normal mammary gland (Figure 18B). The level of RARγ transcript present in equivalent amounts of RNA isolated from 19 adenocarcinomas was assessed by quantitative PCR. Notably, RARγ mRNA expression was increased approximately 2-4 fold in 14 of the 19 (74%) of human colon tumors examined compared to normal human colon tissue (Figure 18C). These results demonstrate that Wnt-1 signaling promotes the expression of RARγ that the receptors are elevated in mouse and human tumors that are driven by the Wnt-1 pathway.

Discussion of Experimental Findings

Gene expression profiling approaches are based on unbiased detection of mRNA transcripts and can therefore lead to unexpected insights into the mechanisms by which gene activation occurs. Here it has been shown that Wnt-1 promoted the induction of the retinoic acid responsive gene Stra6, suggesting a connection between signaling pathways elicited by Wnt and retinoic acid. This connection was further supported by demonstrating a synergistic induction of Stra6 by a combination of Wnt and retinoic acid. There are at least three alternative explanations that could account for this synergy: i) transcription factors directly responsive to Wnt such as the TCF/LEFs might bind to and activate promoter elements in the Stra6 gene; ii) signaling components in the Wnt pathway might directly interact with the appropriate retinoic acid receptor (RAR) and potentiate gene activation mediated by the RAR; or iii) signaling by Wnt-1 could activate expression of the appropriate RAR, which could then sensitize cells to retinoic acid. Although this final proposal is favored because the data presented herein show that Wnt-1 signaling rapidly induces expression of the RARγ receptor, the present invention is not limited by any particular theory or mechanism of action.

Previous work has shown that specific disruption of the RARγ gene greatly reduced induction of the Stra6 gene by retinoids, which was then rescued on re-expression of this receptor (Taneja *et al., supra*). However, it remains possible that mechanisms in addition to the Wnt-1-induced expression of RARγ may also contribute to the observed synergy. The proposal that β-catenin binds to retinoic acid receptors is attractive, but we have not observed any specific association of endogenous RARγ with β-catenin in our cell lines. However, the binding of β-catenin to RARγ, as was shown in vitro (Easwaran *et al.,* 1999, *supra*), might relate to the activation of Stra6 by Wnt-1. The expression pattern of Stra6 in transgenic animals null for RARγ was more widespread than that observed in wild-type littermates, and induction of Stra6 by retinoic acid was enhanced in cells from the null animals compared to controls (Bouillet *et al,* 1997, *supra;* Taneja *et al.,* 1995, *supra).* Thus RARγ might be inhibitory to Stra6 expression, and activation of β-catenin by Wnt-1 could potentially relieve this inhibition if β-catenin inhibited RARγ function.

The synergistic induction of a retinoic acid responsive gene by Wnt-1 signaling implies that human cancers that harbor genetic defects in the Wnt-1 pathway would exhibit overexpression of Stra6. The vast majority of colorectal tumors contain mutations in the genes coding for either the APC tumor suppressor or β-catenin (Polakis, Curr. Opin, Genet. Dev. 9:15-21 [1999]) and, accordingly, we have detected overexpression of the Stra6 transcript in 14 of 14 colorectal tumors relative to matched normal tissue (see Example 12). Activating mutations in β-catenin have also been identified in cancers of the ovary and endometrium, Wilm's kidney tumors and melanomas, demonstrating that defects in Wnt-1 signaling contribute to the progression of these cancers (Kobayashi et al.Jpn.J. Cancer Res. 90:55-9 [1999]; Koesters et al., Cancer Res 59:3880-2 [1999]; Palacios and Hamallo, Cancer Res. 58:1344-7 [1998]; Rimm et al. Am. J. Pathol. 154:325-9 [1999]; Rubinfeld et al. Science 262:1731-1734 [1993]; Wright et al. Int. J. Cancer 82:625-9 [1999]). All four of these human cancers displayed overexpression of Stra6 mRNA as determined by in situ hybridization (see Example 12). Pheochromocytoma, a tumor derived from the adrenal medulla, exhibited extremely high levels of Stra6 mRNA, however, the status of these tumors with respect to mutations in the Wnt-1 signaling pathway has not been reported. It is intriguing that the cell types that give rise to melanomas and pheochromocytomas share in common an embryological derivation from the neural crest. Notably, Wilms' tumor of the kidney, pheochromocytoma, and endometrial carcinomas all arise in organs in which Stra6 is normally expressed. This suggests that tumorigenic signaling, such as that driven by the Wnt-1 pathway, might interact with signals responsible for differentiation, thereby hyper-activating the expression of Stra6.

The human Stra6 protein resides at the cell surface, as determined by the staining of colorectal cancer cells with monoclonal antibodies specific to human Stra6 protein. Moreover, the staining intensity observed at the cell membrane was increased after treatment of cells with retinoic acid. Thus, Stra6 likely encodes a multi-pass transmembrane protein that is localized to the cell surface.

It is noted that ISRI, one of the genes identified in the present screen, has been found to be contiguous with the Stra6 gene. It is believed that any other gene flanking Stra6 and/or ISRL, or any gene located in close proximity to Stra6 and/or ISRL is a good candidate for the methods of the present invention.

**EXAMPLE 14**

Identification of further genes synergistically induced by Wnt-1 and retinoic acid receptor (RAR) signaling

The data disclosed in Example 13 above show that endogenous retinoic acid responsive gene Stra6, was activated upon Wnt-1 expression. Moreover, stra6 was synergistically activated by a combination of retinoic acid and Wnt-1. The synergistic activation of stra6 did not require the overexpression of intracellular signaling components and is therefore mediated entirely by endogenous signaling molecules responsive to their corresponding receptors. Also, human colorectal cancer cell lines that exhibit hyperactive Wnt signaling responded to retinoic acid treatment by producing high levels of Stra6 protein. This suggests that genuine cross talk might occur between the RAR and Wnt signaling pathways under normal physiological or pathological conditions.

That the induction of Stra6 expression by retinoic acid was more robust on an oncogenic background prompted us to consider potential therapeutic applications where this effect could be exploited. As the Stra6 gene codes for a cell surface protein, an appropriate application is immunotherapy in cancer. The proven utility of therapeutic antibodies in treatment of human cancer in the clinic has recently spurned intense activity aimed at the development and refinement of immunotherapeutics. Ideally, these therapies require the presence of cell surface antigens expressed on the cancer cells at significantly higher levels than that present on normal tissues throughout the body. Such criteria for differential expression on tumors relative to normal tissue will obviously limit the number of antigens considered desirable as targets for immunotherapy. Therefore, reagents that selectively enhance the level of antigen expression of cancer cells relative to normal cells are expected to improve the therapeutic index for immunotherapeutics directed against these antigens. To this end we performed a screen to identify further antigens that are preferentially upregulated by the treatment of Wnt-1 transformed cells with retinoic acid.

Materials and Methods

*Cell Culture*

A murine C57MG breast epithelial cell line was engineered to express the Wnt-1 proto-oncogene upon withdrawal of tetracycline from the cell culture media (Korinek et al., Mol Cell Biol 18:1248-56 (1998)). The cells were grown in 10 cm dishes in Dulbecco's Modified Eagle medium supplemented with 10% fetal bovine serum, 2mM L-glutamine, 100 units/ml penicillin/streptomycin, 200 ng/ml tetracycline, 400 μg/ml G418, and 100 μg/ml hygromycin B until approximately 60% confluent. Cells were washed with phosphate buffered saline, cultured in tetracycline-free media for 48 hours either in the presence of 1μM all-*trans*-retinoic acid (ATRA) or an equal volume of DMSO and then harvested. Control cells were maintained entirely in media containing tetracycline either in the presence of 1μM ATRA or DMSO. All dishes were simultaneously harvested and total RNA was extracted. The growth and treatment of cells and the purification of RNA from them was performed 2 independent times. In addition to these experiments, a time course was performed in which the cells grown under the conditions described above were harvested at 24, 48 and 72 hours. The results from the 48 hour time point from this experiment alone with those from the two 48 hour experiments described above comprise the triplicate data set that is presented in the results section.

*Total RNA Extraction*

Cells were lysed in 3.5 ml of lysis buffer (4M guanidine thiocyanate, 25mM sodium citrate, 0.5% N-lauryl sarcosine, 0.7% 2-mercaptoethanol) and layered on 1.5 ml of 5.7M cesium chloride/50mM EDTA (pH8.0) solution. The lysate was spun at 150,000xg overnight. The RNA pellet was dried, resuspended in water, phenol:chloroform extracted, and ethanol precipitated. The RNA was resuspended in water to a final concentration of 1 mg/ml and stored at -70 °C.

*Oligonucleotide Array*

Approximately 10 mg of each RNA sample served as starting material for the preparation of probes required for oligonucleotide array analysis on the miroarray system of Affymetrix (Santa Clara, California). Probes were prepared according to previously described protocols (Wodicka et al. Nat Biotechnol 15:1359-1367 (1997)). Following hybridization, the arrays were washed and stained with streptavidin-phycoerythrin and then scanned with the Gene Array scanner (Agilent Technologies). Default parameters provided in the Affymetrix data analysis software package were applied in determining the signal intensities, referred to as average differences, and the fold differences for the 12,000 gene probe sets represented on the Affymetrix "A" Mu74 chip. The average differences obtained with probes derived from cells expressing Wnt-1 in the absence or presence or ATRA, or treated with ATRA in the absence of Wnt-1 expression, were base-lined against average differences obtained from untreated control cells to generate the fold difference value for each gene call.

*Reverse Transcriptase-PCR (RT-PCR) Analysis*

Confirmation of gene expression was performed using quantitative RT-PCR using Taqman assay reagents in an ABI 7700 Sequence Detector from Perkin-Elmer, Applied Biosystems. RT-PCR reactions consisted of 100 ng RNA, 5 μl 10x Taqman Buffer A, 300 μM of each dNTP, 5 mM $MgCl_2$, 10 units of RNase inhibitor, 12.5 units of MuLV Reverse Transcriptase, 1.25 units of AmpliTaq Gold DNA Polymerase, 200 nM probe, and 500 nM primers. Reaction conditions consisted of reverse transcription at 48°C for 30 minutes, denaturation at 95°C for 10 minutes, and 40 cycles of 95°C for 25 seconds and 65°C for 1 minute. Reaction products were analyzed on 4-20% polyacrylamide gels.

Fold induction was obtained by using the $\Delta\Delta C_t$ method in which all samples are first normalized to the level of glyceraldehyde-3-phosphate dehydrogenase (GAPDH) in each sample. Relative normalized units were then compared between the experimental sample and its control. Mouse GAPDH, Ephrin B1, 4-1BB ligand, and ISLR primer and probe sequences are as follows:

GAPDH forward primer: 5'-TGCACCACCAACTGCTTAG-3' (SEQ ID NO:17);
reverse primer: 5'-GGATGCAGGGATGATGTTC-3' (SEQ ID NO: 18);
probe: 5'-CAGAAGACTGTGGATGGCCCCTC-3' (SEQ ID NO:19).
Ephrin B1 forward primer: 5'- GTAGGCCAGGGCTATTTCTG-3' (SEQ D NO: 20);
reverse primer: 5'-TGTCTCATGAGGGTCCAAAA-3' (SEQ ID NO:21);
probe: 5'-TGGCGCTCTTCTCTCCTCGGT-3' (SEQ ID NO: 22).
41BB ligand forward primer: 5'-TCGCCAAGCTACTGGCTAA-3' (SEQ ID NO: 23);
reverse primer: 5'-CTTGGCTGTGCCAGTTCA-3' (SEQ ID NO: 24);
probe: 5'-AACCAAGCATCGTTGTGCAATACAACTC-3' (SEQ ID NO: 25).
ISLR forward primer: 5'- GCCAGTACAGGATCTGGAAAG-3' (SEQ ID NO: 26);
reverse primer: 5'- CATATCTCATCAGAGAGCATCTAAAA-3' (SEQ ID NO: 27);
probe: 5'- AAG CTT TTA GCC TGC CCA GCC A-3' (SEQ ID NO: 28).

*Western Blotting*

Following indicated treatment, cells were lysed in Triton X-100 lysis buffer (20mM tris-HCl (pH 8.0), 137 mM NaCl, 1 % Triton X-100, 1mM EGTA, 10% glycerol, 1.5mM $MgCl_2$, 1mM dithiothreitol, 1mM sodium vanadate, 50mM sodium fluoride, and Complete protease inhibitor cocktail (Boehringer Mannheim, Mannheim Germany)) and protein-equivalents were subjected to SDS-PAGE and immunoblotting. Blots were incubated with 0.1 μg/ml anti-ERK2 monoclonal antibody (Transduction Laboratories, Lexington, KY), anti-myc tag monoclonal antibody. Blots were developed using the ECL system (Amersham).

*Luciferase Assay*

To determine RAR-dependent transactivation, Cos-7 cells were cotransfected with the indicated expression plasmids and the TREpal luciferase reporter construct (Beyers) using Effectene (Qiagen) transfection reagent per the manufacturer's instructions. Expression plasmids have been described elsewhere. Cells were treated with 1μM ATRA or DMSO control on the day of transfection and harvested 72 hours later by lysis in Triton X-100 lysis buffer. Luciferase activity in 10 μl of lysate was analyzed using a Tropix TR717 microplate luminometer. Activity was normalized to Renilla luciferase activity produced by cotransfection with SV40-Renilla luciferase. Activation of LEF/TCF-dependent transcription was determined by cotransfection with indicated plasmids and Top-tk-luciferase plasmid. Activity was determined as described above.

Results

The murine C57MG breast epithelial cell line undergoes morphological transformation in response to the expression of various Wnt genes (Wong et al., Mol Cell Biol 14:6278-6286 (1994)). A version of this cell line that was engineered to conditionally express Wnt-1 in response to the removal of tetracycline from the culture medium was employed for the gene expression profiling experiments. To identify genes that were preferentially activated by the combination of retinoic acid and Wnt-1 signaling, four different conditions were established for the treatment of cells. As a control, cells were left in medium containing tetracycline plus DMSO, the vehicle for retinoic acid. A second dish of cells was treated with 100nM all-*trans*-retinoic acid (ATRA) in the presence of tetracycline, while a third dish of cells received DMSO control and the tetracycline was removed to activate expression of Wnt-1. Finally, a fourth dish of cells was treated with ATRA and the tetracycline was removed. Following a forty eight hour incubation period, cells were harvested and RNA was extracted and purified. Probes synthesized from the RNA was hybridized to the Affymetrix Mouse Gene Chip "A" Mu74 containing 12,000 oligonucleotide probe sets. The experiment, as initiated from the growth and treatment of cells, was performed three independent times. Data is presented for mRNA transcripts that underwent at least a two-fold increase or a 50% decrease relative to the untreated cells in all three experiments. In addition, a time course was performed in which RNA was collected from treated cells at 24, 48 and 72 hours. These data are included only for select genes.

Treatment of the C57MG cells with retinoic acid alone resulted in the robust activation of numerous genes (Table 1).

**Table 1**

| Up regulated by retinoic acid | | | |
|---|---|---|---|
| Gene | Acc. # | Fold Change | Std. Dev |
| thioether S-methyltransferase | M88694 | 154.67 | 204.10 |
| Casein kappa | M10114 | 33.67 | 35.64 |
| angiotensinogen | AF045887 | 27.63 | 17.34 |
| retinal short-chain dehydrogenase/reductase | X95281 | 25.67 | 28.78 |
| Ribonuclease 1 | X60103 | 18.60 | 10.87 |
| FGF-1 | M30641 | 18.43 | 16.46 |
| LPS-binding protein | X99347 | 15.10 | 6.74 |
| cytochrome P450, 2f2 | M77497 | 10.77 | 8.30 |
| Death-asociated protein kinase 2 | AB018002 | 10.67 | 10.87 |
| Decorin | X53929 | 8.07 | 4.46 |
| 11beta-hydroxysteroid dehydrogenase/carbonyl reductase | X93202 | 7.97 | 4.02 |
| autotaxin | AW122933 | 7.90 | 5.72 |
| type 2 deiodinase | AF096875 | 7.63 | 5.71 |
| v-erb-a homolog-like 3 | X74134 | 7.00 | 2.65 |
| aldehyde dehydrogenase 3 | AF033034 | 6.43 | 3.07 |
| 3-O-sulfotransferase | AF019385 | 6.13 | 3.97 |
| ficolin-A | AB007813 | 5.87 | 3.69 |
| lysosomal acid lipase | Z31689 | 5.60 | 2.46 |
| osteoglycin | D31951 | 4.43 | 2.15 |
| ATP-binding cassette 1, sub-family A, member 1 (Abca1) gene | AI845514 | 4.40 | 2.12 |
| kallikrein | V00829 | 4.33 | 1.22 |
| Cathepsin B | AI851255 | 3.90 | 1.21 |
| Complement component C3 | K02782 | 3.83 | 2.67 |
| Procollogen, type IV, alpha 5 | Z35168 | 3.53 | 1.01 |
| diacylglycerol acyltransferase | AF078752 | 3.40 | 1.45 |
| chaperonin containing TCP-1 epsilon subunit | AW049373 | 3.33 | 1.88 |

(continued)

| Up regulated by retinoic acid | | | |
|---|---|---|---|
| Gene | Acc. # | Fold Change | Std. Dev |
| VEGF C | U73620 | 3.10 | 0.95 |
| Glutathione-S-transferase, alpha 1 | L06047 | 3.10 | 0.80 |
| ceruloplasmin | U49430 | 3.03 | 1.30 |
| sorting nexin 10 | A1746846 | 2.90 | 1.15 |
| receptor interacting protein 140 | AF053062 | 2.83 | 1.62 |
| megakaryocyte potentiating factor | D86370 | 2.73 | 1.35 |
| annexin VI | X13460 | 2.67 | 1.50 |
| MG87 | AW124268 | 2.63 | 0.75 |
| prefoldin | AB023957 | 2.60 | 0.44 |
| myristoylated alanine rich PKC substrate | M60474 | 2.60 | 0.78 |
| Rho quanine nucleotide exchange factor | AJ010045 | 2.43 | 0.55 |
| solute carrier family 12. member 2 | U13174 | 2.40 | 0.61 |
| quanylate nucleotide binding protein 3 (Gbp3) | AW047476 | 2.37 | 0.58 |
| isovaleryl CoA dehydrogenase (Ivd) | AW047743 | 2.37 | 1.16 |
| alpha-2,3-sialyltransferase | D28941 | 2.37 | 0.35 |
| Retinoblastoma-1 | M26391 | 2.37 | 0.40 |
| Caspase 3 | U54803 | 2.37 | 1.07 |
| Acyl-CoA synthetase | AA619207 | 2.30 | 0.36 |
| Lysosomal membrane glycoprotein 2 | M32017 | 2.30 | 0.56 |
| TM6P1 | AA881018 | 2.23 | 0.47 |
| Mad4 | U32395 | 2.17 | 0.21 |
| interleukin 1-beta converting enzyme | L28095 | 2.00 | 0.26 |
| Up regulated by wnt | | | |
| Gene | Acc. # | Fold Change | Std. Dev |
| autotaxin-t | AW122933 | 4.27 | 1.52 |
| 4-1bb Ligand | L15435 | 3.53 | 1.21 |
| prefoldin (MM-1) | AB023957 | 3.00 | 0.26 |
| Semaphorin E | X85994 | 2.90 | 0.90 |
| BTEB2 (IKLF) | AA611766 | 2.80 | 0.26 |
| G28K RHO-RAS family GTP-binding protein | AW121294 | 2.73 | 0.61 |
| SSG-1 steroid sensetive gene-1 protein | AW122012 | 2.57 | 1.10 |
| TCF-1 | AI019193 | 2.57 | 0.81 |
| GADD45 gamma | AF055638 | 2.43 | 0.25 |
| Frizzled-2 | AW123618 | 2.23 | 0.23 |
| ets-2 | J04103 | 2.20 | 0.40 |
| TSA-1 | U47737 | 2.13 | 1.01 |

(continued)

| Up regulated by wnt | | | |
|---|---|---|---|
| Gene | Acc. # | Fold Change | Std. Dev |
| adenosine kinase | AW121801 | 1.87 | 0.35 |
| Drown regulated by wnt | | | |
| Gene | Acc. # | Fold Change | Std. Dev |
| Contactin 1 | X14943 | -7.17 | 4.65 |
| Protaglandin E receptor, EP4 | D13458 | -5.40 | 2.69 |
| Orosomucold 1 | M27008 | -3.93 | 2.51 |
| calpain-like protease | Y12582 | -3.83 | 1.72 |
| RGS2 | U67187 | -3.30 | 1.30 |
| Small Inducible cytokine A7 | X70058 | -3.13 | 1.06 |
| Lymphocyte antigen 84 | D13695 | -2.97 | 1.68 |
| complement factor H-related protein | M29010 | -2.50 | 0.79 |
| Small Inducible cytokine B subfamily, member 5 | U27267 | -2.50 | 0.52 |
| Complement component factor h | M12660 | -2.47 | 1.61 |
| Ceruloplasmin | U49430 | -2.30 | 0.53 |
| embigin | AW061330 | -2.13 | 0.31 |
| Prostaglandin F receptor | D17433 | -2.03 | 0.49 |

Some of these genes, such as decorin (Pearson, D. and Sasse, J., J Biol Chem 267:25364-25370 (1992)), COUPTF-1 (Clotman et al., Neurotoxicol Teratol 20:591-599 (1998)), 11-beta-hydroxysteroid dehydrogenase (Tremblay et al., Biol Reprod 60:541-545 (1999)), 3-0-sulfotransferase (Zhang et al., J Biol Chem 273:27998-28003 (1998)),Abca 1 (Wade, D. P. and Owen, J. S., Lancet 357:161-163 (2001)) and ceruloplasmin (Koj et al., Biol Chem Hoppe Seyler 374: 193-201 (1993)) have been reported previously to be upregulated by retinoic acid in other cell types. The identification of these genes in our screen demonstrates that the C57MG cells respond appropriately to retinoic acid and suggests that common endpoints for retinoic acid signaling exist in diverse cell types. Additional genes induced by retinoic acid, such as retinal short-chain dehydrogenase and aldehyde dehydrogenase 3, code for enzymes that are involved in the metabolism of retinoids and might, therefore, represent feedback or feed-forward responses to retinoic acid itself (Duester, Eur J Biochem 267:4315-4324 (2000)). Finally, several thergenes that were induced by retinoic acid, such as autotaxin, thioether S-methyl transferase and angiotensiogen, bear no obvious connection to retinoic acid signaling or metabolism and have not been previously reported to be activated by receptors responsive to retinoic acid.

To identify genes induced by Wnt-1, tetracyline was removed from the cells in the absence or retinoic acid. Under these conditions, 13 transcripts were identified that were expressed at 2-fold or higher levels relative to control cells in all three experiments. This is likely an underestimate of the number of genes that are actually induced by Wnt-1 signaling in these cells. Indeed, numerous additional genes, including cyclin D1, which is a target of Wnt-1 signaling (Tetsu, 0. and McCormick, F., Nature 398:422-426 (1999)), were identified in only 2 of the 3 experiments or fell short of the 2-fold cutoff. Two of the genes identified in our screen, the TCF1 and BTEB2 transcription factors, were previously reported to be targets of Wnt signaling (Ziemer et al, Mol Cell Biol 21:562-574 (2001); Roose et al., Science 285: 1923-1926 (1999)).

Splice variants of TCF1 that bind DNA but lack β-catenin binding sites have provide negative feedback in Wnt signaling and thus function as tumor suppressors. In addition, one of the Wnt receptors, frizzled-2, was also upregulated by Wnt signaling in the C57MG cell line.

Some of the genes upregulated by Wnt-1, such as GADD45 gamma and 4-1BB ligand, might represent a response to inappropriate growth signaling due to the overexpression of Wnt-1. The 4-1BB ligand (CD137) is a TNF superfamily member that is expressed on various human carcinoma cell lines and stimulates T-cell responses in tumor rejection. The activation of GA0045γ/CR6, suggests that Wnt signaling promotes errors affecting the fidelity of DNA replication. Indeed, p53 mutations cooperate with Wnt-1 overexpression to produce tumors in mice that exhibit chromosomal instability (Donehower et al., Genes Dev 9:882-895 (1995)). Two additional genes upregulated by Wnt-1, semaphorin E and autotaxin, have been implicated in human tumor progression as factors contributing to the motility and metastatic spread of cancer cells (Martin-Satue, M. and Blanco, J., J Surg Oncol 72:18-23 (1999); Yamada et al., Proc Natl Acad Sci USA 94:14713-14718 (1997)); Nam et al., Oncogene 19:241-247 (2000)).

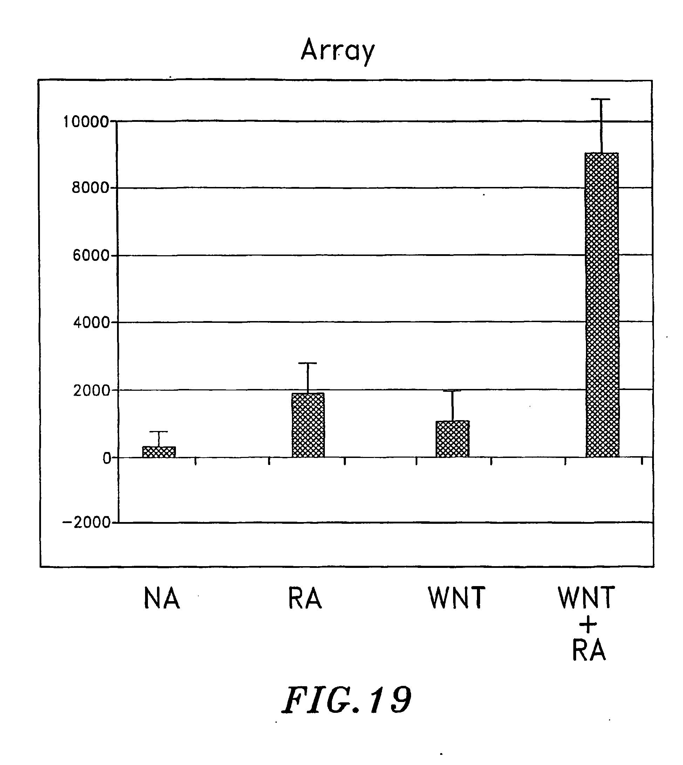

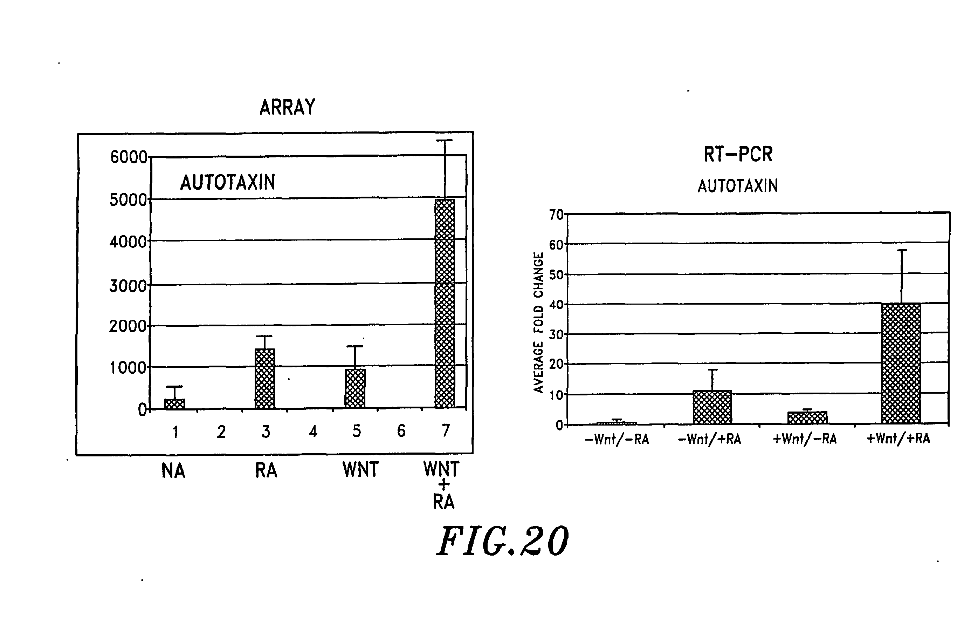

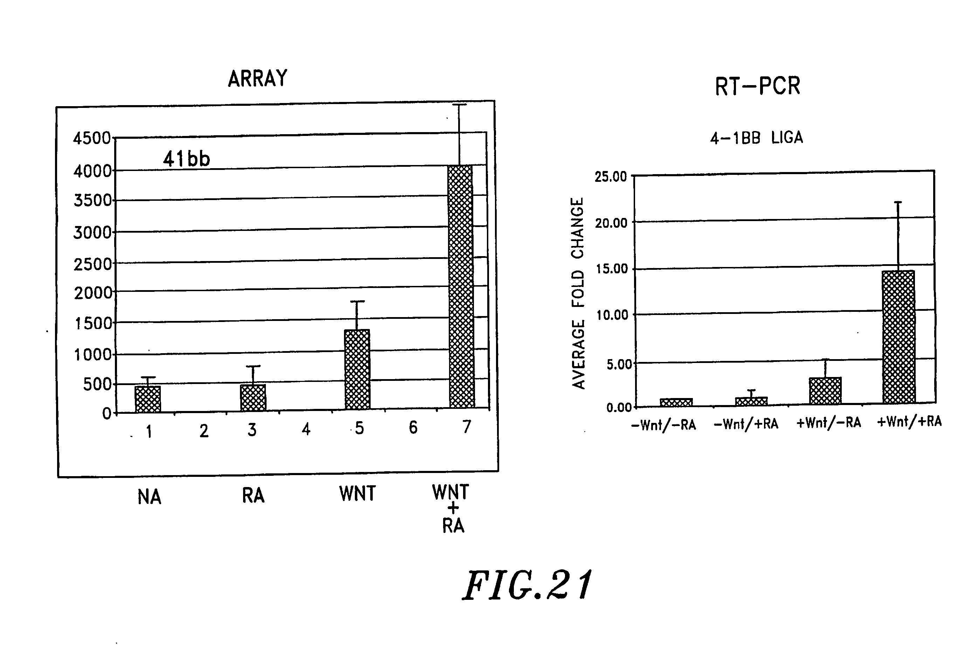

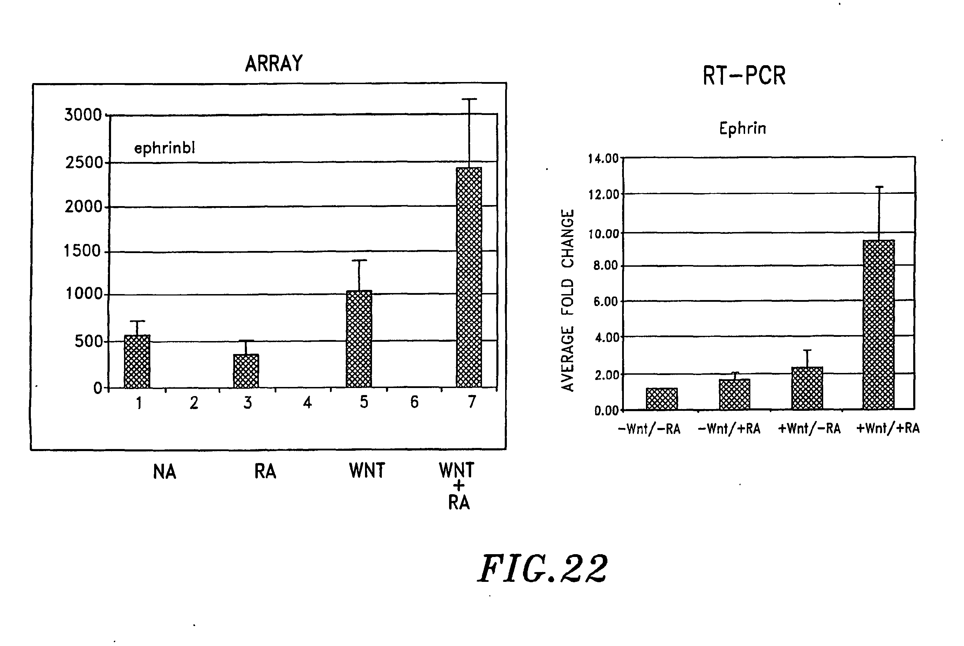

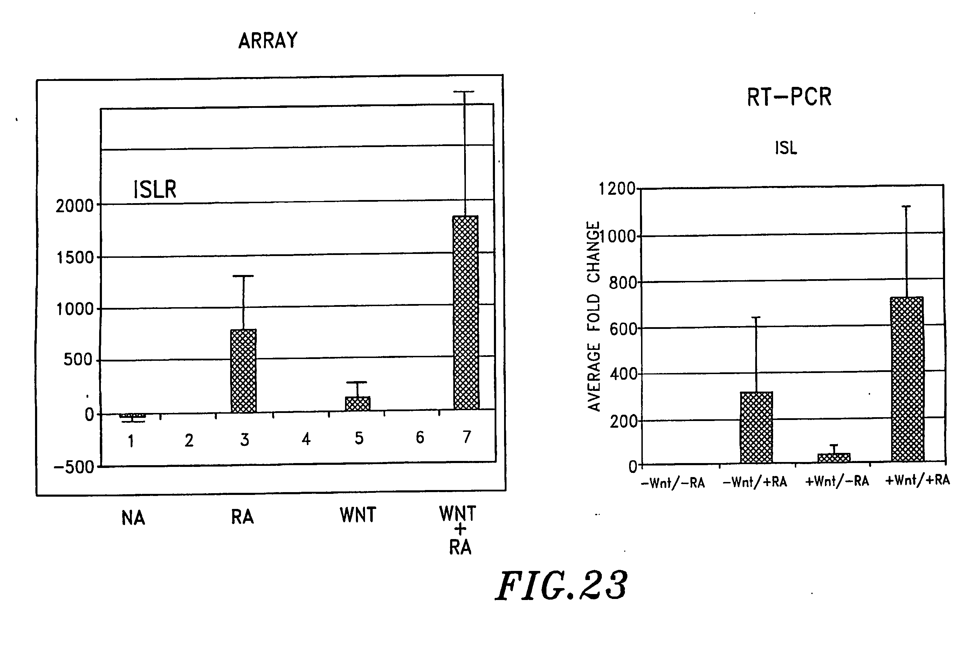

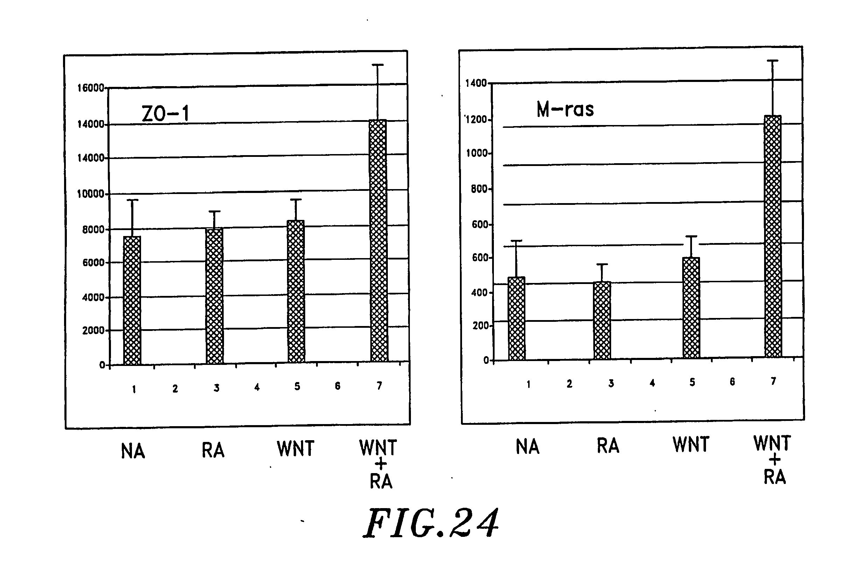

As described above, we identified stra6 as a gene activated by Wnt-1 signaling. Stra6 was originally identified in a screen designed to detect mRNA transcripts induced by retinoic acid receptor signaling (Bouillet et al., Dev Biol 170:420-433 (1995)). Although we found that retinoic acid induced the expression of stra6, we also demonstrated its synergistic activation by a combination of Wnt-1 and retinoic acid (see Example 13 above). Therefore, we were interested in whether genes in addition to stra6 could be activated in a like fashion. Consistent with our previous findings, stra6 was activated by either retinoic acid or Wnt-1, while the combination of these agents resulted in expression levels greatly exceeding that observed with either agent alone (Figure 19). A similar pattern of expression was observed for autotaxin, which underwent modest activation in the presence of retinoic acid or Wnt-1 alone, but more robust induction in response to both stimuli (Figure 20). The synergistic activation of autotaxin was confirmed by performing RT-PCR analysis on RNA extracted from C57MG cells subjected to same treatment as those utilized in the expression profiling screen. The 41BB ligand and ephrin b1 were also synergistically induced by Wnt-1 and retinoic acid, although retinoic acid alone appeared not to have a significant affect on their expression (Figures 21 and 22). The synergistic activation of these two genes was also confirmed by RT-PCR. In the case of ephrin b1, antibody was commercially available, and was used to demonstrate synergistic activation at the level of protein expression (Figure 22). In contrast to the activation of ephrin b1 and 41BB ligand, the ISLR gene was responsive to retinoic acid, but not to Wnt, while the combination of both agents resulted in activation that exceeded that of retinoic acid alone (Figure 23). Some genes, such as M-ras and the tight junction protein Z0-1, appeared not to respond significantly to either retinoic acid or Wnt-1 alone, but were only activated by combined treatment (Figure 24).

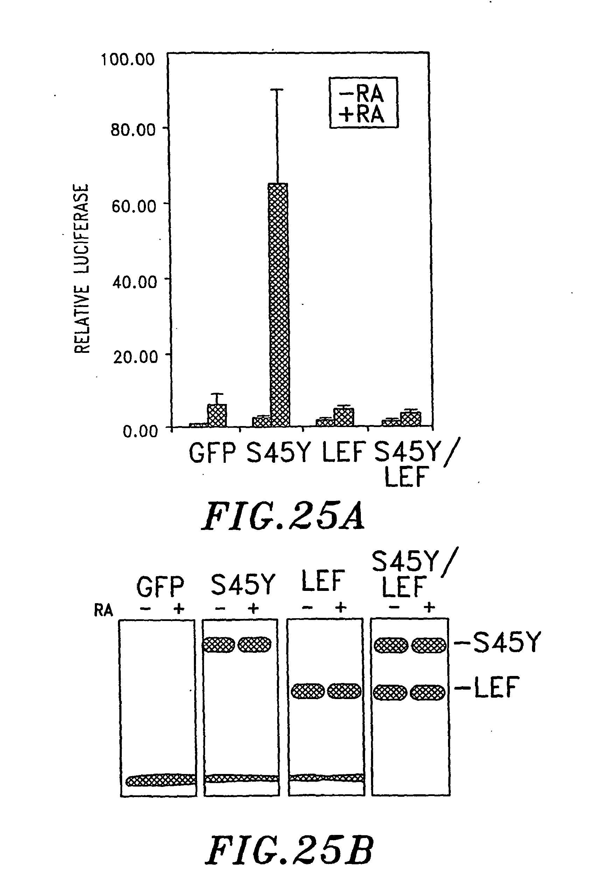

It is apparent that certain genes undergo synergistic activation by a combination of retinoic acid and Wnt-1, although the signaling mechanism that mediates this effect has not been clearly defined. We have found increased levels of RARg protein in cells activated by Wnt-1 expression (data not dislosed). Therefore, cells activated by Wnt might be more sensitive to retinoic acid due to the higher levels of RARg. However, the synergistic activation of stra6 by Wnt-1 and retinoic acid preceded any observable increase in RARg suggesting that additional mechanisms were at work. Moreover, the ability of Wnt-1 alone to activate stra6 was inhibited by a pan-antagonist of RAR signaling. This suggests that even in the absence of exogenously added RA, Wnt-1 was dependent upon retinoic RAR signaling for the induction of stra6. Therefore, it is conceivable that Wnt facilitates RAR signaling in a manner that does not involve the canonical Wnt-1 pathway in which β-catenin interacts the TCF/LEF transcription factors. To examine this we overexpressed an oncogenic mutant of β-catenin and measured the activation of an RAR response element (RARE) in the presence and absence of overexpressed LEF. Expression of β-catenin activated the RARE as determined by the increased production of luciferase (Figure 25A). However, coexpression of β-catenin did not potentiate RARE activity but, conversely, inhibited its activation by β-catenin. This was not the case for a LEF responsive element TopFlash, which was activated further upon coexpression of LEF with b-catenin (Figure 25B). The results indicate that β-catenin is involved in the activation of some retinoic acid responsive genes in a manner that does not involve LEF. The ability of LEF to inhibit the activation of RARE by β-catenin is most likely be due to competitive binding as a b-catenin binding site on LEF was required for this effect (data not shown). This result suggests that LEF binds to b-catenin in a manner that precludes its interaction with RAR signaling components and thereby prevents it from potentiating RAR signaling.

## EXAMPLE 15

Induction of Stra6 in Explanted Mammary Gland Tumors

The results with the C57MG cell line presented in Example 14 above, suggest that tumors driven by Wnt signaling are likely to respond to retinoic acid by expressing high levels of mRNA transcripts that are synergistically induced by Wnt and retinoic acid. To examine this, mammary tumors derived from Wnt-1 transgenic mice prepared as described in Example 13, were transplanted to the mammary fad pad of naïve mice that were subsequently administered retinoic acid. Administration of retinoic acid (ATRA) but not vehicle control resulted in the upregulation Stra6 mRNA in the transplanted mouse mammary tumors but did not have a significant effect on Stra6 expression in normal mammary tissue.

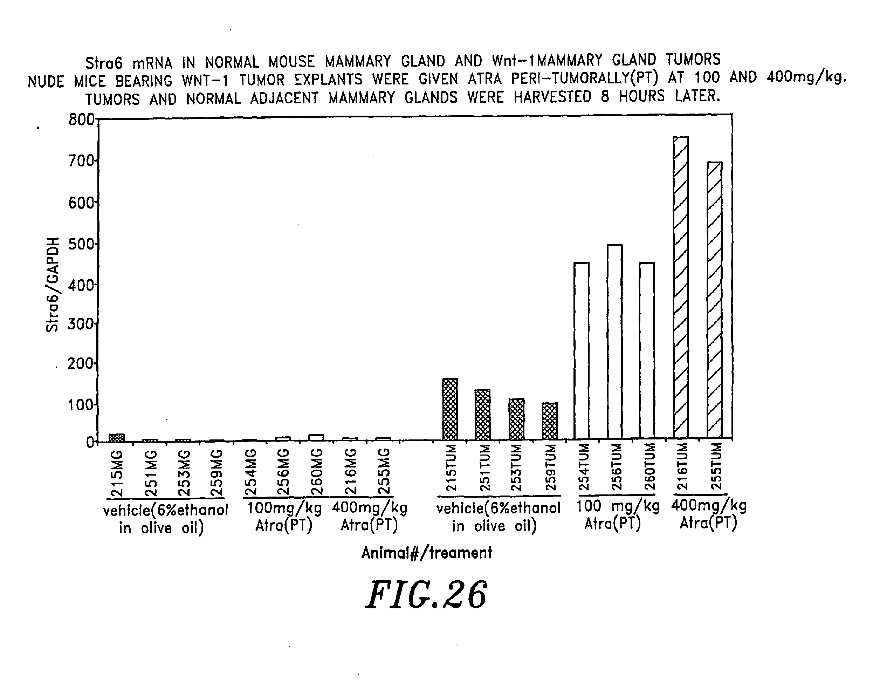

The data presented in Figure 26 shows the effect of induction of Stra6 following peritumor injection of 100mg/kg and 400 mg/kg (about 10 mg total/25 gram mouse) *all-trans* retinoic acid. We have also found that a control gene, retinal DHR, which is responsive to retinoic acid alone, was induced in normal mammary tissue (data not shown). In Figure 26 "Tum" stands for transplanted mouse mammary tumor, "MG" stands for normal mammary tissue, "PT" stands for peritumor, and the numbers immediately preceding the "MG" and "Tum" are used to designate the particular animals from which samples were taken. The data presented are mRNA expression levels normalized to the housekeeping gene, GAPDH. In particular, standard curves were used to determine relative levels of expression for each gene of interest as well as the GAPDH housekeeping gene for each sample analyzed. Relative normalized units were obtained by dividing the mRNA level for the gene of interest by the GAPDH mRNA level. Tumors and nomal adjacent mammary glands were harvested 8 hours after treatment.

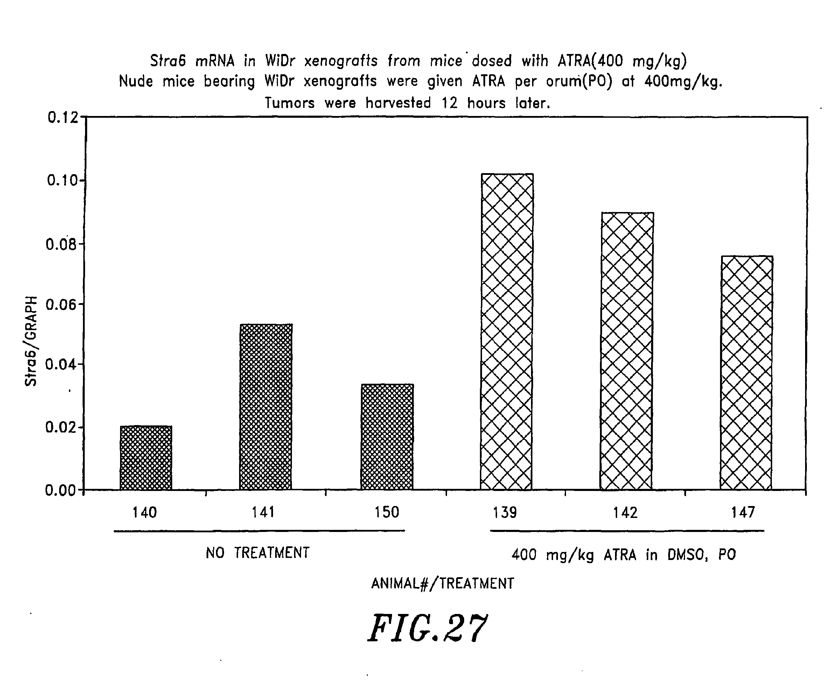

Figure 27 shows that administration of ATRA to nude mice bearing WiDr xenografts also induces Stra6. Specifially the mice were given ATRA per orum (PO) at 400 mg/kg. The tumors were harvested 12 hours following treatment. In this experiment oral administration of ATRA (PO) was tested as well as peritumoral administration (data not shown). The data presented in Figure 27 show that oral administration of 400 mg/kg of ATRA selectively upregulates Stra6 mRNA in WiDr xenografts as compared to untreated control. mRNA expression levels were normalized as discussed above.

Deposit of Material

The following materials have been deposited with the American Type Culture Collection, 10801 University Blvd., Manassas, VA 20110-2209, USA (ATCC):

| Material | ATCC Dep. No. | Deposit Date |
|---|---|---|
| DNA14B380-2827 | PTA-1181 | January 11, 2000 |
| DNA148389-2827-1 | PTA-1405 | February 23, 2000 |

These deposits were made under the provisions of the Budapest Treaty on the International Recognition of the Deposit of Microorganisms for the Purpose of Patent Procedure and the Regulations thereunder (Budapest Treaty). This assures maintenance of a viable culture of the deposit for 30 years from the date of deposit. The deposit will be made available by ATCC under the terms of the Budapest Treaty, and subject to an agreement between Genentech, Inc. and ATCC, which assures permanent and unrestricted availability of the progeny of the culture of the deposit to the public upon issuance of the pertinent U.S. patent or upon laying open to the public of any U.S. or foreign patent application, whichever comes first, and assures availability of the progeny to one determined by the U.S. Commissioner of Patents and Trademarks to be entitled thereto according to 35 U.S.C. § 122 and the Commissioner's rules pursuant thereto (including 37 C.F.R. § 1.14 with particular reference to 886 OG 638).

The assignee of the present application has agreed that if a culture of the materials on deposit should die or be lost or destroyed when cultivated under suitable conditions, the materials will be promptly replaced on notification with another of the same. Availability of the deposited material is not to be construed as a license to practice the invention in contravention of the rights granted under the authority of any government in accordance with its patent laws.

The foregoing written specification is considered to be sufficient to enable one skilled in the art to practice the invention. The present invention is not to be limited in scope by the construct deposited, since the deposited embodiment is intended as a single illustration of certain aspects of the invention. The deposit of material herein does not constitute an admission that the written description herein contained is inadequate to enable the practice of any aspect of the invention, including the best mode thereof, nor is it to be construed as limiting the scope of the claims to the specific illustrations that it represents.

## Claims

1. Use of an anti-tumour agent for the manufacture of a medicament for the treatment in combination with a retinoid of a tumor of a human cancer selected from ovarian cancer, endometrial cancer, Wilm's kidney tumour, colon cancer, breast cancer, prostate cancer, gastric cancer, lung cancer, hepatocellular cancer, melanoma or pheochromocytoma , wherein said anti-tumour agent is an antibody, an antisense molecule, a ribozyme or a triple-helix DNA that binds to a target protein selected from the group consisting of autotaxin, 4-1BB ligand, ephrin b1, ISLR, M-ras, and ZO-1 or to a nucleic acid encoding said target protein.

2. Use of a retinoid and an anti-tumour agent for the manufacture of a medicament for the treatment of a tumor of a human cancer selected from ovarian cancer, endometrial cancer, Wilm's kidney tumour, colon cancer, breast cancer, prostate cancer, gastric cancer, lung cancer, hepatocellular cancer, melanoma or pheochromocytoma, wherein said anti-tumour agent is an antibody, an antisense molecule, a ribozyme or a triple-helix DNA that binds to a target protein selected from the group consisting of autotaxin, 4- 1BB ligand, ephrin b1, ISLR, M-ras, and ZO-1 or to a nucleic acid encoding said target protein.

3. The use of any one of the preceding claims, wherein said protein is a cell surface protein.

4. The use of claim 1 wherein said protein is selected from the group consisting of 4-1BB ligand, ephrin b1, ISLR, M-ras, and ZO-1.

5. The use of claim 4, wherein said protein is selected from the group consisting of 4-1BB ligand, ephrin b1 and ISLR.

6. The use of claim 5 wherein said protein is selected from the group consisting of 4-1BB ligand and ephrin b1.

7. The use of any one of the preceding claims wherein said retinoid is to be administered prior to the administration of said anti-tumor agent.

8. The use of any one of claims 1 to 6 wherein said retinoid is to be administered concurrently with the administration of said anti-tumor agent.

9. The use of any one of claims 1 to 6 wherein said retinoid is to be administered following the administration of said anti-tumor agent.

10. The use of any one of the preceding claims wherein said retinoid is retinoic acid.

11. The use according to claim 1, wherein said antibody is an antibody fragment.

12. The use according to claim 11, wherein said antibody fragment is selected from the group consisting of Fab, Fab', $F(ab')_2$, and Fv fragments, diabodies, single-chain antibody molecules, and multispecific antibodies formed from antibody fragments.

**13.** The use of any one of claims 1 to 12, wherein the antibody is a monoclonal, chimeric, human or humanized antibody.

**14.** The use of any one of claims 1 to 13, wherein the antibody is conjugated to a cytotoxic agent.

**15.** The use according to claim 14, wherein the cytotoxic agent is selected from the group consisting of a chemotherapeutic agent, a maytansinoid, a celicheamicin, an anti folate, an enzymatically active toxin and a radioactive isotope.

**16.** The use according to claim 14, wherein the cytotoxic agent is a maytansinoid.

**17.** A method for diagnosing a tumor of a human cancer selected from ovarian cancer, endometrial cancer, Wilm's kidney tumour, colon cancer, breast cancer, prostate cancer, gastric cancer, lung cancer, hepatocellular cancer, melanoma or pheochromocytoma comprising:

a) contacting a biological sample obtained from said patient with retinoic acid; and
b) detecting enhanced expression of a target protein in the biological sample treated with the retinoid relative to a normal tissue, wherein the target protein is selected from the group consisting of autotaxin, 4-1BB ligand, ephrin b1, ISLR, M-ras, and ZO-1.

**18.** The method of claim 17, wherein said protein is a cell surface protein.

**19.** The method of claim 17, wherein said protein is selected from the group consisting of 4-1BB ligand, ephrin b1, ISLR; M-ras and ZO-1.

**20.** The method of any one of claims 17 to 19, wherein said detecting enhanced expression in the biological sample comprises detecting expression of the protein with an antibody or antibody fragment.

**21.** The method of claim 20, wherein said antibody fragment is selected from the group consisting of Fab, Fab', $F(ab')_2$, and Fv fragments, diabodies, single-chain antibody molecules and multispecific antibodies formed from antibody fragments.

**22.** The method of any one of claims 17 to 21, wherein the antibody is detectably labelled.

**23.** The method of any one of claims 17 to 22, wherein said detecting enhanced expression of a target protein in the biological sample comprises detecting expression of the nucleic acid encoding said target protein using a probe or primers.

**24.** A pharmaceutical composition comprising a retinoid and an anti-tumour agent, wherein the anti-tumour agent is an antibody, an antisense molecule, a ribozyme or a triple-helix DNA that binds to a target protein selected from the group consisting of autotaxin, 4-1BB ligand, ephrin b1, ISLR, M-ras, and ZO-1 or to a nucleic acid encoding said target protein.

**25.** The pharmaceutical composition of claim 24, wherein the target protein is a cell-surface protein.

**26.** The pharmaceutical composition of claim 24, wherein said protein is selected from the group consisting of 4-1BB ligand, ephrin b1, ISLR, M-ras and ZO-1.

**27.** The pharmaceutical composition of any one of claims 24 to 26, wherein the anti-tumour agent is an antibody fragment selected from the group consisting of Fab, Fab', $F(ab')_2$ and Fv fragments, diabodies, single-chain antibody molecules, and multispecific antibodies formed from antibody fragments.

**28.** The pharmaceutical composition of any one of claims 24 to 27, wherein the anti-tumour agent is a monoclonal, human or humanized antibody or antibody fragment.

**29.** The pharmaceutical composition of claim 28, wherein the antibody is conjugated to a cytotoxic agent.

**30.** The pharmaceutical composition of claim 29, wherein the cytotoxic agent is selected from the group consisting of a chemotherapeutic agent, a maytansinoid, a celicheamicin, an anti folate, an enzymatically active toxin and a radioactive isotope.

31. The pharmaceutical composition of claim 30, wherein the cytotoxic agent is a maytansinoid.

32. An anti-tumour agent for use in the treatment in combination with a retinoid of a tumor of a human cancer selected from ovarian cancer, endometrial cancer, Wilm's kidney tumour, colon cancer, breast cancer, prostate cancer, gastric cancer, lung cancer, hepatocellular cancer, melanoma or pheochromocytoma, wherein said anti-tumour agent is an antibody, an antisense molecule, a ribozyme or a triple-helix DNA that binds to a target protein selected from the group consisting of autotaxin, 4-1BB ligand, ephrin b1, ISLR, M-ras, and ZO-1 or to a nucleic acid encoding said target protein.

## Patentansprüche

1. Verwendung eines Antitumormittels zur Herstellung eines Medikaments zur Behandlung in Kombination mit einem Retinoid eines Tumors eines aus Eierstockkrebs, Endometriumkrebs, Wilms-Nierentumor, Kolonkrebs, Brustkrebs, Prostatakrebs, Magenkrebs, Lungenkrebs, Leberzellenkrebs, Melanom oder Phäochromozytom ausgewählten menschlichen Krebses, worin das Antitumormittel ein Antikörper, ein Antisensemolekül, ein Ribozym oder eine Tripelhelix-DNA ist, der/die/das an ein aus der aus Autotaxin, 4-1BB-Ligand, Ephrin b1, ISLR, M-ras und ZO-1 bestehenden Gruppe ausgewähltes Zielprotein oder an eine für dieses Zielprotein kodierende Nucleinsäure bindet.

2. Verwendung eines Retinoids und eines Antitumormittels zur Herstellung eines Medikaments zur Behandlung eines Tumors eines aus Eierstockkrebs, Endometriumkrebs, Wilms-Nierentumor, Kolonkrebs, Brustkrebs, Prostatakrebs, Magenkrebs, Lungenkrebs, Leberzellenkrebs, Melanom oder Phäochromozytom ausgewählten menschlichen Krebses, worin das Antitumormittel ein Antikörper, ein Antisensemolekül, ein Ribozym oder eine Tripelhelix-DNA ist, der/die/das an ein aus der aus Autotaxin, 4-1BB-Ligand, Ephrin b1, ISLR, M-ras und ZO-1 bestehenden Gruppe ausgewähltes Zielprotein oder an eine für dieses Zielprotein kodierende Nucleinsäure bindet.

3. Verwendung nach einem der vorangegangenen Ansprüche, worin das Protein ein Zelloberflächenprotein ist.

4. Verwendung nach Anspruch 1, worin das Protein aus der aus 4-1B8-Ligand, Ephrin b1, ISLR, M-ras und ZO-1 bestehenden Gruppe ausgewählt ist.

5. Verwendung nach Anspruch 4, worin das Protein aus der aus 4-1BB-Ligand, Ephrin b1 und ISLR bestehenden Gruppe ausgewählt ist.

6. Verwendung nach Anspruch 5, worin das Protein aus der aus 4-1BB-Ligand und Ephrin b1 bestehenden Gruppe ausgewählt ist.

7. Verwendung nach einem der vorangegangenen Ansprüche, worin das Retinoid vor der Verabreichung des Antitumormittels zu verabreichen ist.

8. Verwendung nach einem der Ansprüche 1 bis 6, worin das Retinoid gleichzeitig mit der Verabreichung des Antitumormittels zu verabreichen ist.

9. Verwendung nach einem der Ansprüche 1 bis 6, worin das Retinoid nach der Verabreichung des Antitumormittels zu verabreichen ist.

10. Verwendung nach einem der vorangegangenen Ansprüche, worin das Retinoid Retinsäure ist.

11. Verwendung nach Anspruch 1, worin der Antikörper ein Antikörperfragment ist.

12. Verwendung nach Anspruch 11, worin das Antikörperfragment aus der aus Fab-, Fab'-, $F(ab')_2$- und Fv-Fragmenten, Diabodies, einkettigen Antikörpermolekülen und aus Antikörperfragmenten gebildeten multispezifischen Antikörpern bestehenden Gruppe ausgewählt ist.

13. Verwendung nach einem der Ansprüche 1 bis 12, worin der Antikörper ein monoklonaler, chimärer, menschlicher oder humanisierter Antikörper ist.

14. Verwendung nach einem der Ansprüche 1 bis 13, worin der Antikörper an ein zytotoxisches Mittel konjugiert ist.

**15.** Verwendung nach Anspruch 14, worin das zytotoxische Mittel aus der aus einem chemotherapeutischen Mittel, einem Maytansinoid, einem Calicheamicin, einem Antifolat, einem enzymatisch aktiven Toxin und einem radioaktiven Isotop bestehenden Gruppe ausgewählt ist.

**16.** Verwendung nach Anspruch 14, worin das zytotoxische Mittel ein Maytansinoid ist.

**17.** Verfahren zum Diagnostizieren eines Tumors eines aus Eierstockkrebs, Endometriumkrebs, Wilms-Nierentumor, Kolonkrebs, Brustkrebs, Prostatakrebs, Magenkrebs, Lungenkrebs, Leberzellenkrebs, Melanom oder Phäochromozytom ausgewählten menschlichen Krebses, umfassend:

a) das Kontaktieren einer aus dem Patienten erhaltenen biologischen Probe mit Retinsäure; und
b) das Detektieren der verstärkten Expression eines Zielproteins in der mit dem Retinoid behandelten biologischen Probe im Vergleich zu einem normalen Gewebe, worin das Zielprotein aus der aus Autotaxin, 4-1BB-Ligand, Ephrin b1, ISLR, M-ras und ZO-1 bestehenden Gruppe ausgewählt ist.

**18.** Verfahren nach Anspruch 17, worin das Protein ein Zelloberflächenprotein ist.

**19.** Verfahren nach Anspruch 17, worin das Protein aus der aus 4-1BB-Ligand, Ephrin b1, ISLR, M-ras und ZO-1 bestehenden Gruppe ausgewählt ist.

**20.** Verfahren nach einem der Ansprüche 17 bis 19, worin das Detektieren der verstärkten Expression in der biologischen Probe das Detektieren der Expression des Proteins mit einem Antikörper oder Antikörperfragment umfasst.

**21.** Verfahren nach Anspruch 20, worin das Antikörperfragment aus der aus Fab-, Fab'-, $F(ab')_2$- und Fv-Fragmenten, Diabodies, einkettigen Antikörpermolekülen und aus Antikörperfragmenten gebildeten multispezifischen Antikörpern bestehenden Gruppe ausgewählt ist.

**22.** Verfahren nach einem der Ansprüche 17 bis 21, worin der Antikörper detektierbar markiert ist.

**23.** Verfahren nach einem der Ansprüche 17 bis 22, worin das Detektieren der verstärkten Expression eines Zielproteins in der biologischen Probe das Detektieren der Expression der für das Zielprotein kodierenden Nucleinsäure unter Verwendung von einer Sonde oder Primern umfasst.

**24.** Pharmazeutische Zusammensetzung, umfassend ein Retinoid und ein Antitumormittel, worin das Antitumormittel ein Antikörper, ein Antisensemolekül, ein Ribozym oder eine Tripelhelix-DNA ist, der/die/das an ein aus der aus Autotaxin, 4-1 BB-Ligand, Ephrin b1, ISLR, M-ras und ZO-1 bestehenden Gruppe ausgewähltes Zielprotein oder an eine für dieses Zielprotein kodierende Nucleinsäure bindet.

**25.** Pharmazeutische Zusammensetzung nach Anspruch 24, worin das Zielprotein ein Zelloberflächenprotein ist.

**26.** Pharmazeutische Zusammensetzung nach Anspruch 24, worin das Protein aus der aus 4-1BB-Ligand, Ephrin b1, ISLR, M-ras und ZO-1 bestehenden Gruppe ausgewählt ist.

**27.** Pharmazeutische Zusammensetzung nach einem der Ansprüche 24 bis 26, worin das Antitumormittel ein aus der aus Fab-, Fab'-, $F(ab')_2$- und Fv-Fragmenten, Diabodies, einkettigen Antikörpermolekülen und aus Antikörperfragmenten gebildeten multispezifischen Antikörpern bestehenden Gruppe ausgewähltes Antikörperfragment ist.

**28.** Pharmazeutische Zusammensetzung nach einem der Ansprüche 24 bis 27, worin das Antitumormittel ein monoklonaler, menschlicher oder humanisierter Antikörper oder ein solches Antikörperfragment ist.

**29.** Pharmazeutische Zusammensetzung nach Anspruch 28, worin der Antikörper an ein zytotoxisches Mittel konjugiert ist.

**30.** Pharmazeutische Zusammensetzung nach Anspruch 29, worin das zytotoxische Mittel aus der aus einem chemotherapeutischen Mittel, einem Maytansinoid, einem Calicheamicin, einem Antifolat, einem enzymatisch aktiven Toxin und einem radioaktiven Isotop bestehenden Gruppe ausgewählt ist.

**31.** Pharmazeutische Zusammensetzung nach Anspruch 30, worin das zytotoxische Mittel ein Maytansinoid ist.

32. Antitumormittel zur Verwendung bei der Behandlung in Kombination mit einem Retinoid eines Tumors eines aus Eierstockkrebs, Endometriumkrebs, Wilms-Nierentumor, Kolonkrebs, Brustkrebs, Prostatakrebs, Magenkrebs, Lungenkrebs, Leberzellenkrebs, Melanom oder Phäochromozytom ausgewählten menschlichen Krebses, worin das Antitumormittel ein Antikörper, ein Antisensemolekül, ein Ribozym oder eine Tripelhelix-DNA ist, der/die/das an ein aus der aus Autotaxin, 4-1BB-Ligand, Ephrin b1, ISLR, M-ras und ZO-1 bestehenden Gruppe ausgewähltes Zielprotein oder an eine für dieses Zielprotein kodierende Nucleinsäure bindet.

**Revendications**

1. utilisation d'un agent antitumoral pour la production d'un médicament destiné au traitement, en association avec un rétinoïde, d'une tumeur d'un cancer humain choisi entre le cancer des ovaires, le cancer de l'endomètre, la tumeur rénale de Wilm, le cancer du côlon, le cancer du sein, le cancer de la prostate, le cancer gastrique, le cancer du poumon, le cancer hépatocellulaire, un mélanome ou un phéochromocytome, dans laquelle ledit agent antitumoral est un anticorps, une molécule antisens, un ribozyme ou un ADN en triple hélice qui se lie à une protéine cible choisie dans le groupe consistant en l'autotaxine, le ligand de 4-1BB, l'éphrine b1, ISLR, M-ras et ZO-1 ou à un acide nucléique codant pour ladite protéine cible.

2. Utilisation d'un rétinoïde et d'un agent antitumoral pour la production d'un médicament destiné au traitement d'une tumeur d'un cancer humain choisi entre le cancer des ovaires, le cancer de l'endomètre, la tumeur rénale de Wilm, le cancer du côlon, le cancer du sein, le cancer de la prostate, le cancer gastrique, le cancer du poumon, le cancer hépatocellulaire, un mélanome ou un phéochromocytome, dans laquelle ledit agent antitumoral est un anticorps, une molécule antisens, un ribozyme ou un ADN en triple hélice qui se lie à une protéine cible choisie dans le groupe consistant en l'autotaxine, le ligand de 4-1BB, l'éphrine b1, ISLR, M-ras et ZO-1 ou à un acide nucléique codant pour ladite protéine cible.

3. Utilisation suivant l'une quelconque des revendications précédentes, dans laquelle ladite protéine est une protéine de surface cellulaire.

4. Utilisation suivant la revendication 1, dans laquelle ladite protéine est choisie dans le groupe consistant en le ligand de 4-1BB, l'éphrine b1, ISLR, M-ras et ZO-1.

5. Utilisation suivant la revendication 4, dans laquelle ladite protéine est choisie dans le groupe consistant en le ligand de 4-1BB, l'éphrine b1 et ISLR.

6. Utilisation suivant la revendication 5, dans laquelle ladite protéine est choisie dans le groupe consistant en le ligand de 4-1BB et l'éphrine b1.

7. Utilisation suivant l'une quelconque des revendications précédentes, dans laquelle ledit rétinoïde est destiné à être administré avant administration dudit agent antitumoral.

8. Utilisation suivant l'une quelconque des revendications 1 à 6, dans laquelle ledit rétinoïde est destiné à être administré conjointement avec l'administration dudit agent antitumoral.

9. Utilisation suivant l'une quelconque des revendications 1 à 6, dans laquelle ledit rétinioïde est destiné à être administré après l'administration dudit agent antitumoral,

10. Utilisation suivant l'une quelconque des revendications précédentes, dans laquelle ledit rétinoïde est l'acide rétinoïque.

11. Utilisation suivant la revendication 1, dans laquelle ledit anticorps est un fragment d'anticorps.

12. Utilisation suivant la revendication 11, dans laquelle ledit fragment d'anticorps est choisi dans le groupe consistant en des fragments Fab, Fab', $F(ab')_2$ et Fv, des anticorps doubles "diabodies", des molécules d'anticorps monocaténaires et des anticorps multispécifiques formés à partir de fragments d'anticorps.

13. Utilisation suivant l'une quelconque des revendications 1 à 12, dans laquelle l'anticorps est un anticorps monoclonal, chimère, humain ou humanisé.

**14.** Utilisation suivant l'une quelconque des revendications 1 à 13, dans laquelle l'anticorps est conjugué avec un agent cytotoxique.

**15.** Utilisation suivant la revendication 14, dans laquelle l'agent cytotoxique est choisi dans le groupe consistant en un agent chimiothérapeutique, un maytansinoïde, une calichéamicine, un antifolate, une toxine enzymatiquement active et un isotope radioactif.

**16.** Utilisation suivant la revendication 14, dans laquelle l'agent cytotoxique est un maytansinoïde.

**17.** Méthode pour le diagnostic d'une tumeur d'un cancer humain choisi entre le cancer des ovaires, le cancer de l'endomètre, la tumeur rénale de Wilm, le cancer du côlon, le cancer du sein, le cancer de la prostate, le cancer gastrique, le cancer du poumon, un cancer hépatocellulaire, un mélanome ou un phéochromocytome, comprenant :

a) la mise en contact d'un échantillon biologique obtenu à partir dudit patient avec de l'acide rétinoïque ; et
b) la détection de l'expression accrue d'une protéine cible dans l'échantillon biologique traité avec le rétinoïde par rapport à un tissu normal, la protéine cible étant choisie dans le groupe consistant en l'autotaxine, le ligand de 4-1BB, l'éphrine b1, ISLR, M-ras et ZO-1.

**18.** Méthode suivant la revendication 17, dans laquelle ladite protéine est une protéine de surface cellulaire.

**19.** Méthode suivant la revendication 17, dans laquelle ladite protéine est choisie dans le groupe consistant en le ligand de 4-1BB, l'éphrine b1, ISLR, M-ras et ZO-1.

**20.** Méthode suivant l'une quelconque des revendications 17 à 19, dans laquelle ladite détection de l'expression accrue dans l'échantillon biologique comprend la détection de l'expression de la protéine avec un anticorps ou fragment d'anticorps.

**21.** Méthode suivant la revendication 20, dans laquelle ledit fragment d'anticorps est choisi dans le groupe consistant en des fragments Fab, Fab', $F(ab')_2$ et Fv, des anticorps doubles "diabodies", des molécules d'anticorps monocaténaires et des anticorps multispécifiques formés à partir de fragments d'anticorps.

**22.** Méthode suivant l'une quelconque des revendications 17 à 21, dans laquelle l'anticorps est marqué de manière détectable,

**23.** Méthode suivant l'une quelconque des revendications 17 à 22, dans laquelle ladite détection de l'expression accrue d'une protéine cible dans l'échantillon biologique comprend la détection de l'expression de l'acide nucléique codant pour ladite protéine cible en utilisant une sonde ou des amorces.

**24.** Composition pharmaceutique comprenant un rétinoïde et un agent humoral, dans laquelle l'agent antitumoral est un anticorps, une molécule antisens, un ribozyme ou un ADN en triple hélice qui se lie à une protéine cible choisie dans le groupe consistant en l'autotaxine, le ligand de 4-1BB, l'éphrine b1, ISLR, M-ras et ZO-1 ou à un acide nucléique codant pour ladite protéine cible.

**25.** Composition pharmaceutique suivant la revendication 24, dans laquelle la protéine cible est une protéine de surface cellulaire.

**26.** Composition pharmaceutique suivant la revendication 24, dans laquelle ladite protéine est choisie dans le groupe consistant en le ligand de 4-1BB, l'éphrine b1, ISLR, M-ras et ZO-1.

**27.** Composition pharmaceutique suivant l'une quelconque des revendications 24 à 26, dans laquelle l'agent antitumoral est un fragment d'anticorps choisi dans le groupe consistant en des fragments Fab, Fab', $F(ab')_2$ et Fv, des anticorps doubles "diabodies", des molécules d'anticorps monocaténaires et des anticorps multispécifiques formés à partir de fragments d'anticorps.

**28.** composition pharmaceutique suivant l'une quelconque des revendications 24 à 27, dans laquelle l'agent antitumoral est un anticorps ou fragment d'anticorps monoclonal, humain ou humanisé.

**29.** Composition pharmaceutique suivant la revendication 28, dans laquelle l'anticorps est conjugué avec un agent cytotoxique.

**30.** Composition pharmaceutique suivant la revendication 29, dans laquelle l'agent cytotoxique est choisi dans le groupe consistant en un agent chimiothérapeutique, un maytansinoïde, une calichéamicine, un antifolate, une toxine enzymatiquement active et un isotope radioactif.

**31.** Composition pharmaceutique suivant la revendication 30, dans laquelle l'agent cytotoxique est un maytansinoïde.

**32.** Agent antitumoral destiné à être utilisé dans le traitement, en association avec un rétinoïde, d'une tumeur d'un cancer humain choisi entre le cancer des ovaires, le cancer de l'endomètre, la tumeur rénale de Wilm, le cancer du côlon, le cancer du sein, le cancer de la prostate, le cancer gastrique, le cancer du poumon, un cancer hépatocellulaire, un mélanome ou un phéochromocytome, ledit agent antitumoral étant un anticorps, une molécule antisens, un ribozyme ou un ADN en triple hélice qui se lie à une protéine cible choisie dans le groupe consistant en l'autotaxine, le ligand de 4-1BB, l'éphrine b1, ISLR, M-ras et ZO-1 ou à un acide nucléique codant pour ladite protéine cible.

AGTCCCAGACGGGCTTTTCCCAGAGAGCTAAAAGAGAAGGGCCAGAGAATGTCGTCCCAG
CCAGCAGGGAACCAGACCTCCCCCGGGGCCACAGAGGACTACTCCTATGGCAGCTGGTAC
ATCGATGAGCCCCAGGGGGGCGAGGAGCTCCAGCCAGAGGGGGAAGTGCCCTCCTGCCAC
ACCAGCATACCACCCGGCCTGTACCACGCCTGCCTGGCCTCGCTGTCAATCCTTGTGCTG
CTGCTCCTGGCCATGCTGGTGAGGCGCCGCCAGCTCTGGCCTGACTGTGTGCGTGGCAGG
CCCGGCCTGCCCAGCCCTGTGGATTTCTTGGCTGGGGACAGGCCCCGGGCAGTGCCTGCT
GCTGTTTTCATGGTCCTCCTGAGCTCCCTGTGTTTGCTGCTCCCCGACGAGGACGCATTG
CCCTTCCTGACTCTCGCCTCAGCACCCAGCCAAGATGGGAAAACTGAGGCTCCAAGAGGG
GCCTGGAAGATACTGGGACTGTTCTATTATGCTGCCCTCTACTACCCTCTGGCTGCCTGT
GCCACGGCTGGCCACACAGCTGCACACCTGCTCGGCAGCACGCTGTCCTGGGCCCACCTT
GGGGTCCAGGTCTGGCAGAGGGCAGAGTGTCCCCAGGTGCCCAAGATCTACAAGTACTAC
TCCCTGCTGGCCTCCCTGCCTCTCCTGCTGGGCCTCGGATTCCTGAGCCTTTGGTACCCT
GTGCAGCTGGTGAGAAGCTTCAGCCGTAGGACAGGAGCAGGCTCCAAGGGGCTGCAGAGC
AGCTACTCTGAGGAATATCTGAGGAACCTCCTTTGCAGGAAGAAGCTGGGAAGCAGCTAC
CACACCTCCAAGCATGGCTTCCTGTCCTGGGCCCGCGTCTGCTTGAGACACTGCATCTAC
ACTCCACAGCCAGGATTCCATCTCCCGCTGAAGCTGGTGCTTTCAGCTACACTGACAGGG
ACGGCCATTTACCAGGTGGCCCTGCTGCTGCTGGTGGGCGTGGTACCCACTATCCAGAAG
GTGAGGGCAGGGGTCACCACGGATGTCTCCTACCTGCTGGCCGGCTTTGGAATCGTGCTC
TCCGAGGACAAGCAGGAGGTGGTGGAGCTGGTGAAGCACCATCTGTGGGCTCTGGAAGTG
TGCTACATCTCAGCCTTGGTCTTGTCCTGCTTACTCACCTTCCTGGTCCTGATGCGCTCA
CTGGTGACACACAGGACCAACCTTCGAGCTCTGCACCGAGGAGCTGCCCTGGACTTGAGT
CCCTTGCATCGGAGTCCCCATCCCTCCCGCCAAGCCATATTCTGTTGGATGAGCTTCAGT
GCCTACCAGACAGCCTTTATCTGCCTTGGGCTCCTGGTGCAGCAGATCATCTTCTTCCTG
GGAACCACGGCCCTGGCCTTCCTGGTGCTCATGCCTGTGCTCCATGGCAGGAACCTCCTG
CTCTTCCGTTCCCTGGAGTCCTCGTGGCCCTTCTGGCTGACTTTGGCCCTGGCTGTGATC
CTGCAGAACATGGCAGCCCATTGGGTCTTCCTGGAGACTCATGATGGACACCCACAGCTG
ACCAACCGGCGAGTGCTCTATGCAGCCACCTTTCTTCTCTTCCCCCTCAATGTGCTGGTG
GGTGCCATGGTGGCCACCTGGCGAGTGCTCCTCTCTGCCCTCTACAACGCCATCCACCTT
GGCCAGATGGACCTCAGCCTGCTGCCACCGAGAGCCGCCACTCTCGACCCCGGCTACTAC
ACGTACCGAAACTTCTTGAAGATTGAAGTCAGCCAGTCGCATCCAGCCATGACAGCCTTC
TGCTCCCTGCTCCTGCAAGCGCAGAGCCTCCTACCCAGGACCATGGCAGCCCCCCAGGAC
AGCCTCAGACCAGGGGAGGAAGACGAAGGGATGCAGCTGCTACAGACAAAGGACTCCATG
GCCAAGGGAGCTAGGCCCGGGGCCAGCCGCGGCAGGGCTCGCTGGGGTCTGGCCTACACG
CTGCTGCACAACCCAACCCTGCAGGTCTTCCGCAAGACGGCCCTGTTGGGTGCCAATGGT
GCCCAGCCCTGAGGGCAGGGAAGGTCAACCCACCTGCCCATCTGTGCTGAGGCATGTTCC
TGCCTACCATCCTCCTCCCTCCCCGGCTCTCCTCCCAGCATCACACCAGCCATGCAGCCA
GCAGGTCCTCCGGATCACTGTGGTTGGGTGGAGGTCTGTCTGCACTGGGAGCCTCAGGAG
GGCTCTGCTCCACCCACTTGGCTATGGGAGAGCCAGCAGGGGTTCTGGAGAAAAAAACTG
GTGGGTTAGGGCCTTGGTCCAGGAGCCAGTTGAGCCAGGGCAGCCACATCCAGGCGTCTC
CCTACCCTGGCTCTGCCATCAGCCTTGAAGGGCCTCGATGAAGCCTTCTCTGGAACCACT
CCAGCCCAGCTCCACCTCAGCCTTGGCCTTCACGCTGTGGAAGCAGCCAAGGCACTTCCT
CACCCCCTCAGCGCCACGGACCTCTCTGGGGAGTGGCCGGAAAGCTCCCGGTCCTCTGGC
CTGCAGGGCAGCCCAAGTCATGACTCAGACCAGGTCCCACACTGAGCTGCCCACACTCGA
GAGCCAGATATTTTTGTAGTTTTTATGCCTTTGGCTATTATGAAAGAGGTTAGTGTGTTC
CCTGCAATAAACTTGTTCCTGAGAAAAAAAAAAAAAAAAAAAAAAAAAAAAAAAAAAAAA
AAAAAAAAAAAAAAAAAAAAAAAAAAAAAAAA

*FIG. 1*

MSSQPAGNQTSPGATEDYSYGSWYIDEPQGGEELQPEGEVPSCHTSIPPGLYHACLASLS
ILVLLLLAMLVRRRQLWPDCVRGRPGLPSPVDFLAGDRPRAVPAAVFMVLLSSLCLLLPD
EDALPFLTLASAPSQDGKTEAPRGAWKILGLFYYAALYYPLAACATAGHTAAHLLGSTLS
WAHLGVQVWQRAECPQVPKIYKYYSLLASLPLLLGLGFLSLWYPVQLVRSFSRRTGAGSK
GLQSSYSEEYLRNLLCRKKLGSSYHTSKHGFLSWARVCLRHCIYTPQPGFHLPLKLVLSA
TLTGTAIYQVALLLLVGVVPTIQKVRAGVTTDVSYLLAGFGIVLSEDKQEVVELVKHHLW
ALEVCYISALVLSCLLTFLVLMRSLVTHRTNLRALHRGAALDLSPLHRSPHPSRQAIFCW
MSFSAYQTAFICLGLLVQQIIFFLGTTALAFLVLMPVLHGRNLLLFRSLESSWPFWLTLA
LAVILQNMAAHWVFLETHDGHPQLTNRRVLYAATFLLFPLNVLVGAMVATWRVLLSALYN
AIHLGQMDLSLLPPRAATLDPGYYTYRNFLKIEVSQSHPAMTAFCSLLLQAQSLLPRTMA
APQDSLRPGEEDEGMQLLQTKDSMAKGARPGASRGRARWGLAYTLLHNPTLQVFRKTALL
GANGAQP

Important features of the protein:
Signal peptide:

None

Transmembrane domain:

54-69
102-119
148-166
207-222
301-320
364-380
431-451
474-489
560-535

Motif file:
Motif name: N-glycosylation site.

8-12

Motif name: N-myristoylation site.

50-56
176-182
241-247
317-323
341-347
525-531
627-633
631-637
640-646
661-667

Motif name: Prokaryotic membrane lipoprotein lipid attachment site.

364-375

Motif name: ATP/GTP-binding site motif A (P-loop).

132-140

*FIG.2*

| | | |
|---|---|---|
| PRO | XXXXXXXXXXXXXXX | (Length = 15 amino acids) |
| Comparison Protein | XXXXXYYYYYYY | (Length = 12 amino acids) |

% amino acid sequence identity =

(the number of identically matching amino acid residues between the two polypeptide sequences as determined by ALIGN-2) divided by (the total number of amino acid residues of the PRO polypeptide) =

5 divided by 15 = 33.3%

*FIG. 3A*

| | | |
|---|---|---|
| PRO | XXXXXXXXXX | (Length = 10 amino acids) |
| Comparison Protein | XXXXXYYYYYYZZYZ | (Length = 15 amino acids) |

% amino acid sequence identity =

(the number of identically matching amino acid residues between the two polypeptide sequences as determined by ALIGN-2) divided by (the total number of amino acid residues of the PRO polypeptide) =

5 divided by 10 = 50%

*FIG.3B*

PRO-DNA NNNNNNNNNNNNNN (Length = 14 nucleotides)

Comparison DNA NNNNNNLLLLLLLLLL (Length = 16 nucleotides)

% nucleic acid sequence identity =

(the number of identically matching nucleotides between the two nucleic acid sequences as determined by ALIGN-2) divided by (the total number of nucleotides of the PRO-DNA nucleic acid sequence) =

6 divided by 14 = 42.9%

*FIG.3C*

| | | |
|---|---|---|
| PRO-DNA | NNNNNNNNNNNN | (Length = 12 nucleotides) |
| Comparison DNA | NNNNLLLVV | (Length = 9 nucleotides) |

% nucleic acid sequence identity =

(the number of identically matching nucleotides between the two nucleic acid sequences as determined by ALIGN-2) divided by (the total number of nucleotides of the PRO-DNA nucleic acid sequence) =

4 divided by 12 = 33.3%

*FIG.3D*

```
/*
 *
 * C-C increased from 12 to 15
 * Z is average of EQ
 * B is average of ND
 * match with stop is _M; stop-stop = 0; J (joker) match = 0
 */
#define   _M        -8          /* value of a match with a stop */

int       _day[26][26] = {
/*      A  B  C  D  E  F  G  H  I  J  K  L  M  N  O  P  Q  R  S  T  U  V  W  X  Y  Z */
/* A */   { 2, 0,-2, 0, 0,-4, 1,-1,-1, 0,-1,-2,-1, 0,_M, 1, 0,-2, 1, 1, 0, 0,-6, 0,-3, 0},
/* B */   { 0, 3,-4, 3, 2,-5, 0, 1,-2, 0, 0,-3,-2, 2,_M,-1, 1, 0, 0, 0, 0,-2,-5, 0,-3, 1},
/* C */   {-2,-4,15,-5,-5,-4,-3,-3,-2, 0,-5,-6,-5,-4,_M,-3,-5,-4, 0,-2, 0,-2,-8, 0, 0,-5},
/* D */   { 0, 3,-5, 4, 3,-6, 1, 1,-2, 0, 0,-4,-3, 2,_M,-1, 2,-1, 0, 0, 0,-2,-7, 0,-4, 2},
/* E */   { 0, 2,-5, 3, 4,-5, 0, 1,-2, 0, 0,-3,-2, 1,_M,-1, 2,-1, 0, 0, 0,-2,-7, 0,-4, 3},
/* F */   {-4,-5,-4,-6,-5, 9,-5,-2, 1, 0,-5, 2, 0,-4,_M,-5,-5,-4,-3,-3, 0,-1, 0, 0, 7,-5},
/* G */   { 1, 0,-3, 1, 0,-5, 5,-2,-3, 0,-2,-4,-3, 0,_M,-1,-1,-3, 1, 0, 0,-1,-7, 0,-5, 0},
/* H */   {-1, 1,-3, 1, 1,-2,-2, 6,-2, 0, 0,-2,-2, 2,_M, 0, 3, 2,-1,-1, 0,-2,-3, 0, 0, 2},
/* I */   {-1,-2,-2,-2,-2, 1,-3,-2, 5, 0,-2, 2, 2,-2,_M,-2,-2,-2,-1, 0, 0, 4,-5, 0,-1,-2},
/* J */   { 0, 0, 0, 0, 0, 0, 0, 0, 0, 0, 0, 0, 0, 0,_M, 0, 0, 0, 0, 0, 0, 0, 0, 0, 0, 0},
/* K */   {-1, 0,-5, 0, 0,-5,-2, 0,-2, 0, 5,-3, 0, 1,_M,-1, 1, 3, 0, 0, 0,-2,-3, 0,-4, 0},
/* L */   {-2,-3,-6,-4,-3, 2,-4,-2, 2, 0,-3, 6, 4,-3,_M,-3,-2,-3,-3,-1, 0, 2,-2, 0,-1,-2},
/* M */   {-1,-2,-5,-3,-2, 0,-3,-2, 2, 0, 0, 4, 6,-2,_M,-2,-1, 0,-2,-1, 0, 2,-4, 0,-2,-1},
/* N */   { 0, 2,-4, 2, 1,-4, 0, 2,-2, 0, 1,-3,-2, 2,_M,-1, 1, 0, 1, 0, 0,-2,-4, 0,-2, 1},
/* O */                                        {_M,_M,_M,_M,_M,_M,_M,_M,_M,_M,_M,_M,_M,_M,
0,_M,_M,_M,_M,_M,_M,_M,_M,_M,_M,_M},
/* P */   { 1,-1,-3,-1,-1,-5,-1, 0,-2, 0,-1,-3,-2,-1,_M, 6, 0, 0, 1, 0, 0,-1,-6, 0,-5, 0},
/* Q */   { 0, 1,-5, 2, 2,-5,-1, 3,-2, 0, 1,-2,-1, 1,_M, 0, 4, 1,-1,-1, 0,-2,-5, 0,-4, 3},
/* R */   {-2, 0,-4,-1,-1,-4,-3, 2,-2, 0, 3,-3, 0, 0,_M, 0, 1, 6, 0,-1, 0,-2, 2, 0,-4, 0},
/* S */   { 1, 0, 0, 0, 0,-3, 1,-1,-1, 0, 0,-3,-2, 1,_M, 1,-1, 0, 2, 1, 0,-1,-2, 0,-3, 0},
/* T */   {-1, 0,-2, 0, 0,-3, 0,-1, 0, 0, 0,-1,-1, 0,_M, 0,-1,-1, 1, 3, 0, 0,-5, 0,-3, 0},
/* U */   { 0, 0, 0, 0, 0, 0, 0, 0, 0, 0, 0, 0, 0, 0,_M, 0, 0, 0, 0, 0, 0, 0, 0, 0, 0, 0},
/* V */   { 0,-2,-2,-2,-2,-1,-1,-2, 4, 0,-2, 2, 2,-2,_M,-1,-2,-2,-1, 0, 0, 4,-6, 0,-2,-2},
/* W */   {-6,-5,-8,-7,-7, 0,-7,-3,-5, 0,-3,-2,-4,-4,_M,-6,-5, 2,-2,-5, 0,-6,17, 0, 0,-6},
/* X */   { 0, 0, 0, 0, 0, 0, 0, 0, 0, 0, 0, 0, 0, 0,_M, 0, 0, 0, 0, 0, 0, 0, 0, 0, 0, 0},
/* Y */   {-3,-3, 0,-4,-4, 7,-5, 0,-1, 0,-4,-1,-2,-2,_M,-5,-4,-4,-3,-3, 0,-2, 0, 0,10,-4},
/* Z */   { 0, 1,-5, 2, 3,-5, 0, 2,-2, 0, 0,-2,-1, 1,_M, 0, 3, 0, 0, 0, 0,-2,-6, 0,-4, 4}
};
```

Page 1 of day.h

*FIG.4A*

```
/*
 */
#include <stdio.h>
#include <ctype.h>

#define MAXJMP      16      /* max jumps in a diag */
#define MAXGAP      24      /* don't continue to penalize gaps larger than this */
#define JMPS        1024    /* max jmps in an path */
#define MX          4       /* save if there's at least MX-1 bases since last jmp */

#define DMAT        3       /* value of matching bases */
#define DMIS        0       /* penalty for mismatched bases */
#define DINS0       8       /* penalty for a gap */
#define DINS1       1       /* penalty per base */
#define PINS0       8       /* penalty for a gap */
#define PINS1       4       /* penalty per residue */

struct jmp {
        short           n[MAXJMP];      /* size of jmp (neg for dely) */
        unsigned short  x[MAXJMP];      /* base no. of jmp in seq x */
};                                      /* limits seq to 2^16 -1 */

struct diag {
        int             score;          /* score at last jmp */
        long            offset;         /* offset of prev block */
        short           ijmp;           /* current jmp index */
        struct jmp      jp;             /* list of jmps */
};

struct path {
        int     spc;            /* number of leading spaces */
        short   n[JMPS];/* size of jmp (gap) */
        int     x[JMPS];/* loc of jmp (last elem before gap) */
};

char            *ofile;                 /* output file name */
char            *namex[2];              /* seq names: getseqs() */
char            *prog;                  /* prog name for err msgs */
char            *seqx[2];               /* seqs: getseqs() */
int             dmax;                   /* best diag: nw() */
int             dmax0;                  /* final diag */
int             dna;                    /* set if dna: main() */
int             endgaps;                /* set if penalizing end gaps */
int             gapx, gapy;             /* total gaps in seqs */
int             len0, len1;             /* seq lens */
int             ngapx, ngapy;           /* total size of gaps */
int             smax;                   /* max score: nw() */
int             *xbm;                   /* bitmap for matching */
long            offset;                 /* current offset in jmp file */
struct  diag    *dx;                    /* holds diagonals */
struct  path    pp[2];                  /* holds path for seqs */

char            *calloc(), *malloc(), *index(), *strcpy();
char            *getseq(), *g_calloc();
```

*FIG. 4B*

```
/* Needleman-Wunsch alignment program
 *
 * usage: progs file1 file2
 *   where file1 and file2 are two dna or two protein sequences.
 *   The sequences can be in upper- or lower-case an may contain ambiguity
 *   Any lines beginning with ';', '>' or '<' are ignored
 *   Max file length is 65535 (limited by unsigned short x in the jmp struct)
 *   A sequence with 1/3 or more of its elements ACGTU is assumed to be DNA
 *   Output is in the file "align.out"
 *
 * The program may create a tmp file in /tmp to hold info about traceback.
 * Original version developed under BSD 4.3 on a vax 8650
 */
#include "nw.h"
#include "day.h"

static    _dbval[26] = {
          1,14,2,13,0,0,4,11,0,0,12,0,3,15,0,0,0,5,6,8,8,7,9,0,10,0
};

static    _pbval[26] = {
          1, 2|(1<<('D'-'A'))|(1<<('N'-'A')), 4, 8, 16, 32, 64,
          128, 256, 0xFFFFFFF, 1<<10, 1<<11, 1<<12, 1<<13, 1<<14,
          1<<15, 1<<16, 1<<17, 1<<18, 1<<19, 1<<20, 1<<21, 1<<22,
          1<<23, 1<<24, 1<<25|(1<<('E'-'A'))|(1<<('Q'-'A'))
};

main(ac, av)                                                          main
          int       ac;
          char      *av[];
{
          prog = av[0];
          if (ac != 3) {
                    fprintf(stderr,"usage: %s file1 file2\n", prog);
                    fprintf(stderr,"where file1 and file2 are two dna or two protein sequences.\n");
                    fprintf(stderr,"The sequences can be in upper- or lower-case\n");
                    fprintf(stderr,"Any lines beginning with ';' or '<' are ignored\n");
                    fprintf(stderr,"Output is in the file \"align.out\"\n");
                    exit(1);
          }
          namex[0] = av[1];
          namex[1] = av[2];
          seqx[0] = getseq(namex[0], &len0);
          seqx[1] = getseq(namex[1], &len1);
          xbm = (dna)? _dbval : _pbval;

          endgaps = 0;                          /* 1 to penalize endgaps */
          ofile = "align.out";                  /* output file */

          nw();               /* fill in the matrix, get the possible jmps */
          readjmps();         /* get the actual jmps */
          print();            /* print stats, alignment */

          cleanup(0);         /* unlink any tmp files */
}
```

*FIG.4C*

```
/* do the alignment, return best score: main()
 * dna: values in Fitch and Smith, PNAS, 80, 1382-1386, 1983
 * pro: PAM 250 values
 * When scores are equal, we prefer mismatches to any gap, prefer
 * a new gap to extending an ongoing gap, and prefer a gap in seqx
 * to a gap in seq y.
 */
nw()                                                                    nw
{
        char            *px, *py;           /* seqs and ptrs */
        int             *ndely, *dely;      /* keep track of dely */
        int             ndelx, delx;        /* keep track of delx */
        int             *tmp;               /* for swapping row0, row1 */
        int             mis;                /* score for each type */
        int             ins0, ins1;         /* insertion penalties */
        register        id;                 /* diagonal index */
        register        ij;                 /* jmp index */
        register        *col0, *col1;       /* score for curr, last row */
        register        xx, yy;             /* index into seqs */

        dx = (struct diag *)g_calloc("to get diags", len0+len1+1, sizeof(struct diag));

        ndely = (int *)g_calloc("to get ndely", len1+1, sizeof(int));
        dely = (int *)g_calloc("to get dely", len1+1, sizeof(int));
        col0 = (int *)g_calloc("to get col0", len1+1, sizeof(int));
        col1 = (int *)g_calloc("to get col1", len1+1, sizeof(int));
        ins0 = (dna)? DINS0 : PINS0;
        ins1 = (dna)? DINS1 : PINS1;

        smax = -10000;
        if (endgaps) {
                for (col0[0] = dely[0] = -ins0, yy = 1; yy <= len1; yy++) {
                        col0[yy] = dely[yy] = col0[yy-1] - ins1;
                        ndely[yy] = yy;
                }
                col0[0] = 0;        /* Waterman Bull Math Biol 84 */
        }
        else
                for (yy = 1; yy <= len1; yy++)
                        dely[yy] = -ins0;

        /* fill in match matrix
         */
        for (px = seqx[0], xx = 1; xx <= len0; px++, xx++) {
                /* initialize first entry in col
                 */
                if (endgaps) {
                        if (xx == 1)
                                col1[0] = delx = -(ins0+ins1);
                        else
                                col1[0] = delx = col0[0] - ins1;
                        ndelx = xx;
                }
                else {
                        col1[0] = 0;
                        delx = -ins0;
                        ndelx = 0;
                }
```

*FIG. 4D*

...nw

```
for (py = seqx[1], yy = 1; yy <= len1; py++, yy++) {
        mis = col0[yy-1];
        if (dna)
                mis += (xbm[*px-'A']&xbm[*py-'A'])? DMAT : DMIS;
        else
                mis += _day[*px-'A'][*py-'A'];

        /* update penalty for del in x seq;
         * favor new del over ongong del
         * ignore MAXGAP if weighting endgaps
         */
        if (endgaps || ndely[yy] < MAXGAP) {
                if (col0[yy] - ins0 >= dely[yy]) {
                        dely[yy] = col0[yy] - (ins0+ins1);
                        ndely[yy] = 1;
                } else {
                        dely[yy] -= ins1;
                        ndely[yy]++;
                }
        } else {
                if (col0[yy] - (ins0+ins1) >= dely[yy]) {
                        dely[yy] = col0[yy] - (ins0+ins1);
                        ndely[yy] = 1;
                } else
                        ndely[yy]++;
        }

        /* update penalty for del in y seq;
         * favor new del over ongong del
         */
        if (endgaps || ndelx < MAXGAP) {
                if (col1[yy-1] - ins0 >= delx) {
                        delx = col1[yy-1] - (ins0+ins1);
                        ndelx = 1;
                } else {
                        delx -= ins1;
                        ndelx++;
                }
        } else {
                if (col1[yy-1] - (ins0+ins1) >= delx) {
                        delx = col1[yy-1] - (ins0+ins1);
                        ndelx = 1;
                } else
                        ndelx++;
        }

        /* pick the maximum score; we're favoring
         * mis over any del and delx over dely
         */
```

*FIG. 4E*

...DW

```
                id = xx - yy + len1 - 1;
                if (mis >= delx && mis >= dely[yy])
                        col1[yy] = mis;
                else if (delx >= dely[yy]) {
                        col1[yy] = delx;
                        ij = dx[id].ijmp;
                        if (dx[id].jp.n[0] && (!dna || (ndelx >= MAXJMP
                        && xx > dx[id].jp.x[ij]+MX) || mis > dx[id].score+DINS0)) {
                                dx[id].ijmp++;
                                if (++ij >= MAXJMP) {
                                        writejmps(id);
                                        ij = dx[id].ijmp = 0;
                                        dx[id].offset = offset;
                                        offset += sizeof(struct jmp) + sizeof(offset);
                                }
                        }
                        dx[id].jp.n[ij] = ndelx;
                        dx[id].jp.x[ij] = xx;
                        dx[id].score = delx;
                }
                else {
                        col1[yy] = dely[yy];
                        ij = dx[id].ijmp;
if (dx[id].jp.n[0] && (!dna || (ndely[yy] >= MAXJMP
                        && xx > dx[id].jp.x[ij]+MX) || mis > dx[id].score+DINS0)) {
                                dx[id].ijmp++;
                                if (++ij >= MAXJMP) {
                                        writejmps(id);
                                        ij = dx[id].ijmp = 0;
                                        dx[id].offset = offset;
                                        offset += sizeof(struct jmp) + sizeof(offset);
                                }
                        }
                        dx[id].jp.n[ij] = -ndely[yy];
                        dx[id].jp.x[ij] = xx;
                        dx[id].score = dely[yy];
                }
                if (xx == len0 && yy < len1) {
                        /* last col
                         */
                        if (endgaps)
                                col1[yy] -= ins0+ins1*(len1-yy);
                        if (col1[yy] > smax) {
                                smax = col1[yy];
                                dmax = id;
                        }
                }
        }
        if (endgaps && xx < len0)
                col1[yy-1] -= ins0+ins1*(len0-xx);
        if (col1[yy-1] > smax) {
                smax = col1[yy-1];
                dmax = id;
        }
        tmp = col0; col0 = col1; col1 = tmp;
}
(void) free((char *)ndely);
(void) free((char *)dely);
(void) free((char *)col0);(void) free((char *)col1);}
```

*FIG.4F*

```
/*
 *
 * print() -- only routine visible outside this module
 *
 * static:
 * getmat() -- trace back best path, count matches: print()
 * pr_align() -- print alignment of described in array p[]: print()
 * dumpblock() -- dump a block of lines with numbers, stars: pr_align()
 * nums() -- put out a number line: dumpblock()
 * putline() -- put out a line (name, [num], seq, [num]): dumpblock()
 * stars() - -put a line of stars: dumpblock()
 * stripname() -- strip any path and prefix from a seqname
 */

#include "nw.h"

#define SPC      3
#define P_LINE   256      /* maximum output line */
#define P_SPC    3        /* space between name or num and seq */

extern   _day[26][26];
int      olen;            /* set output line length */
FILE     *fx;             /* output file */

print()                                                              print
{
        int     lx, ly, firstgap, lastgap;      /* overlap */

        if ((fx = fopen(ofile, "w")) == 0) {
                fprintf(stderr,"%s: can't write %s\n", prog, ofile);
                cleanup(1);
        }
        fprintf(fx, "<first sequence: %s (length = %d)\n", namex[0], len0);
        fprintf(fx, "<second sequence: %s (length = %d)\n", namex[1], len1);
        olen = 60;
        lx = len0;
        ly = len1;
        firstgap = lastgap = 0;
        if (dmax < len1 - 1) {        /* leading gap in x */
                pp[0].spc = firstgap = len1 - dmax - 1;
                ly -= pp[0].spc;
        }
        else if (dmax > len1 - 1) {   /* leading gap in y */
                pp[1].spc = firstgap = dmax - (len1 - 1);
                lx -= pp[1].spc;
        }
        if (dmax0 < len0 - 1) {       /* trailing gap in x */
                lastgap = len0 - dmax0 -1;
                lx -= lastgap;
        }
        else if (dmax0 > len0 - 1) {  /* trailing gap in y */
                lastgap = dmax0 - (len0 - 1);
                ly -= lastgap;
        }
        getmat(lx, ly, firstgap, lastgap);
        pr_align();
}
```

FIG. 4G

```
/*
 * trace back the best path, count matches
 */
static
getmat(lx, ly, firstgap, lastgap)                                                    getmat
        int     lx, ly;                 /* "core" (minus endgaps) */
        int     firstgap, lastgap;      /* leading trailing overlap */
{
        int             nm, i0, i1, siz0, siz1;
        char            outx[32];
        double          pct;
        register        n0, n1;
        register char   *p0, *p1;

        /* get total matches, score
         */
        i0 = i1 = siz0 = siz1 = 0;
        p0 = seqx[0] + pp[1].spc;
        p1 = seqx[1] + pp[0].spc;
        n0 = pp[1].spc + 1;
        n1 = pp[0].spc + 1;

        nm = 0;
        while ( *p0 && *p1 ) {
                if (siz0) {
                        p1++;
                        n1++;
                        siz0--;
                }
                else if (siz1) {
                        p0++;
                        n0++;
                        siz1--;
                }
                else {
                        if (xbm[*p0-'A']&xbm[*p1-'A'])
                                nm++;
                        if (n0++ == pp[0].x[i0])
                                siz0 = pp[0].n[i0++];
                        if (n1++ == pp[1].x[i1])
                                siz1 = pp[1].n[i1++];
                        p0++;
                        p1++;
                }
        }

        /* pct homology:
         * if penalizing endgaps, base is the shorter seq
         * else, knock off overhangs and take shorter core
         */
        if (endgaps)
                lx = (len0 < len1)? len0 : len1;
        else
                lx = (lx < ly)? lx : ly;
        pct = 100.*(double)nm/(double)lx;
        fprintf(fx, "\n");
        fprintf(fx, "<%d match%s in an overlap of %d: %.2f percent similarity\n",
                nm, (nm == 1)? "" : "es", lx, pct);
```

*FIG.4H*

...getmat

```
        fprintf(fx, "<gaps in first sequence: %d", gapx);
        if (gapx) {
                (void) sprintf(outx, " (%d %s%s)",
                        ngapx, (dna)? "base":"residue", (ngapx == 1)? "":"s");
                fprintf(fx,"%s", outx);

        fprintf(fx, ", gaps in second sequence: %d", gapy);
        if (gapy) {
                (void) sprintf(outx, " (%d %s%s)",
                        ngapy, (dna)? "base":"residue", (ngapy == 1)? "":"s");
                fprintf(fx,"%s", outx);
        }
        if (dna)
                fprintf(fx,
                "\n<score: %d (match = %d, mismatch = %d, gap penalty = %d + %d per base)\n",
                smax, DMAT, DMIS, DINS0, DINS1);
        else
                fprintf(fx,
                "\n<score: %d (Dayhoff PAM 250 matrix, gap penalty = %d + %d per residue)\n",
                smax, PINS0, PINS1);
        if (endgaps)
                fprintf(fx,
                "<endgaps penalized. left endgap: %d %s%s, right endgap: %d %s%s\n",
                firstgap, (dna)? "base" : "residue", (firstgap == 1)? "" : "s",
                lastgap, (dna)? "base" : "residue", (lastgap == 1)? "" : "s");
        else
                fprintf(fx, "<endgaps not penalized\n");
}

static          nm;                     /* matches in core -- for checking */
static          lmax;                   /* lengths of stripped file names */
static          ij[2];                  /* jmp index for a path */
static          nc[2];                  /* number at start of current line */
static          ni[2];                  /* current elem number -- for gapping */
static          siz[2];
static char     *ps[2];                 /* ptr to current element */
static char     *po[2];                 /* ptr to next output char slot */
static char     out[2][P_LINE];         /* output line */
static char     star[P_LINE];           /* set by stars() */

/*
 * print alignment of described in struct path pp[]
 */
static
pr_align()
{
        int             nn;             /* char count */
        int             more;
        register        i;

        for (i = 0, lmax = 0; i < 2; i++) {
                nn = stripname(namex[i]);
                if (nn > lmax)
                        lmax = nn;

                nc[i] = 1;
                ni[i] = 1;
                siz[i] = ij[i] = 0;
                ps[i] = seqx[i];
                po[i] = out[i];
```

pr_align

*FIG. 4I*

...pr_align

```
for (nn = nm = 0, more = 1; more; ) {
        for (i = more = 0; i < 2; i++) {
                /*
                 * do we have more of this sequence?
                 */
                if (!*ps[i])
                        continue;

                more++;

                if (pp[i].spc) {        /* leading space */
                        *po[i]++ = ' ';
                        pp[i].spc--;
                }
                else if (siz[i]) {      /* in a gap */
                        *po[i]++ = '-';
                        siz[i]--;
                }
                else {                  /* we're putting a seq element
                                         */
                        *po[i] = *ps[i];
                        if (islower(*ps[i]))
                                *ps[i] = toupper(*ps[i]);
                        po[i]++;
                        ps[i]++;

                        /*
                         * are we at next gap for this seq?
                         */
                        if (ni[i] == pp[i].x[ij[i]]) {
                                /*
                                 * we need to merge all gaps
                                 * at this location
                                 */
                                siz[i] = pp[i].n[ij[i]++];
                                while (ni[i] == pp[i].x[ij[i]])
                                        siz[i] += pp[i].n[ij[i]++];
                        }
                        ni[i]++;
                }
        }
        if (++nn == olen || !more && nn) {
                dumpblock();
                for (i = 0; i < 2; i++)
                        po[i] = out[i];
                nn = 0;
        }
    }
}

/*
 * dump a block of lines, including numbers, stars: pr_align()
 */
static
dumpblock()
{
        register i;

        for (i = 0; i < 2; i++)
                *po[i]-- = '\0';
```

dumpblock

*FIG.4J*

...dumpblock

```
	(void) putc('\n', fx);
	for (i = 0; i < 2; i++) {
		if (*out[i] && (*out[i] != ' ' || *(po[i]) != ' ')) {
			if (i == 0)
				nums(i);
			if (i == 0 && *out[1])
				stars();
			putline(i);
			if (i == 0 && *out[1])
				fprintf(fx, star);
			if (i == 1)
				nums(i);
		}
	}
}

/*
 * put out a number line: dumpblock()
 */
static
nums(ix)
	int	ix;	/* index in out[] holding seq line */
{
	char		nline[P_LINE];
	register	i, j;
	register char	*pn, *px, *py;

	for (pn = nline, i = 0; i < lmax+P_SPC; i++, pn++)
		*pn = ' ';
	for (i = nc[ix], py = out[ix]; *py; py++, pn++) {
		if (*py == ' ' || *py == '-')
			*pn = ' ';
		else {
			if (i%10 == 0 || (i == 1 && nc[ix] != 1)) {
				j = (i < 0)? -i : i;
				for (px = pn; j; j /= 10, px--)
					*px = j%10 + '0';
				if (i < 0)
					*px = '-';
			}
			else
				*pn = ' ';
			i++;
		}
	}
	*pn = '\0';
	nc[ix] = i;
	for (pn = nline; *pn; pn++)
		(void) putc(*pn, fx);
	(void) putc('\n', fx);
}

/*
 * put out a line (name, [num], seq, [num]): dumpblock()
 */
static
putline(ix)
	int	ix;
{
```

nums

putline

*FIG. 4K*

...putline

```
        int             i;
        register char   *px;

        for (px = namex[ix], i = 0; *px && *px != ':'; px++, i++)
                (void) putc(*px, fx);
        for (; i < lmax+P_SPC; i++)
                (void) putc(' ', fx);

        /* these count from 1:
         * ni[] is current element (from 1)
         * nc[] is number at start of current line
         */
        for (px = out[ix]; *px; px++)
                (void) putc(*px&0x7F, fx);
        (void) putc('\n', fx);
}


/*
 * put a line of stars (seqs always in out[0], out[1]): dumpblock()
 */
static
stars()
{
        int             i;
        register char   *p0, *p1, cx, *px;

        if (!*out[0] || (*out[0] == ' ' && *(po[0]) == ' ') ||
           !*out[1] || (*out[1] == ' ' && *(po[1]) == ' '))
                return;
        px = star;
        for (i = lmax+P_SPC; i; i--)
                *px++ = ' ';

        for (p0 = out[0], p1 = out[1]; *p0 && *p1; p0++, p1++) {
                if (isalpha(*p0) && isalpha(*p1)) {

                        if (xbm[*p0-'A']&xbm[*p1-'A']) {
                                cx = '*';
                                nm++;
                        }
                        else if (!dna && _day[*p0-'A'][*p1-'A'] > 0)
                                cx = '.';
                        else
                                cx = ' ';
                }
                else
                        cx = ' ';
                *px++ = cx;
        }
        *px++ = '\n';
        *px = '\0';
}
```

stars

*FIG.4L*

```
/*
 * strip path or prefix from pn, return len: pr_align()
 */
static
stripname(pn)                                                       stripname
        char    *pn;    /* file name (may be path) */
{
        register char   *px, *py;

        py = 0;
        for (px = pn; *px; px++)
                if (*px == '/')
                        py = px + 1;
        if (py)
                (void) strcpy(pn, py);
        return(strlen(pn));

}
```

*FIG. 4M*

```
/*
 * cleanup() -- cleanup any tmp file
 * getseq() -- read in seq, set dna, len, maxlen
 * g_calloc() -- calloc() with error checkin
 * readjmps() -- get the good jmps, from tmp file if necessary
 * writejmps() -- write a filled array of jmps to a tmp file: nw()
 */
#include "nw.h"
#include <sys/file.h>

char    *jname = "/tmp/homgXXXXXX";             /* tmp file for jmps */
FILE    *fj;

int     cleanup();                              /* cleanup tmp file */
long    lseek();

/*
 * remove any tmp file if we blow
 */
cleanup(i)                                                              cleanup
        int     i;
{
        if (fj)
                (void) unlink(jname);
        exit(i);
}

/*
 * read, return ptr to seq, set dna, len, maxlen
 * skip lines starting with ';', '<', or '>'
 * seq in upper or lower case
 */
char    *
getseq(file, len)                                                       getseq
        char    *file;  /* file name */
        int     *len;   /* seq len */
{
        char            line[1024], *pseq;
        register char   *px, *py;
        int             natgc, tlen;
        FILE            *fp;

        if ((fp = fopen(file,"r")) == 0) {
                fprintf(stderr,"%s: can't read %s\n", prog, file);
                exit(1);
        }
        tlen = natgc = 0;
        while (fgets(line, 1024, fp)) {
                if (*line == ';' || *line == '<' || *line == '>')
                        continue;
                for (px = line; *px != '\n'; px++)
                        if (isupper(*px) || islower(*px))
                                tlen++;
        }
        if ((pseq = malloc((unsigned)(tlen+6))) == 0) {
                fprintf(stderr,"%s: malloc() failed to get %d bytes for %s\n", prog, tlen+6, file);
                exit(1);
        }
        pseq[0] = pseq[1] = pseq[2] = pseq[3] = '\0';
```

*FIG. 4N*

...getseq

```
        py = pseq + 4;
        *len = tlen;
        rewind(fp);

        while (fgets(line, 1024, fp)) {
                if (*line == ';' || *line == '<' || *line == '>')
                        continue;
                for (px = line; *px != '\n'; px++) {
                        if (isupper(*px))
                                *py++ = *px;
                        else if (islower(*px))
                                *py++ = toupper(*px);
                        if (index("ATGCU",*(py-1)))
                                natgc++;
                }
        }
        *py++ = '\0';
        *py = '\0';
        (void) fclose(fp);
        dna = natgc > (tlen/3);
        return(pseq+4);
}

char    *
g_calloc(msg, nx, sz)
        char    *msg;           /* program, calling routine */
        int     nx, sz;         /* number and size of elements */
{
        char            *px, *calloc();

        if ((px = calloc((unsigned)nx, (unsigned)sz)) == 0) {
                if (*msg) {
                        fprintf(stderr, "%s: g_calloc() failed %s (n=%d, sz=%d)\n", prog, msg, nx, sz);
                        exit(1);
                }
        }
        return(px);
}

/*
 * get final jmps from dx[] or tmp file, set pp[], reset dmax: main()
 */
readjmps()
{
        int             fd = -1;
        int             siz, i0, i1;
        register i, j, xx;

        if (fj) {
                (void) fclose(fj);
                if ((fd = open(jname, O_RDONLY, 0)) < 0) {
                        fprintf(stderr, "%s: can't open() %s\n", prog, jname);
                        cleanup(1);
                }
        }
        for (i = i0 = i1 = 0, dmax0 = dmax, xx = len0; ; i++) {
                while (1) {
                        for (j = dx[dmax].ijmp; j >= 0 && dx[dmax].jp.x[j] >= xx; j--)
                                ;
```

g_calloc

readjmps

Page 2 of nwsubr.c

*FIG.40*

...readjmps

```
                        if (j < 0 && dx[dmax].offset && fj) {
                                (void) lseek(fd, dx[dmax].offset, 0);
                                (void) read(fd, (char *)&dx[dmax].jp, sizeof(struct jmp));
                                (void) read(fd, (char *)&dx[dmax].offset, sizeof(dx[dmax].offset));
                                dx[dmax].ijmp = MAXJMP-1;
                        }
                        else
                                break;
                }
                if (i >= JMPS) {
                        fprintf(stderr, "%s: too many gaps in alignment\n", prog);
                        cleanup(1);
                }
                if (j >= 0) {
                        siz = dx[dmax].jp.n[j];
                        xx = dx[dmax].jp.x[j];
                        dmax += siz;
                        if (siz < 0) {                  /* gap in second seq */
                                pp[1].n[i1] = -siz;
                                xx += siz;

                                /* id = xx - yy + len1 - 1
                                 */
                                pp[1].x[i1] = xx - dmax + len1 - 1;
                                gapy++;
                                ngapy -= siz;
/* ignore MAXGAP when doing endgaps */
                                siz = (-siz < MAXGAP || endgaps)? -siz : MAXGAP;
                                i1++;
                        }
                        else if (siz > 0) {  /* gap in first seq */
                                pp[0].n[i0] = siz;
                                pp[0].x[i0] = xx;
                                gapx++;
                                ngapx += siz;
/* ignore MAXGAP when doing endgaps */
                                siz = (siz < MAXGAP || endgaps)? siz : MAXGAP;
                                i0++;
                        }
                }
                else
                        break;
        }

        /* reverse the order of jmps
         */
        for (j = 0, i0--; j < i0; j++, i0--) {
                i = pp[0].n[j]; pp[0].n[j] = pp[0].n[i0]; pp[0].n[i0] = i;
                i = pp[0].x[j]; pp[0].x[j] = pp[0].x[i0]; pp[0].x[i0] = i;
        }
        for (j = 0, i1--; j < i1; j++, i1--) {
                i = pp[1].n[j]; pp[1].n[j] = pp[1].n[i1]; pp[1].n[i1] = i;
                i = pp[1].x[j]; pp[1].x[j] = pp[1].x[i1]; pp[1].x[i1] = i;
        }
        if (fd >= 0)
                (void) close(fd);
        if (fj) {
                (void) unlink(jname);
                fj = 0;
                offset = 0;}}
```

*FIG. 4P*

```
/*
 * write a filled jmp struct offset of the prev one (if any): nw()
 */
writejmps(ix)                                                          writejmps
        int     ix;
{
        char    *mktemp();

        if (!fj) {
                if (mktemp(jname) < 0) {
                        fprintf(stderr, "%s: can't mktemp() %s\n", prog, jname);
                        cleanup(1);
                }
                if ((fj = fopen(jname, "w")) == 0) {
                        fprintf(stderr, "%s: can't write %s\n", prog, jname);
                        exit(1);
                }
        }
        (void) fwrite((char *)&dx[ix].jp, sizeof(struct jmp), 1, fj);
        (void) fwrite((char *)&dx[ix].offset, sizeof(dx[ix].offset), 1, fj);
}
```

*FIG.4Q*

GTGCTCTCCGAGGACAAGCAGGAGGNGGTGGAGCTGGTGAAGCACCATCTGTGGGCTCTG
GAAGTGTGCTACATCTCAGCCTTGGTCTTGTCCTGCTTACTCACCTTCCTGGTCCTGATG
CGCTCACTGGTGACACACAGGACCAACCTTCGAGCTCTGCACCGAGGAGCTGCCCTGGAC
TTGAGTCCCTTGCATCGGAGTCCCCATCCCTCCCGCCAAGCCATATTCTGTTGGATGAGC
TTCAGTGCCTACCAGACAGCCTTTATCTGCCTTGGGCTCCTGGTGCAGCAGATCATCTTC
TTCCTGGGAACCACGGCCCTGGCCTTCCTGGTGCTCATGCCTGTGCTCCATGGCAGGAAC
CTCCTGCTCTTCCGTTCCCTGGAGTCCTCGTGGCCCTTCTGGCTGACTTTGGCCCTGGCT
GTGATCCTGCAGAACATGGCAGCCCATTGGGTCTTCCTGGAGACTCATGATGGACACCCA
CAGCTGACCAACCGGCGAGTGCTCTATGCAGCCACCTTTCTTCTCTTCCCCCTCAATGTG
CTGGTGGGTGCCATGGTGGCCACCTGGCGAGTGCTCCTCTCTGCCCTCTACAACGCCATC
CACCTTGGCCAGATGGACCTCAGCCTGCTGCCACCGAGAGCCGCCACTCTCGACCCCGGC
TACTACACGTACCGAA

*FIG.5*

CACAACCAGCCACCCCTCTAGGATCCCAGCCCAGCTGGTGCTGGGCTCAGAGGAGAAGGC
CCCGTGTTGGGAGCACCCTGCTTGCCTGGAGGGACAAGTTTCCGGGAGAGATCAATAAAG
GAAAGGAAAGAGACAAGGAAGGGAGAGGTCAGGAGAGCGCTTGATTGGAGGAGAAGGGCC
AGAGAATGTCGTCCCAGCCAGCAGGGAACCAGACCTCCCCCGGGGCCACAGAGGACTACT
CCTATGGCAGCTGGTACATCGATGAGCCCCAGGGGGGCGAGGAGCTCCAGCCAGAGGGGG
AAGTGCCCTCCTGCCACACCAGCATACCACCCGGCCTGTACCACGCCTGCCTGGCCTCGC
TGTCAATCCTTGTGCTGCTGCTCCTGGCCATGCTGGTGAGGCGCCGCCAGCTCTGGCCTG
ACTGTGTGCGTGGCAGGCCCGGCCTGCCCAGGCCCCGGGCAGTGCCTGCTGCTGTTTTCA
TGGTCCTCCTGAGCTCCCTGTGTTTGCTGCTCCCCGACGAGGACGCATTGCCCTTCCTGA
CTCTCGCCTCAGCACCCAGCCAAGATGGGAAAACTGAGGCTCCAAGAGGGGCCTGGAAGA
TACTGGGACTGTTCTATTATGCTGCCCTCTACTACCCTCTGGCTGCCTGTGCCACGGCTG
GCCACACAGCTGCACACCTGCTCGGCAGCACGCTGTCCTGGGCCCACCTTGGGGTCCAGG
TCTGGCAGAGGGCAGAGTGTCCCCAGGTGCCCAAGATCTACAAGTACTACTCCCTGCTGG
CCTCCCTGCCTCTCCTGCTGGGCCTCGGATTCCTGAGCCTTTGGTACCCTGTGCAGCTGG
TGAGAAGCTTCAGCCGTAGGACAGGAGCAGGCTCCAAGGGGCTGCAGAGCAGCTACTCTG
AGGAATATCTGAGGAACCTCCTTTGCAGGAAGAAGCTGGGAAGCAGCTACCACACCTCCA
AGCATGGCTTCCTGTCCTGGGCCCGCGTCTGCTTGAGACACTGCATCTACACTCCACAGC
CAGGATTCCATCTCCCGCTGAAGCTGGTGCTTTCAGCTACACTGACAGGGACGGCCATTT
ACCAGGTGGCCCTGCTGCTGCTGGTGGGCGTGGTACCCACTATCCAGAAGGTGAGGGCAG
GGGTCACCACGGATGTCTCCTACCTGCTGGCCGGCTTTGGAATCGTGCTCTCCGAGGACA
AGCAGGAGGTGGTGGAGCTGGTGAAGCACCATCTGTGGGCTCTGGAAGTGTGCTACATCT
CAGCCTTGGTCTTGTCCTGCTTACTCACCTTCCTGGTCCTGATGCGCTCACTGGTGACAC
ACAGGACCAACCTTCGAGCTCTGCACCGAGGAGCTGCCCTGGACTTGAGTCCCTTGCATC
GGAGTCCCCATCCCTCCCGCCAAGCCATATTCTGTTGGATGAGCTTCAGTGCCTACCAGA
CAGCCTTTATCTGCCTTGGGCTCCTGGTGCAGCAGATCATCTTCTTCCTGGGAACCACGG
CCCTGGCCTTCCTGGTGCTCATGCCTGTGCTCCATGGCAGGAACCTCCTGCTCTTCCGTT
CCCTGGAGTCCTCGTGGCCCTTCTGGCTGACTTTGGCCCTGGCTGTGATCCTGCAGAACA
TGGCAGCCCATTGGGTCTTCCTGGAGACTCATGATGGACACCCACAGCTGACCAACCGGC
GAGTGCTCTATGCAGCCACCTTTCTTCTCTTCCCCCTCAATGTGCTGGTGGGTGCCATAG
TGGCCACCTGGCGAGTGCTCCTCTCTGCCCTCTACAACGCCATCCACCTTGGCCAGATGG
ACCTCAGCCTGCTGCCACCGAGAGCCGCCACTCTCGACCCCGGCTACTACACGTACCGAA
ACTTCTTGAAGATTGAAGTCAGCCAGTCGCATCCAGCCATGACAGCCTTCTGCTCCCTGC
TCCTGCAAGCGCAGAGCCTCCTACCCAGGACCATGGCAGCCCCCCAGGACAGCCTCAGAC
CAGGGGAGGAAGACGAAGGGATGCAGCTGCTACAGACAAAGGACTCCATGGCCAAGGGAG
CTAGGCCCGGGGCCAGCCGCGGCAGGGCTCGCTGGGGTCTGGCCTACACGCTGCTGCACA
ACCCAACCCTGCAGGTCTTCCGCAAGACGGCCCTGTTGGGTGCCAATGGTGCCCAGCCCT
GAGGGCAGGGAAGGTCAACCCACCTGCCCATCTGTGCTGAGGCATGTTCCTGCCTACCAC
CTCCTCCCTCCCCGGCTCTCCTCCCAGCATCACACCAGCCATGCAGCCAGCAGGTCCTCC
GGATCACTGTGGTTGGGTGGAGGTCTGTCTGCACTGGGAGCCTCAGGAGGGCTCTGCTCC
ACCCACTTGGCTATGGGAGAGCCAGCAGGGGTTCTGGAGAAAGAAACTGGTGGGTTAGGG
CCTTGGTCCAGGAGCCAGTTGAGCCAGGGCAGCCACATCCAGGCGTCTCCCTACCCTGGC
TCTGCCATCAGCCTTGAAGGGCCTCGATGAAGCCTTCTCTGGAACCACTCCAGCCCAGCT
CCACCTCAGCCTTGGCCTTCACGCTGTGGAAGCAGCCAAGGCACTTCCTCACCCCCTCAG
CGCCACGGACCTCTCTGGGGAGTGGCCGGAAAGCTCCCGGGCCTCTGGCCTGCAGGGCAG
CCCAAGTCATGACTCAGACCAGGTCCCACACTGAGCTGCCCACACTCGAGAGCCAGATAT
TTTTGTAGTTTTTATGCCTTTGGCTATTATGAAAGAGGTTAGTGTGTTCCCTGCAATAAA
CTTGTTCCTGAGAAAAA

*FIG.6*

MSSQPAGNQTSPGATEDYSYGSWYIDEPQGGEELQPEGEVPSCHTSIPPGLYHACLASL
SILVLLLLAMLVRRRQLWPDCVRGRPGLPRPRAVPAAVFMVLLSSLCLLLPDEDALPFL
TLASAPSQDGKTEAPRGAWKILGLFYYAALYYPLAACATAGHTAAHLLGSTLSWAHLGV
QVWQRAECPQVPKIYKYYSLLASLPLLLGLGFLSLWYPVQLVRSFSRRTGAGSKGLQSS
YSEEYLRNLLCRKKLGSSYHTSKHGFLSWARVCLRHCIYTPQPGFHLPLKLVLSATLTG
TAIYQVALLLLVGVVPTIQKVRAGVTTDVSYLLAGFGIVLSEDKQEVVELVKHHLWALE
VCYISALVLSCLLTFLVLMRSLVTHRTNLRALHRGAALDLSPLHRSPHPSRQAIFCWMS
FSAYQTAFICLGLLVQQIIFFLGTTALAFLVLMPVLHGRNLLLFRSLESSWPFWLTLAL
AVILQNMAAHWVFLETHDGHPQLTNRRVLYAATFLLFPLNVLVGAIVATWRVLLSALYN
AIHLGQMDLSLLPPRAATLDPGYYTYRNFLKIEVSQSHPAMTAFCSLLLQAQSLLPRTM
AAPQDSLRPGEEDEGMQLLQTKDSMAKGARPGASRGRARWGLAYTLLHNPTLQVFRKTA
LLGANGAQP

Important features of the protein:

Signal peptide:
none

Transmembrane domain:
54-71
93-111
140-157
197-214
291-312
356-371
425-444
464-481
505-522

Motif name: N-glycosylation site.

8-12

Motif name: N-myristoylation site.

50-56
167-173
232-238
308-314
332-338
516-522
618-624
622-628
631-637
652-658

Motif name: Prokaryotic membrane lipoprotein lipid attachment site.

355-366

Motif name: ATP/GTP-binding site motif A (P-loop).

123-131

*FIG. 7*

STRA6 VARIANT CLONES

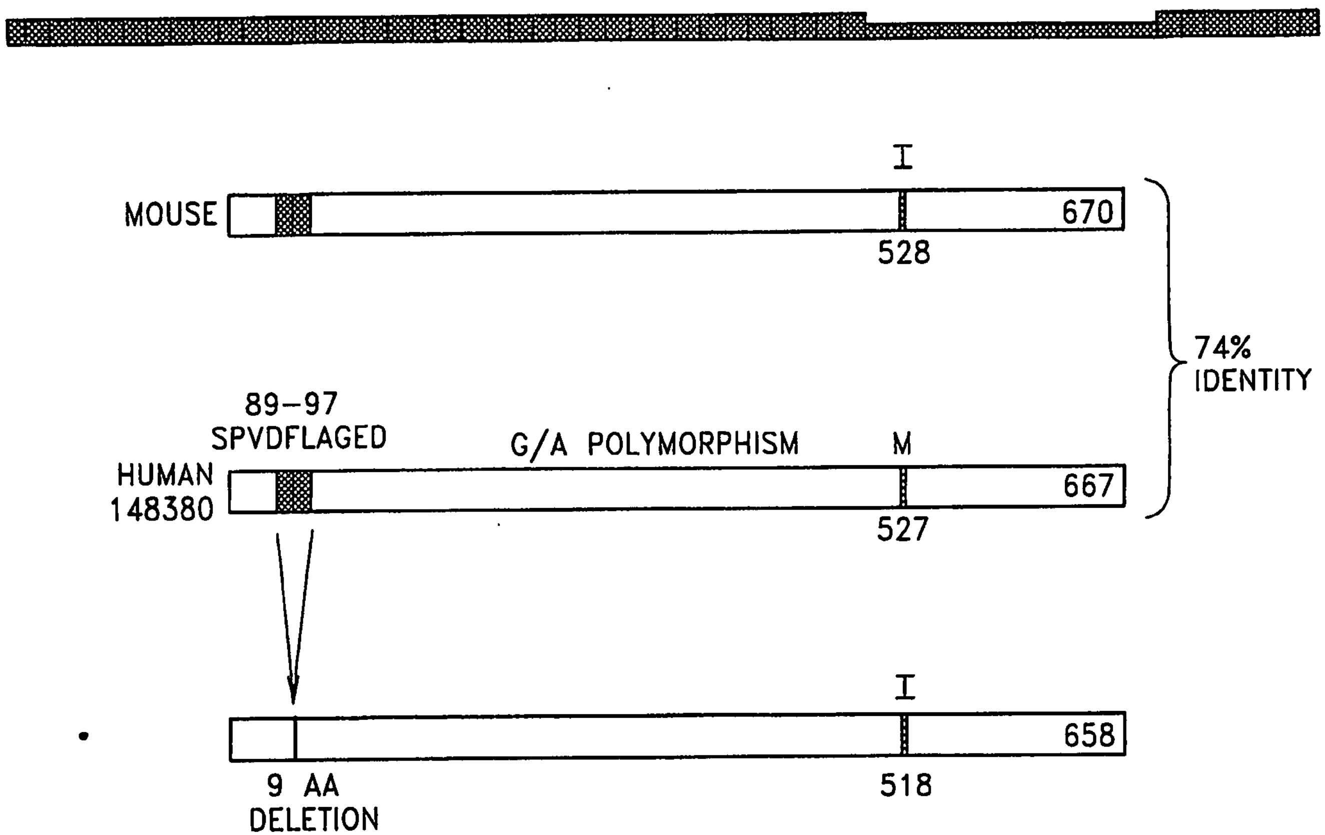


*FIG.8*

HYDROPHOBICITY PLOT OF HUMAN STRA6

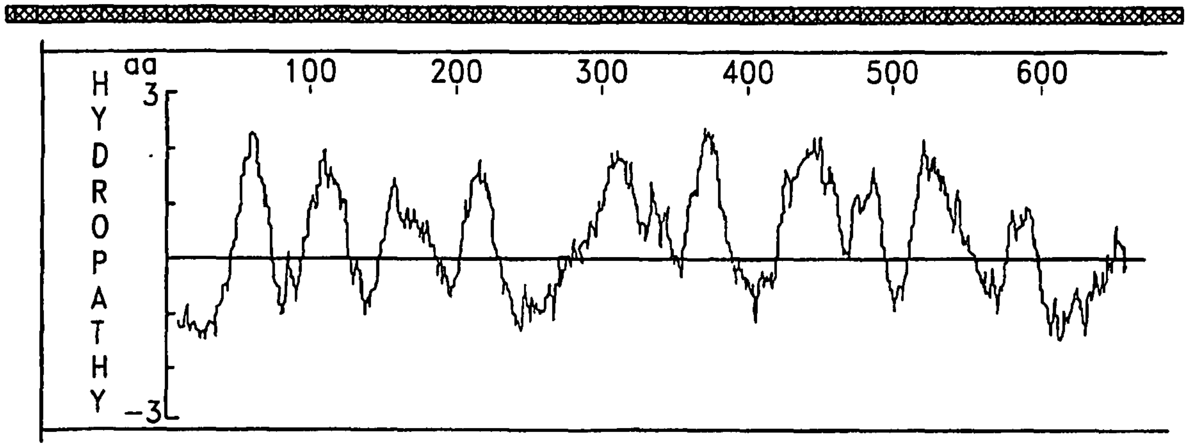


- 3 kb mRNA
- 667 AMINO ACIDS --->50% RESIDUES HYDROPHOBIC
- 73.5 kDa PROTEIN

- 9 POTENTIAL TRANSMEMBRANE DOMAINS

*FIG.9*

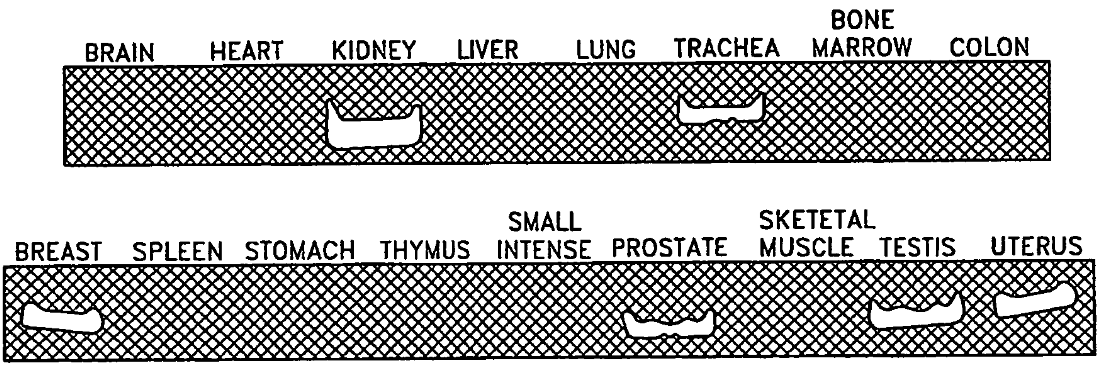
BRAIN
HEART
KIDNEY
LIVER
LUNG
TRACHEA
BONE MARROW
COLON
BREAST
SPLEEN
STOMACH
THYMUS
SMALL INTENSE
PROSTATE
SKETETAL MUSCLE
TESTIS
UTERUS

*FIG. 10*

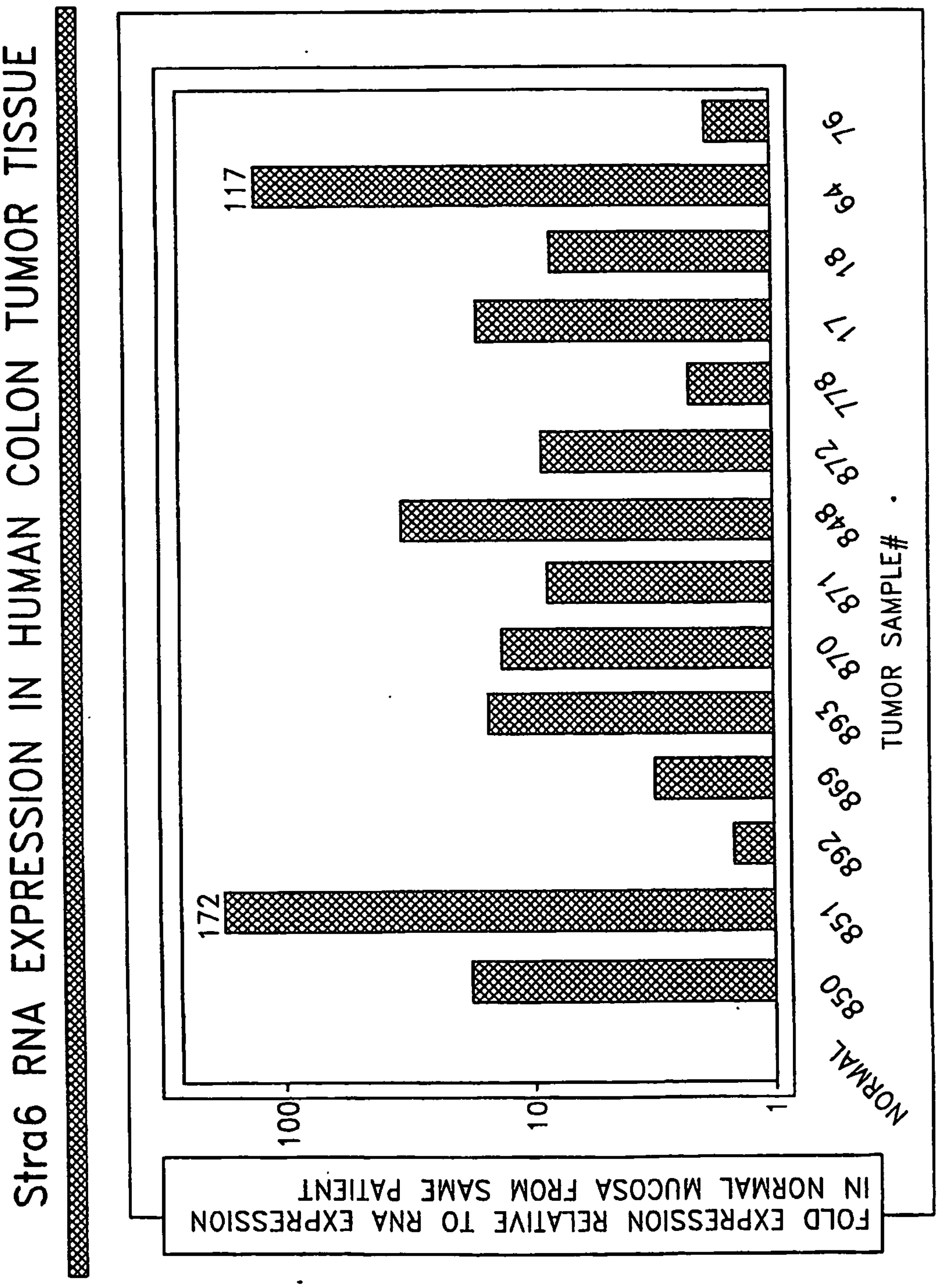
Stra6 RNA EXPRESSION IN HUMAN COLON TUMOR TISSUE
FOLD EXPRESSION RELATIVE TO RNA EXPRESSION IN NORMAL MUCOSA FROM SAME PATIENT
100
10
1
172
117
NORMAL
850
851
892
869
893
870
871
848
872
778
17
18
64
76
TUMOR SAMPLE#

*FIG. 11*

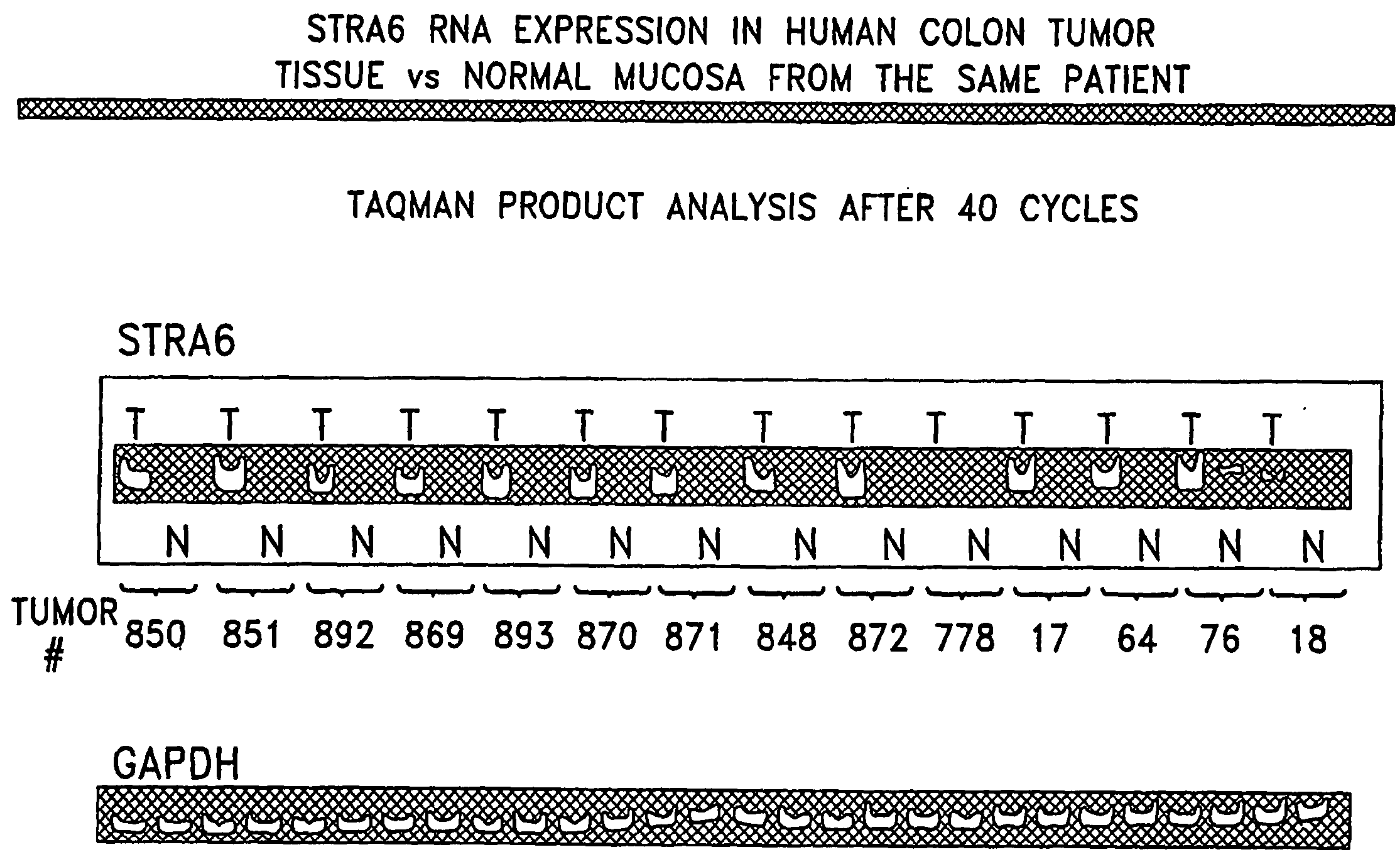
STRA6 RNA EXPRESSION IN HUMAN COLON TUMOR
TISSUE vs NORMAL MUCOSA FROM THE SAME PATIENT
TAQMAN PRODUCT ANALYSIS AFTER 40 CYCLES
STRA6
T N
TUMOR #
850 851 892 869 893 870 871 848 872 778 17 64 76 18
GAPDH

FIG. 12A

C

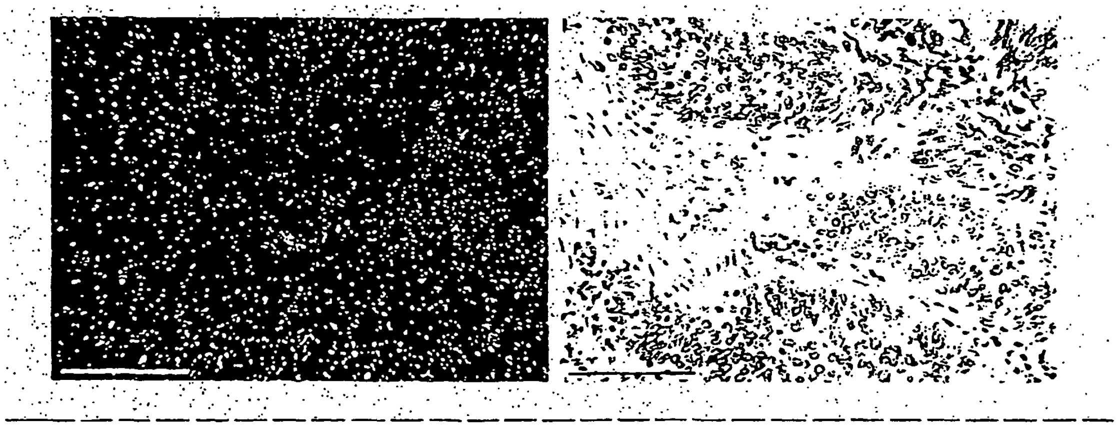

FIG. 12B

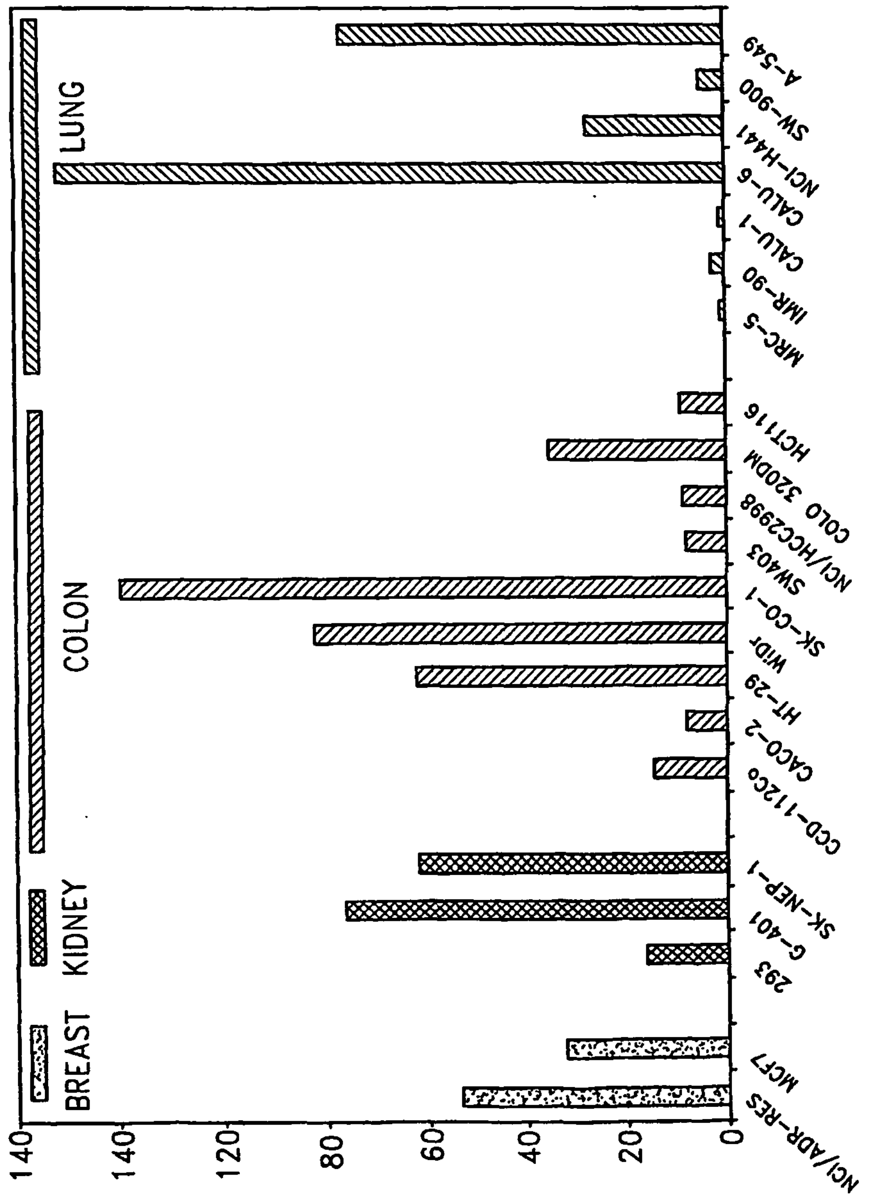
BREAST
KIDNEY
COLON
LUNG
140
140
120
80
60
40
20
0
NCI/ADR-RES
MCF7
293
G-401
SK-NEP-1
CCD-112Co
CACO-2
HT-29
WiDr
SK-CO-1
SW403
NCI/HCC2998
COLO 320DM
HCT116
MRC-5
IMR-90
CALU-1
CALU-6
NCI-H441
SW-900
A-549

FIG. 13

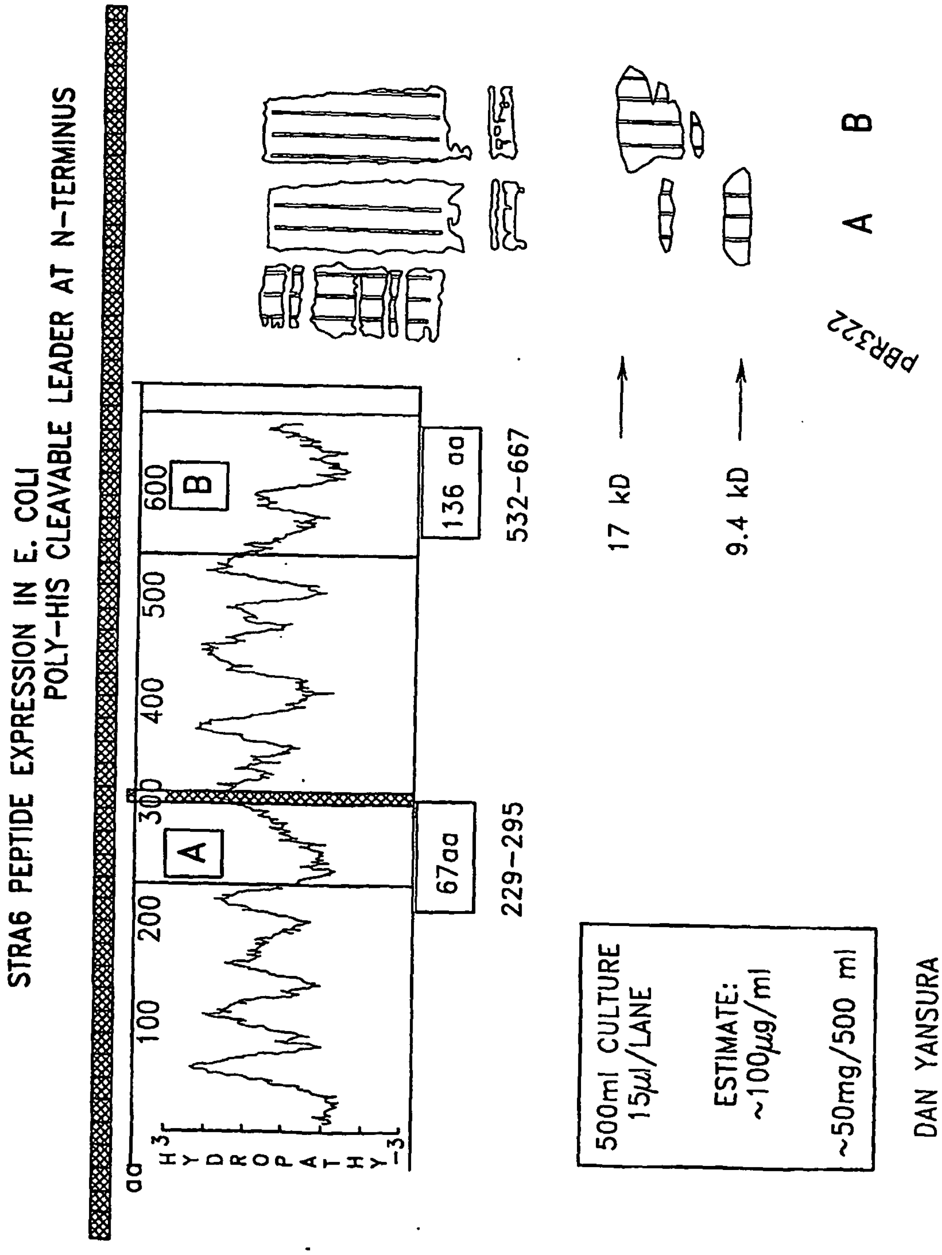
STRA6 PEPTIDE EXPRESSION IN E. COLI
POLY-HIS CLEAVABLE LEADER AT N-TERMINUS
aa
100
200
300
400
500
600
HYDROPATHY
3
-3
A
B
67aa
136 aa
229-295
532-667
17 kD
9.4 kD
pBR322
A
B
500ml CULTURE
15μl/LANE
ESTIMATE:
~100μg/ml
~50mg/500 ml
DAN YANSURA

FIG.14

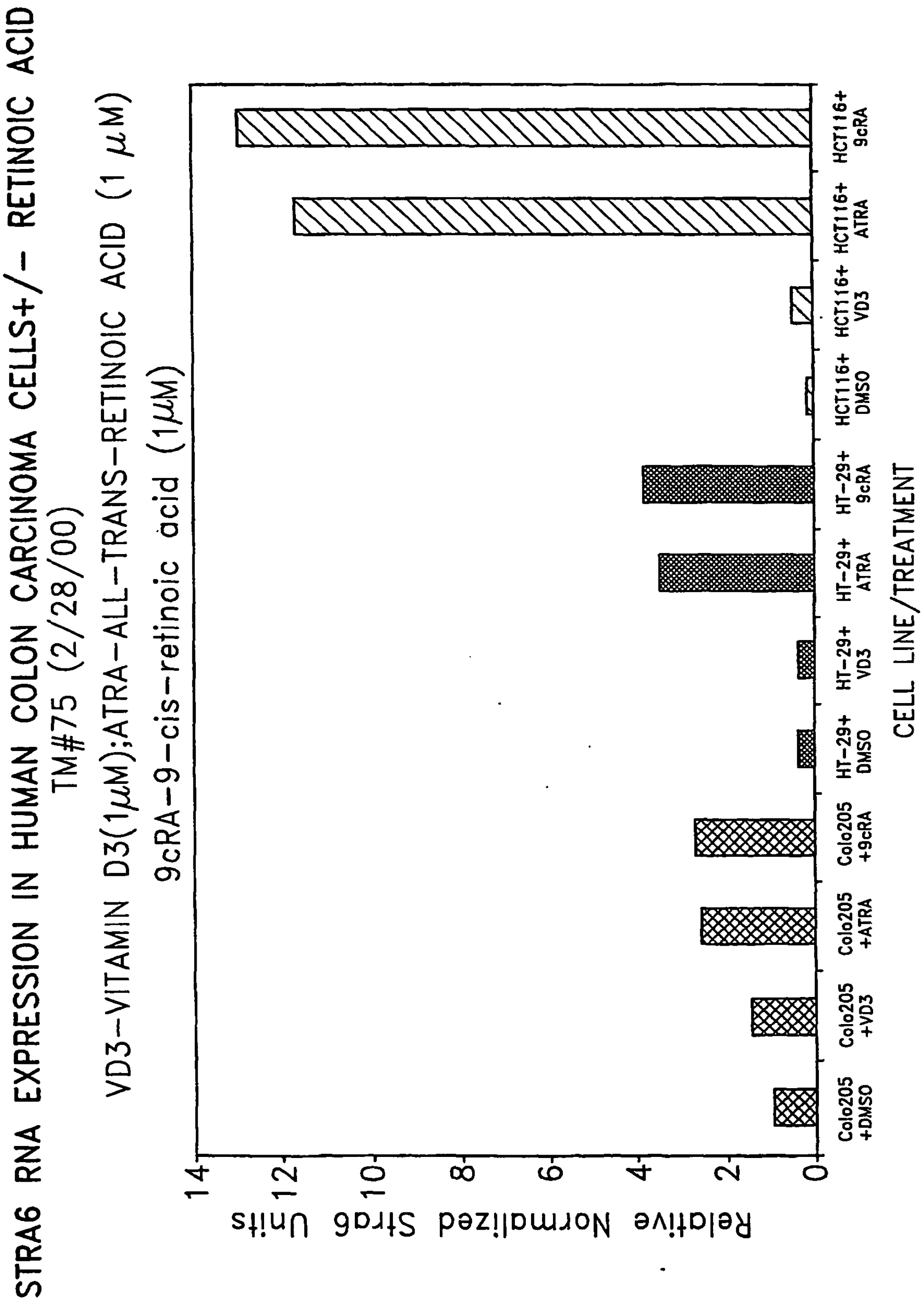
STRA6 RNA EXPRESSION IN HUMAN COLON CARCINOMA CELLS+/- RETINOIC ACID
TM#75 (2/28/00)
VD3-VITAMIN D3(1μM);ATRA-ALL-TRANS-RETINOIC ACID (1 μM)
9cRA-9-cis-retinoic acid (1μM)
Relative Normalized Stra6 Units
0
2
4
6
8
10
12
14
Colo205 +DMSO
Colo205 +VD3
Colo205 +ATRA
Colo205 +9cRA
HT-29+ DMSO
HT-29+ VD3
HT-29+ ATRA
HT-29+ 9cRA
HCT116+ DMSO
HCT116+ VD3
HCT116+ ATRA
HCT116+ 9cRA
CELL LINE/TREATMENT

*FIG. 15*

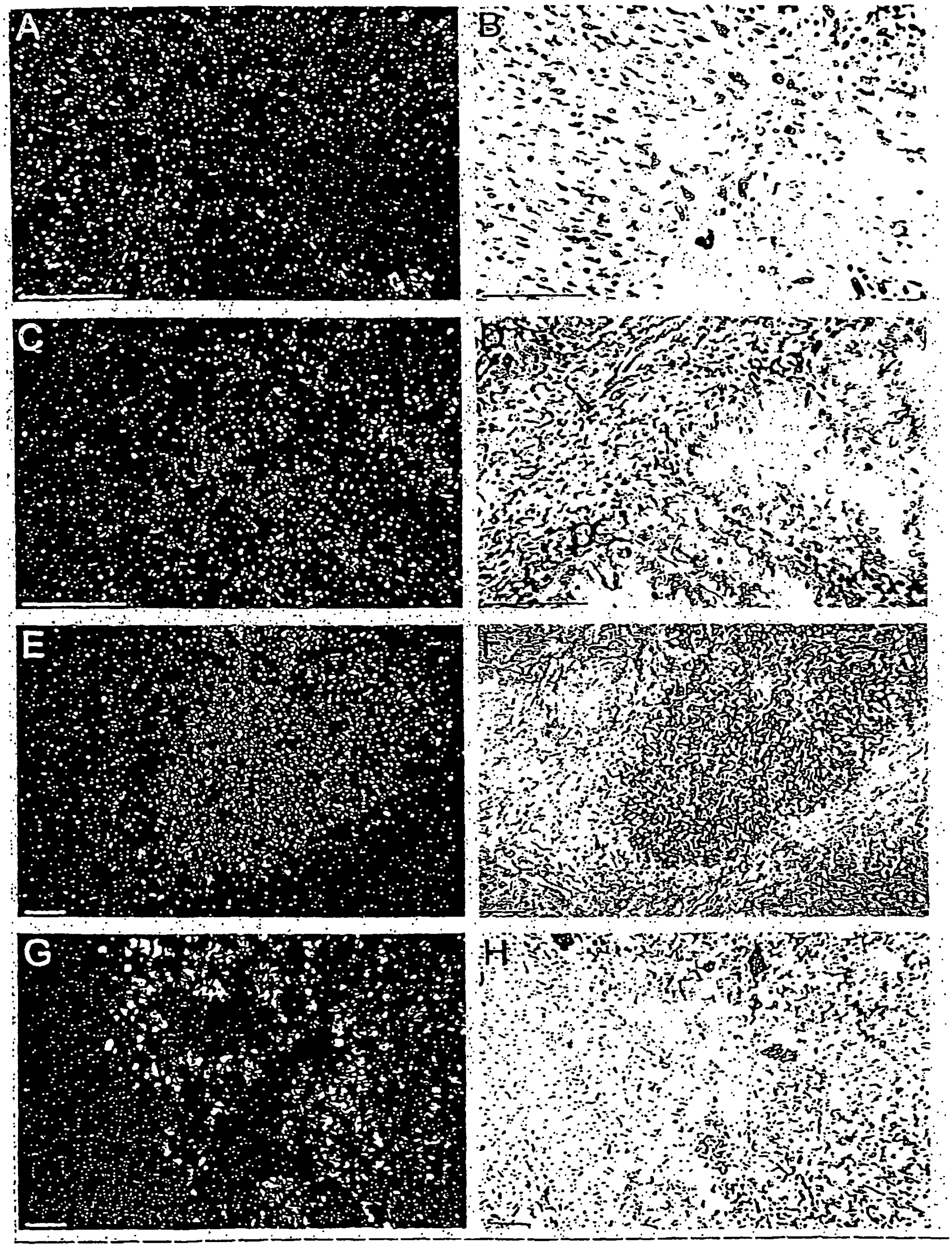


FIG.16

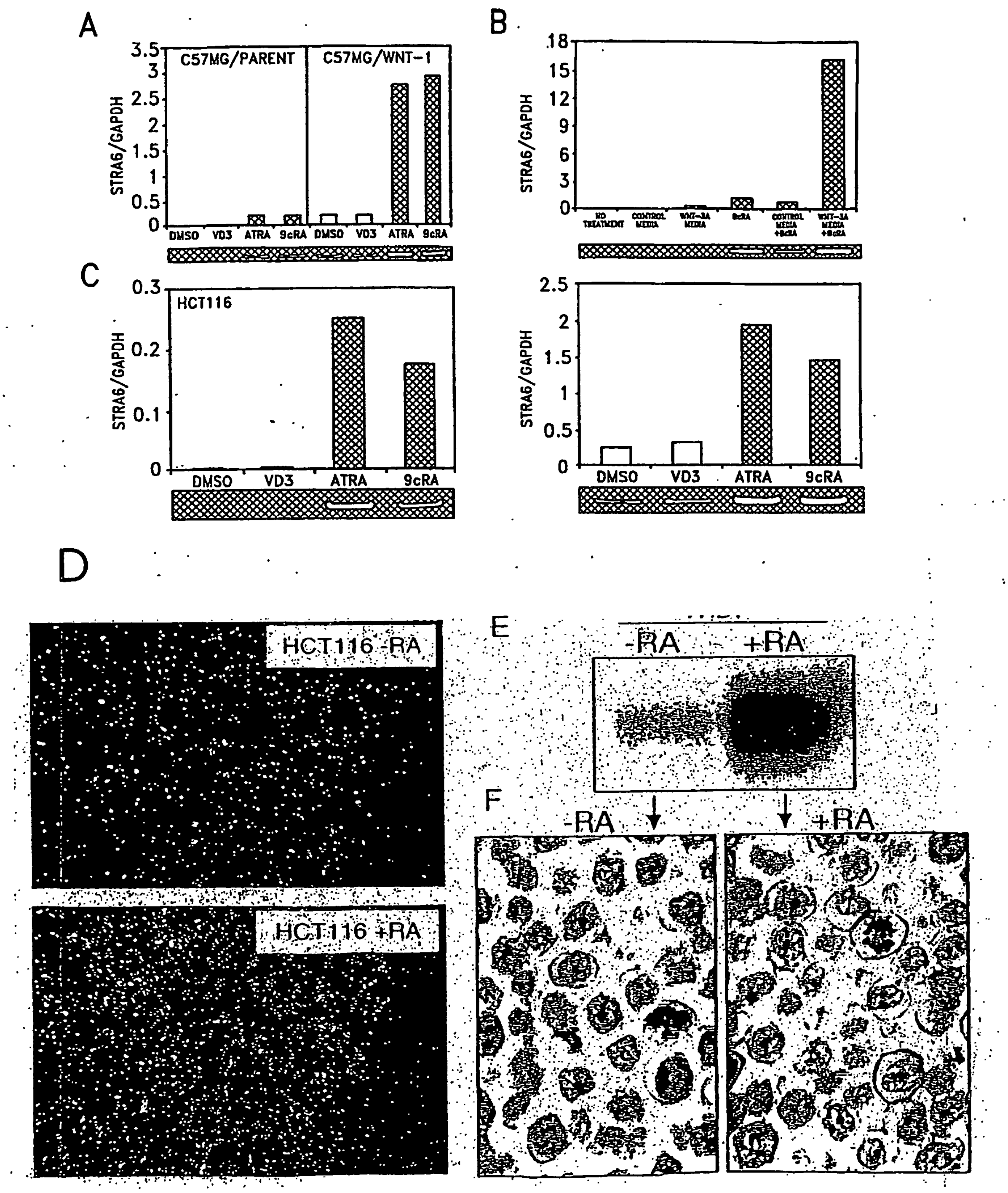
A
B
C
D
E
F
C57MG/PARENT
C57MG/WNT-1
STRA6/GAPDH
3.5
3
2.5
2
1.5
1
0.5
0
DMSO
VD3
ATRA
9cRA
18
15
12
9
6
3
0
HCT116
0.3
0.2
0.1
2.5
HCT116 -RA
HCT116 +RA
-RA
+RA

*FIG. 17*

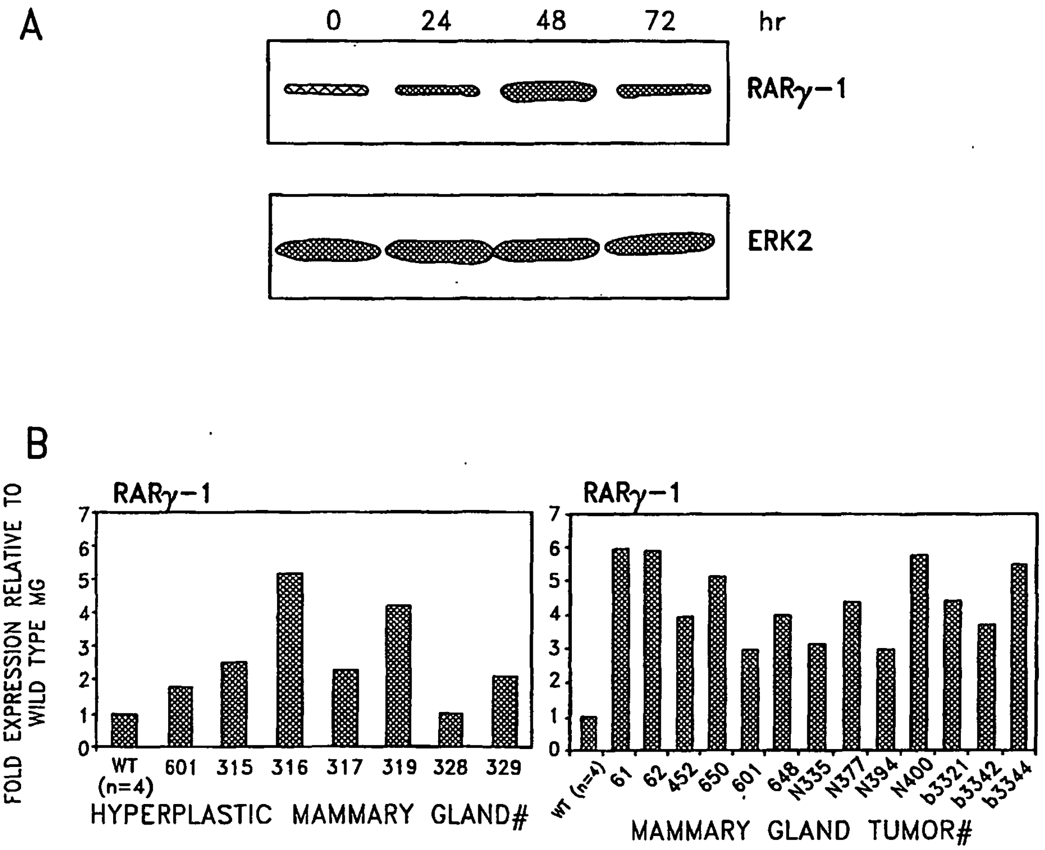
A
0
24
48
72
hr
RARγ-1
ERK2
B
FOLD EXPRESSION RELATIVE TO WILD TYPE MG
RARγ-1
7
6
5
4
3
2
1
0
WT (n=4)
601
315
316
317
319
328
329
HYPERPLASTIC MAMMARY GLAND#
RARγ-1
WT (n=4)
61
62
452
650
601
648
N335
N377
N394
N400
b3321
b3342
b3344
MAMMARY GLAND TUMOR#

*FIG. 18*

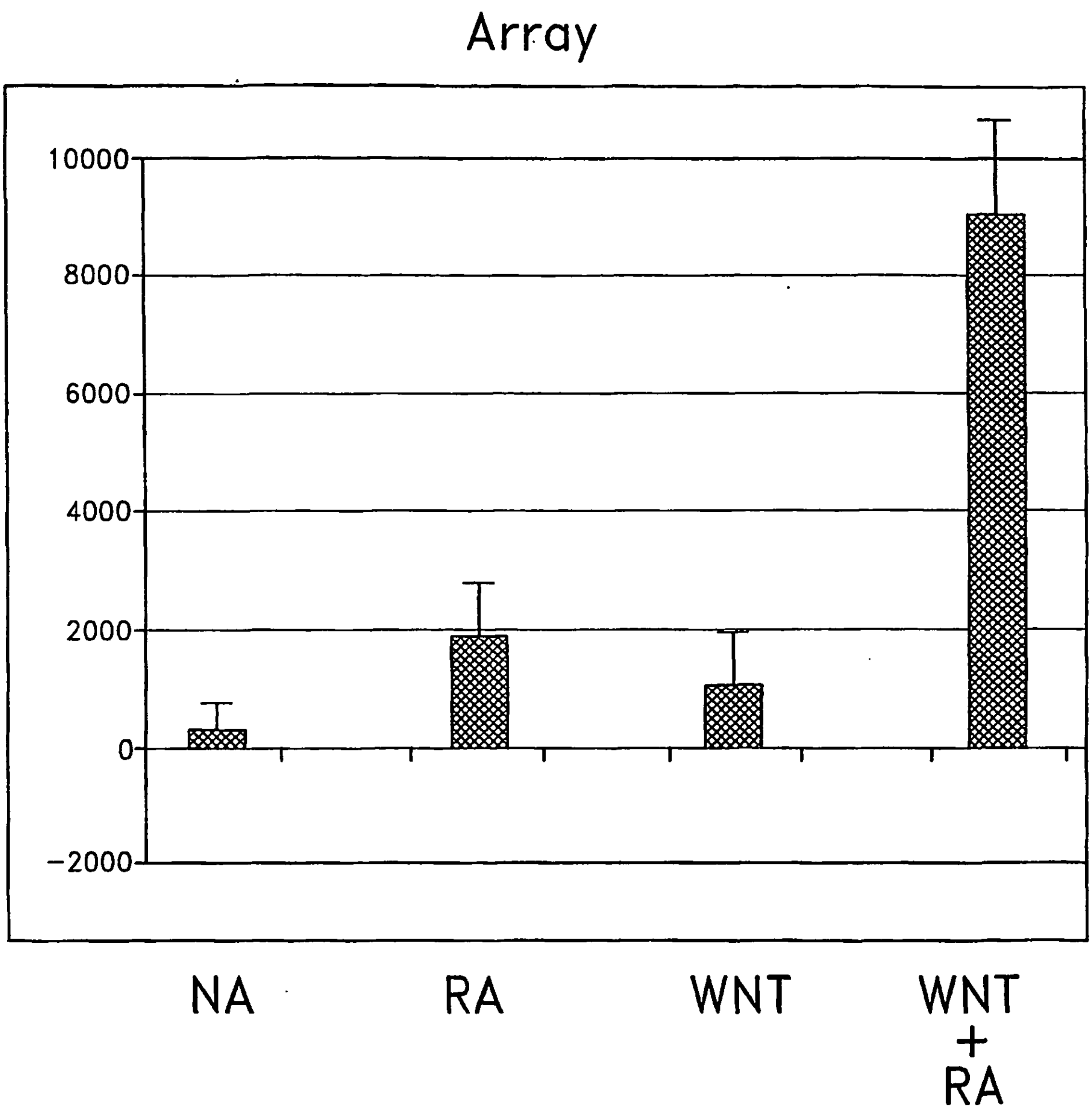


*FIG. 19*

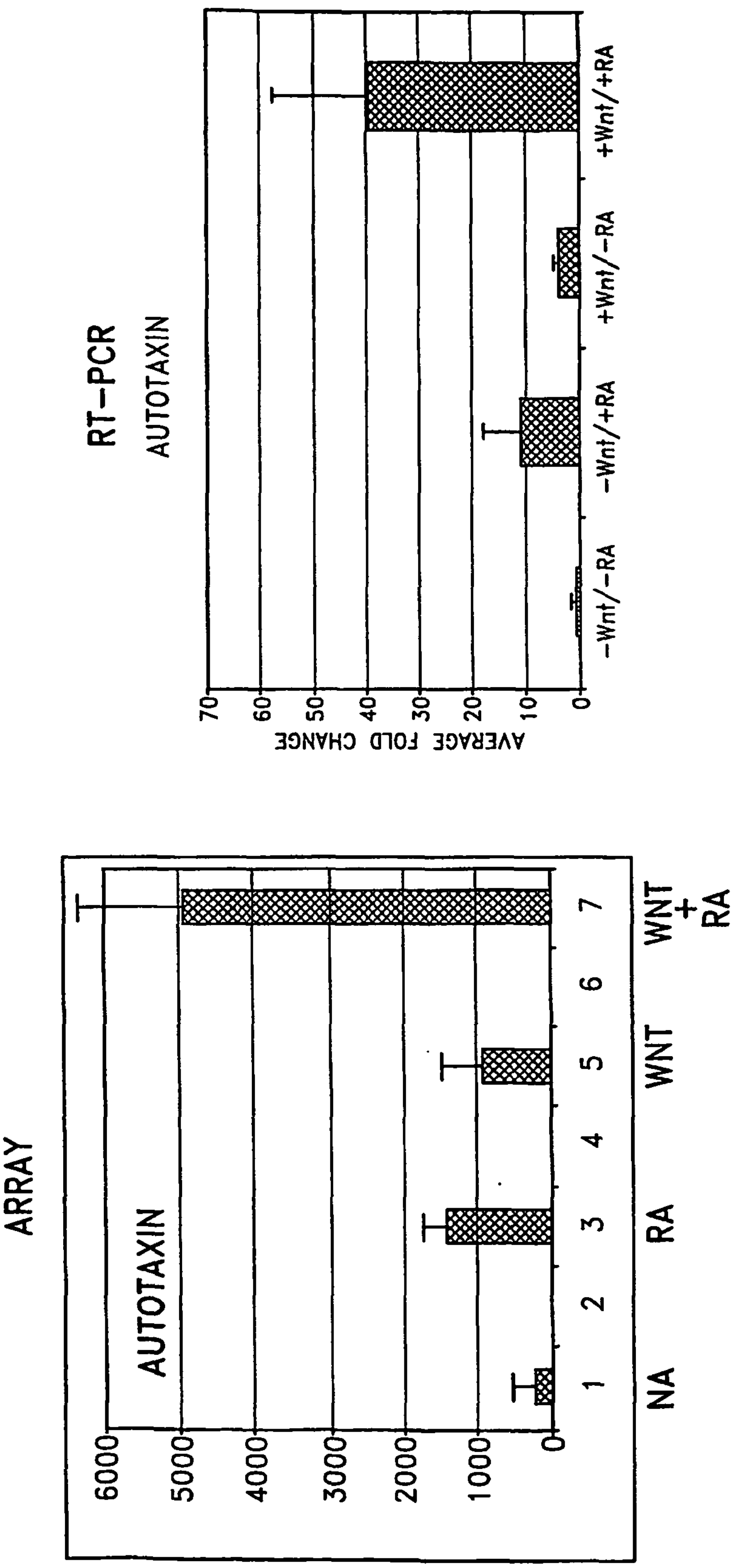
RT–PCR
AUTOTAXIN
AVERAGE FOLD CHANGE
70
60
50
40
30
20
10
0
–Wnt/–RA
–Wnt/+RA
+Wnt/–RA
+Wnt/+RA
ARRAY
AUTOTAXIN
6000
5000
4000
3000
2000
1000
0
1
2
3
4
5
6
7
NA
RA
WNT
WNT
+
RA

FIG.20

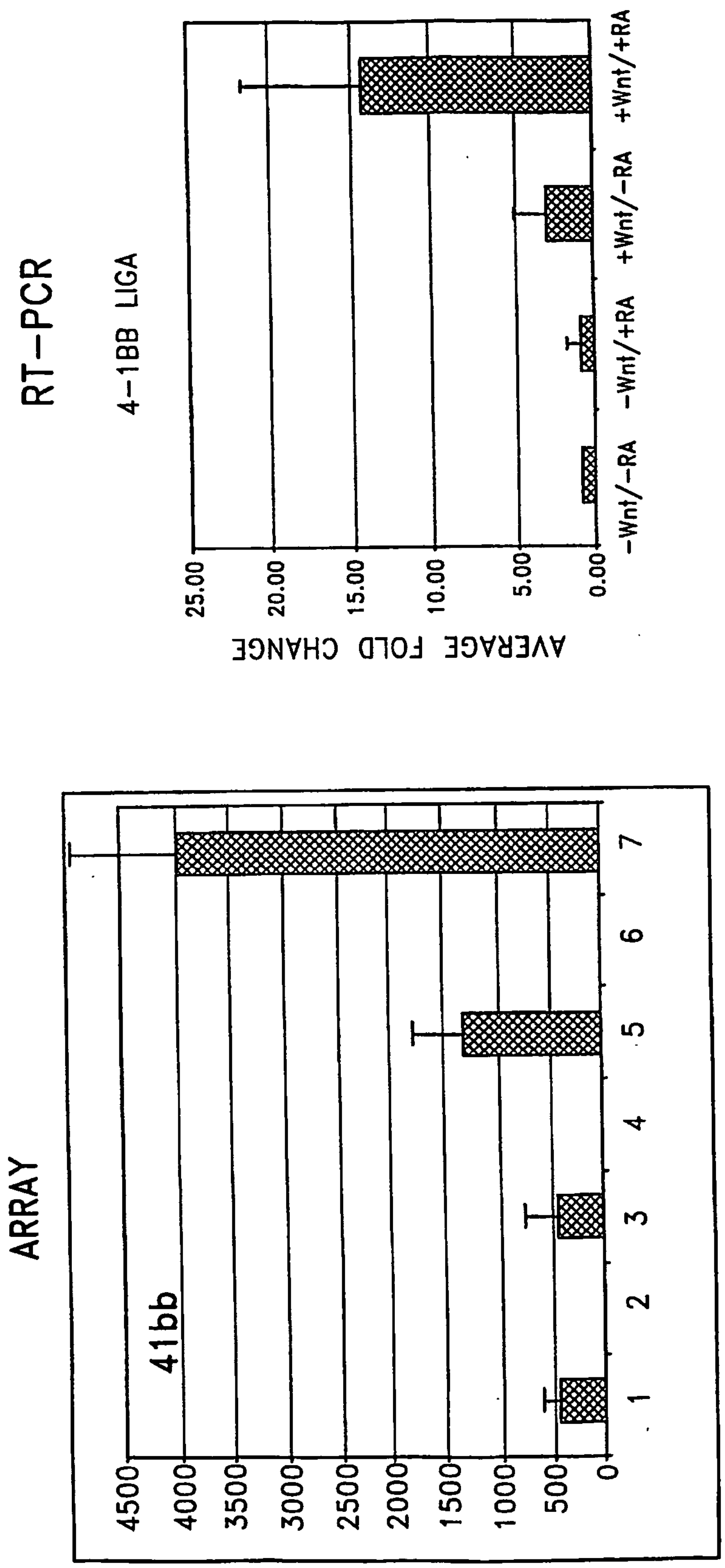
ARRAY
41bb
4500
4000
3500
3000
2500
2000
1500
1000
500
0
1
2
3
4
5
6
7
NA
RA
WNT
WNT + RA
RT-PCR
4-1BB LIGA
AVERAGE FOLD CHANGE
25.00
20.00
15.00
10.00
5.00
0.00
-Wnt/-RA
-Wnt/+RA
+Wnt/-RA
+Wnt/+RA

FIG. 21

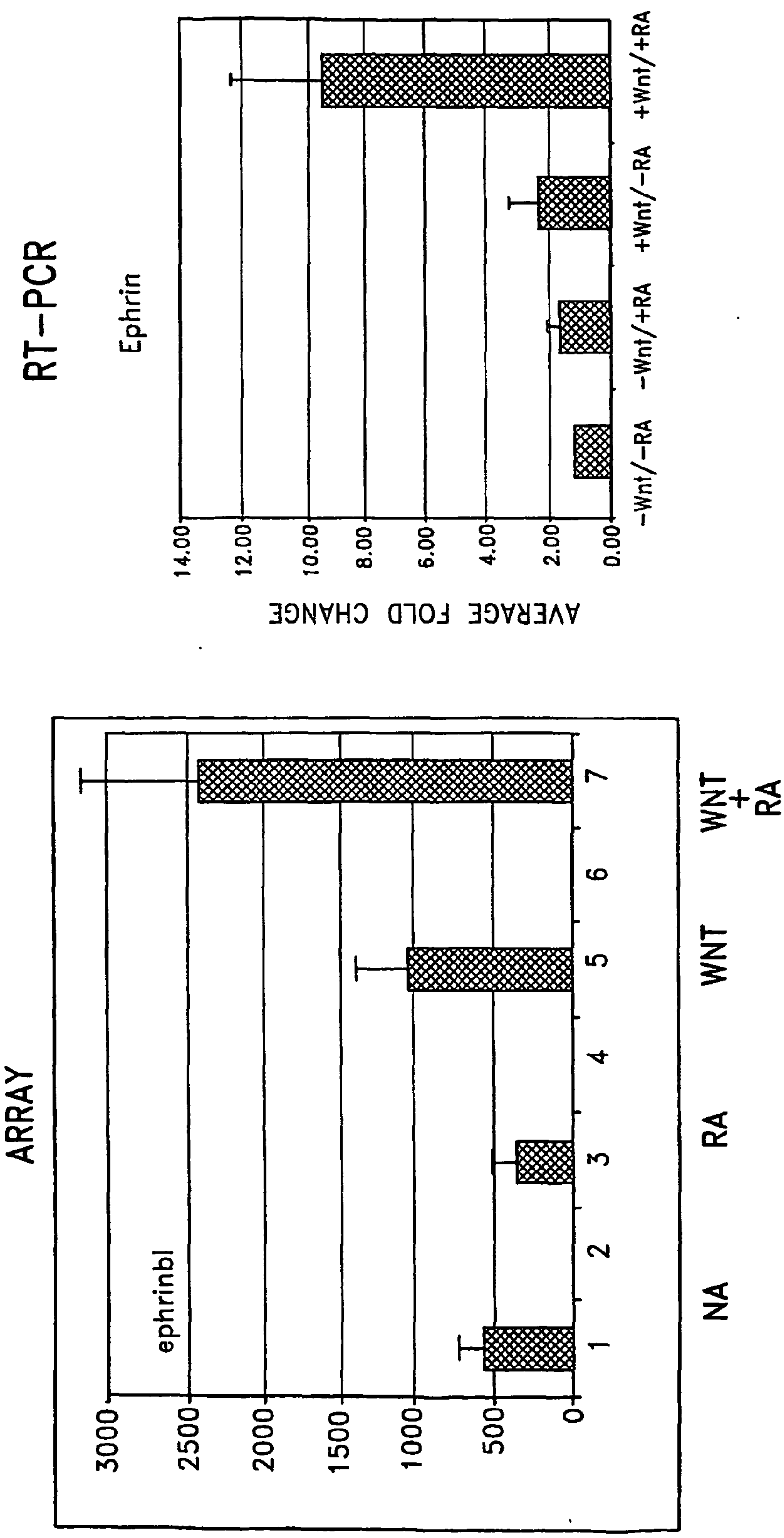
ARRAY
ephrinbl
3000
2500
2000
1500
1000
500
0
1
2
3
4
5
6
7
NA
RA
WNT
WNT + RA
RT–PCR
Ephrin
AVERAGE FOLD CHANGE
14.00
12.00
10.00
8.00
6.00
4.00
2.00
0.00
–Wnt/–RA
–Wnt/+RA
+Wnt/–RA
+Wnt/+RA

*FIG.22*

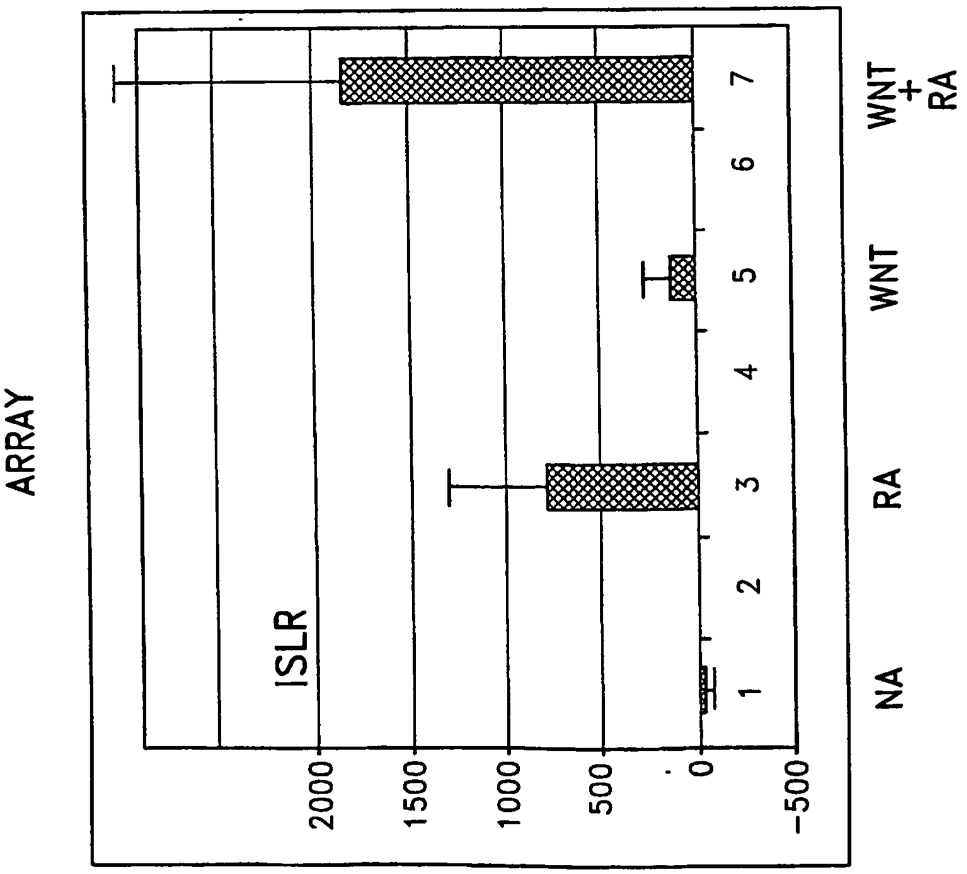
ARRAY
ISLR
2000
1500
1000
500
0
-500
1
2
3
4
5
6
7
NA
RA
WNT
WNT + RA

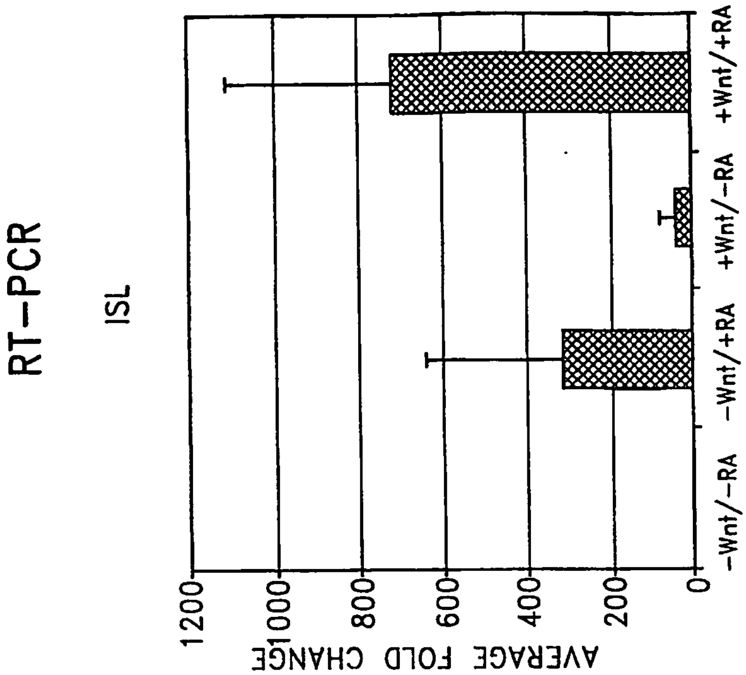
RT-PCR
ISL
AVERAGE FOLD CHANGE
1200
1000
800
600
400
200
0
-Wnt/-RA
-Wnt/+RA
+Wnt/-RA
+Wnt/+RA

*FIG.23*

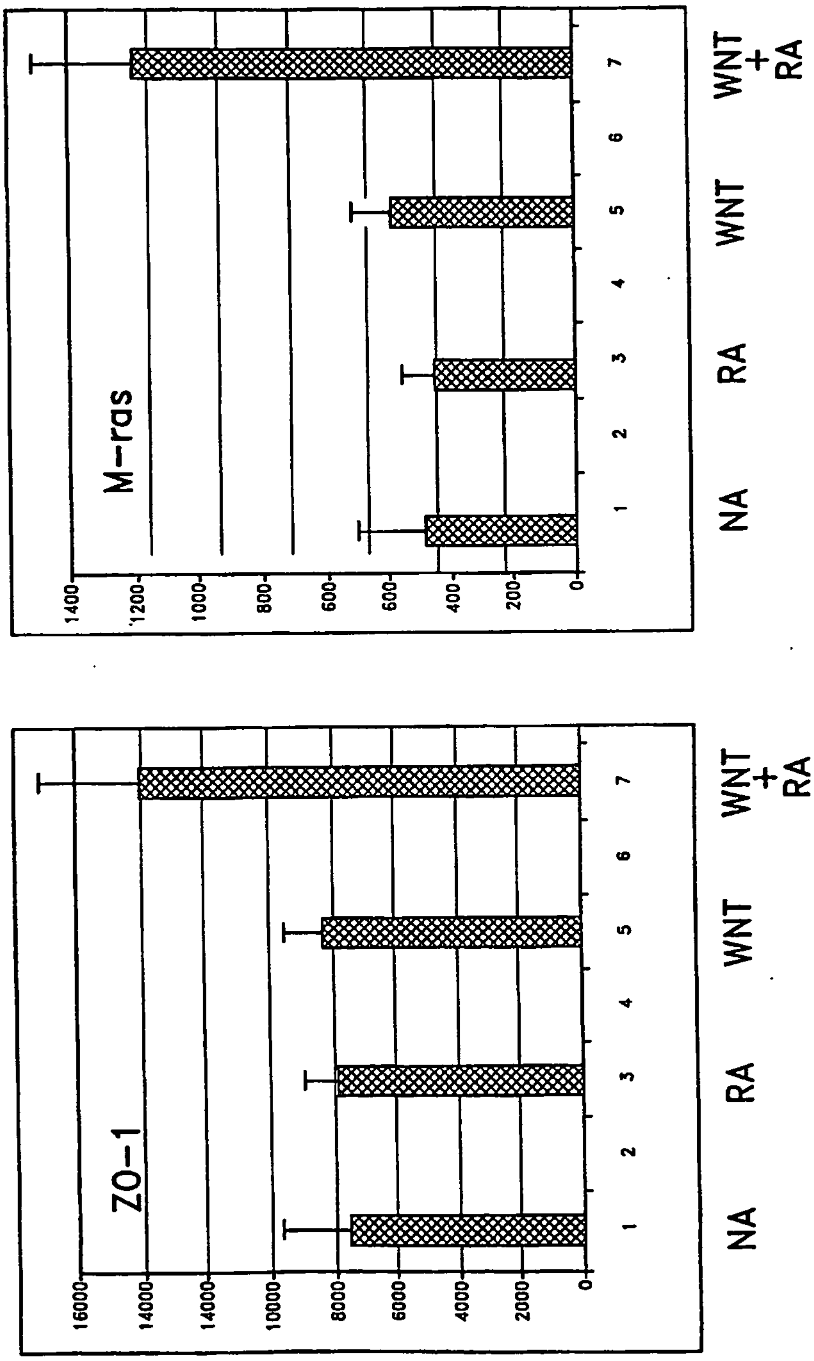


FIG.24

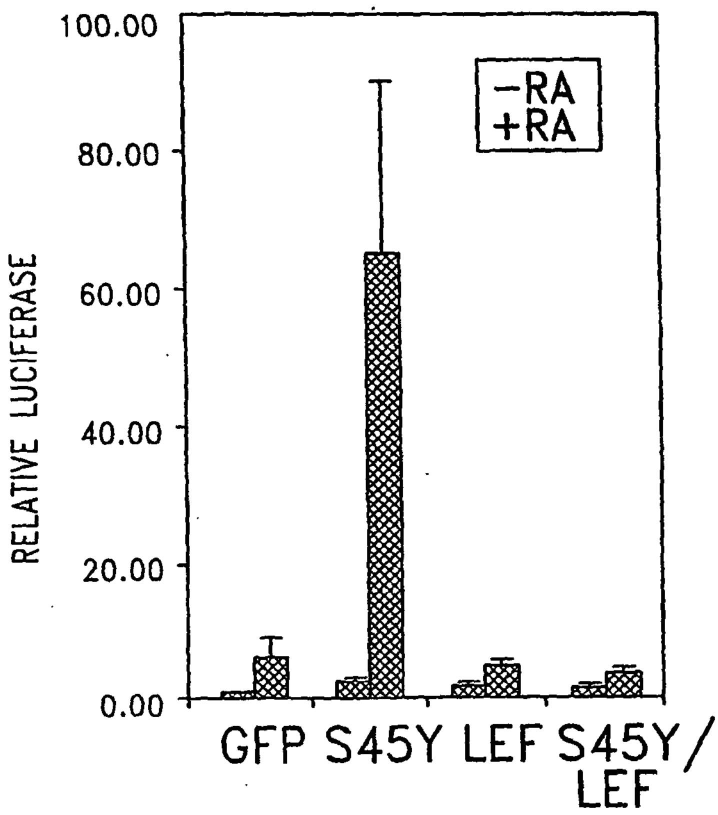


*FIG.25A*

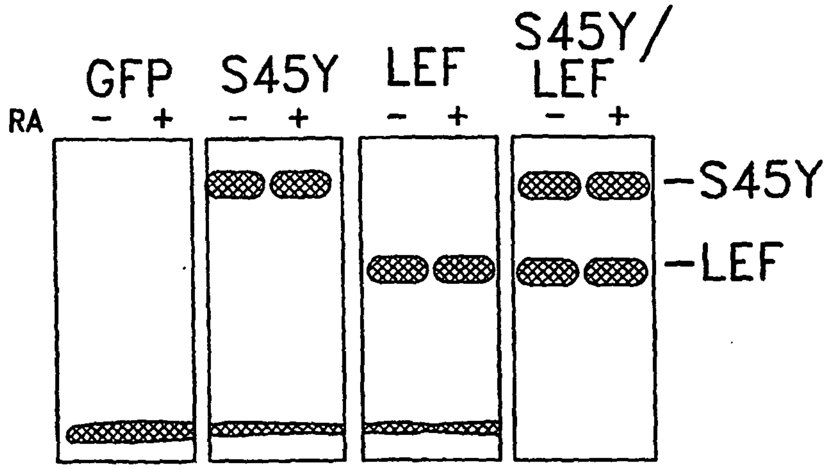


*FIG.25B*

Stra6 mRNA IN NORMAL MOUSE MAMMARY GLAND AND Wnt-1MAMMARY GLAND TUMORS
NUDE MICE BEARING WNT-1 TUMOR EXPLANTS WERE GIVEN ATRA PERI-TUMORALLY(PT) AT 100 AND 400mg/kg.
TUMORS AND NORMAL ADJACENT MAMMARY GLANDS WERE HARVESTED 8 HOURS LATER.

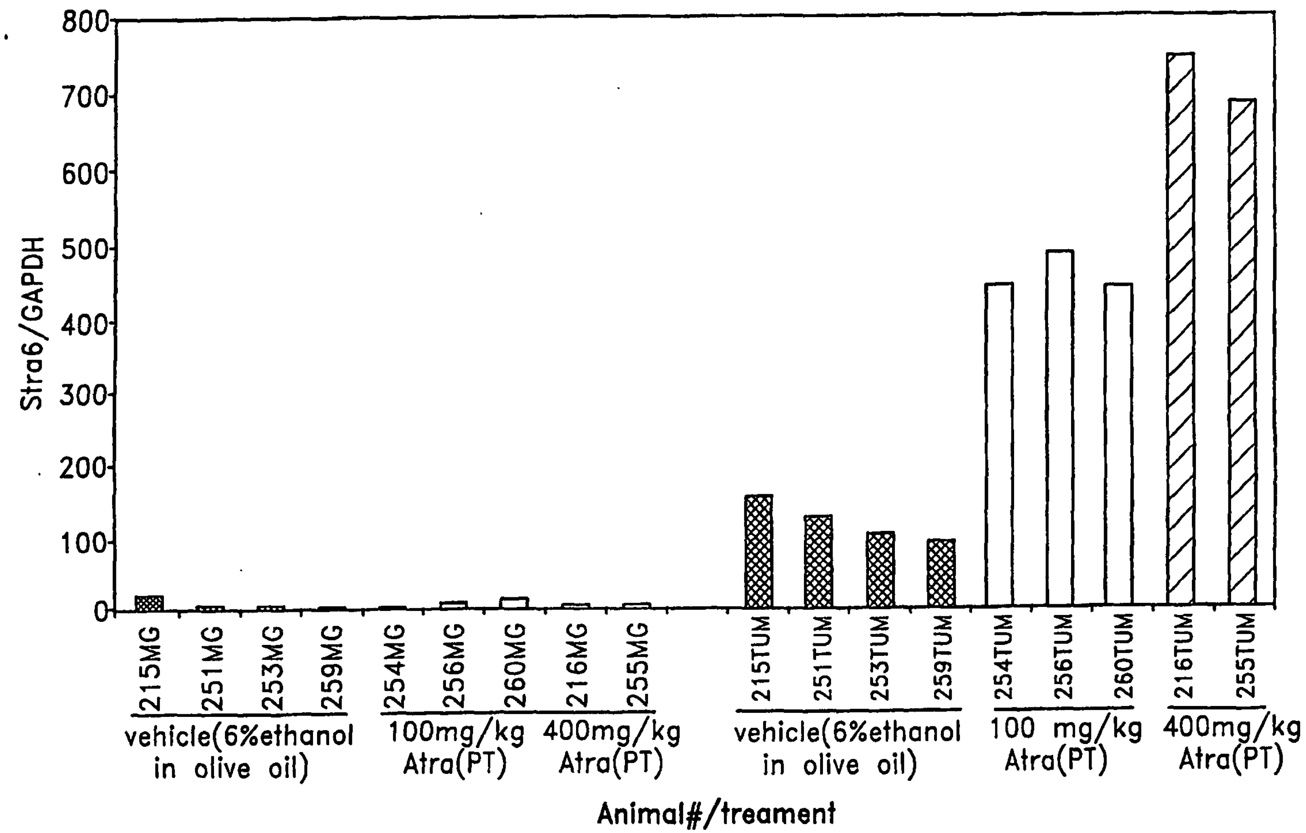


FIG.26

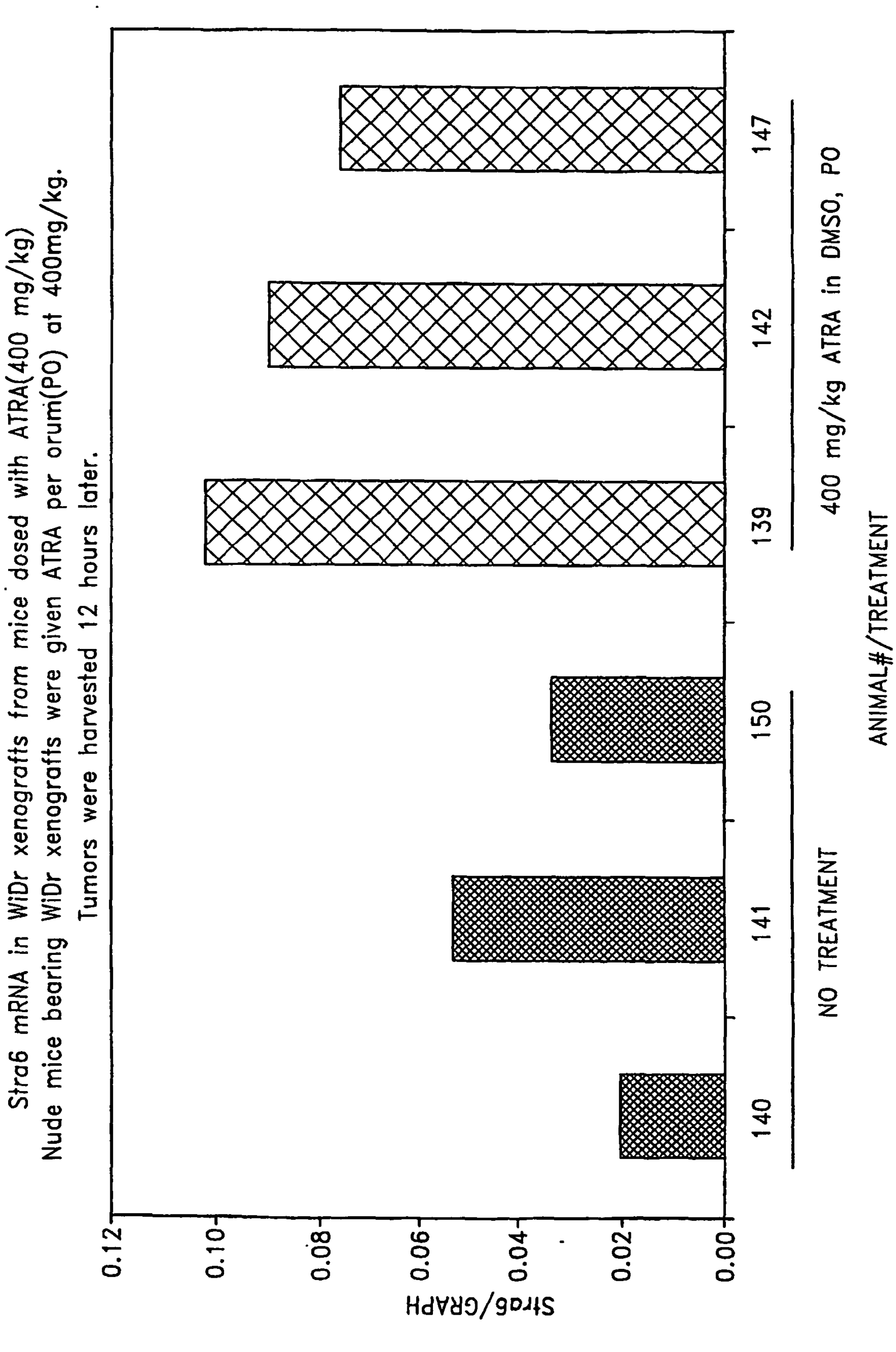
Stra6 mRNA in WiDr xenografts from mice dosed with ATRA(400 mg/kg)
Nude mice bearing WiDr xenografts were given ATRA per orum(PO) at 400mg/kg.
Tumors were harvested 12 hours later.
Stra6/GRAPH
0.12
0.10
0.08
0.06
0.04
0.02
0.00
140
141
150
139
142
147
NO TREATMENT
400 mg/kg ATRA in DMSO, PO
ANIMAL#/TREATMENT

FIG.27

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 4816567 A
- EP 404097 A
- WO 9311161 A
- US 4275149 A
- US 5053395 A
- US 5770710 A
- US 5877296 A
- US 4675187 A
- US 0110482 W
- WO 97133551 A
- US 4873191 A, Hoppe and Wanger
- US 4736866 A
- WO 9733551 A
- US 5545807 A
- US 5545806 A
- US 5569825 A
- US 5625126 A
- US 5633425 A
- US 5661016 A
- WO 9308829 A
- WO 9627011 A
- US 4676980 A
- WO 9100360 A
- WO 92200373 A
- EP 03089 A
- WO 9411026 A
- US 5208020 A
- US 5416064 A
- EP 0425235 B1
- US 4137230 A
- US 4248870 A
- US 4256746 A
- US 4260608 A
- US 4265814 A
- US 4294747 A
- US 4307016 A
- US 4308268 A
- US 4308269 A
- US 4309428 A
- US 4313946 A
- US 4315929 A
- US 4317821 A
- US 4322348 A
- US 4331598 A
- US 4361650 A
- US 4364866 A
- US 4424219 A
- US 4450254 A
- US 4362663 A
- US 4371533 A
- US 5712374 A
- US 5714586 A
- US 5739116 A
- US 5767285 A
- US 5770701 A
- US 5773001 A
- US 4485045 A
- US 4544545 A
- US 5013556 A
- WO 8101145 A
- WO 8807378 A
- US 4975278 A
- US 3773919 A
- EP 616812 A
- PE 148380 A
- PE 148380 B
- EP 307247 A
- US 5122469 A
- WO 8403564 A
- EP 05023231 A
- EP 01950996 A
- US 0121635 W
- US 60228914 B
- US 09759056 B
- US 09901812 B


### Non-patent literature cited in the description


- **Zhang et al.** *Science,* 1997, vol. 276, 1268-1272
- **He et al.** *Science,* 1998, vol. 281, 1509-1512
- **Tetsu ; McCormick.** *Nature,* 1999, vol. 38, 422-426
- **Barker et al.** *Adv Cancer Res,* 2000, vol. 77, 1-24
- **Polakis.** *Genes Dev,* 2000, vol. 14, 1837-1851
- **Rubinfeld et al.** *Science,* 1997, vol. 275, 1790-1792
- **Korinek et al.** *Science,* 1997, vol. 275, 1784-1787

- **Wodarz ; Nusse.** *Annu Rev Cell Dev Biol,* 1998, vol. 14, 59-88
- **Nusse ; Varmus.** *Cell,* 1982, vol. 31, 99-109
- **Polakis.** *Curr. Opin. Genet. Dev.,* 1999, vol. 9, 15-21
- **Kobayashi et al.** *Jpn J Cancer Res,* 1999, vol. 90, 55-9
- **Koesters et al.** *Cancer Res,* 1999, vol. 59, 3880-2
- **Palacios ; Hamallo.** *Cancer Res,* 1998, vol. 58, 1344-7
- **Rimm et al.** *Am J Pathol,* 1999, vol. 154, 325-9
- **Rubinfeld et al.** *Science,* 1993, vol. 262, 1731-1734
- **Wright et al.** *Int J Cancer,* 1999, vol. 82, 625-9
- **Peifer ; Polakis.** *Science,* 2000, vol. 287, 1606-1609
- **Miller ; Moon.** *Genes & Dev.,* 1996, vol. 10, 2527-2539
- **Molenaar.** *Cell,* 1996, vol. 86, 391-399
- **Behrens et al.** *Nature,* 1996, vol. 382, 638-642
- **Easwaran et al.** *Curr Biol,* 1999, vol. 9, 1415-1418
- **Glennie ; Johnson.** *Immunol Today,* 2000, vol. 21, 403-410
- **Singleton et al.** Dictionary of Microbiology and Molecular Biology. J. Wiley & Sons, 1994
- **Sambrook et al.** Molecular Cloning, A laboratory Manual. Cold Springs Harbor Press, 1989
- **Bouillet et al.** *Mechanisms of Development,* 1997, vol. 63, 173-186
- **Altschul et al.** *Nucleic Acids Res.,* 1997, vol. 25, 3389-3402
- **Zhou et al.** *Immunol Lett,* 1995, vol. 45, 67-73
- **Davis et al.** *Science,* 1994, vol. 266, 816
- **Beckmann et al.** *EMBO J,* 1994, vol. 13, 3657
- **L'Allemain et al.** *Bull Cancer,* 2000, vol. 87, 529-530
- **Nagasawa et al.** *Genomics,* 1997, vol. 44, 173-179
- **Nagasawa et al.** *Genomics,* 1999, vol. 61, 37-43
- **Murata et al.** *J Biol Chem,* 1994, vol. 269, 30479-84
- **Lee et al.** *Biochem Biophys Res Commun,* 1996, vol. 218, 714-719
- **Nam et al.** *Oncogene,* 2000, vol. 19, 241-247
- **Moss, G.P.** *Arch. Biochem. Biophys.,* 1983, vol. 224, 728-731
- *Eur. J. Biochem.,* 1982, vol. 129, 1-5
- *J. Biol. Chem.,* 1983, vol. 258, 5329-5333
- *Pure Appl. Chem.,* 1983, vol. 55, 721-726
- Biochemical Nomenclature and Related Documents. Portland Press, 1992, 247-251
- **van Ooyen et al.** *EMBO J,* 1985, vol. 4, 2905-9
- **Wainwright et al.** *EMBO J,* 1988, vol. 7, 1743-1748
- **Katoh et al.** *Oncogene,* 1996, vol. 13, 873-876
- **Roelink et al.** *Genomics,* 1993, vol. 17, 790-792
- **Huguet et al.** *Cancer Res,* 1994, vol. 54, 2615-2521
- **Clark et al.** *Genomics,* 1993, vol. 18, 249-260
- **Rankin et al.** *Cytogenet Cell Genet,* 1999, vol. 84, 50-52
- **Ikegawa et al.** *Cytogenet Cell Genet,* 1996, vol. 74, 149-152
- **Lako et al.** *Genomics,* 1996, vol. 35, 386-388
- **Bui et al.** *Oncogene,* 1997, vol. 14, 1249-1253
- **Lako et al.** *Gene,* 1998, vol. 219, 101-110
- **Bergstein et al.** *Genomics,* 1997, vol. 46, 450-458
- **McWhirter et al.** *Proc Natl Acad Sci USA,* 1999, vol. 96, 11464-11469
- **Fear et al.** *Biochem Biophys Res Commun,* 2000, vol. 278, 814-820
- **Wong et al.** *Mol Cell Biol,* 1994, vol. 14, 6278-86
- **Morin et al.** *Science,* 1997, vol. 275, 1787-1790
- **Pennica et al.** *Proc. Natl. Acad. Sci. USA,* 1998, vol. 95, 14717-14722
- **Chothia et al.** *J. Mol. Biol.,* 1985, vol. 186, 651
- **Novotny ; Haber.** *Proc. Natl. Acad. Sci. U.S.A.,* 1985, vol. 82, 4592
- **Chothia et al.** *Nature,* 1989, vol. 342, 877-883
- **Kohler et al.** *Nature,* 1975, vol. 256, 495
- **Clackson et al.** *Nature,* 1991, vol. 352, 624-628
- **Marks et al.** *J. Mol. Biol.,* 1991, vol. 222, 581-597
- **Morrison et al.** *Proc. Natl. Acad Sci. USA,* 1984, vol. 81, 6851-6855
- **Jones et al.** *Nature,* 1986, vol. 321, 522-525
- **Reichmann et al.** *Nature,* 1988, vol. 332, 323-329
- **Presta.** *Curr. Op. Struct. Biol.,* 1992, vol. 2, 593-596
- **Clark.** *Immunol. Today,* 2000, vol. 21, 397-402
- **Pluckthun.** The Pharmacology of Monoclonal Antibodies. Springer-Verlag, 1994, vol. 113, 269-315
- **Dall'Acqua ; Carter.** *Curr. Opin. Struct. Biol.,* 1998, vol. 8, 443-450
- **Hudson.** *Curr. Opin. Immunol.,* 1999, vol. 11, 548-557
- **Hollinger et al.** *Proc. Natl. Acad. Sci. USA,* 1993, vol. 90, 6444-6448

- Cell cycle regulation, oncogens, and antineoplastic drugs. **Murakami et al.** The Molecular Basis of Cancer. WB Saunders, 1995, 13
- **Wilman.** Prodrugs in Cancer Chemotherapy. *Biochemical Society Transactions,* 1986, vol. 14, 375-382
- Prodrugs: A Chemical Approach to Targeted Drug Delivery. **Stella et al.** Directed Drug Delivery. Humana Press, 1985, 147-267
- **Warzocha ; Wotowiec.** Antisense strategy'' biological utility and prospects int he treatment of hematological malignancies. *Leuk. Lymphoma,* 1997, vol. 24, 267-281
- **Nusse ; Varnus.** *Cell,* 1982, vol. 31, 99-109
- **Polakis.** *Biochem. Biophys. Acta,* 1997, vol. 1332 (3), F127-47
- **Morin et al.** *Science,* 1997, vol. 275, 1752-53
- **Polakis.** *Genes Dev.,* 2000, vol. 14, 1837-51
- **Bienz ; Clevers.** *Cell,* 2000, vol. 103, 311-20
- **Parker ; Barnes.** *Methods in Molecular Biology,* 1999, vol. 106, 247-283
- **Hod.** *Biotechniques,* 1992, vol. 13, 852-854
- **Weis et al.** *Trends in Genetics,* 1992, vol. 8, 283-264
- **Ausubel et al.** Current Protocols of Molecular Biology. John Wiley and Sons, 1997
- **Rupp ; Locker.** *Lab Invest.,* 1987, vol. 56, A67
- **De Andrés et al.** *BioTechniques,* 1995, vol. 18, 42044
- **Wright et al.** *Int J Cancer,* 1989, vol. 82, 825-9
- **Schena et al.** *Proc. Natl. Acad Sci. USA,* 1996, vol. 93 (20), 106-49
- **Small et al.** *Mol. Cell. Biol.,* 1985, vol. 5, 642-648
- The Nude Mouse in Oncology Research. CRC Press, Inc, 1991
- **Diatchenko et al.** *Proc. Natl. Acad. Sci. USA,* 1996, vol. 93, 6025-6030
- **Karmali et al.** *Br. J. Cancer,* 1983, vol. 48, 689-696
- **Drebin et al.** *PNAS USA,* 1986, vol. 83, 9129-9133
- **Wang et al.** *Cancer Research,* 1994, vol. 54, 4726-4728
- **Too et al.** *Cancer Research,* 1995, vol. 55, 681-684
- **DeLeo et al.** *J. Exp. Med.,* 1977, vol. 146, 720
- **Palladino et al.** *J. Immunol.,* 1987, vol. 138, 4023-4032
- **Zupi et al.** *Br. J. Cancer,* 1980, vol. 41 (4), 309
- **Zacharski.** *Haemostasis,* 1986, vol. 16, 300-320
- **Rygaard ; Spang-Thomsen.** Proc. 6th Int. Workshop on Immune-Deficient Animals. 1989, 301
- **Van der Putten et al.** *Proc. Natl. Acad. Sci. USA,* 1985, vol. 82, 6148-615
- **Thompson et al.** *Cell,* 1989, vol. 56, 313-321
- **Lo.** *Mol. Cell Biol.,* 1983, vol. 3, 1803-1814
- **Lavitrano et al.** *Cell,* 1989, vol. 57, 717-73
- **Lasko et al.** *Proc. Natl. Acad. Sci. USA,* 1992, vol. 89, 6232-636
- **Thomas ; Capecchi.** *Cell,* 1987, vol. 51, 503
- **Li et al.** *Cell,* 1992, vol. 69, 915
- **Bradley.** Teratocarcinomas and Embryonic Stem Cells: A Practical Approach. IRL, 1987, 113-152
- **Lee et al.** *Nucl. Acids Res.,* 1979, vol. 6, 3073
- **Cooney et al.** *Science,* 1988, vol. 241, 456
- **Dervan et al.** *Science,* 1991, vol. 251, 1360
- **Okano.** *Neurochem.,* 1991, vol. 56, 560
- Oligodeoxynucleotides as Antisense Inhibitors of Gene Expression. CRC Press, 1988
- **Rossi.** *Current Biology,* 1994, vol. 4, 469-471
- **Kohler ; Milstein.** *Nature,* 1975, vol. 256, 495
- **Goding.** Monoclonal Antibodies: Principles and Practice. Academic Press, 1986, 59-103
- **Kozbor.** *J. Immunol,* 1984, vol. 133, 3001
- **Brodeur et al.** Monoclonal Antibody Production Techniques and Applications. Marcel Dekker, Inc, 1987, 51-63
- **Munson ; Pollard.** *Anal. Biochem.,* 1980, vol. 107, 220
- **Riechmann et al.** *Nature,* 1988, vol. 332, 323-329
- **Riechmann et al.** *Nature,* 1988, vol. 332, 323-327
- **Verhoeyen et al.** *Science,* 1988, vol. 239, 1534-1536
- **Hoogenboom ; Winter.** *J. Mol. Biol.,* 1991, vol. 227, 381
- **Marks et al.** *J. Mol. Biol.,* 1991, vol. 222, 581
- **Cole et al.** Monoclonal Antibodies and Cancer Therapy. Alan R. Liss, 1985, 77
- **Boerner et al.** *J. Immunol.,* 1991, vol. 147 (1), 86-95
- **Marks et al.** *Bio/Technology,* 1992, vol. 10, 779-783
- **Lonberg et al.** *Nature,* 1994, vol. 368, 856-859
- **Morrison.** *Nature,* 1994, vol. 368, 812-13
- **Fishwild et al.** *Nature Biotechnology,* 1996, vol. 14, 845-51
- **Neuberger.** *Nature Biotechnology,* 1996, vol. 14, 826
- **Lonberg ; Huszar.** *Intern. Rev. Immunol.,* 1995, vol. 13, 65-93
- **Milstein ; Cuello.** *Nature,* 1983, vol. 305, 537-539
- **Traunecker et al.** *EMBO J.,* 1991, vol. 10, 3655-3659
- **Suresh et al.** *Methods in Enzymology,* 1986, vol. 121, 210
- **Brennan et al.** *Science,* 1985, vol. 229, 81

- **Shalaby et al.** *J. Exp. Med.,* 1992, vol. 175, 217-225
- **Kostelny et al.** *J. Immunol.,* 1992, vol. 148 (5), 1547-1553
- **Gruber et al.** *J. Immunol.,* 1994, vol. 152, 5368
- **Tutt et al.** *J. Immunol,* 1991, vol. 147, 60
- **Caron et al.** *J. Exp Med.,* 1992, vol. 176, 1191-1195
- **Shopes.** *J. Immunol.,* 1992, vol. 148, 2918-2922
- **Wolff et al.** *Cancer Research,* 1993, vol. 53, 2560-2565
- **Stevenson et al.** *Anti-Cancer Drug Design,* 1989, vol. 3, 219-230
- **Vitetta et al.** *Science,* 1987, vol. 238, 1098
- **Liu et al.** *Proc Natl Acad Sci USA,* 1996, vol. 93, 8618-8623
- **Chari et al.** *Cancer Research,* 1992, vol. 52, 127-131
- **Hinman et al.** *CancerRes,* 1993, vol. 53, 3336-3342
- **Lode et al.** *Cancerres,* 1998, vol. 58, 2925-2928
- **Epstein et al.** *Proc. Natl. Acad Sci. USA,* 1985, vol. 82, 3688
- **Hwang et al.** *Proc. Natl Acad. Sci. USA,* 1980, vol. 77, 4030
- **Martin et al.** *J. Biol. Chem.,* 1982, vol. 257, 286-288
- **Gabizon et al.** *J. National Cancer Inst.,* 1989, vol. 81 (19), 1484
- **Massey.** *Nature,* 1987, vol. 328, 457-458
- **Neuberger et al.** *Nature,* 1984, vol. 312, 604-608
- **Marasco et al.** *Proc. Natl. Acad Sci. USA,* 1993, vol. 90, 7889-7893
- Chemotherapy Service. Williams & Wilkins, 1992
- **Bolivar et al.** *Gene,* 1977, vol. 2, 95
- **Kikuchi et al.** *Nucleic Acids Res.,* 1981, vol. 9, 5671-5678
- **Yanofsky et al.** *Nucleic Acids Res.,* 1981, vol. 9, 6647-6668
- **Scholtissek ; Grosse.** *Nucleic Acids Res.,* 1987, vol. 15, 3185
- **Komine et al.** *J. Mol. Biol.,* 1990, vol. 212, 579-598
- **Fournier ; Ozeki.** *Microbiol. Rev.,* 1985, vol. 49, 379-397
- **Thimmappaya et al.** *Cell,* 1982, vol. 31, 543
- **Somparyrac et al.** *Proc. Natl. Acad. Sci.,* 1981, vol. 12, 7575
- **Lucas et al.** *Nucl. Acids Res.,* 1996, vol. 24 (9), 1774-1779
- **O'Reilley et al.** Baculovirus expression vectors: A Laboratory Manual. Oxford University Press, 1994
- **Rupert et al.** *Nature,* 1993, vol. 362, 175-179
- **Hongo et al.** *Hybridoma,* 1995, vol. 14, 253-260
- **Freud ; Blair.** *J. Immunol.,* 1982, vol. 129, 2826-2830
- **Hodgson.** *Bio/Technology,* 1991, vol. 9, 19-21
- **Braxton ; Wells.** *Biochemistry,* 1992, vol. 31, 7796-7801
- **Athauda et al.** *J. Biochem.,* 1993, vol. 113, 742-746
- **Pennica et al.** *Proc. Natl. Acad. Sci USA,* 1998, vol. 95, 14717-22
- **Korinek et al.** *Mol. Cell Biol.,* 1998, vol. 18, 1248-56
- **Willert et al.** *Genes Dev.,* 1999, vol. 13, 1768-73
- **Eberhard et al.** *Am. J. Pathol.,* 1994, vol. 145, 640-9
- **Tsukamoto et al.** *Cel,* 1988, vol. 55, 619-625
- **Easwaran et al.** *Curr. Biol.,* 1999, vol. 9, 1415-1418
- **Bouillet et al.** *Mech Dev.,* 1997, vol. 63, 173-186
- **McGrew et al.** *Mech. Dev.,* 1999, vol. 87, 21-32
- **Taneja et al.** *Proc. Natl. Acad. Sci. USA,* 1995, vol. 92, 7854-8
- **Polakis.** *Curr. Opin, Genet. Dev.,* 1999, vol. 9, 15-21
- **Kobayashi et al.** *Jpn.J. Cancer Res.,* 1999, vol. 90, 55-9
- **Palacios ; Hamallo.** *Cancer Res.,* 1998, vol. 58, 1344-7
- **Rimm et al.** *Am. J. Pathol.,* 1999, vol. 154, 325-9
- **Wright et al.** *Int. J. Cancer,* 1999, vol. 82, 625-9
- **Korinek et al.** *Mol Cell Biol,* 1998, vol. 18, 1248-56
- **Wodicka et al.** *Nat Biotechnol,* 1997, vol. 15, 1359-1367
- **Wong et al.** *Mol Cell Biol,* 1994, vol. 14, 6278-6286
- **Pearson, D. ; Sasse, J.** *J Biol Chem,* 1992, vol. 267, 25364-25370
- **Clotman et al.** *Neurotoxicol Teratol,* 1998, vol. 20, 591-599
- **Tremblay et al.** *Biol Reprod,* 1999, vol. 60, 541-545
- **Zhang et al.** *J Biol Chem,* 1998, vol. 273, 27998-28003
- **Wade, D. P. ; Owen, J. S.** *Lancet,* 2001, vol. 357, 161-163
- **Koj et al.** *Biol Chem Hoppe Seyler,* 1993, vol. 374, 193-201
- **Duester.** *Eur J Biochem,* 2000, vol. 267, 4315-4324

- **Tetsu, 0. ; McCormick, F.** *Nature,* 1999, vol. 398, 422-426
- **Ziemer et al.** *Mol Cell Biol,* 2001, vol. 21, 562-574
- **Roose et al.** *Science,* 1999, vol. 285, 1923-1926
- **Donehower et al.** *Genes Dev,* 1995, vol. 9, 882-895
- **Martin-Satue, M. ; Blanco, J.** *J Surg Oncol,* 1999, vol. 72, 18-23
- **Yamada et al.** *Proc Natl Acad Sci USA,* 1997, vol. 94, 14713-14718
- **Bouillet et al.** *Dev Biol,* 1995, vol. 170, 420-433